# EUROPEAN PATENT SPECIFICATION

(11) **EP 2 686 007 B1**
(45) Date of publication and mention of the grant of the patent: **24.04.2019**
(21) Application number: 12757910.0
(22) Date of filing: 15.03.2012
(51) Int. Cl.: C12Q 1/6883, A61K 31/4422, A61K 31/225, A61K 31/015

(54) **METHODS OF DIAGNOSING AND TREATING VASCULAR ASSOCIATED MACULOPATHY AND SYMPTOMS THEREOF**
VERFAHREN ZUR DIAGNOSE UND BEHANDLUNG VON GEFÄSSMAKULOPATHIE UND SYMPTOMEN DAVON
PROCÉDÉS DE DIAGNOSTIC ET DE TRAITEMENT DE LA MACULOPATHIE VASCULAIRE ASSOCIÉE ET DES SYMPTÔMES CORRESPONDANTS

(30) Priority: 15.03.2011 US 201161452964 P; 15.03.2011 US 201161452944 P; 15.03.2011 US 201161452950 P
(43) Date of publication of application: 22.01.2014
(73) Proprietor: University of Utah Research Foundation, Salt Lake City, UT 84108 (US)
(72) Inventor: HAGEMAN, Gregory, Salt Lake City, UT (US); FU, Yingbin, Salt Lake City, UT 84109 (US)
(74) Representative: Michalski Hüttermann & Partner Patentanwälte mbB
(86) International application number: PCT/US2012/029303
(87) International publication number: WO 2012/125872

(56) References cited:
- EP-A1- 0 968 716
- WO-A1-2005/112971
- WO-A1-2008/137107
- WO-A2-2008/067040
- WO-A2-2008/103299
- US-A1- 2003 109 500
- US-A1- 2009 012 030
- HIRATA Y ET AL: "The choroidal circulation assessed by laser-targeted angiography", PROGRESS IN RETINAL AND EYE RESEARCH, OXFORD, GB, vol. 25, no. 2, 1 March 2006 (2006-03-01), pages 129-147, XP028074809, ISSN: 1350-9462, DOI: 10.1016/J.PRETEYERES.2005.08.001 [retrieved on 2006-03-01]
- DANIEL PAULEIKHOFF: "A Fluorescein and Indocyanine Green Angiographic Study of Choriocapillaris in Age-related Macular Disease", ARCHIVES OF OPHTHALMOLOGY, vol. 117, no. 10, 1 October 1999 (1999-10-01), page 1353, XP055129079, ISSN: 0003-9950, DOI: 10.1001/archopht.117.10.1353
- EFSTRATIOS MENDRINOS ET AL: "Topographic variation of the choroidal watershed zone and its relationship to neovascularization in patients with age-related macular degeneration", ACTA OPHTHALMOLOGICA, vol. 87, no. 3, 1 May 2009 (2009-05-01), pages 290-296, XP055129084, ISSN: 1755-375X, DOI: 10.1111/j.1755-3768.2008.01247.x
- LINDA TRUEBESTEIN ET AL: "Substrate-induced remodeling of the active site regulates human HTRA1 activity", NATURE STRUCTURAL & MOLECULAR BIOLOGY, vol. 18, no. 3, 6 February 2011 (2011-02-06), pages 386-388, XP055124206, ISSN: 1545-9993, DOI: 10.1038/nsmb.2013
- DEANGELIS M M ET AL: "Alleles in the HtrA Serine Peptidase 1 Gene Alter the Risk of Neovascular Age-Related Macular Degeneration", OPHTHALMOLOGY, J. B. LIPPINCOTT CO., PHILADELPHIA, PA, US, vol. 115, no. 7, 1 July 2008 (2008-07-01), pages 1209-1215.e7, XP022852668, ISSN: 0161-6420, DOI: 10.1016/J.OPHTHA.2007.10.032 [retrieved on 2007-12-27]
- NORIMOTO GOTOH ET AL: "Haplotype analysis of theregion in Japanese patients with typical neovascular age-related macular degeneration or polypoidal choroidal vasculopathy", JAPANESE JOURNAL OF OPHTHALMOLOGY ; THE OFFICIAL ENGLISH-LANGUAGE JOURNAL OF THE JAPANESE OPHTHALMOLOGICAL SOCIETY, SPRINGER-VERLAG, TO, vol. 54, no. 6, 30 December 2010 (2010-12-30), pages 609-614, XP019855674, ISSN: 1613-2246, DOI: 10.1007/S10384-010-0865-2
- ANDREOLI M T ET AL: "Comprehensive Analysis of Complement Factor H and LOC387715/ARMS2/HTRA1 Variants With Respect to Phenotype in Advanced Age-Related Macular Degeneration", AMERICAN JOURNAL OF OPHTHALMOLOGY, ELSEVIER, AMSTERDAM, NL, vol. 148, no. 6, 1 December 2009 (2009-12-01), pages 869-874, XP026777779, ISSN: 0002-9394, DOI: 10.1016/J.AJO.2009.07.002 [retrieved on 2009-10-01]
- MATTHEW ET AL.: 'Lyngbyastatin 4, a dolastatin 13 analogue with elastase and chymotrypsin inhibitory activity from the marine cyanobacterium Lyngbya confervoides' J. NAT. PROD. vol. 70, no. 1, January 2007, pages 124 - 127, XP002465450
- HAYREH.: 'Macular lesions secondary to choroidal vascular disorders' IND. J. OPHTHAMOL. vol. 31, no. 3, 1983, pages 158 - 164, XP008170715 Retrieved from the Internet: <URL:http://www.ijo.in/text.asp?1983/31/3/1 58/29774> [retrieved on 2012-05-31]
- POVAZAY ET AL.: 'Wide-field optical coherence tomography of the choroid in vivo.' INVEST. OPHTHAMOL. VIS. SCI. vol. 50, no. 4, April 2009, pages 1856 - 1863, XP055124203
- SKEIE ET AL.: 'Elastin-mediated choroidal endothelial cell migration: possible role in age-related macular degeneration.' INVEST. OPHTHAMOL. VIS. SCI. vol. 49, no. 12, December 2008, pages 5574 - 5580, XP055124205
- LEE ET AL.: 'Association analysis of CFH, C2, BF, and HTRA1 gene polymorphisms in Chinese patients with polypoidal choroidal vasculopathy.' INVEST. OPHTHAMOL. VIS. SCI. vol. 49, no. 6, June 2008, pages 2613 - 2619, XP055124217
- TRUEBESTEIN ET AL.: 'Substrate-induced remodeling of the active site regulates human HTRA1 activity.' NAT. STRUCT. MOL. BIOL. vol. 18, no. 3, 06 February 2011, pages 386 - 386, XP055124206
- HAYREH.: 'Segmental nature of the choroidal vasculature.' BR. J. OPHTHAMOL. November 1975, pages 631 - 648, XP055124208
- KONDO ET AL.: 'Elastin gene polymorphisms in neovascular age-related macular degeneration and polypoidal choroidal vasculopathy.' INVEST. OPHTHAMOL. VIS. SCI. vol. 49, no. 3, March 2008, pages 1101 - 1105, XP055124213
- ZARBIN.: 'Current concepts in the pathogenesis of age-related macular degeneration.' ARCH. OPHTHAMOL. vol. 122, no. 4, April 2004, pages 598 - 614, XP055030722
- JONES ET AL.: 'Increased expression of multifunctional serine protease, HTRA1, in retinal pigment epithelium induces polypoidal choroidal vasculopathy in mice.' PROC. NATL. ACAD. SCI. USA vol. 108, no. 35, 15 August 2011, pages 14578 - 14583, XP055124215

## Description

### FIELD OF THE INVENTION

The present disclosure relates generally to the diagnosis and treatment of Vascular Associated Maculopathy, and symptoms thereof, in a subject. Specifically, disclosed are a) methods of diagnosing Vascular Associated Maculopathy, and symptoms thereof, in a subject, b) methods of identifying a predisposition for developing Vascular Associated Maculopathy, and symptoms thereof, in a subject, c) methods of treating Vascular Associated Maculopathy, and symptoms thereof, in a subject, and d) methods of screening for agents effective in treating Vascular Associated Maculopathy, and symptoms thereof, in a subject.

### BACKGROUND

Vascular Associated Maculopathy (VAM) represents a previously unidentified disease that can be associated with a variety of vascular symptoms. Currently, patients with Vascular Associated Maculopathy are often misdiagnosed. Moreover, it can be difficult to make a diagnosis of Vascular Associated Maculopathy, as well as the symptoms thereof, because there are a wide variety of symptoms that a person can exhibit. Therefore, what is needed are more effective methods of diagnosing Vascular Associated Maculopathy and its associated symptoms in a subject. Furthermore, what is needed are methods of treating Vascular Associated Maculopathy and its associated symptoms in a subject.

International patent application WO 2008/103299 A2 discloses methods for determining whether a subject is at risk of developing age-related macular degeneration, as well as a method of treating or slowing the progression of age-related macular degeneration by reducing the expression of the HTRA1 gene, or reducing the biological activity of the HTRA1 gene product.

### SUMMARY

The present invention relates to an elastase inhibitor for use in the treatment of a disease characterized by the presence of aberrant choriocapillaris lobules in the macula of an eye of a subject, wherein said aberrant choriocapillaris lobules are characterized by decreased or absent perfusion associated with partial or complete closure or occlusion of the choriocapillaris capillaries. The elastase inhibitor inhibits the elastase activity of High Temperature Requirement Serine Peptidase 1 (HTRA1).

Described herein are furthermore methods of diagnosing Vascular Associated Maculopathy, or symptoms thereof, in a subject comprising detecting the presence of aberrant choriocapillaris lobules and/or ocular pathology that recapitulates the lobular nature of the choriocapillaris in the eye of a subject, wherein the presence of aberrant choriocapillaris lobules and/or ocular pathology that recapitulates the lobular nature of the choriocapillaris in the eye of the subject indicates the presence of Vascular Associated Maculopathy, or a symptom thereof, in the subject.

Also described herein are methods for predicting a subject's risk for having or developing Vascular Associated Maculopathy in a human subject, comprising: determining in the subject the identity of one or more SNPs in the HTRA1, ARMS2, or CFH genes, wherein the one or more SNPs are (i) the rs11200638 in the HTRA1 gene, rs1049331 in the HTRA1 gene, rs2672587 in the HTRA1 gene, rs10490924 in the ARMS2 gene, rs3750848 in the ARMS2 gene, rs1061170 in the CFH gene, or rs800292 in the CFH gene, or (ii) a SNP in linkage disequilibrium with the SNPS of (i), wherein the presence of one or more of the SNPs is predictive of the subject's risk for having or developing Vascular Associated Maculopathy.

Also described herein are methods for predicting a subject's risk for having or developing Vascular Associated Maculopathy in a human subject, comprising: generating an image of an eye of the subject, detecting the presence of aberrant choriocapillaris lobules in the eye of the subject using the image, wherein the presence of aberrant choriocapillaris lobules in the eye of the subject is predictive of the subject's risk for having or developing Vascular Associated Maculopathy.

Also described herein are methods for predicting a subject's risk for having or developing Vascular Associated Maculopathy in a human subject, comprising: generating an image of an eye of the subject, detecting the presence of aberrant choriocapillaris lobules in the eye of the subject using the image, wherein the presence of aberrant choriocapillaris lobules in the eye of the subject is predictive of the subject's risk for having or developing Vascular Associated Maculopathy.

Also described herein are methods for diagnosing a subject with Vascular Associated Maculopathy, comprising: in a sample obtained from the subject, determining the identity of one or more SNPs in the HTRA1, ARMS2, or CFH genes, wherein the one or more SNPs are (i) the rs11200638 in the HTRA1 gene, rs1049331 in the HTRA1 gene, rs2672587 in the HTRA1 gene, rs10490924 in the ARMS2 gene, rs3750848 in the ARMS2 gene, rs1061170 in the CFH gene, or rs800292 in the CFH gene, or (ii) a SNP in linkage disequilibrium with the SNPs of (i), thereby diagnosing the subject based on the presence of the one or more variants of the SNPs in (i) or (ii).

Also described herein are methods of diagnosing a subject with Vascular Associated Maculopathy, comprising: generating an image of an eye of the subject, detecting the presence of aberrant choriocapillaris lobules in the image of eye of the subject, whereby the presence of aberrant choriocapillaris lobules in the eye of the subject results in a diagnosis of Vascular Associated Maculopathy.

Also described herein are methods for predicting a subject's risk for having or developing severe maculopathy or last stage maculopathy in a human subject, comprising: determining in the subject the identity of one or more SNPs in the HTRA1, ARMS2, or CFH genes, wherein the one or more SNPs are (i) the rs11200638 in the HTRA1 gene, rs1049331 in the HTRA1 gene, rs2672587 in the HTRA1 gene, rs10490924 in the ARMS2 gene, rs3750848 in the ARMS2 gene, rs1061170 in the CFH gene, or rs800292 in the CFH gene, or (ii) a SNP in linkage disequilibrium with the SNPs of (i), wherein the presence of one or more of the SNPs is predictive of the subject's risk for having or developing having or developing severe maculopathy or last stage maculopathy.

Also described herein are methods for predicting a subject's risk for having or developing severe maculopathy or last stage maculopathy in a human subject, comprising: generating an image of an eye of the subject, detecting the presence of aberrant choriocapillaris lobules in the eye of the subject using the image, wherein the presence of aberrant choriocapillaris lobules in the eye of the subject is predictive of the subject's risk for having or developing severe maculopathy or last stage maculopathy.

Also described herein are methods for diagnosing a subject with severe maculopathy or last stage maculopathy, comprising: in a sample obtained from the subject, determining the identity of one or more SNPs in the HTRA1, ARMS2, or CFH genes, wherein the one or more SNPs are (i) the rs11200638 in the HTRA1 gene, rs1049331 in the HTRA1 gene, rs2672587 in the HTRA1 gene, rs10490924 in the ARMS2 gene, rs3750848 in the ARMS2 gene, rs1061170 in the CFH gene, or rs800292 in the CFH gene, or (ii) a SNP in linkage disequilibrium with the SNPs of (i), thereby diagnosing the subject based on the presence of the one or more SNPs in (i) or (ii).

Also described herein are methods for diagnosing a subject with severe maculopathy or last stage maculopathy, comprising: generating an image of an eye of the subject, detecting the presence of aberrant choriocapillaris lobules in the eye of the subject using the image, thereby diagnosing the subject based on the presence of aberrant choriocapillaris lobules in the eye of the subject.

Also described herein are methods for predicting a subject's risk for having or developing aberrant choriocapillaris lobules in a human subject, comprising: determining in the subject the identity of one or more SNPs in the HTRA1, ARMS2, or CFH genes, wherein the one or more SNPs are (i) the rs11200638 in the HTRA1 gene, rs1049331 in the HTRA1 gene, rs2672587 in the HTRA1 gene, rs10490924 in the ARMS2 gene, rs3750848 in the ARMS2 gene, rs1061170 in the CFH gene, or rs800292 in the CFH gene, or (ii) a SNP in linkage disequilibrium with the SNPs of (i), wherein the presence of one or more of the SNPs is predictive of the subject's risk for having or developing aberrant choriocapillaris lobules.

Also described herein are methods of determining a subject's risk of developing Vascular Associated Maculopathy, or a symptom thereof, comprising determining in a subject the identity of one or more single nucleotide polymorphisms (SNPs) in the HTRA1, ARMS2, or PLEKHA1 genes. wherein certain SNPs indicate an increased or decreased risk of developing Vascular Associated Maculopathy, or a symptom thereof

Also described herein are methods of determining a subject's risk of developing Vascular Associated Maculopathy, or a symptom thereof, in a subject comprising determining in a subject the identity of one or more single nucleotide polymorphisms (SNPs) in the HTRA1, ARMS2, or PLEKHA1 genes, wherein certain SNPs indicate the or decreased risk of developing Vascular Associated Maculopathy, or a symptom thereof.

Also described herein are methods of determining a subject's risk of developing Vascular Associated Maculopathy, or a symptom thereof, in a subject comprising determining in a subject the identity of one or more single nucleotide polymorphisms (SNPs) in the CFH, C3, C2, CFB, or APOE genes, wherein certain SNPs indicate an increased or decreased risk of developing Vascular Associated Maculopathy, or a symptom thereof.

Also described herein are methods of diagnosing Vascular Associated Maculopathy, or a symptom thereof, in a subject comprising determining in a subject the identity of one or more single nucleotide polymorphisms (SNPs) in the HTRA1, ARMS2, PLEKHA1, single nucleotide polymorphisms (SNPs) in the CFH, C3, C2, CFB, or APOE genes, wherein certain SNPs indicate the presence of a Vascular Associated Maculopathy, or a symptom thereof.

Also described herein are methods of diagnosing Vascular Associated Maculopathy, or a symptom thereof, in a subject comprising determining in a subject the identity of one or more single nucleotide polymorphisms (SNPs) in the HTRA1, ARMS2, PLEKHA1, CFH, C3, C2, CFB, or APOE genes, wherein certain SNPs indicate the lack of or protection from developing Vascular Associated Maculopathy, or a symptom thereof.

Also described herein are methods of identifying a predisposition for developing Vascular Associated Maculopathy, or a symptom thereof, in a subject, comprising detecting aberrant choriocapillaris lobules in an eye of a subject, wherein detecting aberrant choriocapillaris lobules in the eye of the subject indicates a predisposition for developing a Vascular Associated Maculopathy, or a symptom thereof, in a tissue or organ in the subject.

Also described herein are methods of identifying a predisposition for developing severe or late stage maculopathy in a subject, comprising detecting aberrant choriocapillaris lobules in an eye of a subject, wherein detecting aberrant choriocapillaris lobules in the eye of the subject indicates a predisposition for developing severe or late stage maculopathy in the subject.

Also described herein are methods of identifying a predisposition for developing aberrant choriocapillaris lobules in a subject, comprising detecting in a subject specific SNPs in the HTRA1 or ARMS2 genes, wherein detection of the specific SNPs in the HTRA1 or ARMS2 genes indicates a predisposition for developing aberrant choriocapillaris lobules in the subject.

Also described herein are methods of treating Vascular Associated Maculopathy, or a symptom thereof, in a subject, wherein aberrant choriocapillaris lobules are present in an eye of a subject, comprising administering to the subject a therapeutically effective amount of one or more active or therapeutic agents that inhibit occlusion or closure of choriocapillaris lobules and/or larger arterioles and arteries upstream in the vasculature of the choriocapillaris.

Also described herein are methods of enhancing clinical trials comprising choosing appropriate patient populations for those clinical trials. In one aspect, the methods can be used to ensure patients are identified to participate in clinical trials based upon whether or not they are likely to be responsive to the experimental therapeutic agent or agents being studied.

Also described herein are kits comprising an assay for detecting one or more SNPs in a nucleic acid sample of a subject, wherein the SNP is (i) rs11200638 in the HTRA1 gene, rs1049331 in the HTRA1 gene, rs2672587 in the HTRA1 gene, rs10490924 in the ARMS2 gene, rs3750848 in the ARMS2 gene, rs1061170 in the CFH gene, or rs800292 in the CFH gene; (ii) combinations of the SNPs of (i); or (iii) a SNP in linkage disequilibrium with the SNPs of (i).

Also described herein are non-human animal models of Vascular Associated Maculopathy, wherein one or more cells of the animal express recombinant HTRA1 polypeptide.

Also described herein are systems for identifying aberrant choriocapillaris lobules comprising
a device for imaging an eye, the device configured to provide a recognizable image of aberrant choriocapillaris lobules, and instructions for identifying aberrant choriocapillaris lobules.

Also described herein are methods of treating Vascular Associated Maculopathy, treating one or more symptoms associated with Vascular Associated Maculopathy; treating severe maculopathy, treating late stage maculopathy or resolving aberrant choriocapillaris lobules in a subject.

Also described herein are methods of treating Vascular Associated Maculopathy, treating one or more symptoms associated with Vascular Associated Maculopathy; treating severe maculopathy, treating late stage maculopathy or resolving aberrant choriocapillaris lobules in a subject comprising administering an effective amount of one or more of the disclosed compositions, therapeutic agents, active agents, or functional nucleic acids to the subject.

Also described herein are methods of treating Vascular Associated Maculopathy, or a symptom thereof, in a subject, wherein aberrant choriocapillaris lobules are present in an eye of a subject, comprising administering to the subject a therapeutically effective amount of one or more active or therapeutic agents that increase perfusion in choriocapillaris lobules in the eye of the subject.

Also described herein are methods of treating Vascular Associated Maculopathy, or a symptom thereof, in a subject, wherein aberrant choriocapillaris lobules are present in an eye of a subject, comprising administering to the subject a therapeutically effective amount of one or more active or therapeutic agents that mimic, hybridize to, or otherwise modulate the activity of HTRA1 or ARMS2 nucleic acids or peptides.

Also described herein are methods of treating Vascular Associated Maculopathy, severe maculopathy or late stage maculopathy, or resolving aberrant choriocapillaris lobules in a subject, comprising administering an effective amount of an elastase inhibitor to the subject.

Also described herein are methods of treating one or more symptoms associated with Vascular Associated Maculopathy in a subject, comprising administering an effective amount of an elastase inhibitor to the subject.

Also described herein are methods of selecting a subject for the disclosed methods of (i) treating Vascular Associated Maculopathy; (ii) treating one or more symptoms associated with Vascular Associated Maculopathy; (iii) treating severe maculopathy or last stage maculopathy or (iv) resolving aberrant choriocapillaris lobules.

Also described herein are methods of diagnosing a subject with (i) Vascular Associated Maculopathy; (ii) one or more symptoms associated with Vascular Associated Maculopathy; (iii) severe maculopathy or last stage maculopathy or (iv) aberrant choriocapillaris lobules.

Also described herein are methods of screening for an agent or combination of agents effective in (i) treating Vascular Associated Maculopathy; (ii) treating one or more symptoms associated with Vascular Associated Maculopathy; (iii) treating severe maculopathy or last stage maculopathy; or (iv) resolving aberrant choriocapillaris lobules.

Also described herein are methods of determining the efficacy of a treatment of Vascular Associated Maculopathy, or a symptom thereof, in a subject diagnosed with Vascular Associated Maculopathy, or a symptom thereof.

### BRIEF DESCRIPTION OF THE FIGURES

The accompanying figures, which are incorporated in and constitute a part of this specification, illustrate several aspects and together with the description serve to explain the principles of the invention.
Figure 1 shows aberrant choriocapillaris lobules on near infrared (IR), fundus autofluorescence (AF), and red-free (RF) images, along with the corresponding fluorescein angiographs (FA) and indocyanin green angiograpjhs (ICG).
Figure 2 shows Infrared-imaged (IR) aberrant choriocapillaris lobules as distinct units of hypo-reflectance - both with and without a distinct hyper-reflective central 'spot' -- against a background of mild hyper-reflectance.
Figure 3 shows the progression of the phenotype over time.
Figure 4 shows the progression of the phenotype over time.
Figure 5 shows and aberrant choriocapillaris lobule in the subretinal space, as visualized by SD-OCT.
Figure 6 shows poor perfusion of the choriocapillaris lobules and/or masking of the fluorescent signal.
Figure 7 shows the overall pattern observed in the fundus recapitulates that of the macular choriocapillaris anatomy.
Figure 8 shows high resolution OCT 'C' scans taken from patients with macular aberrant choriocapillaris lobules.
Figure 9 shows the 'segmental' distribution of aberrant choriocapillaris lobules centered on the fovea.
Figure 10 shows color, FA and IR images taken in March 2011 and depicting the presence of aberrant choriocapillaris lobules.
Figure 11 shows an eye taken from a donor 3 hours and 45 minutes after the time of death, opened to reveal the inside layers.
Figure 12 shows a standard histological section (stained with Mallory trichrome).
Figure 13 shows distinct morphological features of the choriocapillaris that are observed at higher magnification.
Figure 14 shows an example of reduced macular retinal function (dark area in the right panel).
Figure 15 shows the alignment of the HTRA1 protease structure.
Figure 16 shows three views of the structural alignment of HTRA1.
Figure 17 shows HTRA1 expression data in the extramacular and macular regions.
Figure 18 shows HTRA1 expression data in the extramacular and macular regions.
Figure 19 shows a western blot of HTRA1 and ARMS2 expression.
Figure 20 shows antibody detection of HTRA1 and ARMS2 in serum.
Figure 21 shows serum samples from patients with different genotypes were analyzed by Western blot with antibodies: A- SC-15465 (left), SC-50335 (right), B-NEP-1688 (left), NEP-1693 (right), C- NEP-1694 (left), NEP-1695 (right), D- NEP-2414.
Figure 22 shows Western blot probing with anti-HtrA1 antibodies.
Figure 23 shows Western blot probing with anti-HtrA1 antibodies.
Figure 24 shows detection of HtrA1 and ARMS2 Proteins in Retina/RPE Protein Extraction and Western Blots.
Figure 25 shows HTRA1 degradation of elastin.
Figure 26 shows HTRA1 elastase activity.
Figure 27 shows inhibition of HTRA1 elastase activity.
Figure 28 shows the HTRA1 transgene construct employed in generating the mouse lines.
Figure 29 shows *hHTRA1*⁺ mice exhibited cardinal features of PCV.
Figure 30 shows *hHTRA1*⁺ mice exhibited cardinal features of PCV.
Figure 31 shows degradation of the elastic lamina of Bruch's membrane in the *hHTRA1*⁺ mice.
Figure 32 shows degradation of the elastic lamina of the choroidal vessels in *hHTRA1*⁺ mice.
Figure 33 shows that NP-injected eyes had normal retinal architecture with no signs of toxicity compared to the control.
Figure 34 shows that treating the *hHTRA1*⁺ mice with HTRA1 inhibitors reversed PCV phenotype.
Figure 35 shows the human HTRA1 cDNA containing a full-length coding region and the C-terminal myc-His₆ tag², subcloned into an AAV shuttle vector pAAV-CAG-Shuttle-WPRE under the CAG promoter.
Figure 36 shows PCV lesions were observed in AAV2-HTRA1 infected mice.
Figure 37 shows the level of tropoelastin, the soluble precursor of elastin, was similar in both the *hHTRA1*⁺ mice and WT.
Figure 38 shows that the largest risk for ACL is any diplotype that is homozygous risk at chromosome 10.
Figure 39 shows the results from DNA samples screened for SNPs in the control of complement region (CFH-to-F13B), as well as the HTRA1, ARMS2, C3, CFB, C2 and APOE genes.
Figure 40 shows allele frequencies of the various SNPs, characterized in the aberrant choriocapillaris lobules cohort and compared to a cohort of 416 individuals with no aberrant choriocapillaris lobules.
Figure 41 shows allele frequencies of the various SNPs, characterized in the aberrant choriocapillaris lobules cohort and compared to a cohort of 416 individuals with no aberrant choriocapillaris lobules.
Figure 42 shows allele frequencies of the various SNPs, characterized in the aberrant choriocapillaris lobules cohort and compared to a cohort of 416 individuals with no aberrant choriocapillaris lobules.

Additional advantages of the invention will be set forth in part in the description which follows, and in part will be obvious from the description, or can be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### DESCRIPTION

The present invention can be understood more readily by reference to the following detailed description and the Examples included therein.

Unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is in no way intended that an order be inferred, in any respect. This holds for any possible non-express basis for interpretation, including matters of logic with respect to arrangement of steps or operational flow, plain meaning derived from grammatical organization or punctuation, or the number or type of aspects described in the specification.

### Definitions

As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another significant both in relation to the other endpoint, and independently of the other endpoint. It is also understood that there are a number of values described herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10" as well as "greater than or equal to 10" is also disclosed. It is also understood that throughout the application, data are provided in a number of different formats, and that these data, represent endpoints, starting points, and ranges for any combination of the data points. For example, if a particular data point "10" and a particular data point 15 are disclosed, it is understood that greater than, greater than or equal to, less than, less than or equal to, and equal to 10 and 15 are considered disclosed as well as between 10 and 15. It is also understood that each unit between two particular units is also disclosed. For example, if 10 and 15 are disclosed, then 11, 12, 13, and 14 are also disclosed.

"Optional" or "optionally" means that the subsequently described event or circumstance may or may not occur and that the description includes instances where said event or circumstance occurs and instances where it does not.

The word "or" as used herein means any one member of a particular list and also includes any combination of members of that list.

"Severe maculopathy" or "late stage maculopathy" as used herein means any geographic atrophy (Grade 4A), neovascular AMD (RPE detachment, [peri]retinal hemorrhages, and/or scars in the absence of other retinal [vascular] disorders; Grade 4B; includes all phenotypes of CNV, including occult, classic, polypoidal choroidopathy [PPCV], and retinal angiomatous proliferation [RAP]), or both (Grade 4C; includes all phenotypes of GA).

"HTRA1" as used herein means the High Temperature Requirement Serine Peptidase 1 (HTRA1) gene. HTRA1 can also mean any HTRA1 protein encoded by the HTRA1 gene. HTRA1's NCBI Gene identification number is 5654.

"ARMS2" as used herein means the age-related maculopathy susceptibility 2 (ARMS2) gene. ARMS2 can also mean any ARMS2 protein encoded by the ARMS2 gene. ARMS2's NCBI Gene identification number is 387715.

### • METHODS OF DIAGNOSING VASCULAR ASSOCIATED MACULOPATHY AND SYMPTOMS HEREOF

The present disclosure reveals the surprising discovery that aberrant choriocapillaris lobules are not subretinal drusenoid deposits, and represent a sign of a new disease, Vascular Associated Maculopathy, as well as macular degeneration. Rather than being composed of cellular debris, complement proteins or lipids like classical drusen, aberrant choriocapillaris lobules represent areas of ischemic injury and damage to the outer segments of the retinal photoreceptors, retinal pigment epithelium, and Bruch's membrane overlying and corresponding to areas of closure or occlusion of capillaries in the choriocapillaris lobules and of small arterioles in the choroid in a human eye. Closure or occlusion of choriocapillaris capillaries and/or small choroidal arterioles in the choroid in a human eye is associated with an increased incidence of Vascular Associated Maculopathy, which can manifest as choroidal neovascularization (CNV), subretinal hemorrhage, and loss of central vision. Detecting the aberrant choriocapillaris lobules represents a previously unrecognized means of diagnosing diseases of the eye, as well as vascular diseases affecting other tissues and organs in the body of a subject.

Currently, patients with Vascular Associated Maculopathy are often misdiagnosed. Moreover, it can be difficult to make a diagnosis of Vascular Associated Maculopathy, as well as the symptoms thereof, because there are a wide variety of symptoms that a person can exhibit. Therefore, what is needed are more effective methods of diagnosing Vascular Associated Maculopathy and its associated symptoms in a subject. Furthermore, what is needed are methods of treating Vascular Associated Maculopathy and its associated symptoms in a subject.

### 1. ABERRANT CHORIOCAPILLARIS LOBULES

Described herein are methods of diagnosing Vascular Associated Maculopathy, or a symptom thereof, in a subject comprising detecting the presence of aberrant choriocapillaris lobules in the eye of a subject, wherein the presence of aberrant choriocapillaris lobules in the eye of the subject indicates Vascular Associated Maculopathy, or a symptom or consequence thereof, in the subject.

Also described herein are methods for predicting a subject's risk for having or developing Vascular Associated Maculopathy in a human subject, comprising: generating an image of an eye of the subject, detecting the presence of aberrant choriocapillaris lobules in the eye of the subject using the image, wherein the presence of aberrant choriocapillaris lobules in the eye of the subject is predictive of the subject's risk for having or developing Vascular Associated Maculopathy, severe or late state maculopathy, or aberrant choriocapillaris lobules. As used herein Vascular Associated Maculopathy can further comprise one or more symptoms associated with Vascular Associated Maculopathy, wherein the one or more symptoms of Vascular Associated Maculopathy is macular degeneration, age-related macular degeneration, cardiac microvascular disease, Stargardt's, Pseudoxanthoma Elasticum (PXE), Alagille syndrome, subcortical leukoencephalopathy, preeclampsia, hypertension, diabetes mellitus, myocardial ischemia, aneurysms, vasculitis due to autoimmune disease or infectious agents, deep vein thrombosis, lymphedema, varicose veins, peripheral artery disease, renal artery disease, Raynaud's disease, Buerger's disease, peripheral venous disease, venous blood clots, atherosclerosis, arteriosclerosis, arteriolar sclerosis, coronary artery disease, angina, congestive heart failure, hardening of the arteries, thrombotic or embolic stroke, or myocardial infarction.

In one aspect, the methods described herein can further comprise determining in the subject the identity of one or more SNPs in the HTRA1, ARMS2, or CFH genes, wherein the one or more SNPs are (i) the rs11200638 in the HTRA1 gene, rs1049331 in the HTRA1 gene, rs2672587 in the HTRA1 gene, rs10490924 in the ARMS2 gene, rs3750848 in the ARMS2 gene, rs1061170 in the CFH gene, or rs800292 in the CFH gene, or (ii) a SNP in linkage disequilibrium with the SNPs of (i), wherein the presence of one or more variants of the SNPs is predictive of the subject's risk for having or developing Vascular Associated Maculopathy, severe or late state maculopathy, or aberrant choriocapillaris lobules. In one aspect, determining the identity of an A at the rs11200638 SNP, a T at the rs1049331 SNP, a G at the rs2672587 SNP, a T at the rs10490924 SNP, a G at the rs3750848 SNP, a C at the rs1061170 SNP, or a G at the rs800292 SNP can be predictive of the subject's risk or increased risk for having or developing Vascular Associated Maculopathy, severe or late state maculopathy, or aberrant choriocapillaris lobules.

In one aspect, determining the identity of a G at the rs11200638 SNP, a C at the rs1049331 SNP, a C at the rs2672587 SNP, a G at the rs10490924 SNP, a T at the rs3750848 SNP, a T at the rs1061170 SNP, or an A at the rs800292 SNP can predictive of the subject's risk or decreased risk for having or developing Vascular Associated Maculopathy, severe or late state maculopathy, or aberrant choriocapillaris lobules.

Also described herein are methods of identifying a predisposition for developing a symptom associated with Vascular Associated Maculopathy in a tissue or organ in a subject, comprising detecting the presence of aberrant choriocapillaris lobules in an eye of a subject, wherein detecting the presence of aberrant choriocapillaris lobules in the eye of the subject indicates a predisposition for developing a symptom associated with Vascular Associated Maculopathy in a tissue or organ in the subject. The tissue or organ can include, but is not limited to, the heart, blood vessels, brain, spinal cord, muscle, bone, eye, ear, kidney, uterus, placenta, skin, mucous membranes, stomach, liver, lung, gall bladder, spleen, appendix, small intestine, large intestine, pancreas, prostate, or urinary bladder.

The term "subject" means an individual. In one aspect, a subject is a mammal such as a primate, and, more preferably, a human. Non-human primates include marmosets, monkeys, chimpanzees, gorillas, orangutans, and gibbons, to name a few. The term "subject" also includes domesticated animals, such as cats, dogs, etc., livestock (for example, cattle (cows), horses, pigs, sheep, goats, etc.), laboratory animals (for example, ferret, chinchilla, mouse, rabbit, rat, gerbil, guinea pig, etc.) and avian species (for example, chickens, turkeys, ducks, pheasants, pigeons, doves, parrots, cockatoos, geese, etc.). Subjects can also include, but are not limited to fish (for example, zebrafish, goldfish, tilapia, salmon, and trout), amphibians and reptiles. As used herein, a "subject" is the same as a "patient," and the terms can be used interchangeably.

As used herein, "aberrant" means differing from the norm or the expected type. Aberrant can be the same as "abnormal" or "anomalous," and the terms can be used interchangeably. In one aspect, aberrant can also mean recapitulate.

A "Choriocapillaris lobule," as used herein, is a circular-shaped capillary bed, *i.e.,* capillary in the choroid, that is unique to the macula of the eye. Oxygenated blood enters the center of each choriocapillaris lobule, and deoxygenated blood leaves via a venous drainage field which surrounds most of the periphery of each lobule. The drainage field can be the same diameter as the choriocapillaris lobule.

"Aberrant choriocapillaris lobules" can be choriocapillaris lobules that appear abnormal and/or do not function normally. Aberrant choriocapillaris lobules can also be pathology that recapitulates the lobular nature of the macular choriocapillaris, including, but not limited to, dead retinal pigment epithelium (RPE), changes in Bruch's membrane or the sub-RPE space, changes in the interphotoreceptor or subretinal space, other retinal abnormalities or pathologies associated with the development of aberrant choriocapillaris lobules, such as changes in the choroidal stroma, changes in the upstream vasculature, changes in the downstream vasculature, or ischemia, dead photoreceptors, or cobblestone atrophy in the periphery of the eye. Aberrant choriocapillaris lobules can be imaged via a variety of instruments known in the art, including, but not limited to, the Heidelberg Spectralis, the Zeiss Cirrus, the Topcon 3D OCT 2000, the Optivue RTVue SD-OCT, the Opko OCT SLO, the NIDEK F-10, or the Optopol SOCT Copernicus HR. Functional consequences of aberrant choriocapillaris lobules can be detected using, for example, and not to be limiting, angiography, ERG, blood flow, or color Doppler optical coherence tomography (CDOCT). In one aspect, the improper functioning can be due to occlusion of the lobules. As such, "aberrant choriocapillaris lobules" can be choriocapillaris lobules that are occluded, partially occluded, or functionally occluded. "Occlusion" of a lobule means the same as complete or partial "closure" of a lobule. For example, a choriocapillaris lobule can become partially or totally closed or occluded. "Occlusion" can also mean functionally occluded. As used herein functional occlusion can mean occlusion of upstream vasculature, for example, and not to be limiting, the arterioles that feed the choriocapillaris. Occlusion of choriocapillaris lobules can be due to abnormal thickening of a blood vessel wall that obliterates part or all of the lumen; by degradation of support matrices associated with choroidal vessels, for example degradation of elastin and/or extracellular matrix; by the accumulation of lipid, calcium, fibrous tissue, platelets, fibrin, or thrombi on the inner surface of the blood vessel; or by emboli that partially or totally obstruct, inhibit, or decrease blood flow in the lumen of the blood vessel. Under normal conditions, for example, when a subject is not suffering from Vascular Associated Maculopathy, or a symptom thereof, aberrant choriocapillaris lobules are not present or cannot be seen in the macula of an eye of a subject. In a further aspect, when a subject is suffering from a visual disturbance, but is not suffering from Vascular Associated Maculopathy, or a symptom thereof, aberrant choriocapillaris lobules can be present or can be seen in an eye of a subject, thereby serving as an early prognosticator of Vascular Associated Maculopathy and its associated symptoms. As used herein, "visual disturbance" means any condition that causes a change in vision away from normal, for example, and not to be limiting, blurred vision, halos, blind spots, floaters, and other symptoms.

The presence of aberrant choriocapillaris lobules in the macula of an eye of a subject indicates a previously unrecognized method of diagnosing Vascular Associated Maculopathy, or a symptom thereof, characterized by, in one aspect, decreased or absent perfusion of the choriocapillaris lobules in the choroid in an eye of the subject. As used herein, "decreased perfusion" means that blood flow in the choriocapillaris lobules is either reduced below normal levels or absent in all or part of the macular area. "Decreased perfusion" can also mean that there is insufficient blood flow in the choriocapillaris lobules to provide the necessary oxygen and nutrients to target tissues, including, but not limited to, for example, the choroid, the sclera, Bruch's membrane, the retinal pigment epithelium, or the outer segments of the retinal photoreceptors.

Decreased perfusion or absence of perfusion in the choriocapillaris lobules can be associated with partial or complete closure or occlusion of the choriocapillaris capillaries, choroidal arterioles, or even larger arteries supplying the choroid, including but not limited to, for example, the ciliary arteries, the ophthalmic arteries, the internal carotid arteries, and the common carotid arteries. Decreased or absent perfusion of the choriocapillaris lobules can be detected when partial or total occlusion of a blood vessel leading to or away from an eye occurs in a variety of locations including, but not limited to, the orbit or the optic nerve region of a subject. We also want to say something about "down the vascular tree" or on the venous side

In one aspect, the methods of diagnosing Vascular Associated Maculopathy, or a symptom thereof, or identifying the predisposition for developing Vascular Associated Maculopathy, or a symptom thereof, in a subject described herein can further comprise examining the subject with an ophthalmological procedure.

Various ophthalmological procedures known to persons of ordinary skill in the art can be performed to detect the presence of aberrant choriocapillaris lobules including, but not limited to, autofluorescent imaging techniques, infrared imaging techniques, optical coherence tomography (OCT), Stratus optical coherence tomography (Stratus OCT), Fourier-domain optical coherence tomography (Fd-OCT), two-photon-excited fluorescence (TPEF) imaging, adaptive optics scanning laser ophthalmoscopy (AOSLO), scanning laser ophthalmoscopy, near-infrared imaging combined with spectral domain optical coherence tomography (SD-OCT), color fundus photography, fundus autofluorescence imaging, red-free imaging, fluorescein angiography, indocyanin green angiography, multifocal electroretinography (ERG) recording, microperimetry, color Doppler optical coherence tomography (CDOCT), and visual field assessment. Additionally, the presence of aberrant choriocapillaris lobules can be detected by using the Heidelberg Spectralis, the Zeiss Cirrus, the Topcon 3D OCT 2000, the Optivue RTVue SD-OCT, the Opko OCT SLO, the NIDEK F-10, or the Optopol SOCT Copernicus HR.

In one aspect, the presence of aberrant choriocapillaris lobules can be detected by observing changes in the retinal pigment epithelium (RPE) overlying and corresponding to the choriocapillaris lobules. Observation of the RPE can be accomplished using, for example, and not to be limiting, autofluorescent imaging techniques known in the art. When an aberrant choriocapillaris lobule is present, the RPE cells overlying and corresponding to the aberrant lobule can die. Thus, the presence of aberrant choriocapillaris lobules can be detected by observing dead RPE cells overlying and corresponding to the choriocapillaris lobule. Various procedures known to persons of ordinary skill in the art can be utilized to detect the dead RPE cells, including, but not limited to, autofluorescent imaging techniques, infrared imaging techniques, optical coherence tomography (OCT), Stratus optical coherence tomography (Stratus OCT), Fourier-domain optical coherence tomography (Fd-OCT), two-photon-excited fluorescence (TPEF) imaging, adaptive optics scanning laser ophthalmoscopy (AOSLO), scanning laser ophthalmoscopy, near-infrared imaging combined with spectral domain optical coherence tomography (SD-OCT), color fundus photography, fundus autofluorescence imaging, red-free imaging, fluorescein angiography, indocyanin green angiography, multifocal electroretinography (ERG) recording, microperimetry, color Doppler optical coherence tomography (CDOCT), and visual field assessment. Additionally, the RPE can be observed by using the Heidelberg Spectralis, the Zeiss Cirrus, the Topcon 3D OCT 2000, the Optivue RTVue SD-OCT, the Opko OCT SLO, the NIDEK F-10, or the Optopol SOCT Copernicus HR.

In a further aspect, the presence of aberrant choriocapillaris lobules can be detected by observing other pathology that recapitulates the lobular nature of the macular choriocapillaris, including, but not limited to, changes in Bruch's membrane or the sub-RPE space, changes in the interphotoreceptor or subretinal space, other retinal abnormalities or pathologies associated with the development of aberrant choriocapillaris lobules, such as changes in the choroidal stroma, changes in the upstream vasculature, changes in the downstream vasculature, or ischemia, dead photoreceptors, or cobblestone atrophy in the periphery of the eye. Various procedures known to persons of ordinary skill in the art, and described herein, can be utilized to detect the pathology that recapitulates the lobular nature of the macular choriocapillaris.

In one aspect, the methods described herein can comprise diagnosing Vascular Associated Maculopathy, or a symptom thereof, in a subject comprising detecting the presence of aberrant choriocapillaris lobules in an eye of a subject, wherein the presence of aberrant choriocapillaris lobules in the eye of the subject indicates the diagnosis of a Vascular Associated Maculopathy, or a symptom thereof, in the subject, and wherein the symptom of Vascular Associated Maculopathy is macular degeneration, age-related macular degeneration, cardiac microvascular disease, Stargardt's, Pseudoxanthoma Elasticum (PXE), Alagille syndrome, subcortical leukoencephalopathy, preeclampsia, hypertension, diabetes mellitus, myocardial ischemia, aneurysms, vasculitis due to autoimmune disease or infectious agents, deep vein thrombosis, lymphedema, varicose veins, peripheral artery disease, renal artery disease, Raynaud's disease, Buerger's disease, peripheral venous disease, venous blood clots, atherosclerosis, arteriosclerosis, arteriolar sclerosis, coronary artery disease, angina, congestive heart failure, hardening of the arteries, thrombotic or embolic stroke, or myocardial infarction. In a further aspect, the symptom of Vascular Associated Maculopathy can be a pathology that recapitulates the lobular nature of the macular choriocapillaris, including, but not limited to, dead retinal pigment epithelium (RPE), changes in Bruch's membrane or the sub-RPE space, changes in the interphotoreceptor or subretinal space, other retinal abnormalities or pathologies associated with the development of aberrant choriocapillaris lobules, such as changes in the choroidal stroma, changes in the upstream vasculature, changes in the downstream vasculature, or ischemia, dead photoreceptors, or cobblestone atrophy in the periphery of the eye.

In a further aspect, the methods described herein can comprise identifying a predisposition for developing a symptom of Vascular Associated Maculopathy in a tissue or organ in a subject, comprising detecting the presence of aberrant choriocapillaris lobules in an eye of a subject, wherein detecting aberrant choriocapillaris lobules in the macula of the eye of the subject indicates a predisposition for developing a symptom of Vascular Associated Maculopathy in a tissue or organ in the subject, and wherein the symptom is age-related macular degeneration (AMD), cardiac microvascular disease, subcortical leukoencephalopathy, or preeclampsia.

As used herein, "a symptom of Vascular Associated Maculopathy " means any condition that affects the circulatory system of a subject. For example, and not to be limiting, a symptom of Vascular Associated Maculopathy can be macular degeneration, age-related macular degeneration, cardiac microvascular disease, Stargardt's, Pseudoxanthoma Elasticum (PXE), Alagille syndrome, subcortical leukoencephalopathy, preeclampsia, hypertension, diabetes mellitus, myocardial ischemia, aneurysms, vasculitis due to autoimmune disease or infectious agents, deep vein thrombosis, lymphedema, varicose veins, peripheral artery disease, renal artery disease, Raynaud's disease, Buerger's disease, peripheral venous disease, venous blood clots, atherosclerosis, arteriosclerosis, arteriolar sclerosis, coronary artery disease, angina, congestive heart failure, hardening of the arteries, thrombotic or embolic stroke, or myocardial infarction. A "symptom of Vascular Associated Maculopathy" can also be a pathology that recapitulates the lobular nature of the macular choriocapillaris, including, but not limited to, dead retinal pigment epithelium (RPE), changes in Bruch's membrane or the sub-RPE space, changes in the interphotoreceptor or subretinal space, other retinal abnormalities or pathologies associated with the development of aberrant choriocapillaris lobules, such as changes in the choroidal stroma, changes in the upstream vasculature, changes in the downstream vasculature, or ischemia, dead photoreceptors, or cobblestone atrophy in the periphery of the eye. A "symptom of Vascular Associated Maculopathy" can also be any visual condition that causes a change in vision away from normal, for example, and not to be limiting, blurred vision, halos, blind spots, floaters, and other symptoms.

Symptoms Vascular Associated Maculopathy are commonly associated with the heart; however, the condition can also affect a variety of other tissues or organs, such as the brain, spinal cord, muscle, bone, eye, ear, kidney, uterus, placenta, skin, mucous membranes, stomach, liver, lung, gall bladder, spleen, appendix, small intestine, large intestine, pancreas, prostate, or urinary bladder.

A symptom of Vascular Associated Maculopathy appearing in the heart, such as coronary microvascular disease, can lead to a myocardial infarction in a human subject, even in the absence of significant disease in the subject's coronary arteries. Moreover, a human subject with Vascular Associated Maculopathy can develop, for example, subcortical leukoencephalopathy which can present as a form of dementia. Additionally, symptoms of Vascular Associated Maculopathy appearing in the brain can be seizures, memory loss, gait disturbances, neuromuscular atrophy, paralysis, stroke, and blindness. A symptom of Vascular Associated Maculopathy appearing in a human subject's placenta can manifest as pre-eclampsia, with signs and symptoms of hypertension and proteinuria.

Examples of tissues or organs that can be affected by a symptom of Vascular Associated Maculopathy include, but are not limited to, heart, blood vessels, brain, spinal cord, muscle, bone, eye, ear, kidney, uterus, placenta, skin, mucous membranes, stomach, liver, lung, gall bladder, spleen, appendix, small intestine, large intestine, pancreas, prostate, or urinary bladder.

In one aspect, a symptom of Vascular Associated Maculopathy can be a small vessel disease. A "small Vessel Disease" or "SVD" as used herein refers to a condition that causes narrowing of the smaller blood vessels that provide blood flow to or from an organ or tissue. Examples of SVD include, but are not limited to, cerebral autosomal recessive arteriopathy with subcortical infarcts and leukoencephalopathy (CARASIL), retinal vasculopathy with cerebral leukodystrophy (RVCL) and the Collagen type IV, alpha 1 (COL4A1)-related disorders. Cerebral autosomal dominant arteriopathy with subcortical infarcts and leukoencephalopathy (CADASIL), remain the most common *hereditary* small vessel disease (SVD) caused by >190 different mutations in the NOTCH3 gene, which encodes a cell-signaling receptor.

Therefore the presence of aberrant choriocapillaris lobules in an eye of a subject can identify to a physician the possibility of a small vessel disease affecting other tissues or organs in the subject's body including, but not limited to, heart, blood vessels, brain, spinal cord, muscle, bone, eye, ear, kidney, uterus, placenta, skin, mucous membranes, stomach, liver, lung, gall bladder, spleen, appendix, small intestine, large intestine, pancreas, prostate, or urinary bladder.

For example, a symptom of Vascular Associated Maculopathy appearing in the heart can include coronary microvascular disease, which can lead to a myocardial infarction in a human subject, even in the absence of significant disease in the subject's coronary arteries. Coronary microvascular disease is a condition in which the small arteries in the heart become narrowed and cause signs and symptoms of heart disease, such as chest pain (angina). Small vessel disease is usually diagnosed after a physician checks for blockages in the main arteries of the heart that cause coronary artery disease and finds little or no narrowing. Thus, the presence of aberrant choriocapillaris lobules in the macula of an eye can alert a physician to a subject's possibly having a symptom of Vascular Associated Maculopathy, like a small vessel disease of the heart causing the subject's signs and symptoms.

Moreover, a subject with Vascular Associated Maculopathy can develop, for example, subcortical leukoencephalopathy which can present as a form of dementia. Non-limiting examples of other neurologic symptoms of Vascular Associated Maculopathy appearing in the brain include, but are not limited to, aphasia, seizures, memory loss, gait disturbances, neuromuscular atrophy, paralysis, loss of equilibrium, hearing loss, stroke, and blindness.

A symptom of Vascular Associated Maculopathy appearing in a subject's placenta can manifest as preeclampsia, with signs and symptoms of hypertension and proteinuria.

In one aspect, a symptom of Vascular Associated Maculopathy can present as macular degeneration. For example, and not to be limiting, macular degeneration can be age-related macular disorder (AMD), North Carolina macular dystrophy, Sorsby's fundus dystrophy, Stargardt's disease, pattern dystrophy, Best's disease, dominant drusen, malattia leventinese, serous retinal detachment, chorioretinal degenerations, retinal degenerations, photoreceptor degenerations, retinal pigment epithelial (RPE) degenerations, mucopolysaccharidoses, rod-cone dystrophies, cone-rod dystrophies, or cone degenerations.

In one aspect, Vascular Associated Maculopathy can cause decreased perfusion in the choriocapillaris lobules in the macula of an eye. In a further aspect, Vascular Associated Maculopathy can cause partial or total closure of choriocapillaris lobules in the macula of an eye. Examples of symptoms of Vascular Associated Maculopathy that can cause partial or total closure or occlusion of the choriocapillaris lobules, choroidal arterioles, ciliary arteries, the ophthalmic arteries, the internal carotid arteries, and the common carotid arteries include, but are not limited to, atherosclerosis, arteriosclerosis, arteriolar sclerosis, vasculitis, polycythemia vera, leukemia, hyperviscosity syndromes, thromboembolic disease, diabetes mellitus, infectious diseases, septic emboli, mycotic emboli, allergic diseases, neoplasms, autoimmune diseases, and the like.

Additionally, partial or total occlusion of the choriocapillaris lobules, choroidal arterioles, ciliary arteries, the ophthalmic arteries, the internal carotid arteries, or the common carotid arteries can cause a variety of symptoms associated with Vascular Associated Maculopathy including, but not limited to, atherosclerosis, arteriosclerosis, arteriolar sclerosis, vasculitis, polycythemia vera, leukemia, hyperviscosity syndromes, thromboembolic disease, diabetes mellitus, infectious diseases, septic emboli, mycotic emboli, allergic diseases, neoplasms, autoimmune diseases, and the like. Other causes of partial or total closure or occlusion of the choriocapillaris lobules, choroidal arterioles, ciliary arteries, the ophthalmic arteries, the internal carotid arteries, and the common carotid arteries include, but are not limited to, trauma, physical agents, radiation, toxins, and drugs.

In one aspect, a symptom of Vascular Associated Maculopathy can appear in a tissue or organ other than an eye. Therefore, also described herein are methods of diagnosing a symptom of Vascular Associated Maculopathy in a subject comprising detecting the presence of aberrant choriocapillaris lobules in an eye of the subject, wherein the presence of aberrant choriocapillaris lobules in the eye of the subject indicates the diagnosis of a symptom of Vascular Associated Maculopathy in the subject, and wherein the symptom of Vascular Associated Maculopathy does not appear as a disease or vascular disease of the eye.

Additionally, described herein are methods of identifying a predisposition for developing a symptom of Vascular Associated Maculopathy in a tissue or organ in a subject, comprising detecting aberrant choriocapillaris lobules in an eye of a subject, wherein detecting aberrant choriocapillaris lobules in the eye of the subject indicates a predisposition for developing a symptom of Vascular Associated Maculopathy in a tissue or organ in the subject, and wherein the symptom of Vascular Associated Maculopathy does not appear as a disease or vascular disease of the eye.

In one aspect, the methods described herein can further comprise determining the ratio of the diameters of retinal arteries (A) or veins (V), wherein a ratio (A:V) of the diameters of about 0.60 to about 1.48 further indicates the diagnosis of Vascular Associated Maculopathy, or a symptom thereof. In a further aspect, the ratio (A:V) of the diameters of about 0.60 to about 1.48 can also indicate that a subject is likely to have aberrant choriocapillaris lobules. In a further aspect, a ratio (A:V) of the diameters of about 0.8 can further indicate the diagnosis of Vascular Associated Maculopathy, or a symptom thereof, or can indicate that a subject is likely to have aberrant choriocapillaris lobules. In yet a further aspect, determining individual measurements of arteries and veins that are not normal can indicate the diagnosis of Vascular Associated Maculopathy, or a symptom thereof, or can indicate that a subject is likely to have aberrant choriocapillaris lobules. In still a further aspect, a diagnosis of Vascular Associated Maculopathy, or a symptom thereof, or a diagnosis of aberrant choriocapillaris lobules can be made by measuring blood flow, for example, in orbital vessels, the carotid artery, the ophthalmic artery, or the posterior ciliary artery. The blood flow can be measured using Doppler or other techniques known in the art. In yet a further aspect, diagnosis of Vascular Associated Maculopathy, or a symptom thereof, or a diagnosis of aberrant choriocapillaris lobules can be made by assessing the vascular architecture in the orbit using, for example, 3T MRI or other techniques known in the art.

Also described herein are methods of identifying severe or late stage maculopathy in a subject comprising detecting aberrant choriocapillaris lobules in an eye of a subject, wherein detecting aberrant choriocapillaris lobules in an eye of the subject indicates a predisposition for developing a severe or late stage maculopathy in the subject. This method can further comprise detecting areas of geographic atrophy in an eye of the subject. Geographic atrophy can result from the loss of large areas of choriocapillaries. Therefore, in one aspect, the methods described herein can be used to predict the rate at which macular disease will progress in a subject. This method can still further comprise detecting choroidal neovascularization (CNV) in an eye of the subject. CNV can result from aberrant choriocapillaris lobule driven ischemia in the retina or the choroid.

Also described herein are methods of identifying a symptom of Vascular Associated Maculopathy in a subject comprising detecting aberrant choriocapillaris lobules in an eye of a subject, wherein detecting aberrant choriocapillaris lobules in an eye of the subject indicates a predisposition for developing a symptom of Vascular Associated Maculopathy in the subject. This method can further comprise detecting areas of geographic atrophy in an eye of the subject. Geographic atrophy can result from the loss of large areas of choriocapillaries. Therefore, in one aspect, the methods described herein can be used to predict the rate at which a symptom of Vascular Associated Maculopathy or macular disease will progress in a subject.

Also described herein are methods method for diagnosing a subject with Vascular Associated Maculopathy, severe or late state maculopathy, or aberrant choriocapillaris lobules, comprising: generating an image of an eye of the subject, detecting the presence of aberrant choriocapillaris lobules in the image of eye of the subject, whereby the presence of aberrant choriocapillaris lobules in the eye of the subject results in a diagnosis of Vascular Associated Maculopathy, severe or late state maculopathy, or aberrant choriocapillaris lobules, In one aspect, the absence of aberrant choriocapillaris lobules in the eye of the subject can result in a diagnoses that the subject does not have Vascular Associated Maculopathy, severe or late state maculopathy, or aberrant choriocapillaris lobules.

In one aspect, the methods can further comprise in a sample obtained from the subject, determining the identity of one or more SNPs in the HTRA1, ARMS2, or CFH genes, wherein the one or more SNPs are (i) the rs11200638 in the HTRA1 gene, rs1049331 in the HTRA1 gene, rs2672587 in the HTRA1 gene, rs10490924 in the ARMS2 gene, rs3750848 in the ARMS2 gene, rs1061170 in the CFH gene, or rs800292 in the CFH gene, or (ii) a SNP in linkage disequilibrium with the SNPS of (i), thereby diagnosing the subject based on the presence of the one or more SNPs in (i) or (ii).

Also described herein are systems for identifying aberrant choriocapillaris lobules comprising a device for imaging an eye, the device configured to provide a recognizable image of aberrant choriocapillaris lobules, and instructions for identifying aberrant choriocapillaris lobules. Any device described herein, or known in the art, for imaging an eye can be used with the system. For example, and not to be limiting, the device can be n near-infrared reflectance and SD-OCT device or a Doppler imaging device.

### 2. HTRA1 AND ARMS2 SINGLE NUCLEOTIDE POLYMORPHISMS

Described herein are a collection of polymorphisms and haplotypes comprised of multiple variations in the HTRA1 and ARMS2 genes. One or more of these polymorphisms and haplotypes are associated with Vascular Associated Maculopathy, or a symptom thereof. Detection of these and other polymorphisms and sets of polymorphisms (e.g., haplotypes) can be useful in designing and performing diagnostic assays for Vascular Associated Maculopathy, or a symptom thereof. Polymorphisms and sets of polymorphisms can be detected by analysis of nucleic acids, by analysis of polypeptides encoded by HTRA1 or ARMS2 coding sequences (including polypeptides encoded by splice variants), by analysis of HTRA1 or ARMS2 non-coding sequences, or by other means known in the art. Analysis of such polymorphisms and haplotypes can also be useful in designing prophylactic and therapeutic regimes for Vascular Associated Maculopathy, or a symptom thereof.

The term "polymorphism" refers to the occurrence of one or more genetically determined alternative sequences or alleles in a population. A "polymorphic site" is the locus at which sequence divergence occurs. Polymorphic sites have at least one allele. A diallelic polymorphism has two alleles. A triallelic polymorphism has three alleles. Diploid organisms may be homozygous or heterozygous for allelic forms. A polymorphic site can be as small as one base pair. Examples of polymorphic sites include: restriction fragment length polymorphisms (RFLPs), variable number of tandem repeats (VNTRs), hypervariable regions, minisatellites, dinucleotide repeats, trinucleotide repeats, tetranucleotide repeats, and simple sequence repeats. As used herein, reference to a "polymorphism" can encompass a set of polymorphisms (i.e., a haplotype).

A "single nucleotide polymorphism (SNP)" can occur at a polymorphic site occupied by a single nucleotide, which is the site of variation between allelic sequences. The site can be preceded by and followed by highly conserved sequences of the allele. A SNP usually arises due to substitution of one nucleotide for another at the polymorphic site. Replacement of one purine by another purine or one pyrimidine by another pyrimidine is called a transition. Replacement of a purine by a pyrimidine or vice versa is called a transversion. A synonymous SNP refers to a substitution of one nucleotide for another in the coding region that does not change the amino acid sequence of the encoded polypeptide. A non-synonymous SNP refers to a substitution of one nucleotide for another in the coding region that changes the amino acid sequence of the encoded polypeptide. A SNP may also arise from a deletion or an insertion of a nucleotide or nucleotides relative to a reference allele.

A "set" of polymorphisms means one or more polymorphism, e.g., at least 1, at least 2, at least 3, at least 4, at least 5, at least 6, or more than 6 polymorphisms known, for example, in the HTRA1 or ARMS2 genes.

As used herein, "haplotype" means a DNA sequence comprising one or more polymorphisms of interest contained on a subregion of a single chromosome of an individual. A haplotype can refer to a set of polymorphisms in a single gene, an intergenic sequence, or in larger sequences including both gene and intergenic sequences, e.g., a collection of genes, or of genes and intergenic sequences. For example, a haplotype can refer to a set of polymorphisms in the chromosome 10q26 locus, which includes gene sequences for ARMS2, HTRA1, and intergenic sequences (i.e., intervening intergenic sequences, upstream sequences, and downstream sequences that are in linkage disequilibrium with polymorphisms in the genie region). The term "haplotype" can refer to a set of single nucleotide polymorphisms (SNPs) found to be statistically associated with each other on a single chromosome. A haplotype can also refer to a combination of polymorphisms (e.g., SNPs) and other genetic markers (e.g., an insertion or a deletion) found to be statistically associated with each other on a single chromosome. A "diplotype" is a haplotype pair. For example, a diplotype can comprise a protective and a risk haplotype, two protective haplotypes, two risk haplotypes, a neutral and a risk haplotype, a neutral and a protective haplotype, or two neutral haplotypes. In some circumstances, one haplotype may be dominant or one haplotype may be recessive.

HTRA1, also known as HtrA serine peptidase 1, L56, HtrA, ARMD7, ORF480 or PRSS11, is a gene encoding the HTRA1 protein, a protein that is a member of the trypsin family of serine proteases. HTRA1's NCBI Gene identification number is 5654, and one of skill in the art can readily obtain additional information regarding HTRA1 by accessing http://www.ncbi.nlm.nih.gov/gene/ and searching for HTRA1. For example, and not to be limiting, by accessing the NCBI Gene page for HTRA1, one of skill in the art can readily obtain information such as the genomic location of HTRAl, a summary of the properties of the HTRAl protein, information on cellular localization of HTRAl, information on homologs and variants (for example, splice variants) of HTRAl, as well as numerous HTRAl reference sequences, such as the genomic sequence of HTRA, the mRNA sequence of HTRAl and the protein sequence of HTRAl.

ARMS2, also known as age-related maculopathy susceptibility 2 or ARMD8, is a gene encoding the ARMS2 protein. ARMS2's NCBI Gene identification number is 387715, and one of skill in the art can readily obtain additional information regarding ARMS2 by accessing http://www.ncbi.nlm.nih.gov/gene/ and searching for ARMS2. By accessing the NCBI Gene page for ARMS2, one of skill in the art can readily obtain information such as the genomic location of ARMS2, a summary of the properties of the ARMS2 protein, information on cellular localization of ARMS2, information on homologs and variants (for example, splice variants) of ARMS2, as well as numerous ARMS2 reference sequences, such as the genomic sequence of ARMS2, the mRNA sequence of ARMS2 and the protein sequence of ARMS2.

Unless otherwise noted, "HTRAl" or "ARMS2" as used herein, includes any HTRAl or ARMS2 gene, nucleic acid (DNA or RNA), or protein from any organism that retains at least one activity of HTRAl or ARMS2. In addition, "HTRAl" or "ARMS2" as used herein, includes any non-coding sequence present in or intergenic sequence present between the genomic DNA or RNA of the HTRAl or ARMS2 genes. When referring to HTRAl or ARMS2, this also includes any HTRAl or ARMS2 gene, nucleic acid (DNA or RNA), or protein from any organism that and can function as an HTRAl or ARMS2 nucleic acid or protein associated with Vascular Associated Maculopathy, or a symptom thereof. For example, the nucleic acid or protein sequence can be from or in a cell of a subject.

As used herein, a "nucleic acid", "polynucleotide" or "oligonucleotide" is a polymeric form of nucleotides of any length, may be DNA or RNA, and may be single- or double-stranded. Nucleic acids may include promoters or other regulatory sequences. Oligonucleotides are usually prepared by synthetic means. Nucleic acids include segments of DNA, or their complements spanning or flanking any one of the polymorphic sites known in the HTRA1 or ARMS2 genes. The segments are usually between 5 and 100 contiguous bases and often range from a lower limit of 5, 10, 15, 20, or 25 nucleotides to an upper limit of 10, 15, 20, 25, 30, 50, or 100 nucleotides (where the upper limit is greater than the lower limit). Nucleic acids between 5-10, 5-20, 10-20, 12-30, 15-30, 10-50, 20-50, or 20-100 bases are common. The polymorphic site can occur within any position of the segment. A reference to the sequence of one strand of a double-stranded nucleic acid defines the complementary sequence and except where otherwise clear from context, a reference to one strand of a nucleic acid also refers to its complement.

For certain applications, nucleic acid (e.g., RNA) molecules may be modified to increase intracellular stability and half-life. Possible modifications include, but are not limited to, the use of phosphorothioate or 2'-O-methyl rather than phosphodiesterase linkages within the backbone of the molecule. Modified nucleic acids include peptide nucleic acids (PNAs) and nucleic acids with nontraditional bases such as inosine, queosine and wybutosine and acetyl-, methyl-, thio-, and similarly modified forms of adenine, cytidine, guanine, thymine, and uridine which are not as easily recognized by endogenous endonucleases.

As used herein, "hybridization probes" are nucleic acids capable of binding in a base-specific manner to a complementary strand of nucleic acid. Such probes include nucleic acids and peptide nucleic acids (Nielsen et al., 1991). Hybridization may be performed under stringent conditions which are known in the art. For example, see Berger and Kimmel (1987) Methods In Enzymology, Vol. 152: Guide To Molecular Cloning Techniques, San Diego: Academic Press, Inc.; Sambrook et al. (1989) Molecular Cloning: A Laboratory Manual, 2nd Ed., Vols. 1-3, Cold Spring Harbor Laboratory; Sambook (2001) 3rd Edition; Rychlik, W. and Rhoads, R. E., 1989, Nucl. Acids Res. 17, 8543; Mueller, P. R. et al. (1993) In: Current Protocols in Molecular Biology 15.5, Greene Publishing Associates, Inc. and John Wiley and Sons, New York; and Anderson and Young, Quantitative Filter Hybridization in Nucleic Acid Hybridization (1985)). As used herein, the term "probe" includes primers. Probes and primers are sometimes referred to as "oligonucleotides."

The term "primer" refers to a single-stranded oligonucleotide capable of acting as a point of initiation of template-directed DNA synthesis under appropriate conditions, in an appropriate buffer and at a suitable temperature. The appropriate length of a primer depends on the intended use of the primer but typically ranges from 15 to 30 nucleotides. A primer sequence need not be exactly complementary to a template but must be sufficiently complementary to hybridize with a template. The term "primer site" refers to the area of the target DNA to which a primer hybridizes. The term "primer pair" means a set of primers including a 5' upstream primer, which hybridizes to the 5' end of the DNA sequence to be amplified and a 3' downstream primer, which hybridizes to the complement of the 3' end of the sequence to be amplified.

Exemplary hybridization conditions for short probes and primers is about 5 to 12 degrees C, below the calculated Tm. Formulas for calculating Tm are known and include: Tm = 4°C x (number of G's and C's in the primer) + 2°C x (number of A's and T's in the primer) for oligos <14 bases and assumes a reaction is carried out in the presence of 50 mM monovalent cations. For longer oligos, the following formula can be used: Tm = 64.9°C + 41°C x (number of G's and C's in the primer-16.4)/N, where N is the length of the primer. Another commonly used formula takes into account the salt concentration of the reaction (Rychlik, supra, Sambrook, supra, Mueller, supra): Tm = 81.5°C + 16.6°C x (log 10[Na+]+[K+]) + 0.41°C x (% GC) - 675/N, where N is the number of nucleotides in the oligo. The aforementioned formulas provide a starting point for certain applications; however, the design of particular probes and primers may take into account additional or different factors. Methods for design of probes and primers for use in the methods described herein are well known in the art.

As used herein, the terms "risk," "protective," and "neutral" are used to describe variations, SNPs, haplotypes, diplotypes, and proteins in a population encoded by genes characterized by such patterns of variations. A risk SNP is an allelic form of a gene, for example an HTRAl or ARMS2 gene, comprising at least one variant polymorphism associated with increased risk for developing Vascular Associated Maculopathy, or a symptom thereof. The term "variant," when used in reference to an HTRA1 or ARMS2 gene, refers to a nucleotide sequence in which the sequence differs from the sequence most prevalent in a population. The variant polymorphisms can be in the coding or non-coding portions of the gene. An example of a risk HTRA1 SNP is an A allele at the rs11200638 SNP in the HTRA1 gene. The risk SNP can be naturally occurring or can be synthesized by recombinant techniques. A protective SNP is an allelic form of a gene, herein an HTRA1 or ARMS2 gene, comprising at least one variant polymorphism associated with decreased risk of developing Vascular Associated Maculopathy, or a symptom thereof. For example, one protective HTRA1 SNP is a G allele at the rs11200638 SNP in the HTRA1 gene. The protective SNP can be naturally occurring or synthesized by recombinant techniques. Thus, the "protective" forms of HTRAl or ARMS2 can provide therapeutic benefit when administered to, for example, a subject with Vascular Associated Maculopathy or at risk for developing Vascular Associated Maculopathy, and thus can "protect" the subject from disease.

The term "variant" can also refer to a nucleotide sequence in which the sequence differs from the sequence most prevalent in a population, for example by one nucleotide, in the case of the SNPs described herein. For example, some variations or substitutions in the nucleotide sequence of the HTRA1 or ARMS2 genes alter a codon so that a different amino acid is encoded resulting in a variant polypeptide. The term "variant," can also refer to a polypeptide in which the sequence differs from the sequence most prevalent in a population at a position that does not change the amino acid sequence of the encoded polypeptide (i.e., a conserved change). Variant polypeptides can be encoded by a risk haplotype, encoded by a protective haplotype, or can be encoded by a neutral haplotype. Variant HTRA1 or ARMS2 polypeptides can be associated with risk, associated with protection, or can be neutral.

By "isolated nucleic acid" or "purified nucleic acid" is meant DNA that is free of the genes that, in the naturally-occurring genome of the organism from which the DNA is derived, flank the gene. The term therefore includes, for example, a recombinant DNA which is incorporated into a vector, such as an autonomously replicating plasmid or virus; or incorporated into the genomic DNA of a prokaryote or eukaryote (e.g., a transgene); or which exists as a separate molecule (for example, a cDNA or a genomic or cDNA fragment produced by PCR, restriction endonuclease digestion, or chemical or in vitro synthesis). It also includes a recombinant DNA which is part of a hybrid gene encoding additional polypeptide sequence. The term "isolated nucleic acid" also refers to RNA, e.g., an mRNA molecule that is encoded by an isolated DNA molecule, or that is chemically synthesized, or that is separated or substantially free from at least some cellular components, for example, other types of RNA molecules or polypeptide molecules.

By "isolated polypeptide" or "purified polypeptide" is meant a polypeptide (or a fragment thereof) that is substantially free from the materials with which the polypeptide is normally associated in nature. The polypeptides, or fragments thereof, can be obtained, for example, by extraction from a natural source (for example, a mammalian cell), by expression of a recombinant nucleic acid encoding the polypeptide (for example, in a cell or in a cell-free translation system), or by chemically synthesizing the polypeptide. In addition, polypeptide fragments may be obtained by any of these methods, or by cleaving full length polypeptides.

Two amino acid sequences are considered to have "substantial identity" when they are at least about 80% identical, at least about 90% identical, at least about 95% identical, at least about 98% identical, or at least about 99% identical. Percentage sequence identity is typically calculated by determining the optimal alignment between two sequences and comparing the two sequences. Optimal alignment of sequences may be conducted by inspection, or using the local homology algorithm of Smith and Waterman, 1981, Adv. Appl. Math. 2: 482, using the homology alignment algorithm of Needleman and Wunsch, 1970, J. Mol. Biol. 48: 443, using the search for similarity method of Pearson and Lipman, 1988, Proc. Natl. Acad. Sei. U.S.A. 85: 2444, by computerized implementations of these algorithms (e.g., in the Wisconsin Genetics Software Package, Genetics Computer Group, 575 Science Dr., Madison, Wis.) using default parameters for amino acid comparisons (e.g., for gap-scoring, etc.). It is sometimes desirable to describe sequence identity between two sequences in reference to a particular length or region (e.g., two sequences may be described as having at least 95% identity over a length of at least 500 base pairs). Usually the length will be at least about 50, 100, 200, 300, 400, 500, 600, 700, 800, 900, or 1000 amino acids, or the full length of the reference protein. Two amino acid sequences can also be considered to have substantial identity if they differ by 1, 2, or 3 residues, or by from 2-20 residues, 2-10 residues, 3-20 residues, or 3-10 residues.

It should be understood that polymorphic sites in the HTRAl or ARMS2 genes, may be associated with Vascular Associated Maculopathy, or a symptom thereof, wherein the symptom of Vascular Associated Maculopathy can be macular degeneration, age-related macular degeneration, cardiac microvascular disease, Stargardt's, Pseudoxanthoma Elasticum (PXE), Alagille syndrome, subcortical leukoencephalopathy, preeclampsia, hypertension, diabetes mellitus, myocardial ischemia, aneurysms, vasculitis due to autoimmune disease or infectious agents, deep vein thrombosis, lymphedema, varicose veins, peripheral artery disease, renal artery disease, Raynaud's disease, Buerger's disease, peripheral venous disease, venous blood clots, atherosclerosis, arteriosclerosis, arteriolar sclerosis, coronary artery disease, angina, congestive heart failure, hardening of the arteries, thrombotic or embolic stroke, myocardial infarction, cerebral autosomal recessive arteriopathy with subcortical infarcts and leukoencephalopathy (CARASIL), retinal vasculopathy with cerebral leukodystrophy (RVCL) and the Collagen type IV, alpha 1 (COL4Al)-related disorders, or Cerebral autosomal dominant arteriopathy with subcortical infarcts or leukoencephalopathy (CADASIL).

Exemplary polymorphic sites in the HTRAl or ARMS2 genes are described herein as examples and are not intended to be limiting. These polymorphic sites, or SNPs, can also be used in carrying out methods described herein. Moreover, it will be appreciated that these HTRAl or ARMS2 polymorphisms are useful for linkage and association studies, genotyping clinical populations, correlation of genotype information to phenotype information, loss of heterozygosity analysis, identification of the source of a cell sample and can also be useful to target potential therapeutics to cells.

It will be appreciated that additional polymorphic sites in the HTRAl or ARMS2 genes, which are not explicitly described in the Tables herein, may further refine this analysis. A SNP analysis using non-synonymous polymorphisms in the HTRA1 or ARMS2 genes can be useful to identify variant HTRA1 or ARMS2 polypeptides. Other SNP associated with risk may encode a protein with the same sequence as a protein encoded by a neutral or protective SNP but contain an allele in a promoter or intron, for example, changes the level or site of HTRA1 or ARMS2 expression. It will also be appreciated that a polymorphism in the HTRA1 or ARMS2 gene may be linked to a variation in a neighboring gene. The variation in the neighboring gene may result in a change in expression or form of an encoded protein and have detrimental or protective effects in the carrier.

The methods and materials provided herein can be used to determine whether an HTRA1 or ARMS2 nucleic acid of a subject (e.g., human) contains a polymorphism, such as a single nucleotide polymorphism (SNP). For example, methods and materials provided herein can be used to determine whether a subject has a variant SNP. Any method can be used to detect a polymorphism in an HTRA1 or ARMS2 nucleic acid. For example, polymorphisms can be detected by sequencing exons, introns, or untranslated sequences, denaturing high performance liquid chromatography (DHPLC), allele-specific hybridization, allele-specific restriction digests, mutation specific polymerase chain reactions, single-stranded conformational polymorphism detection, and combinations of such methods.

Described herein are methods for predicting a subject's risk for having or developing Vascular Associated Maculopathy in a human subject, comprising: determining in the subject the identity of one or more SNPs in the HTRA1, ARMS2, or CFH genes, wherein the one or more SNPs are (i) the rs11200638 in the HTRA1 gene, rs1049331 in the HTRA1 gene, rs2672587 in the HTRA1 gene, rs10490924 in the ARMS2 gene, rs3750848 in the ARMS2 gene, rs1061170 in the CFH gene, or rs800292 in the CFH gene, or (ii) a SNP in linkage disequilibrium with the SNPS of (i), wherein the presence of one or more of the SNPs is predictive of the subject's risk for having or developing Vascular Associated Maculopathy. In one aspect, the method can predict a subject's risk for having or developing severe maculopathy or last stage maculopathy. In a further aspect, the methods can predict a subject's risk for having or developing aberrant choriocapillaris lobules.

In one aspect, determining the identity of an A at the rs11200638 SNP, a T at the rs1049331 SNP, a G at the rs2672587 SNP, a T at the rs10490924 SNP, a G at the rs3750848 SNP, a C at the rs1061170 SNP, or a G at the rs800292 SNP can be predictive of the subject's risk for having or developing Vascular Associated Maculopathy, severe or late stage maculopathy, or aberrant choriocapillaris lobules.

In another aspect, determining the identity of an A at the rs11200638 SNP, a T at the rs1049331 SNP, a G at the rs2672587 SNP, a T at the rs10490924 SNP, a G at the rs3750848 SNP, a C at the rs1061170 SNP, or a G at the rs800292 SNP can be predictive of the subject's increased risk for having or developing Vascular Associated Maculopathy, severe or late state maculopathy, or aberrant choriocapillaris lobules.

In a further aspect, determining the identity of a G at the rs11200638 SNP, a C at the rs1049331 SNP, a C at the rs2672587 SNP, a G at the rs10490924 SNP, a T at the rs3750848 SNP, a T at the rs1061170 SNP, or an A at the rs800292 SNP can be predictive of the subject's risk for having or developing Vascular Associated Maculopathy, severe or late state maculopathy, or aberrant choriocapillaris lobules.

In still a further aspect, determining the identity of a G at the rs11200638 SNP, a C at the rs1049331 SNP, a C at the rs2672587 SNP, a G at the rs10490924 SNP, a T at the rs3750848 SNP, a T at the rs1061170 SNP, or an A at the rs800292 SNP can be predictive of the subject's decreased risk for having or developing Vascular Associated Maculopathy, severe or late state maculopathy, or aberrant choriocapillaris lobules.

In one aspect, the methods described herein can further comprise: generating an image of an eye of the subject, detecting the presence of aberrant choriocapillaris lobules in the eye of the subject using the image, wherein the presence of aberrant choriocapillaris lobules in the eye of the subject is predictive of the subject's risk for having or developing Vascular Associated Maculopathy. As used herein, Vascular Associated Maculopathy can further comprise one or more symptoms associated with Vascular Associated Maculopathy, wherein the one or more symptoms of Vascular Associated Maculopathy is macular degeneration, age-related macular degeneration, cardiac microvascular disease. Staregardt's. Pseudoxanthoma Elasticum (PXE). Alagille syndrome, subcortical leukoencephalopathy, preeclampsia, hypertension, diabetes mellitus, myocardial ischemia, aneurysms, vasculitis due to autoimmune disease or infectious agents, deep vein thrombosis, lymphedema, varicose veins, peripheral artery disease, renal artery disease, Raynaud's disease, Buerger's disease, peripheral venous disease, venous blood clots, atherosclerosis, arteriosclerosis, arteriolar sclerosis, coronary artery disease, angina, congestive heart failure, hardening of the arteries, thrombotic or embolic stroke, or myocardial infarction.

In a further aspect, the methods described herein can further comprise determining the ratio of the diameters of retinal arteries or veins, wherein a ratio of the diameters of retinal arteries (A) or veins (V) of about 0.60 to about 1.48 can be predictive of the subject's risk for having or developing Vascular Associated Maculopathy, severe or late state maculopathy, or aberrant choriocapillaris lobules.

Also described herein are methods for diagnosing a subject with Vascular Associated Maculopathy, comprising: in a sample obtained from the subject, determining the identity of one or more SNPs in the HTRA1, ARMS2, or CFH genes, wherein the one or more SNPs are (i) the rs11200638 in the HTRA1 gene, rs1049331 in the HTRA1 gene, rs2672587 in the HTRA1 gene, rs10490924 in the ARMS2 gene, rs3750848 in the ARMS2 gene, rs1061170 in the CFH gene, or rs800292 in the CFH gene, or (ii) a SNP in linkage disequilibrium with the SNPs of (i), thereby diagnosing the subject based on the presence of the one or more variants of the SNPs in (i) or (ii), wherein an A at the rs11200638 SNP, a T at the rs1049331 SNP, a G at the rs2672587 SNP, a T at the rs10490924 SNP, a G at the rs3750848 SNP, a C at the rs1061170 SNP, or a G at the rs800292 SNP can diagnose the subject with Vascular Associated Maculopathy, severe or late state maculopathy, or aberrant choriocapillaris lobules. In one aspect, determining the identity of a G at the rs11200638 SNP, a C at the rs1049331 SNP, a C at the rs2672587 SNP, a G at the rs10490924 SNP, a T at the rs3750848 SNP, a T at the rs1061170 SNP, or an A at the rs800292 SNP can diagnose the subject without Vascular Associated Maculopathy, severe or late state maculopathy, or aberrant choriocapillaris lobules.

Described herein are methods of diagnosing Vascular Associated Maculopathy, or a symptom thereof in a subject, comprising determining in the subject the identity of one or more SNPs identified in Table 1.

Also described herein are methods of diagnosing Vascular Associated Maculopathy, or a symptom thereof in a subject, comprising determining in the subject the identity of one or more SNPs in the HTRA1 or AMRS2 genes. For example the SNPs can include, but are not limited to, rs11200638 in the HTRA1 gene and rs10490924 in the ARMS2 gene, wherein an A allele at the HTRA1 SNP and a T allele at the ARMS2 SNP indicates the presence of Vascular Associated Maculopathy.

Also described herein are methods of diagnosing Vascular Associated Maculopathy, or a symptom thereof in a subject, comprising determining in the subject the identity of rs11200638 in the HTRA1 gene and rs10490924 in the ARMS2 gene, wherein an A allele at rs11200638 in the HTRA1 gene and a T allele at rs10490924 in the ARMS2 gene indicates the presence of Vascular Associated Maculopathy, or a symptom thereof.

In addition, described herein are methods of diagnosing Vascular Associated Maculopathy, or a symptom thereof in a subject, comprising determining in the subject the identity of one or more SNPs in the HTRA1 or ARMS2 genes. For example the SNPs can include, but are not limited to, rs1049331, rs3750848, or rs2672587, wherein the presence of one or more of rs1049331, rs3750848, or rs2672587 indicates the presence of Vascular Associated Maculopathy, or a symptom thereof.

In one aspect, diagnosing Vascular Associated Maculopathy, or a symptom thereof, in a subject can comprise detecting a SNP, or multiple SNPs, that are in linkage disequilibrium with the HTRA1 or ARMS2 polymorphisms described herein.

Furthermore, described herein are methods of diagnosing Vascular Associated Maculopathy, or a symptom thereof, in a subject, comprising determining in the subject the identity of one or more aberrant choriocapillaris lobule-associated SNPs. As used herein, aberrant choriocapillaris lobule-associated SNPs means SNPs that indicate the presence, or likelihood of developing, aberrant choriocapillaris lobules. For example the SNPs can include, but are not limited to, an A allele at rs11200638 SNP in the HTRA1 gene, a T allele at the rs10490924 SNP in the ARMS2 gene, a C allele at rs1061170 in the CFH gene, or a G allele at rs2230199 SNP in the C3 gene, wherein the presence of one or more of the aberrant choriocapillaris lobule-associated SNPs indicates the presence of Vascular Associated Maculopathy, or a symptom thereof.

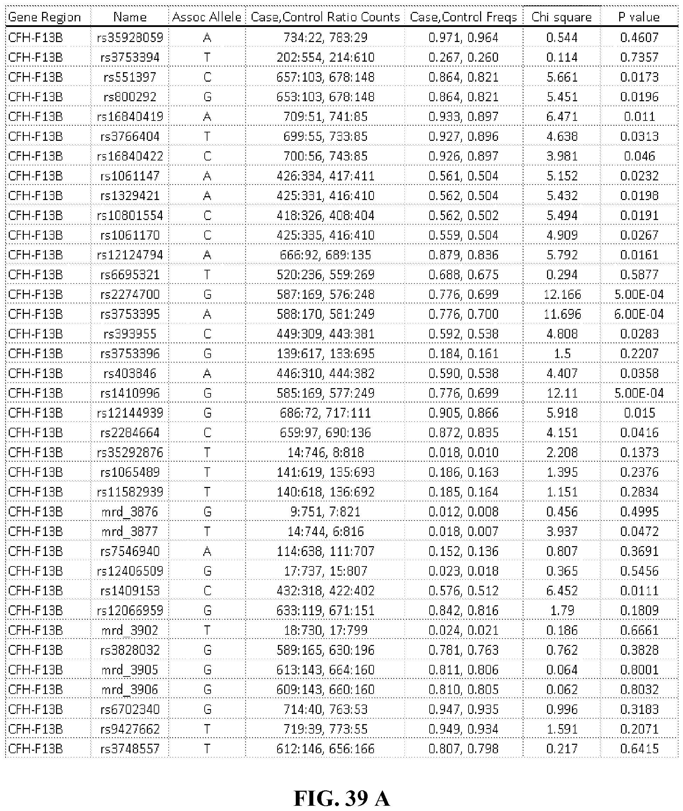

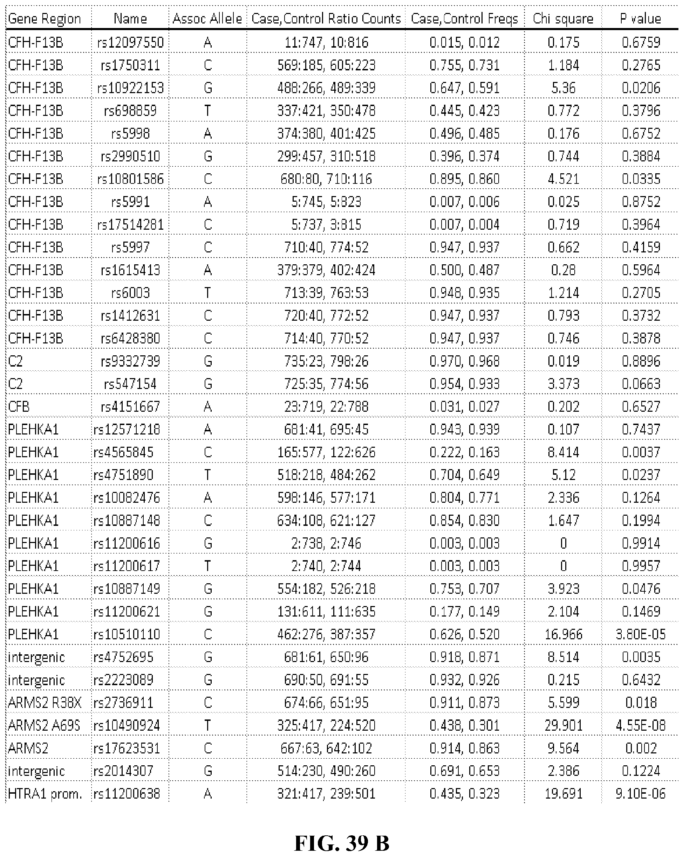

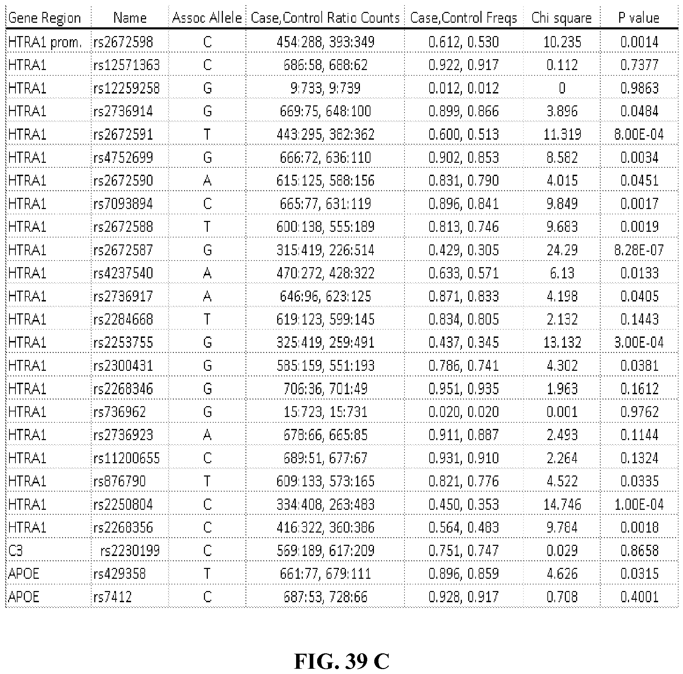

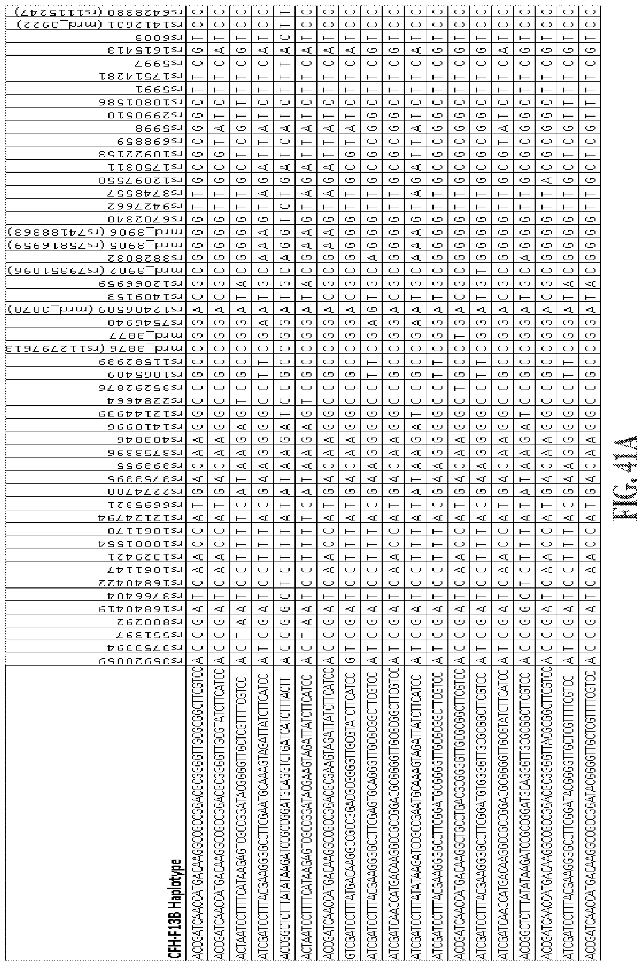

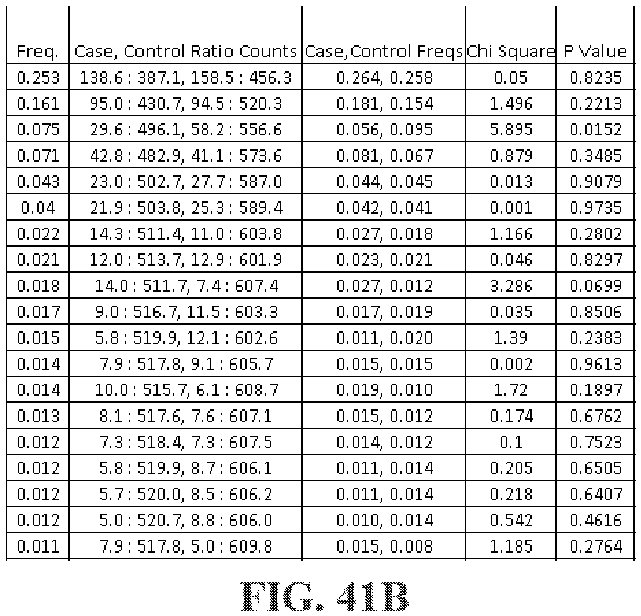

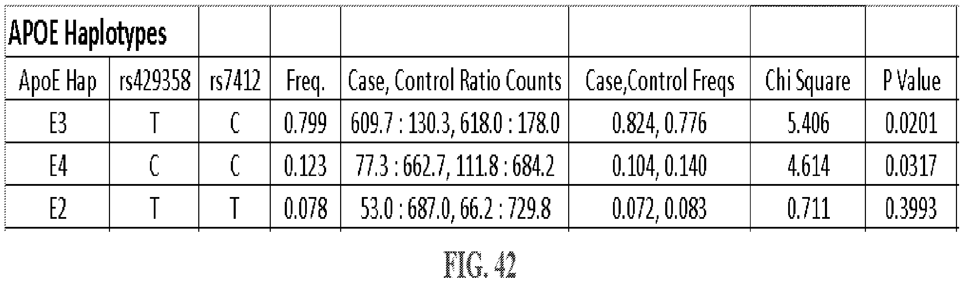

In one aspect, "aberrant choriocapillaris lobule-associated SNPs" can also mean a SNP, or multiple SNPs, that are in linkage disequilibrium with the HTRA1, ARMS2, CFH, or C3 polymorphisms described herein. Examples of aberrant choriocapillaris lobule-associated SNPs include, but are not limited to the SNPs listed in Table 1 and Figures 39-42.

Also described herein are kits comprising an assay for detecting one or more SNPs in a nucleic acid sample of a subject, wherein the SNP is (i) rs11200638 in the HTRA1 gene, rs1049331 in the HTRA1 gene, rs2672587 in the HTRA1 gene, rs10490924 in the ARMS2 gene, rs3750848 in the ARMS2 gene, rs1061170 in the CFH gene, or rs800292 in the CFH gene; (ii) combinations of the SNPs of (i); or (iii) a SNP in linkage disequilibrium with the SNPs of (i). In one aspect, the kits can further comprise instructions for correlating the assay results with the subject's risk for having or developing Vascular Associated Maculopathy, severe or late stage maculopathy, or aberrant choriocapillaris lobules.

**Table 1**

| **Gene** | **SNP** | **Reference (Nucleotide)** | **Reference (Protein)** | **Variation (Nucleotide)** | **Variation (Protein)** | **Nucleotide** | **Peptide** | **RPD** |
|---|---|---|---|---|---|---|---|---|
| HTRA1 | rs11200638 | NG_011554.1 | NP_002766.1 | g.4504G>A | promoter | G | non-coding | protective |
| | | | | | | A | non-coding | risk |
| HTRA1 | rs1049331 | NG_011554.1 | NP_002766.1 | g.5230C>T | A34A | C | A | |
| | | | | | | T | A | |
| HTRA1 | rs2672587 | NG_011554.1 | NP_002766.1 | g.19315G>C | intronic | G | non-coding | risk |
| | | | | | | C | non-coding | protective |
| ARMS2 | rs10490924 | NG_ 011725.1 | NP_001093137.1 | g.5270G>T | A69S | G | A | protective |
| | | | | | | T | S | risk |
| ARMS2 | rs3750848 | NG_011725.1 | NP_001093137.1 | g.6137T>G | intronic | T | non-coding | |
| | | | | | | G | non-coding | |
| CFH | rs1061170 | NG_007259.1 | NP_000177.2 | g.43097T>C | Y402H | T | Y | protective |
| | | | | | | C | H | risk |
| CFH | rs800292 | NG_007259.1 | NP_000177.2 | g.26093G>A | V62I | G | V | risk |
| | | | | | | A | I | protective |
| C3 | rs2230199 | NG_009557.1 | NP_000055.2 | g.7276C>G | R102G | C | R | |
| | | | | | | G | G | |
| C2 | rs9332739 | NG_011730.1 | NP_000054.2 | g.13539G>C | E318D | G | E | |
| | | | | | | C | D | |
| C2 | rs547154 | NG_011730.1 | NP_000054.2 | g.20673G>T | intronic | G | non-coding | |
| | | | | | | T | non-coding | |
| CFB | rs4151667 | NG_008191.1 | NP_001701.2 | g.5304T>A | L9H | T | L | |
| | | | | | | A | H | |
| CFB | rs641153 | NG_008191.1 | NP_001701.2 | g.5460G>A | R32Q | G | R | |
| | | | | | | A | Q | |
| ApoE | rs429358 | NG_007084.2 | NP_ 000032.1 | g.7903T>C | C130R | T | C | |
| | | | | | | C | R | |
| ApoE | rs7412 | NG_007084.2 | NP_000032.1 | g.8041C>T | R176C | C | R | |
| | | | | | | T | C | |

As used herein, "linkage" describes the tendency of genes, alleles, loci or genetic markers to be inherited together as a result of their location on the same chromosome. Linkage can be measured by percent recombination between the two genes, alleles, loci or genetic markers. Typically, loci occurring within a 50 centimorgan (cM) distance of each other are linked. Linked markers may occur within the same gene or gene cluster. As used herein, "linkage disequilibrium" is the non-random association of alleles at two or more loci, not necessarily on the same chromosome. It is not the same as linkage, which describes the association of two or more loci on a chromosome with limited recombination between them. Linkage disequilibrium describes a situation in which some combinations of alleles or genetic markers occur more or less frequently in a population than would be expected from a random formation of haplotypes from alleles based on their frequencies. Non-random associations between polymorphisms at different loci are measured by the degree of linkage disequilibrium (LD). The level of linkage disequilibrium can be influenced by a number of factors including genetic linkage, the rate of recombination, the rate of mutation, random drift, non-random mating, and population structure. "Linkage disequilibrium" or "allelic association" thus means the non-random association of a particular allele or genetic marker with another specific allele or genetic marker more frequently than expected by chance for any particular allele frequency in the population. A marker in linkage disequilibrium with an informative marker, such as one of the HTRA1 or ARMS2 SNPs described herein can be useful in detecting susceptibility to Vascular Associated Maculopathy, or a symptom thereof. A SNP that is in linkage disequilibrium with a risk, protective, or otherwise informative SNP or genetic marker described herein can be referred to as a "proxy" or "surrogate" SNP. A proxy SNP may be in at least 50%, 60%, or 70% in linkage disequilibrium with risk, protective, or otherwise informative SNP or genetic marker described herein, and in one aspect is at least about 80%, 90%, and in another aspect 91%, 92%, 93%, 94%, 95%, 96%, 97%, 98%, 99%, or about 100% in LD with a risk, protective, or otherwise informative SNP or genetic marker described herein.

Publicly available databases such as the HapMap database (http://ftp.hapmap.org/ld_data/latest/) and Haploview (Barrett, J. C. et al., Bioinformatics 21, 263 (2005)) may be used to calculate linkage disequilibrium between two SNPs. The frequency of identified alleles in disease versus control populations can be determined using the methods described herein. Statistical analyses can be employed to determine the significance of a non-random association between the two SNPs (e.g., Hardy-Weinberg Equilibrium, Genotype likelihood ratio (genotype p value), Chi Square analysis, Fishers Exact test). A statistically significant non-random association between the two SNPs indicates that they are in linkage disequilibrium and that one SNP can serve as a proxy for the second SNP.

In addition, described herein are methods of determining the risk a subject will develop Vascular Associated Maculopathy, or a symptom thereof, comprising determining in the subject the identity of one or more aberrant choriocapillaris lobule-associated SNPs, which include, but are not limited to, an A allele at rs800292 SNP in the CFH gene, an A allele at rs641153 SNP in the CFB gene, or an A allele at rs4151667 SNP in the CFB gene, wherein the presence of one or more of the SNPs indicates decreased risk of developing Vascular Associated Maculopathy, or a symptom thereof.

Furthermore, described herein are methods of determining the risk a subject will develop Vascular Associated Maculopathy, or a symptom thereof,, comprising determining in the subject the identity of a G allele at the rs11200638 SNP in the HTRA1 gene, or a T allele at the rs10490924 SNP in the ARMS2 gene, wherein a G allele at the rs11200638 SNP in the HTRA1 gene and a T allele at the rs10490924 SNP in the ARMS2 gene indicates decreased risk of developing Vascular Associated Maculopathy, or a symptom thereof.

In one aspect, the methods described herein can further comprise determining the ratio of the diameters of retinal arteries (A) or veins (V), wherein a ratio (A:V) of the diameters of about 0.60 to about 1.48 further indicates the diagnosis of Vascular Associated Maculopathy, or a symptom thereof. In a further aspect, the ratio (A:V) of the diameters of about 0.60 to about 1.48 can also indicate that a subject is likely to have aberrant choriocapillaris lobules. In a further aspect, a ratio (A:V) of the diameters of about 0.8 can further indicate the diagnosis of Vascular Associated Maculopathy, or a symptom thereof, or can indicate that a subject is likely to have aberrant choriocapillaris lobules. In yet a further aspect, determining individual measurements of arteries and veins that are not normal can indicate the diagnosis of Vascular Associated Maculopathy, or a symptom thereof, or can indicate that a subject is likely to have aberrant choriocapillaris lobules. In still a further aspect, a diagnosis of Vascular Associated Maculopathy, or a symptom thereof, or a diagnosis of aberrant choriocapillaris lobules can be made by measuring blood flow, for example, in orbital vessels, the carotid artery, the ophthalmic artery, or the posterior ciliary artery. The blood flow can be measured using Doppler or other techniques known in the art. In yet a further aspect, diagnosis of Vascular Associated Maculopathy, or a symptom thereof, or a diagnosis of aberrant choriocapillaris lobules can be made by assessing the vascular architecture in the orbit using, for example, 3T MRI or other techniques known in the art.

The discovery that polymorphic sites in the HTRA1 or ARMS2 genes are associated with Vascular Associated Maculopathy, or a symptom thereof, has a number of specific applications including, but not limited to, screening individuals to ascertain risk of developing Vascular Associated Maculopathy, or a symptom thereof, and identification of new and optimal therapeutic approaches for individuals afflicted with, or at increased risk of developing, Vascular Associated Maculopathy, or a symptom thereof. Without intending to be limited to a specific mechanism, polymorphisms in the HTRA1 or ARMS2 genes can contribute to the phenotype of an individual in different ways. Polymorphisms that occur within the protein coding region of HTRA1 or ARMS2 may contribute to a phenotype by affecting the protein structure and/or function. Polymorphisms that occur in the non-coding regions of HTRA1 or ARMS2 may exert phenotypic effects indirectly via their influence on replication, transcription and/or translation. Certain polymorphisms in the HTRA1 or ARMS2 genes may predispose an individual to a distinct mutation that is causally related to Vascular Associated Maculopathy, or a symptom thereof. Alternatively, as noted above, a polymorphism in the HTRA1 or ARMS2 gene may be linked to a variation in a neighboring gene (including, but not limited to, PLEKHA1). The variation in the neighboring gene may result in a change in expression or form of an encoded protein and have detrimental or protective effects in the carrier.

Polymorphisms can be detected in a target nucleic acid isolated from a subject. Typically genomic DNA is analyzed. For assay of genomic DNA, virtually any biological sample containing genomic DNA or RNA, e.g., nucleated cells, is suitable. For example, in the experiments described in the Examples section herein, genomic DNA was obtained from peripheral blood leukocytes collected from case and control subjects (QIAamp DNA Blood Maxi kit, Qiagen, Valencia, Calif.). Other suitable samples include, but are not limited to, saliva, cheek scrapings, biopsies of retina, kidney or liver or other organs or tissues; skin biopsies; amniotic fluid or CNS samples; and the like. In one aspect RNA or cDNA can be assayed. In one aspect, the assay can detect variant HTRA1 or ARMS2 proteins. Methods for purification or partial purification of nucleic acids or proteins from patient samples for use in diagnostic or other assays are known in the art.

The identity of bases occupying the polymorphic sites in the HTRA1 and ARMS2 genes can be determined in an individual, e.g., in a patient being analyzed, using any of several methods known in the art. For example, and not to be limiting use of allele-specific probes, use of allele-specific primers, direct sequence analysis, denaturing gradient gel electrophoresis (DGGE) analysis, single-strand conformation polymorphism (SSCP) analysis, and denaturing high performance liquid chromatography (DHPLC) analysis. Other well known methods to detect polymorphisms in DNA include use of: Molecular Beacons technology (see, e.g., Piatek et al., 1998; Nat. Biotechnol. 16:359-63; Tyagi, and Kramer, 1996, Nat. Biotechnology 14:303-308; and Tyagi, et al., 1998, Nat. Biotechnol. 16:49-53), Invader technology (see, e.g., Neri et al., 2000, Advances in Nucleic Acid and Protein Analysis 3826:117-125 and U.S. Pat. No. 6,706,471), nucleic acid sequence based amplification (Nasba) (Compton, 1991), Scorpion technology (Thelwell et al., 2000, Nuc. Acids Res, 28:3752-3761 and Solinas et al., 2001, "Duplex Scorpion primers in SNP analysis and FRET applications" Nuc. Acids Res, 29:20.), restriction fragment length polymorphism (RFLP) analysis, and the like. Additional methods will be apparent to one of skill in the art.

The design and use of allele-specific probes for analyzing polymorphisms are described by e.g., Saiki et al., 1986; Dattagupta, EP 235,726, Saiki, WO 89/11548. Briefly, allele-specific probes are designed to hybridize to a segment of target DNA from one individual but not to the corresponding segment from another individual, if the two segments represent different polymorphic forms. Hybridization conditions are chosen that are sufficiently stringent so that a given probe essentially hybridizes to only one of two alleles. Typically, allele-specific probes can be designed to hybridize to a segment of target DNA such that the polymorphic site aligns with a central position of the probe.

Allele-specific probes can be used in pairs, one member of a pair designed to hybridize to the reference allele of a target sequence and the other member designed to hybridize to the variant allele. Several pairs of probes can be immobilized on the same support for simultaneous analysis of multiple polymorphisms within the same target gene sequence.

The design and use of allele-specific primers for analyzing polymorphisms are described by, e.g., WO 93/22456 and Gibbs, 1989. Briefly, allele-specific primers are designed to hybridize to a site on target DNA overlapping a polymorphism and to prime DNA amplification according to standard PCR protocols only when the primer exhibits perfect complementarity to the particular allelic form. A single-base mismatch prevents DNA amplification and no detectable PCR product is formed. The method works best when the polymorphic site is at the extreme 3'-end of the primer, because this position is most destabilizing to elongation from the primer.

In some embodiments, genomic DNA can be used to detect HTRA1 or ARMS2 polymorphisms. Genomic DNA is typically extracted from a sample, such as a peripheral blood sample or a tissue sample. Standard methods can be used to extract genomic DNA from a sample, such as phenol extraction. In some cases, genomic DNA can be extracted using a commercially available kit (e.g., from Qiagen, Chatsworth, Calif.; Promega, Madison, Wis.; or Gentra Systems, Minneapolis, Minn.).

Other methods for detecting polymorphisms can involve amplifying a nucleic acid from a sample obtained from a subject (e.g., amplifying the segments of the HTRA1 gene of an individual using HTRA1-specific primers) and analyzing the amplified gene. This can be accomplished by standard polymerase chain reaction (PCR & RT-PCR) protocols or other methods known in the art. The amplifying can result in the generation of HTRA1 or ARMS2 allele-specific oligonucleotides, which span the single nucleotide polymorphic sites in the HTRA1 or ARMS2 genes. The HTRA1 or ARMS2 specific primer sequences and HTRA1 and ARMS2 allele-specific oligonucleotides can be derived from the coding (exons) or non-coding (promoter, 5' untranslated, introns or 3' untranslated) regions of the HTRA1 or ARMS2 genes. In one aspect Genomic DNA from all subjects can be isolated from peripheral blood leukocytes with QIAamp DNA Blood Maxi kits (Qiagen, Valencia, CA). DNA samples can be screened for SNPs in CFH (I62V, rs800292; Y402H, rs1061170), HTRA1 (Promoter, rs11200638), ARMS2 (A69S, rs10490924), C3 (R80G, rs2230199), CFB (L9H, rs4151667; R32Q, rs641153), C2 (IVS10, rs547154; E318D, rs9332739), or APOE. Genotyping can be performed byTaqMan assays (Applied Biosystems, Foster City, California) using 10 ng of template DNA in a 5uL reaction. The thermal cycling conditions in the 384-well thermocycler (PTC-225, MJ Research) can consist of an initial hold at 95°C for 10 minutes, followed by 40 cycles of a 15-second 95°C denaturation step and a 1-minute 60°C annealing and extension step. Plates can be read in the 7900HT Fast Real-Time PCR System (Applied Biosystems).

Amplification products generated using PCR can be analyzed by the use of denaturing gradient gel electrophoresis (DGGE). Different alleles can be identified based on sequence-dependent melting properties and electrophoretic migration in solution. See Erlich, ed., PCR Technology, Principles and Applications for DNA Amplification, Chapter 7 (W.H. Freeman and Co, New York, 1992).

Alleles of target sequences can be differentiated using single-strand conformation polymorphism (SSCP) analysis. Different alleles can be identified based on sequence- and structure-dependent electrophoretic migration of single stranded PCR products (Orita et al., 1989). Amplified PCR products can be generated according to standard protocols and heated or otherwise denatured to form single stranded products, which may refold or form secondary structures that are partially dependent on base sequence.

Alleles of target sequences can be differentiated using denaturing high performance liquid chromatography (DHPLC) analysis. Different alleles can be identified based on base differences by alteration in chromatographic migration of single stranded PCR products (Frueh and Noyer-Weidner, 2003). Amplified PCR products can be generated according to standard protocols and heated or otherwise denatured to form single stranded products, which may refold or form secondary structures that are partially dependent on the base sequence.

Direct sequence analysis of polymorphisms can be accomplished using DNA sequencing procedures that are well-known in the art. See Sambrook et al., Molecular Cloning, A Laboratory Manual (2nd Ed., CSHP, New York 1989) and Zyskind et al., Recombinant DNA Laboratory Manual (Acad. Press, 1988).

A wide variety of other methods are known in the art for detecting polymorphisms in a biological sample. See, e.g., Ullman et al. "Methods for single nucleotide polymorphism detection" U.S. Pat. No. 6,632,606; Shi, 2002, "Technologies for individual genotyping: detection of genetic polymorphisms in drug targets and disease genes" Am J Pharmacogenomics 2:197-205; Kwok et al., 2003, "Detection of single nucleotide polymorphisms" Curr Issues Biol. 5:43-60).

Described herein are nucleic acids, disease polymorphic sites, adjacent to or spanning the HTRA1 or ARMS2 polymorphic sites. The nucleic acids can be used as probes or primers (including Invader, Molecular Beacon and other fluorescence resonance energy transfer (FRET) type probes) for detecting HTRA1 or ARMS2 polymorphisms.

Also described herein are vectors comprising a nucleotide sequence that encodes a full length HTRA1 or ARMS2 polypeptide. The vector can also comprise a nucleotide sequence that encodes a sub-domain of the HTRA1 polypeptide. Therefore, the HTRA1 or ARMS2 polypeptides can comprise the amino acid sequence found at NCBI Protein accession numbers NP_002766 or NP_001093137, or a variant thereof (e.g., a risk variant or a protective variant) and may be a full-length form or a truncated form. The nucleic acid may be DNA or RNA and may be single-stranded or double-stranded.

Some nucleic acids can encode full-length, variant forms of HTRA1 or ARMS2 polypeptides. The variant HTRA1 or ARMS2 polypeptides can differ from NP_002766.1 or NP_001093137.1 at an amino acid encoded by a codon including one of any non-synonymous polymorphic position known in the HTRA1 or ARMS2 genes. In one aspect, the variant HTRA1 or ARMS2 polypeptides differ from NP_002766.1 or NP_001093137.1 at an amino acid encoded by a codon including one of the non-synonymous polymorphic positions described herein. It is understood that variant HTRA1 or ARMS2 genes can be generated that encode variant HTRA1 or ARMS2 polypeptides that have alternate amino acids at multiple polymorphic sites in the HTRA1 or ARMS2 genes.

Expression vectors for production of recombinant proteins and peptides are well known in the art (see Ausubel et al., 2004, Current Protocols In Molecular Biology, Greene Publishing and Wiley-Interscience, New York). Such expression vectors can include the nucleic acid sequence encoding the HTRA1 or ARMS2 polypeptides linked to regulatory elements, such as a promoter, which drives transcription of the DNA and is adapted for expression in prokaryotic (e.g., E. coli) and eukaryotic (e.g., yeast, insect or mammalian cells) hosts. A variant HTRA1 or ARMS2 polypeptide can be expressed in an expression vector in which a variant HTRA1 or ARMS2 gene is operably linked to a promoter. The promoter can be a eukaryotic promoter for expression in a mammalian cell. The transcription regulatory sequences can comprise a heterologous promoter and optionally an enhancer, which is recognized by the host cell. Commercially available expression vectors can be used. Expression vectors can include host-recognized replication systems, amplifiable genes, selectable markers, host sequences useful for insertion into the host genome, and the like. For example, and not to be limiting, an HTRA1-S328A plasmid can be synthesized by PCR using oligonucleotides as template to produce human HtrA1 amino acid sequence using codons optimized for expression in *Escherichia coli* bacterial cells. The sequence can contain a S328A mutation. The PCR product can contain NdeI and XhoI restrictions sites at the ends which can be used to ligate the PCR product into the pET-21a(+) plasmid.

Also described herein are isolated host cells comprising a vector that encodes a full length HTRA1 or ARMS2 polypeptide. The host cells can also comprise a vector that encodes a sub-domain of the HTRA1 polypeptide. Suitable host cells can include bacteria such as E. coli, yeast, filamentous fungi, insect cells, and mammalian cells, which are typically immortalized, including mouse, hamster, human, and monkey cell lines, and derivatives thereof. Host cells may be able to process the HTRAl or ARMS2 gene product to produce an appropriately processed, mature polypeptide. Such processing may include glycosylation, ubiquitination, disulfide bond formation, and the like.

Expression constructs containing an HTRAl or ARMS2 gene can be introduced into a host cell, depending upon the particular construction and the target host. Appropriate methods and host cells, both procarytic and eukaryotic, are well-known in the art. For example, full-length human HTRAl and sub-domains of HTRAl were expressed in NEB T7 Express lysY cells, and full-length human ARMS2 was also expressed in NEB T7 Express lysY cells. Constructs expressing Arms2 protein can be transformed and expressed using BL21-AI™ One Shot® Chemically Competent E. coli (Invitrogen #C607003). Constructs that produce a protein whose native fold contains disulfide bonds, including all HtrAl constructs whose nucleotide sequence encodes the IGFBP and/or Kazal subdomains, can be expressed using either NEB SHuffle Express competent E. coli (New England BioLab #C3028H) or NEB SHuffle T7 Express competent E. coli (New England BioLabs #C3026H) for constructs driving protein expression through the T7 promoter).

Also described herein are adenoviral vectors comprising a nucleotide sequence that encodes a full length HTRAl or ARMS2 polypeptides, including variants. In one aspect, described herein are infectious adenoviral particles that encode a full length or variant HTRAl or ARMS2 polypeptides. Disclosed is also an isolated host cell containing therein an adenoviral particle that encodes a full length or variant HTRAl or ARMS2 polypeptide. Suitable host cells can include mammalian cells, such as mouse, hamster, human, and monkey cell lines, and derivatives thereof. Host cells may be able to process the HTRAl or ARMS2 gene product to produce an appropriately processed, mature polypeptide. Such processing may include glycosylation, ubiquitination, disulfide bond formation, and the like. The adenoviral construct can allow for the expression of HTRAl or ARMS2 polypeptides in mammalian systems. In one aspect, a mouse can be infected with the adenoviral particle and HTRAl or ARMS2 polypeptides or variants thereof can be expressed in order to drive phenotypes associated with Vascular Associated Maculopathy, or a symptom thereof.

Also described herein are purified or isolated proteins comprising the amino acid sequence of the full length HTRA1 or ARMS2 polypeptide. In one aspect, the purified or isolated protein can comprise the amino acid sequence of an HTRA1 polypeptide sub-domain. A protein assay can be carried out to identify the proteins and to characterize polymorphisms in a subject's HTRA1 or ARMS2 genes. Methods that can be adapted for detection of the HTRA1 or ARMS2 proteins are well known. These methods include analytical biochemical methods such as electrophoresis (including capillary electrophoresis and two-dimensional electrophoresis), chromatographic methods such as high performance liquid chromatography (HPLC), thin layer chromatography (TLC), hyperdiffusion chromatography, mass spectrometry, and various immunological methods such as fluid or gel precipitin reactions, immunodiffusion (single or double), immunoelectrophoresis, radioimmnunoassay (RIA), enzyme-linked immunosorbent assays (ELISAs), immunofluorescent assays, western blotting and others. For example, and not to be limiting, a number of well established immunological binding assay formats are known (see, e.g., Harlow, E.; Lane, D. Antibodies: a laboratory manual. Cold Spring Harbor, N.Y.: Cold Spring Harbor Laboratory; 1988; and Ausubel et al., (2004) Current Protocols in Molecular Biology, John Wiley & Sons, New York N.Y. The assay can be competitive or non-competitive. Typically, immunological binding assays (or immunoassays) utilize a "capture agent" to specifically bind to and, often, immobilize the analyte. In one aspect, the capture agent can be a moiety that specifically binds to a variant HTRA1 or ARMS2 polypeptide or subsequence. The bound protein may be detected using, for example, a detectably labeled anti-HTRAl or ARMS2 antibody. In one aspect, at least one of the antibodies is specific for a variant form of a HTRA1 or ARMS2 polypeptide. In one aspect, the variant polypeptide can be detected using an immunoblot (Western blot) format.

Also described herein are purified polyclonal antibodies or fragments thereof that bind an HTRA1 or ARMS2 polypeptide. Also described herein are isolated antibodies or fragments thereof that bind an HTRA1 or ARMS2 polypeptide.

Described herein are antibodies that bind to different epitopes of HTRA1 including, but not limited to antibodies that bind to the IGFBP (Insulin-like Growth Factor Binding Protein domain), Kazal, Linker-protease, Protease-linker, or the PDZ (Post synaptic density protein (**P**SD95), Drosophila disc large tumor suppressor (**D**lg1), and zonula occludens-1 protein (**Z**o-1)) domains of HTRA1.

For example, described herein are antibodies that bind to different amino acids 36-49, 96-106, 119-129, 136-148, 155-168, 367-379, 419-431 of HTRA1 (See NCBI Gene Accession No. 5654).

Also described herein are antibodies that hybridize ARMS2 including, but not limited to antibodies that bind to amino acids 42-58 of ARMS2 (See NCBI Gene Accession No. 387715).

Also described herein are antibodies that hybridize to one or more of SEQ. ID.Nos. 1, 2, 3, 4, 5, 6, 7, or 8.

The antibodies described herein can recognize and hybridize to a reference HTRA1 or ARMS2 polypeptide or a variant HTRA1 or ARMS2 polypeptide, in which one or more non-synonymous single nucleotide polymorphisms (SNPs) are present in the HTRA1 or ARMS2 coding region. In one aspect, the antibodies can specifically hybridize to variant HTRA1 or ARMS2 polypeptides or fragments thereof, but not HTRA1 or ARMS2 polypeptides without a variation at the polymorphic site. The antibodies can be polyclonal, and can be made according to standard protocols. Antibodies can be made by injecting a suitable animal with a wild type or variant HTRA1 or ARMS2 polypeptide, or fragment thereof, or synthetic peptide fragments thereof.

Also described herein are methods of detecting HTRA1 or ARMS2 in a subject comprising detecting HTRA1 or ARMS2 levels using an antibody that specifically hybridizes to HTRA1 or ARMS2. Methods to identify antibodies that specifically hybridizes to a polypeptide are well-known in the art. For methods, including antibody screening and subtraction methods; see Harlow & Lane, Antibodies, A Laboratory Manual, Cold Spring Harbor Press, New York (1988); Current Protocols in Immunology (J. E. Coligan et al., eds., 1999, including supplements through 2005); Goding, Monoclonal Antibodies, Principles and Practice (2d ed.) Academic Press, New York (1986); Burioni et al., 1998, "A new subtraction technique for molecular cloning of rare antiviral antibody specificities from phage display libraries" Res Virol. 149(5):327-30; Ames et al., 1994, Isolation of neutralizing anti-C5a monoclonal antibodies from a filamentous phage monovalent Fab display library. J Immunol. 152(9):4572-81; Shinohara et al., 2002, Isolation of monoclonal antibodies recognizing rare and dominant epitopes in plant vascular cell walls by phage display subtraction. J Immunol Methods 264(1-2):187-94. Immunization or screening can be directed against a full-length protein or, alternatively (and often more conveniently), against a peptide or polypeptide fragment comprising an epitope known to differ between the variant and wild-type forms. Polyclonal antibodies specific for HTRA1 or ARMS2 polypeptides can be useful in diagnostic assays for detection of the variant forms of HTRA1 or ARMS2, or as an active ingredient in a pharmaceutical composition.

Also described herein are animal models comprising a transgenic non-human animal that overexpresses human HTRA1. In one aspect, described herein are transgenic mice that overexpress human HTRA1 in mouse RPE. Also described herein are animal models that comprise an HTRA1 or ARMS2 knockout, wherein the animal is a mouse. The transgenic non-human animal can have one or both of alleles of the endogenous HTRA1 or ARMS2 gene inactivated. In a further aspect, one or more cells of the animal can comprise a nucleic acid encoding a recombinant HTRA1 polypeptide operably linked to a RPE-specific human vitelliform macular dystrophy 2 promoter. Expression of an exogenous variant HTRA1 or ARMS2 gene can be achieved by operably linking the gene to a promoter and optionally an enhancer and then microinjecting the construct into a zygote following standard protocols. See Hogan et al., "Manipulating the Mouse Embryo, A Laboratory Manual," Cold Spring Harbor Laboratory. The endogenous HTRA1 or ARMS2 genes can be inactivated by methods known in the art (Capecchi, 1989). HTRA1 or ARMS2 deficient mice are available for the introduction of exogenous human variant HTRA1 or ARMS2. Transgenic animals expressing human or non-human variant HTRA1 or ARMS2 polypeptides can provide useful drug screening systems and can serve as models of Vascular Associated Maculopathy, or a symptom thereof, and other related diseases. Transgenic animals may also be used for production of recombinant HTRA1 or ARMS2 proteins described herein (see, e.g. U.S. Pat. Nos. 6,066,725; 6,013,857; 5,994,616; and 5,959,171; Lillico et al., 2005; Houdebine, 2000).

Also described herein are methods of screening for polymorphic sites in other genes that are in linkage disequilibrium with a risk, protective, or otherwise informative SNP or genetic marker described herein, including but not limited to the polymorphic sites in the HTRA1 or ARMS2 genes. These methods can involve identifying a polymorphic site in a gene that is in linkage disequilibrium with a polymorphic site in the HTRA1 or ARMS2 gene, wherein the polymorphic form of the polymorphic site in the HTRA1 or ARMS2 gene is associated with Vascular Associated Maculopathy, or a symptom thereof, (e.g., increased or decreased risk), and determining haplotypes in a population of individuals to indicate whether the linked polymorphic site has a polymorphic form in linkage disequilibrium with the polymorphic form of the HTRA1 or ARMS2 gene that correlates with the Vascular Associated Maculopathy phenotype.

Polymorphisms in the HTRA1 or ARMS2 genes, such as those described herein, can be used to establish physical linkage between a genetic locus associated with a trait of interest and polymorphic markers that are not associated with the trait, but are in physical proximity with the genetic locus responsible for the trait and co-segregate with it. Mapping a genetic locus associated with a trait of interest facilitates cloning the gene(s) responsible for the trait following procedures that are well-known in the art.

In one aspect, described herein are biological compounds, in particular, proteins, peptides, or nucleic acids, that are differentially present in samples from subjects with Vascular Associated Maculopathy, or a symptom thereof, as compared to age-matched control subjects (individuals without the disease). In another aspect, described herein are biological conditions, including, but not limited to, preeclampsia, hypertension, stroke, or myocardial infarction, that can be differentially present in subjects with Vascular Associated Maculopathy, or a symptom thereof, as compared to age-matched control subjects (individuals without the disease). These proteins or conditions can therefore be associated with Vascular Associated Maculopathy, or a symptom thereof, and termed Vascular Associated Maculopathy-associated biomarkers or biomarkers. In another aspect, these proteins or conditions can be associated with a symptom of Vascular Associated Maculopathy, such as small-vessel vascular disease and termed small-vessel vascular disease associated biomarkers. In yet another aspect, these proteins or conditions can be associated with the presence or risk of developing aberrant choriocapillaris lobules and termed aberrant choriocapillaris lobules-associated biomarkers. These biomarkers can be present at different levels in individuals with Vascular Associated Maculopathy, or a symptom thereof, as compared to individuals without the disease. These biomarkers can be present in individuals with Vascular Associated Maculopathy, or a symptom thereof, at either elevated or reduced levels compared to healthy individuals. Exemplary biomarkers shown to be present in individuals with Vascular Associated Maculopathy, or a symptom thereof, at different levels compared to age-matched control individuals are HTRA1 and/or ARMS2. Therefore, described herein are methods of determining HTRA1 and/or ARMS2 expression levels in a subject. In practicing the methods described herein, biomarkers can be obtained in a sample, preferably a fluid sample, of the individual. The biomarkers are preferentially obtained in a sample of the individual's saliva, cheek scrapings, biopsies of retina, kidney or liver or other organs or tissues; skin biopsies; amniotic fluid or CNS samples; and the like.

As used herein, the term "biomarker" can refer to a protein found at different levels in a sample from a subject with Vascular Associated Maculopathy, or a symptom thereof, compared to an age-matched control subject. The term "biomarker" can also refer to nucleic acid sequences, for example DNA or RNA sequences, such as the HTRA1 or ARMS2 nucleic acid sequences described herein.

The biomarkers described herein can be in any form that provides information regarding presence or absence of a variant or SNP disclosed herein. For example, a disclosed biomarker can be, but is not limited to, a nucleic acid molecule, for example a DNA or RNA molecule, a polypeptide, or an antibody.

The term "level" refers to the amount of a biomarker in a sample obtained from an individual. The amount of the biomarker can be determined by any method known in the art and will depend in part on the nature of the biomarker (e.g., electrophoresis, including capillary electrophoresis, 1- and 2-dimensional electrophoresis, 2-dimensional difference gel electrophoresis DIGE followed by MALDI-ToF mass spectroscopy, chromatographic methods such as high performance liquid chromatography (HPLC), thin layer chromatography (TLC), hyperdiffusion chromatography, mass spectrometry (MS), various immunological methods such as fluid or gel precipitin reactions, single or double immunodiffusion, immunoelectrophoresis, radioimmunoassay (RIA), enzyme-linked immunosorbant assays (ELISA), immunofluorescent assays, Western blotting and others, and enzyme- or function-based activity assays. It is understood that the amount of the biomarker need not be determined in absolute terms, but can be determined in relative terms. For example, the amount of the biomarker may be expressed by its concentration in a sample, by the concentration of an antibody that binds to the biomarker, or by the functional activity (i.e., binding or enzymatic activity) of the biomarker.

The level(s) of a biomarker(s) can be determined as described above for a single biomarker or for a "set" of biomarkers. A set of biomarkers refers to a group of more than one biomarkers that have been grouped together, for example and not for limitation, by a shared property such as their presence at elevated levels in Vascular Associated Maculopathy patients compared to controls, by their presence at reduced levels in Vascular Associated Maculopathy patients compared to controls, by their ratio or difference in levels between Vascular Associated Maculopathy patients and controls (e.g., difference between 1.25- and 2-fold, difference between 2- and 3-fold, difference between 3- and 5-fold, and difference of at least 5-fold), or by function.

The term "difference" as it relates to the level of a biomarker disclosed herein refers to a difference that is statistically different. A difference is statistically different, for example and not to be limiting, if the expectation is <0.05, the p value determined using the Student's t-test is <0.05, or if the p value determined using the Student's t-test is <0.1. The difference in level of a biomarker between an individual with Vascular Associated Maculopathy, or a symptom thereof, and a control individual or population can be, for example and not to be limiting, at least 10% different (1.10 fold), at least 25% different (1.25-fold), at least 50% different (1.5-fold), at least 100% different (2-fold), at least 200% different (3-fold), at least 400% different (5-fold), at least 10-fold different, at least 20-fold different, at least 50-fold different, at least 100-fold different, at least 150-fold, or at least 200-fold different.

Vascular Associated Maculopathy biomarkers can be detected in any of a number of methods including immunological assays (e.g., ELISA), separation-based methods (e.g., gel electrophoresis), protein-based methods (e.g., mass spectroscopy), function-based methods (e.g., enzymatic or binding activity), or the like. In one aspect, determining HTRA1 or ARMS2 expression levels comprises using an antibody that specifically binds to HTRA1 or ARMS2. Other methods are known to those of skill in the art guided by this specification. The particular method for determining the levels will depend, in part, on the identity and nature of the biomarker protein. In one aspect, normal or baseline values (or ranges) can be established for biomarker expression levels. Normal levels can be determined for any particular population, subpopulation, or group of organisms according to standard methods well known to those of skill in the art. Generally, baseline (normal) levels of biomarkers can be determined by quantifying the amount of biomarker in biological samples (e.g., fluids, cells or tissues) obtained from normal (healthy) subjects. Application of standard statistical methods used in medicine permits determination of baseline levels of expression, as well as significant deviations from such baseline levels. It will be appreciated that the assay methods do not necessarily require measurement of absolute values of biomarker, unless it is so desired, because relative values can be sufficient for many applications of the methods described herein. Where quantification is desirable, described herein are reagents such that virtually any known method for quantifying gene products can be used.

In one aspect, the method for separating and determining the levels of the one or more biomarkers described herein, including, but not limited to, HTRA1 and ARMS2, can involve obtaining a biological sample from a individual, separating and determining the levels of the biomarkers by 2-dimensional difference gel electrophoresis (DIGE), and identifying the biomarkers by MALDI-ToF mass spectroscopy. In another aspect, the biomarkers separated by DIGE can be identified by comparison to a known separation pattern of biomarkers using DIGE.

In a further aspect, the method for separating, detecting, and determining the levels of the biomarkers described herein, including, but not limited to, HTRA1 and ARMS2, involves obtaining a biological sample from an individual, separating the proteins by chromatography, if appropriate, capturing the proteins on a biochip (i.e., an adsorbent of a SELDI probe), and detecting and determining the levels of the captured biomarkers by mass spectrometry (i.e., ToF-MS).

A biochip can comprise a solid substrate and can have a generally planar surface to which a capture reagent (also called an adsorbent or affinity reagent) can be attached. Frequently, the surface of a biochip comprises a plurality of addressable locations, each of which can have the capture reagent bound thereto.

A "protein biochip" as used herein refers to a biochip adapted for the capture of proteins. Protein biochips are known to those of skill in the art, including, but not limited to, those produced by Ciphergen Biosystems, Inc. (Fremont, Calif.), Packard BioScience Company (Meriden, Conn.), Zyomyx (Hayward, Calif.), Phylos (Lexington, Mass.) and Biacore (Uppsala, Sweden). Examples of such protein biochips are described in, e.g., U.S. Pat. Nos. 6,225,047, 6,329,209 and 5,242,828, and PCT Publication Nos. WO 99/51773 and WO 00/56934.

In one aspect, the biomarkers disclosed herein can be detected by mass spectrometry (MS) methods. Examples of mass spectrometers include, but are not limited to, time-of-flight (ToF), magnetic sector, quadrupole filter, ion trap, ion cyclotron resonance, electrostatic sector analyzer, and hybrids of these.

In one aspect, the mass spectrometer can be a laser desorption/ionization mass spectrometer. In laser desorption/ionization mass spectrometry, the analytes (i.e., proteins) are placed on the surface of a MS probe, which engages a probe interface of the mass spectrometer and presents an analyte to ionizing energy for ionization and introduction into the mass spectrometer. A laser desorption mass spectrometer employs laser energy, typically from an ultraviolet laser, but also from an infrared laser, which desorbs the analytes from the surface, and volatilizes and ionizes the analytes, thereby making them available to the ion optics of the mass spectrometer.

A mass spectrometry method for use in the methods described herein can be "Surface Enhanced Laser Desorption and Ionization" or "SELDI," as described, for example, in U.S. Pat. Nos. 5,719,060 and 6,225,047. SELDI refers to a method of desorption/ionization gas phase ion spectrometry in which the analyte (i.e., at least two of the biomarkers) is captured on the surface of a SELDI MS probe. There are several versions of SELDI, including "affinity capture mass spectrometry," "Surface-Enhanced Affinity Capture" or "SEAC," "Surface-Enhanced Neat Desorption" or "SEND," and "Surface-Enhanced Photolabile Attachment and Release" or "SEPAR".

SEAC involves the use of probes having a material on the probe surface that captures analytes (i.e., proteins) through non-covalent affinity interactions (i.e., adsorption) between the material and the analyte. The material is variously called an "adsorbent," a "capture reagent," an "affinity reagent, or a "binding moiety." Such probes are called "affinity capture probes" having "adsorbent surfaces." The capture reagent can be any material capable of binding an analyte. The capture reagent can be attached directly to the substrate of the selective surface, or the substrate can have a reactive surface that carries a reactive moiety capable of binding the capture reagent, e.g., through a reaction forming a covalent or coordinate covalent bond. Epoxide and carbodiimidizole can be reactive moieties used to covalently bind protein capture reagents, such as antibodies or cellular receptors. Nitriloacetic acid and iminodiacetic acid can be reactive moieties that function as chelating agents to bind metal ions that interact non-covalently with histidine containing peptides. Adsorbents can be generally classified as either chromatographic adsorbents or biospecific adsorbents.

A "chromatographic adsorbent" refers to an adsorbent material typically used in chromatography. Chromatographic adsorbents include, for example, anion and cation exchange materials, metal chelators (e.g., nitriloacetic acid or iminodiacetic acid), immobilized metal chelates, hydrophobic interaction adsorbents, hydrophilic interaction adsorbents, dyes, simple biomolecules (e.g., nucleotides, amino acids, simple sugars and fatty acids) and mixed mode adsorbents (e.g., hydrophobic attraction/electrostatic repulsion adsorbents).

A "biospecific adsorbent" refers to an adsorbent comprising a biomolecule, e.g., a nucleic acid molecule (e.g., an aptamer), a polypeptide, a polysaccharide, a lipid, a steroid or a conjugate of these (e.g., a glycoprotein, a lipoprotein, a glycolipid, or a nucleic acid (e.g., DNA)-protein conjugate). In some aspects, the biospecific adsorbent can be a macromolecular structure, such as a multi-protein complex, a biological membrane or a virus. Examples of biospecific adsorbents include, but are not limited to, antibodies, receptor proteins and nucleic acids. Biospecific adsorbents can have higher specificity for a target analyte than chromatographic adsorbents. Further examples of adsorbents for use in SELDI can be found in U.S. Pat. No. 6,225,047. A "bioselective adsorbent" refers to an adsorbent that binds to an analyte with an affinity typically of at least 10⁻⁸ M.

Protein biochips produced by Ciphergen Biosystems, Inc. comprise surfaces having chromatographic or biospecific adsorbents attached thereto at addressable locations. Ciphergen PROTEINCHIP arrays include NP20 (hydrophilic); H4 and HSO (hydrophobic); SAX2, Q10 and LSAX30 (anion exchange); WCX2, CM10 and LWCX30 (cation exchange); IMAC3, IMAC30 and IMAC40 (metal chelate); and PS10, PS20 (reactive surface with carboimidizole, expoxide) and PG20 (protein G coupled through carboimidizole). Hydrophobic PROTEINCHIP arrays have isopropyl or nonylphenoxy-poly(ethylene glycol)methacrylate functionalities. Anion exchange PROTEINCHIP arrays have quaternary ammonium functionalities. Cation exchange PROTEINCHIP arrays have carboxylate functionalities. Immobilized metal chelate PROTEINCHIP arrays have nitriloacetic acid functionalities that adsorb transition metal ions, such as copper, nickel, zinc, and gallium, by chelation. Preactivated PROTEINCHIP arrays have carboimidizole or epoxide functional groups that can react with groups on proteins for covalent binding.

Protein biochips are further described in U.S. Pat. Nos. 6,579,719 and 6,555,813, PCT Publication Nos. WO 00/66265 and WO 03/040700, U.S. Patent Application Nos. US 20030032043 A1, US 20030218130 A1 and US 20050059086 A1.

In one aspect, a probe with an adsorbent surface can be contacted with the sample for a period of time sufficient to allow proteins present in the sample to bind to the adsorbent. After the incubation period, the substrate can be washed to remove unbound material. Any suitable washing solutions can be used; for example, aqueous solutions can be employed. The extent to which proteins remain bound to the adsorbent can be manipulated by adjusting the stringency of the wash. The elution characteristics of a wash solution can depend, for example, on pH, ionic strength, hydrophobicity, degree of chaotropism, detergent strength, temperature, and the like. Unless the probe has both SEAC and SEND properties (as described herein), an energy absorbing molecule can then applied to the substrate with the bound proteins.

The biomarkers bound to the substrates can be detected in a gas phase ion spectrometer such as a ToF mass spectrometer. The biomarkers can be ionized by an ionization source such as a laser, the generated ions can be collected by an ion optic assembly, and then a mass analyzer can disperse and analyze the passing ions. The detector can then translate information of the detected ions into mass-to-charge ratios. Detection of a biomarker can involve detection of signal intensity. Thus, both the quantity and mass of the biomarker can be determined.

SEND involves the use of probes comprising energy absorbing molecules that are chemically bound to the probe surface ("SEND probe"). The phrase "energy absorbing molecules" (EAM) denotes molecules that are capable of absorbing energy from a laser desorption/ionization source and, thereafter, contribute to desorption and ionization of analyte molecules in contact therewith. The EAM category includes molecules used in MALDI, frequently referred to as "matrix," and is exemplified by cinnamic acid derivatives, sinapinic acid (SPA), cyano-hydroxy-cinnamic acid (CHCA) and dihydroxybenzoic acid, ferulic acid, and hydroxyaceto-phenone derivatives. In one aspect, the EAM can be incorporated into a linear or cross-linked polymer, e.g., a polymethacrylate. For example, the composition can be a co-polymer of α-cyano-4-methacryloyloxycinnamic acid and acrylate. In another aspect, the composition is a co-polymer of α-cyano-4-methacryloyloxycinnamic acid, acrylate and 3-(tri-ethoxy)silyl propyl methacrylate. In another aspect, the composition can be a co-polymer of α-cyano-4-methacryloyloxycinnamic acid and octadecylmethacrylate ("C18 SEND"). SEND is further described in U.S. Pat. No. 6,124,137 and PCT Publication No. WO 03/64594.

SEAC/SEND is a version of SELDI in which both a capture reagent and an EAM can be attached to the sample presenting surface. SEAC/SEND probes can therefore allow the capture of analytes through affinity capture and ionization/desorption without the need to apply an external matrix. The C18 SEND biochip is a version of SEAC/SEND, comprising a C18 moiety which functions as a capture reagent, and a CHCA moiety which functions as an EAM.

SEPAR involves the use of probes having moieties attached to the surface that can covalently bind an analyte, and then release the analyte through breaking a photolabile bond in the moiety after exposure to light, e.g., to laser light (see U.S. Pat. No. 5,719,060). SEPAR and other forms of SELDI can be readily adapted to detecting a biomarker or biomarker profile, pursuant to the methods described herein.

In another MS method, the biomarkers can first be captured on a resin having chromatographic properties that bind biomarkers. This can include a variety of methods. For example, the biomarkers can be captured on a cation exchange resin, such as CM CERAMIC HYPERD F resin, the resin can be washed, biomarkers can be eluted and the eluted biomarkers can be detected by MALDI. Alternatively, this method can be preceded by fractionating the sample on an anion exchange resin, such as Q CERAMIC HYPERD F resin, before application to the cation exchange resin. In another aspect, the sample on an anion exchange resin can be fractionated and detected by MALDI directly. In yet another aspect, the biomarkers can be captured on an immuno-chromatographic resin comprising antibodies that bind particular biomarkers, resin can be washed to remove unbound material, the biomarkers can be eluted from the resin and the eluted biomarkers can be detected by MALDI or by SELDI.

Analysis of analytes by ToF-MS generates a time-of-flight spectrum. The time-of-flight spectrum ultimately analyzed typically does not represent the signal from a single pulse of ionizing energy against a sample, but rather the sum of signals from a number of pulses. This reduces noise and increases dynamic range. This time-of-flight data can then be subject to data processing using Ciphergen's PROTEINCHIP software, or any equivalent data processing software. Data processing can include TOF-to-M/Z transformation to generate a mass spectrum, baseline subtraction to eliminate instrument offsets and high frequency noise filtering to reduce high frequency noise.

Data generated by desorption and detection of biomarkers can be analyzed with the use of a programmable digital computer. The computer program can analyze the data to indicate the number of biomarkers detected, the strength of the signal, or the determined molecular mass for each biomarker detected. Data analysis can include steps of determining signal strength of a biomarker and removing data deviating from a predetermined statistical distribution. For example, the observed peaks can be normalized, by calculating the height of each peak relative to some reference. The reference can be background noise generated by the instrument and chemicals such as the energy absorbing molecule which can be set at zero in the scale.

The computer can transform the resulting data into various formats for display. The standard spectrum can be displayed, but in one aspect only the peak height and mass-to-charge information can be retained from the spectrum view, thereby yielding a cleaner image and enabling biomarkers with nearly identical molecular weights to be more easily seen. In another aspect, two or more spectra can be compared, conveniently highlighting unique biomarkers and biomarkers that are up- or down-regulated between samples. Using any of these formats, it can readily be determined whether a particular biomarker is present in a sample.

Analysis can involve the identification of peaks in the spectrum that represent signal from an analyte. Peak selection can be done visually, but software is available, for example, as part of Ciphergen's PROTEINCHIP software package, which can automate the detection of peaks. In general, this software functions by identifying signals having a signal-to-noise ratio above a selected threshold and labeling the mass of the peak at the centroid of the peak signal. In one aspect, many spectra can be compared to identify identical peaks present in some selected percentage of the mass spectra. One version of this software clusters all peaks appearing in the various spectra within a defined mass range, and assigns a mass (M/Z) to all the peaks that are near the mid-point of the mass (M/Z) cluster.

Software used to analyze the data can include code that applies an algorithm to the analysis of the signal to determine whether the signal represents a peak in a signal that corresponds to a biomarker described herein. The software also can subject the data regarding observed biomarker peaks to classification tree or ANN analysis, to determine whether a biomarker peak or combination of biomarker peaks is present that indicates the status of the particular clinical parameter under examination. Analysis of the data can be "keyed" to a variety of parameters that are obtained, either directly or indirectly, from the mass spectrometric analysis of the sample. These parameters include, but are not limited to, the presence or absence of at least two peaks, the shape of a peak or group of peaks, the height of at least two peaks, the log of the height of at least two peaks, and other arithmetic manipulations of peak height data.

An example protocol for the detection of biomarkers described herein is as follows. The biological sample to be tested can be obtained a subject, depleted of albumin and IgG or pre-fractionated on an anion exchange chromatographic resin or other chromatographic resin, as appropriate, and then contacted with an affinity capture SELDI probe comprising a cation exchange adsorbant (e.g., CM 10 or WCX2 PROTEINCHIP array from Ciphergen Systems, Inc.), an anion exchange adsorbant (e.g., Q10 PROTEINCHIP array from Ciphergen Systems, Inc.), a hydrophobic exchange adsorbant (e.g., HSO PROTEINCHIP array from Ciphergen Systems, Inc.), or an IMAC adsorbant (e.g., IMAC3 or IMAC30 PROTEINCHIP array from Ciphergen Systems, Inc.). The SELDI probe can be washed with a suitable buffer that retains the biomarkers disclosed herein, while washing away unbound biomolecules. The biomarkers specifically retained on the SELDI probe can then be detected by laser desorption/ionization mass spectrometry.

The biological sample, e.g., serum, plasma or urine, can be depleted of albumin and IgG or subjected to pre-fractionation before binding to a SELDI probe. In one aspect, pre-fractionation can involve contacting the biological sample with an anion exchange chromatographic resin. The bound biomolecules can then be subjected to stepwise pH elution using buffers at various pH. Various fractions containing biomolecules can be collected and subjected to binding to a SELDI probe.

In a further aspect, if analysis of particular proteins and various forms thereof are desired, antibodies which recognize specific proteins can be attached to the surface of a SELDI probe (e.g., pre-activated PS10 or PS20 PROTEINCHIP array from Ciphergen Systems, Inc.). The antibodies capture the target proteins from a biological sample onto the SELDI probe. The captured proteins can then be detected by, for example, laser desorption/ionization mass spectrometry. The antibodies can also capture the target proteins on immobilized support, and the target proteins can be eluted and captured on a SELDI probe and detected as described herein.

Antibodies to target proteins are either commercially available or can be produced by methods known in the art, e.g., by immunizing animals with the target proteins isolated by standard purification techniques or with synthetic peptides of the target proteins.

In some aspects it will be desirable to establish normal or baseline values (or ranges) for biomarker expression levels. Normal levels can be determined for any particular population, subpopulation, or group of organisms according to standard methods well known to those of skill in the art. Generally, baseline (normal) levels of biomarkers are determined by quantifying the amount of biomarker in biological samples (e.g., fluids, cells or tissues) obtained from normal (healthy) subjects. Application of standard statistical methods used in medicine permits determination of baseline levels of expression, as well as significant deviations from such baseline levels.

In carrying out the diagnostic and prognostic methods described herein, it will sometimes be useful to refer to "diagnostic" and "prognostic" values. As used herein, "diagnostic value" refers to a value that is determined for the biomarker gene product detected in a sample which, when compared to a normal (or "baseline") range of the biomarker gene product is indicative of the presence of a disease. "Prognostic value" refers to an amount of the biomarker that is consistent with a particular diagnosis and prognosis for the disease. The amount of the biomarker gene product detected in a sample is compared to the prognostic value for the biomarker such that the relative comparison of the values indicates the presence of disease or the likely outcome of the disease progression. In one aspect, for example, to assess Vascular Associated Maculopathy prognosis, data are collected to obtain a statistically significant correlation of biomarker levels with different symptoms of Vascular Associated. A predetermined range of biomarker levels is established from subjects having known clinical outcomes. A sufficient number of measurements is made to produce a statistically significant value (or range of values) to which a comparison will be made.

It will be appreciated that the assay methods described herein do not necessarily require measurement of absolute values of a biomarker, unless it is so desired, because relative values are sufficient for many applications of the methods described herein. Where quantification is desirable, the presently described methods provide reagents such that virtually any known method for quantifying gene products can be used.

In one aspect, described herein are methods for diagnosing or determining the risk a subject may develop Vascular Associated Maculopathy, or a symptom thereof, by determining levels of at least one Vascular Associated Maculopathy-associated biomarker in a sample from the individual, and comparing the levels of the biomarker in the sample to reference levels of the biomarker characteristic of a control population of individuals without Vascular Associated Maculopathy, where a difference in the levels of the biomarker between the sample from the individual and the control population indicates that the individual has or has an increased risk of having Vascular Associated Maculopathy, or a symptom thereof. A biomarker can be, but is not limited to, HTRA1 or ARMS2 protein. For example, the biomarker can be HTRA1 or ARMS2 protein expressed at elevated levels in individuals with Vascular Associated Maculopathy, or a symptom thereof. In another aspect, the biomarker can be an HTRA1 or ARMS2 nucleic acid, such as DNA or RNA.

Also described herein are methods for assessing the efficacy of a treatment for Vascular Associated Maculopathy, or a symptom thereof, in an individual, by (a) determining levels of at least one biomarker in a sample from the individual either before treatment or at a first time point after treatment with an agent and (b) determining levels of the biomarker in a sample from the individual at a later time point during treatment or after treatment with the agent, where a difference in the levels of the biomarker measured in (b) compared to (a) in which the levels of the biomarkers moves closer to reference levels of the biomarker characteristic of a control population of individuals without Vascular Associated Maculopathy, or a symptom thereof, indicates that the treatment is effective. At least one of the biomarkers can be an HTRA1 or ARMS2 protein.

Furthermore, described herein are methods of determining the efficacy of an agent for (i) treating Vascular Associated Maculopathy; (ii) treating one or more symptoms associated with Vascular Associated Maculopathy; (iii) treating severe maculopathy or last stage maculopathy (iv) resolving aberrant choriocapillaris lobules diagnosed with (i), (ii), (iii) or (iv), comprising: a) determining a number of aberrant choriocapillaris lobules in an eye of the subject before beginning treatment of (1) Vascular Associated Maculopathy; (2) one or more symptoms associated with Vascular Associated Maculopathy; (3) severe maculopathy or last stage maculopathy or (4) resolving aberrant choriocapillaris lobules; b) beginning treatment of (1), (2), (3) or (4), for an interval of time; c) determining a subsequent number of aberrant choriocapillaris lobules in the eye of step a) after the interval of time; and d) comparing the number of aberrant choriocapillaris lobules in the eye of the subject of step c) to the number of aberrant choriocapillaris lobules in the eye of the subject of step a), wherein detecting an increase in the number of aberrant choriocapillaris lobules in the eye of the subject of step c) indicates an agent not effective in (i) treating Vascular Associated Maculopathy; (ii) treating one or more symptoms associated with Vascular Associated Maculopathy; (iii) treating severe maculopathy or last stage maculopathy; or (iv) resolving aberrant choriocapillaris lobules in the subject.

Also described herein are methods of determining the efficacy of an agent for (i) treating Vascular Associated Maculopathy; (ii) treating one or more symptoms associated with Vascular Associated Maculopathy; (iii) treating severe maculopathy or last stage maculopathy (iv) resolving aberrant choriocapillaris lobules diagnosed with (i), (ii), (iii) or (iv), comprising: a) determining a number of aberrant choriocapillaris lobules in an eye of the subject before beginning treatment of (1) Vascular Associated Maculopathy; (2) one or more symptoms associated with Vascular Associated Maculopathy; (3) severe maculopathy or last stage maculopathy or (4) aberrant choriocapillaris lobules; b) beginning treatment of (1), (2), (3) or (4), for an interval of time; c) determining a subsequent number of aberrant choriocapillaris lobules in the eye of step a) after the interval of time; and d) comparing the number of aberrant choriocapillaris lobules in the eye of the subject of step c) to the number of aberrant choriocapillaris lobules in the eye of the subject of step a), wherein detecting no increase in the number of aberrant choriocapillaris lobules in the eye of the subject of step c) indicates an agent is effective in (i) treating Vascular Associated Maculopathy; (ii) treating one or more symptoms associated with Vascular Associated Maculopathy; (iii) treating severe maculopathy or last stage maculopathy; or (iv) resolving aberrant choriocapillaris lobules in the subject.

Additionally, described herein are methods of determining the efficacy of a treatment of (i) treating Vascular Associated Maculopathy; (ii) treating one or more symptoms associated with Vascular Associated Maculopathy; (iii) treating severe maculopathy or last stage maculopathy; or (iv) resolving aberrant choriocapillaris lobules diagnosed with (i), (ii), (iii) or (iv), comprising: a) determining the presence of RPE cells overlying and corresponding to the choriocapillaris lobule in an eye of the subject before beginning treatment of (1) Vascular Associated Maculopathy; (2) one or more symptoms associated with Vascular Associated Maculopathy; (3) severe maculopathy or last stage maculopathy or (4) aberrant choriocapillaris lobules; b) beginning treatment of (1), (2), (3) or (4), for an interval of time; c) determining the regrowth or regeneration of RPE cells overlying and corresponding to the choriocapillaris lobule in the eye of step a) after the interval of time; and d) comparing the regrowth or regeneration of RPE cells overlying and corresponding to the choriocapillaris lobule in the eye of the subject of step c) to the regrowth or regeneration of RPE cells overlying and corresponding to the choriocapillaris lobule in the eye of the subject of step a), wherein detecting no change or a decrease in the regrowth or regeneration of RPE cells overlying and corresponding to the choriocapillaris lobule in the eye of the subject of step c) indicates an agent is effective in (i) treating Vascular Associated Maculopathy; (ii) treating one or more symptoms associated with Vascular Associated Maculopathy; (iii) treating severe maculopathy or last stage maculopathy; or (iv) resolving aberrant choriocapillaris lobules in the subject.

Also described herein are methods of determining the efficacy of a treatment of (i) treating Vascular Associated Maculopathy; (ii) treating one or more symptoms associated with Vascular Associated Maculopathy; (iii) treating severe maculopathy or last stage maculopathy; or (iv) resolving aberrant choriocapillaris lobules diagnosed with (i), (ii), (iii) or (iv), comprising: a) determining the presence of RPE cells overlying and corresponding to the choriocapillaris lobule in an eye of the subject before beginning treatment of (1) Vascular Associated Maculopathy; (2) one or more symptoms associated with Vascular Associated Maculopathy; (3) severe maculopathy or last stage maculopathy or (4) aberrant choriocapillaris lobules; b) beginning treatment of (1), (2), (3) or (4), for an interval of time; c) determining the regrowth or regeneration of RPE cells overlying and corresponding to the choriocapillaris lobule in the eye of step a) after the interval of time; and d) comparing the regrowth or regeneration of RPE cells overlying and corresponding to the choriocapillaris lobule in the eye of the subject of step c) to the regrowth or regeneration of RPE cells overlying and corresponding to the choriocapillaris lobule in the eye of the subject of step a), wherein detecting an increase in the regrowth or regeneration of RPE cells overlying and corresponding to the choriocapillaris lobule in the eye of the subject of step c) indicates an agent is not effective in (i) treating Vascular Associated Maculopathy; (ii) treating one or more symptoms associated with Vascular Associated Maculopathy; (iii) treating severe maculopathy or last stage maculopathy; or (iv) resolving aberrant choriocapillaris lobules in the subject. Regrowth or regeneration of RPE cells is determined by autofluorescent imaging techniques, infrared imaging techniques, optical coherence tomography (OCT), Stratus optical coherence tomography (Stratus OCT), Fourier-domain optical coherence tomography (Fd-OCT), two-photon-excited fluorescence (TPEF) imaging, adaptive optics scanning laser ophthalmoscopy (AOSLO), scanning laser ophthalmoscopy, near-infrared imaging combined with spectral domain optical coherence tomography (SD-OCT), color fundus photography, fundus autofluorescence imaging, red-free imaging, fluorescein angiography, indocyanin green angiography, multifocal electroretinography (ERG) recording, microperimetry, color Doppler optical coherence tomography (CDOCT), visual field assessment, the Heidelberg Spectralis, the Zeiss Cirrus, the Topcon 3D OCT 2000, the Optivue RTVue SD-OCT, the Opko OCT SLO, the NIDEK F-10, or the Optopol SOCT Copernicus HR.

Furthermore, described herein are methods of determining the efficacy of a treatment of (i) treating Vascular Associated Maculopathy; (ii) treating one or more symptoms associated with Vascular Associated Maculopathy; (iii) treating severe maculopathy or last stage maculopathy (iv) resolving aberrant choriocapillaris lobules diagnosed with (i), (ii), (iii) or (iv), comprising: a) determining the perfusion of the choriocapillaris lobules in an eye of the subject before beginning treatment of (1) Vascular Associated Maculopathy; (2) one or more symptoms associated with Vascular Associated Maculopathy; (3) severe maculopathy or last stage maculopathy or (4) aberrant choriocapillaris lobules; b) beginning treatment of (1), (2), (3) or (4), for an interval of time; c) determining perfusion of the choriocapillaris lobules in the eye of step a) after the interval of time; and d) comparing the perfusion of the choriocapillaris lobules in the eye of the subject of step c) to the perfusion of the choriocapillaris lobules in the eye of the subject of step a), wherein detecting no increase in perfusion of the choriocapillaris lobules in the eye of the subject of step c) indicates an agent is not effective in (i) treating Vascular Associated Maculopathy; (ii) treating one or more symptoms associated with Vascular Associated Maculopathy; (iii) treating severe maculopathy or last stage maculopathy; or (iv) resolving aberrant choriocapillaris lobules in the subject.

Also described herein are methods of determining the efficacy of a treatment of (i) treating Vascular Associated Maculopathy; (ii) treating one or more symptoms associated with Vascular Associated Maculopathy; (iii) treating severe maculopathy or last stage maculopathy (iv) resolving aberrant choriocapillaris lobules diagnosed with (i), (ii), (iii) or (iv), comprising: a) determining the perfusion of the choriocapillaris lobules in an eye of the subject before beginning treatment of (1) Vascular Associated Maculopathy; (2) one or more symptoms associated with Vascular Associated Maculopathy; (3) severe maculopathy or last stage maculopathy or (4) aberrant choriocapillaris lobules; b) beginning treatment of (1), (2), (3) or (4), for an interval of time; c) determining perfusion of the choriocapillaris lobules in the eye of step a) after the interval of time; and d) comparing the perfusion of the choriocapillaris lobules in the eye of the subject of step c) to the perfusion of the choriocapillaris lobules in the eye of the subject of step a), wherein detecting an increase in perfusion of the choriocapillaris lobules in the eye of the subject of step c) indicates an agent is effective in (i) treating Vascular Associated Maculopathy; (ii) treating one or more symptoms associated with Vascular Associated Maculopathy; (iii) treating severe maculopathy or last stage maculopathy; or (iv) resolving aberrant choriocapillaris lobules in the subject. The perfusion of the choriocapillaris lobules in the eye are determined by autofluorescent imaging techniques, infrared imaging techniques, optical coherence tomography (OCT), Stratus optical coherence tomography (Stratus OCT), Fourier-domain optical coherence tomography (Fd-OCT), two-photon-excited fluorescence (TPEF) imaging, adaptive optics scanning laser ophthalmoscopy (AOSLO), scanning laser ophthalmoscopy, near-infrared imaging combined with spectral domain optical coherence tomography (SD-OCT), color fundus photography, fundus autofluorescence imaging, red-free imaging, fluorescein angiography, indocyanin green angiography, multifocal electroretinography (ERG) recording, microperimetry, color Doppler optical coherence tomography (CDOCT), visual field assessment, the Heidelberg Spectralis, the Zeiss Cirrus, the Topcon 3D OCT 2000, the Optivue RTVue SD-OCT, the Opko OCT SLO, the NIDEK F-10, or the Optopol SOCT Copernicus HR.

Also disclosed are imaging agents, wherein the agent specifically binds a HTRA1 or ARMS2, or a variant HTRA1 or ARMS2, encoding nucleic acid. For example, disclosed are arrays comprising polynucleotides capable of specifically hybridizing to one or more risk, protective, or netural HTRA1 or ARMS2 SNPs described herein. Also disclosed are imaging agents, wherein the agent is capable of specifically hybridizing to one or more of the one or more risk or protective HTRA1 or ARMS2 SNPs including, but not limited to, rs11200638, rs1049331, or rs2672587 in the HTRA1 gene, rs10490924 or rs3750848 in the ARMS2 gene, rs1061170 in the CFH gene, rs2230199 in the C3 gene, rs800292 SNP in the CFH gene, rs641153 SNP in the CFB gene, or rs4151667 SNP in the CFB gene.

Also described herein are methods of identifying a predisposition for developing aberrant choriocapillaris lobules in a subject comprising detecting one or more HTRA1 or ARMS2 SNPs in the subject, wherein detecting one or more HTRA1 or ARMS2 SNPs in the subject indicates a predisposition for developing aberrant choriocapillaris lobules in the subject. For example, and not to be limiting, detecting one or more of rs11200638 in the HTRA1 gene or rs10490924 in the ARMS2 gene, wherein an A allele is present at the HTRA1 SNP or a T allele is present at the ARMS2 SNP, can indicate the presence or increased risk of developing aberrant choriocapillaris lobules. In a further aspect, the methods can comprise determining in the subject the identity of one or more SNPs in the HTRA1 or ARMS2 genes, including, but not limited to, rs1049331, rs3750848, or rs2672587, wherein the presence of one or more of the SNPs indicates presence or increased risk of developing aberrant choriocapillaris lobules. In yet a further aspect, the methods can comprise detecting a SNP that is in linkage disequilibrium with a risk, protective, or otherwise informative SNP or genetic marker described herein, for example, and not to be limiting, an HTRA1 or ARMS2 SNP, wherein detecting a SNP that is in linkage disequilibrium with an HTRA1 or ARMS2 SNP can indicate the presence or increased risk of developing aberrant choriocapillaris lobules.

It is understood that the methods described herein, of diagnosing and/or determining a subject's predisposition to develop Vascular Associated Maculopathy, or a symptom thereof, comprising determining in the subject the identity of one or more SNPs described herein, can be performed in combination with any of the other methods described herein. Therefore, described herein are methods of diagnosing and/or determining a subject's predisposition to develop Vascular Associated Maculopathy, or a symptom thereof, comprising detecting the presence of aberrant choriocapillaris lobules in an eye of the subject, wherein the presence of aberrant choriocapillaris lobules in the eye of the subject indicates the diagnosis of or increased risk of the subject to develop Vascular Associated Maculopathy, or a symptom thereof,, further comprising determining in the subject the identity of one or more SNPs in the HTRA1 or ARMS2 genes, including, but not limited to, rs11200638 in the HTRA1 gene or rs10490924 in the ARMS2 gene, wherein an A allele at the HTRA1 SNP or a T allele at the ARMS2 SNP indicates presence or increased risk of developing Vascular Associated Maculopathy, or a symptom thereof.

Also described herein are methods of diagnosing and/or determining a subject's predisposition to develop Vascular Associated Maculopathy, or a symptom thereof, comprising detecting the presence of aberrant choriocapillaris lobules in an eye of the subject, wherein the presence of aberrant choriocapillaris lobules in the eye of the subject indicates the diagnosis of or increased risk of developing Vascular Associated Maculopathy, or a symptom thereof, in the subject, further comprising determining in the subject the identity of rs11200638 in the HTRA1 gene or rs10490924 in the ARMS2 gene, wherein an A allele at rs11200638 in the HTRA1 gene or a T allele at rs10490924 in the ARMS2 gene indicates presence of or increased risk of developing Vascular Associated Maculopathy, or a symptom thereof.

Additionally, described herein are methods of diagnosing Vascular Associated Maculopathy, or a symptom thereof, in a subject comprising detecting the presence of aberrant choriocapillaris lobules in an eye of the subject, wherein the presence of aberrant choriocapillaris lobules in the eye of the subject indicates the diagnosis of Vascular Associated Maculopathy, or a symptom thereof, in the subject, further comprising determining in the subject the identity of one or more SNPs in the HTRA1 or ARMS2 genes, including, but not limited to, rs1049331, rs3750848, or rs2672587, wherein the presence of one or more of the SNPs indicates presence of Vascular Associated Maculopathy, or a symptom thereof.

Furthermore, described herein are methods of diagnosing Vascular Associated Maculopathy, or a symptom thereof, in a subject comprising detecting the presence of aberrant choriocapillaris lobules in an eye of the subject, wherein the presence of aberrant choriocapillaris lobules in the eye of the subject indicates the diagnosis of Vascular Associated Maculopathy, or a symptom thereof, in the subject, further comprising determining in the subject the identity of one or more aberrant choriocapillaris lobule-associated SNPs including, but not limited to, an A allele at rs11200638 SNP in the HTRA1 gene, a T allele at the rs10490924 SNP in the ARMS2 gene, a C allele at rs1061170 in the CFH gene, or a G allele at rs2230199 SNP in the C3 gene, wherein the presence of one or more of the aberrant choriocapillaris lobule-associated SNPs indicates presence of Vascular Associated Maculopathy, or a symptom thereof.

Also described herein are methods of diagnosing Vascular Associated Maculopathy, or a symptom thereof, in a subject comprising detecting the presence of aberrant choriocapillaris lobules in an eye of the subject, wherein the presence of aberrant choriocapillaris lobules in the eye of the subject indicates the presence of Vascular Associated Maculopathy, or a symptom thereof, in the subject, the method further comprising determining in the subject the identity of a SNP that is in linkage disequilibrium with a risk, protective, or otherwise informative SNP or genetic marker described herein, for example, and not to be limiting, an HTRA1 or ARMS2 SNP, wherein detecting a SNP that is in linkage disequilibrium with an HTRA1 or ARMS2 SNP indicates increased risk of Vascular Associated Maculopathy, or a symptom thereof.

Also described herein are methods of diagnosing Vascular Associated Maculopathy, or a symptom thereof, in a subject comprising detecting the presence of aberrant choriocapillaris lobules in an eye of the subject, wherein the presence of aberrant choriocapillaris lobules in the eye of the subject indicates the presence of Vascular Associated Maculopathy, or a symptom thereof, in the subject, the method further comprising determining in the subject the identity of a SNP that is in linkage disequilibrium with a risk, protective, or otherwise informative SNP or genetic marker described herein, for example, and not to be limiting, an HTRA1 or ARMS2 SNP, wherein detecting a SNP that is in linkage disequilibrium with an HTRA1 or ARMS2 SNP indicates the presence of Vascular Associated Maculopathy, or a symptom thereof.

Furthermore, described herein are methods of diagnosing Vascular Associated Maculopathy, or a symptom thereof, in a subject comprising detecting the presence of aberrant choriocapillaris lobules in an eye of the subject, wherein the presence of aberrant choriocapillaris lobules in the eye of the subject indicates the presence of Vascular Associated Maculopathy, or a symptom thereof, in the subject, further comprising determining in the subject the identity of one or more aberrant choriocapillaris lobule-associated SNPs including, but not limited to, an A allele at rs800292 SNP in the CFH gene, an A allele at rs641153 SNP in the CFB gene, or an A allele at rs4151667 SNP in the CFB gene, wherein the presence of one or more of the SNPs indicates decreased risk of developing Vascular Associated Maculopathy, or a symptom thereof.

Also described herein are methods of methods of diagnosing Vascular Associated Maculopathy, or a symptom thereof, in a subject comprising detecting the presence of aberrant choriocapillaris lobules in an eye of the subject, wherein the presence of aberrant choriocapillaris lobules in he eye of the subject indicates the presence of Vascular Associated Maculopathy, or a symptom thereof, in the subject, further comprising determining in the subject the identity of a G allele at the rs11200638 SNP in the HTRA1 gene, wherein a G allele at the rs11200638 SNP in the HTRA1 gene indicates decreased risk of developing Vascular Associated Maculopathy, or a symptom thereof.

### . METHODS OF TREATING VASCULAR ASSOCIATED MACULOPATHY, OR A SYMPTOM HEREOF

Described herein are methods of treating Vascular Associated Maculopathy, or a symptom thereof, in a subject, wherein aberrant choriocapillaris lobules are present in the macula of an eye of the subject, comprising administering to a subject a therapeutically effective amount of one or more agents that inhibit decreased perfusion of and/or closure of choriocapillaris lobules in the macula of the eye of the subject. In one aspect, the symptom of Vascular Associated Maculopathy can appear in an eye of the subject. Therefore, also described herein are methods of treating a symptom of Vascular Associated Maculopathy in an eye of a subject, wherein aberrant choriocapillaris lobules are present in an eye of the subject, comprising administering to a subject a therapeutically effective amount of one or more agents that inhibit closure of choriocapillaris lobules in the eye of the subject.

Also described herein are methods of treating Vascular Associated Maculopathy, treating one or more symptoms associated with Vascular Associated Maculopathy; treating severe maculopathy, treating late stage maculopathy or resolving aberrant choriocapillaris lobules in a subject.

Further described herein are methods of treating Vascular Associated Maculopathy, treating one or more symptoms associated with Vascular Associated Maculopathy; treating severe maculopathy, treating late stage maculopathy or resolving aberrant choriocapillaris lobules in a subject comprising administering an effective amount of one or more of the disclosed compositions, therapeutic agents, active agents, or functional nucleic acids to the subject.

As used herein, "treating" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. In various aspects, the term covers any treatment of a subject, including a mammal (e.g., a human), and includes: (i) preventing the disease from occurring in a subject that can be predisposed to the disease but has not yet been diagnosed as having it; (ii) inhibiting the disease, i.e., arresting its development; or (iii) relieving the disease, i.e., causing regression of the disease.

The terms "administering" and "administration" refer to any method of providing a pharmaceutical preparation to a subject. Such methods are well known to those skilled in the art and include, but are not limited to, oral administration, sublingual administration, trans-buccal mucosa administration, transdermal administration, administration by inhalation, nasal administration, topical administration, intravaginal administration, ophthalmic administration, intraaural administration, intracerebral administration, intrathecal administration, rectal administration, intraperitoneal administration, and parenteral administration, including injectable such as intravenous administration, intra-arterial administration, intramuscular administration, intradermal administration, and subcutaneous administration. Ophthalmic administration can include topical administration, subconjunctival administration, sub-Tenon's administration, epibulbar administration, retrobulbar administration, intra-orbital administration, and intraocular administration, which includes intra-vitreal administration. Administration can be continuous or intermittent. In various aspects, a preparation can be administered therapeutically; that is, administered to treat an existing disease or condition. In further various aspects, a preparation can be administered prophylactically; that is, administered for prevention of a disease or condition.

As used herein, the term "therapeutically effective amount" refers to an amount that is sufficient to achieve the desired result or to have an effect on an undesired condition. For example, a "therapeutically effective amount" can refer to an amount that is sufficient to achieve the desired result, such as inhibiting closure of the choriocapillaris lobules or increasing perfusion in choriocapillaris lobules.

Also described herein are methods of modulating the expression or activity of HTRA1 or ARMS2 comprising administering to a subject a therapeutically effective amount of one or more of an antisense molecule, an siRNA, a peptide, or a small molecule. For example, modulating can mean either increasing or decreasing the expression or activity of HTRA1 or ARMS2. In the methods described herein, inhibiting HTRA1 or ARMS2 transcription, or inhibiting translation of the HTRA1 or ARMS2 gene product can modulate the expression or activity of HTRA1 or ARMS2. Similarly, the activity of an HTRA1 or ARMS2 gene product (for example, an mRNA, a polypeptide or a protein) can be inhibited, either directly or indirectly. Modulation in expression or activity does not have to be complete. For example, expression or activity can be modulated by about 10%, 20%, 30%, 40%, 50%, 60%, 70%, 80%, 90%, 95%, 99%, 100% or any percentage in between as compared to a control cell wherein the expression or activity of HTRA1 or ARMS2 has not been modulated.

By "modulate", "modulating" or "modulated" is meant to alter, by increasing or decreasing.

As used herein, a "modulator" can mean a composition that can either increase or decrease the expression or activity of a gene or gene product such as a peptide. Modulation in expression or activity does not have to be complete. For example, expression or activity can be modulated by about 10%, 20%, 30%, 40%, 50%, 60%, 70%, 80%, 90%, 95%, 99%, 100% or any percentage in between as compared to a control cell wherein the expression or activity of a gene or gene product has not been modulated by a composition. For example, a "candidate modulator" can be an active agent or a therapeutic agent.

The term "active agent" or "therapeutic agent" is defined as an agent, such as a drug, chemotherapeutic agent, chemical compound, etc. For example, and not to be limiting, an active agent or a therapeutic agent can be a naturally occurring molecule or may be a synthetic compound, including, for example and not to be limiting, a small molecule (e.g., a molecule having a molecular weight <1000), a peptide, a protein, an antibody, or a nucleic acid, such as an siRNA or an antisense molecule. An active or therapeutic agent can be used individually or in combination with any other active or therapeutic agent.

Described herein are methods of treating Vascular Associated Maculopathy, treating one or more symptoms associated with Vascular Associated Maculopathy; treating severe maculopathy, treating late stage maculopathy or resolving aberrant choriocapillaris lobules in a subject comprising administering an effective amount of one or more agents that inhibit decreased perfusion of and/or closure of choriocapillaris lobules, inhibit closure (anatomical or functional) or occlusion of choroidal arterioles or choriocapillaris capillaries, increase perfusion in choroidal arterioles and choriocapillaris capillaries, modulate the expression of HTRA1, modulate the expression of ARMS2, modulate the activity of HTRA1, modulate the activity of ARMS2, modulates HTRA1 or ARMS2 transcription, modulates translation of the HTRA1 or ARMS2 gene product, modulates the expression or activity of HTRA1 or ARMS2, modulates elastase activity, or modulates the elastase activity of HTRA1, thereby treating Vascular Associated Maculopathy, treating one or more symptoms associated with Vascular Associated Maculopathy; treating severe maculopathy, treating late stage maculopathy or resolving aberrant choriocapillaris lobules in the subject.

The one or more agents that inhibit decreased perfusion of and/or closure of choriocapillaris lobules, inhibit closure (anatomical or functional) or occlusion of choroidal arterioles or choriocapillaris capillaries, increase perfusion in choroidal arterioles and choriocapillaris capillaries, modulate the expression of HTRA1, modulate the expression of ARMS2, modulate the activity of HTRA1, modulate the activity of ARMS2, modulates HTRA1 or ARMS2 transcription, modulates translation of the HTRA1 or ARMS2 gene product, modulates the expression or activity of HTRA1 or ARMS2, modulates elastase activity, or modulates the elastase activity of HTRA1 include, but are not limited to aspirin, anti-inflammatory medications, cholesterol-lowering medications, anti-hyperglycemic medications, vasodilators, vasopressors, diuretics, anticoagulants, thrombolytic medications, anti-vascular endothelial growth factor medications, anti-post-ischemic injury medications, anti-hypertensive medications, abnormal clotting therapeutics, other vascular therapeutics, Amturnide (aliskiren + amlodipine + hydrochlorothiazide), Pradaxa (dabigatran etexilate mesylate), Tekamlo (aliskiren + amlodipine), Tribenzor (olmesartan medoxomil + amlodipine + hydrochlorothiazide), Cialis (tadalafil), Atryn (antithrombin recombinant lyophilized powder for reconstitution), Efient (prasugrel), Livalo (pitavastatin), Multaq (dronedarone), Tyvaso (treprostinil), Cleviprex (clevidipine), Trilipix (fenofibric acid), Azor (amlodipine besylate; olmesartan medoxomil), Fenofibrate, Letairis (ambrisentan), Soliris (eculizumab), Tekturna (aliskiren), Ranexa (ranolazine), BiDil (isosorbide dinitrate/hydralazine hydrochloride), Caduet (amlodipine/atorvastatin), Crestor (rosuvastatin calcium), Levitra (vardenafil), Altocor (lovastatin), Benicar, Imagent (perflexane lipid microspheres), Inspra (eplerenone tablets), Plavix (clopidogrel bisulfate), Remodulin (treprostinil), Advicor (extended-release niacin/lovastatin), Diovan (valsartan), Natrecor (nesiritide), Teveten (eprosartan mesylate plus hydrochlorothiazide), Tricor (fenofibrate), Angiomax (bivalirudin), Argatroban Injection, Atacand (candesartan cilexetil), Betapace AF Tablet, Diltiazem HCL, Innohep (tinzaparin sodium), Lescol XL (fluvastatin sodium), Micardis HCT (telmisartan and hydrochlorothiazide), Nitrostat (nitroglycerin), Lescol (fluvastatin sodium), Mevacor (lovastatin), Niaspan, Agrylin (anagrelide HCL), Atacand (candesartan cilexetil), Atacand (candesartan cilexetil), CellCept, Diovan HCT (valsartan), Integrilin, Micardis (telmisartan), Rythmol, Tiazac (diltiazem hydrochloride), Tiazac (diltiazem hydrochloride), Tricor (fenofibrate), Viagra, Baycol (cerivastatin sodium), Captopril and hydrochlorotiazide, Cardizem, Corlopam, Diovan (valsartan), DynaCirc CR, EDEX, Lescol (fluvastatin sodium), Lexxel (enalapril maleate-felodipine ER), Microzide (hydrochlorothiazide), Normiflo, Pentoxifylline, Pindolol, Plavix (clopidogrel bisulfate), Posicor, ReoPro, REPRONEX(menotropins for injection, USP), Teveten (eprosartan mesylate), Verapamil, Warfarin Sodium, Zocor, Covera-HS (verapamil), Mavik (trandolapril), Muse, Pravachol (pravastatin sodium), Pravachol (pravastatin sodium), ProAmatine (midodrine), Procanbid (procainamide hydrochloride extended-release tablets), Retavase (reteplase), Teczem (enalapril maleate/diltiazem malate), Tiazac (diltiazem hydrochloride), Visipaque (iodixanol), Androderm (Testosterone Transdermal System), Corvert Injection (ibutilide fumarate injection), Prinivil or Zestril (Lisinopril), or Toprol-XL (metoprolol succinate), β-adrenergic blockers such as betaxolol, propranolol, nadolol, metoprolol, atenolol, carvedilol, metoprolol, nebivolol, labetalol, sotalol, timolol, esmolol, carteolol, penbutolol, acebutolol, pindolol, and bisoprolol; abnormal clotting drugs such as, heparin, enoxaparin, dalteparin, coumadin, TPA, streptokinase, urokinase, diypyramidole, Ticlopidine (Ticlid), clopidrogel (Plavix), abciximab (Reopro), eptifabitide (Integrilin), and tirofiban (Aggrastat); diuretics, such as acetazolamide, dichlorphenamide, methazolamide, torsemide, furosemide, bumetanide, ethacrynic acid, pamabrom, spironolactone, spironolactone, amiloride, triamterene, indapamide, methyclothiazide, hydrochlorothiazide, chlorothiazide, metolazone, bendroflumethiazide, polythiazide, hydroflumethiazide, and chlorthalidone; Ca²⁺ channel blockers such as diltiazem, nimodipine, verapamil, nifedipine, amlodipine, felodipine, isradipine, clevidipine, bepridil, and nisoldipine; nitrodilators such as nitroglycerin, alprostadil, hydralazine, minoxidil, nesiritide, isosorbide mononitrate , and nitroprusside; α-adrenoceptor antagonists or alpha-blockers such as doxazosin, prazosin, terazosin, alfuzosin, tamsulosin, and silodosin; angiotensin converting enzyme inhibitors such as Trandolapril, fosinopril, enalapril, ramipril, captopril, moexipril, lisinopril, quinapril, benazepril, and perindopril; angiotensin receptor blockers or ARBs such as eprosartan, olmesartan, telmisartan, losartan, valsartan, irbesartan, and candesartan; renin inhibitors such as Aliskiren; peripheral pressors such as cyclandelate, papaverine, and isoxsuprine; β-agonists such as epinephrine, norepinephrine, dopamine, dobutamine, and isoproterenol; cardiac glycosides such as ouabain, digitalis, digoxin, and digitoxin; phosphodiesterase inhibitors such as milrinone, inamrinone, cilostazol, sildenafil, and tadalafil; vassopressin analogs such as arginine vasopressin and terlipressin; anti-coagulants; coumarins and indandiones such as warfarin and anisindione; factor Xa inhibitors such as fondaparinux; heparins such as dalteparin, tinzaparin, enoxaparin, ardeparin, and danaparoid; platelet aggregation inhibitors such as aspirin, prasugrel, cilostazol, clopidogrel, dipyridamole, and ticlopidine; thrombin inhibitors such as argatroban, bivalirudin, desirudin, and lepirudin; anti-inflammatory drugs; steroids such as prednisone, prednisolone, and hydrocortisone; ophthalmic anti-inflammatory agents such as nepafenac, ketorolac, flurbiprofen, suprofen, cyclosporine, triamcinolone, diclofenac, and bromfena; non-steroidal anti-inflammatory drugs (NSAIDS) such as ibuprofen,. ketoprofen, etodolac, fenoprofen, naproxen, sulindac, indomethacin, piroxicam, mefenamic acid, oxaprozin, and tolmetin; cholesterol-lowering medications; statins such as atorvastatin, simvastatin, pravastatin, lovastatin, fluvastatin, cerivastatin, pitavastatin, and rosuvastatin; niacin, aspirin, ezetimibe, and dextrothyroxine; fibric acids such as Gemfibrozil, fenofibrate, and clofibrate; Anti-hyperglycemics; secretagogues such as glipizide, glimepiride, glyburide, chlorpropamide, acetohexamide, tolbutamide, tolazamide, repaglinide, and nateglinide; insulin sensitizers such as rosiglitazone, pioglitazone, troglitazone, metformin, buformin, and phenformin; alpha-glucosidase inhibitors such as miglitol, acarbose; peptide analogs such as exenatide, saxagliptin, sitagliptin, liraglutide, taspoglutide, and pramlinitide; and drugs that inhibit post-ischemic injury such as lycopene, glutamate, Anti-T-Lymphocyte Globulin, EPO, ethyl pyruvate, diannexin, A-002 (Athera Pharmaceuticals, PLA2 inhibition), Pycnogenol®, DP-b99 (D-Pharm, metal chelator), Rivaroxaban alone or in combination with thienopyridine, Viprinex™ (Ancrod), Omecamtiv mecarbil (CK-1827452), Ginsenoside-Rd, Tenecteplase, Rotigaptide, desmoteplase, Otamixaban (XRP0673), eplerenone, SCH 530348, ranibizumab, Alteplase, argatroban, Human Recombinant Fibroblast Growth Factor-1 (FGF 1-141), Eptifibatide, FX06 (fibrin-derived peptide), apixaban, functional nucleic acid, aptamers, ribozymes, triplex forming molecules, and external guide sequences, a peptide nucleic acid, an antisense molecule, an siRNA, an shRNA, a morpholino, an antibody, a peptide, small molecules, alpha-1 antitrypsin, ZD0892, SPIPm2, ONO-5046, 1,4-bisphenyl-1,4-dihydropyridine, 2-hydroxy and 2-aminodihydropyridines, a dolastatin or dolasatin analog, or a lyngbyastatin or a lyngbyastatin analog or DPMFKLboroV.

Also described herein are methods of treating or alleviating a symptom of Vascular Associated Maculopathy, or a symptom thereof, comprising modulating the expression or activity of HTRA1 or ARMS2 by administering to a subject a therapeutically effective amount of an active agent or a therapeutic agent, for example, one or more of an antisense molecule, an siRNA, a peptide, or a small molecule. For example, and not to be limiting, the therapeutic agent can be DPMFKLboroV.

In one aspect, the methods of treating or alleviating Vascular Associated Maculopathy, or a symptom thereof, comprise modulating the expression or activity of HTRA1 or ARMS2 by administering to a subject a therapeutically effective amount of one or more of an antisense molecule, an siRNA, a peptide, or a small molecule capable of hybridizing to HTRA1 or ARMS2, can further comprise administering to a subject a therapeutically effective amount of one or more agents that inhibit closure or occlusion of choroidal arterioles or choriocapillaris capillaries in combination with one or more agents that increase perfusion in choroidal arterioles and choriocapillaris capillaries in the subject.

Also described herein are methods of predicting the success of an active or therapeutic agent in a patient undergoing treatment for exudative AMD comprising determining the identity of one or more SNPs in the HTRA1 or ARMS2 gene, wherein the presence of a SNP indicates that the patient is less likely to respond to the active or therapeutic agent. For example, and not to be limiting, detecting one or more of rs11200638 in the HTRA1 gene or rs10490924 in the ARMS2 gene, wherein an A allele at the HTRA1 SNP or a T allele at the ARMS2 SNP, can indicate that the patient is less likely to respond to the active or therapeutic agent. In a further aspect, the methods can comprise determining in the subject the identity of one or more SNPs in the HTRA1 or ARMS2 genes, including, but not limited to, rs1049331, rs3750848, or rs2672587, wherein the presence of one or more of the SNPs indicates that the patient is less likely to respond to the active or therapeutic agent. In yet a further aspect, the methods can comprise detecting a SNP that is in linkage disequilibrium with a risk, or otherwise informative SNP or genetic marker described herein, for example, and not to be limiting, an HTRA1 or ARMS2 SNP, wherein detecting a SNP that is in linkage disequilibrium with an HTRA1 or ARMS2 SNP can indicate that the patient is less likely to respond to the active or therapeutic agent.

Also described herein are methods of predicting the success of anti-VEGF (vascular endothelial growth factor) therapy in patients undergoing treatment for exudative AMD comprising determining the identity of one or more SNPs in the ARMS2 gene, wherein the presence of the one or more SNPs in the ARMS2 gene indicates that the patient is less likely to respond to anti-VEGF therapy. For example, and not to be limiting, detecting rs10490924 in the ARMS2 gene, wherein a T allele at this ARMS2 SNP, can indicate that the patient is less likely to respond to anti-VEGF therapy. In a further aspect, the methods can comprise determining in the subject the identity of one or more SNPs in the ARMS2 gene, including, but not limited to rs3750848, wherein a G allele at this ARMS2 SNP indicates that the patient is less likely to respond to anti-VEGF therapy. In yet a further aspect, the methods can comprise detecting a SNP that is in linkage disequilibrium with a risk, or otherwise informative SNP or genetic marker described herein, for example, and not to be limiting, an ARMS2 SNP, wherein detecting a SNP that is in linkage disequilibrium with an ARMS2 SNP can indicate that the patient is less likely to respond to anti-VEGF therapy.

Also described herein are methods of modulating the expression or activity of HTRA1 and ARMS2 comprising administering to a subject a therapeutically effective amount of one or more of an antisense molecule, an siRNA, a peptide, or a small molecule.

Also described herein are methods of treating or alleviating Vascular Associated Maculopathy, or a symptom thereof, comprising modulating the expression or activity of HTRA1 and ARMS2 by administering to a subject a therapeutically effective amount of one or more of an antisense molecule, an siRNA, a peptide, or a small molecule.

In one aspect, the methods of treating or alleviating Vascular Associated Maculopathy, or a symptom thereof, can comprise modulating the expression or activity of HTRA1 and ARMS2 by administering to a subject a therapeutically effective amount of one or more of an antisense molecule, an siRNA, a peptide, or a small molecule, can further comprise administering to a subject a therapeutically effective amount of one or more agents that inhibit closure of choroidal arterioles or choriocapillaris capillaries in combination with one or more agents that increase perfusion in choroidal arterioles and choriocapillaris capillaries in the subject.

### 1. HTRA1 - ELASTASE

Also described herein are methods of modulating the elastase activity of HTRA1 comprising administering to a subject a therapeutically effective amount of one or more of an antisense molecule, an siRNA, a peptide, or a small molecule. In one aspect, the method comprises preventing degradation of Bruch's membrane by administering to a subject a therapeutically effective amount of one or more active agents or therapeutic agents. For example, and not to be limiting the agent can be DPMFKLboroV.

Bruch's membrane, an extracellular layer comprised of the structural proteins elastin and collagen, functions as a physical barrier to the egress of cells and vessels from the choroid into the sub-RPE and subretinal spaces. Disruption of, or damage to, this barrier is associated with loss of vision in AMD, often resulting from the growth of new blood vessels from the choroid into the sub-RPE and/or subretinal spaces (a process referred to as choroidal neovascularization, or CNV). Moreover, morphometric assessment of the macular and extramacular regions of human donor eyes, with and without AMD, revealed a statistically significant difference in both the integrity and thickness of the elastin-containing layer (EL) of Bruch's membrane between the macular and extramacular regions. Importantly, the protease recognition pocket of HTRA1, which plays a critical role in substrate recognition, resembles that of elastase, and the Kazal domain, an integral part of the regulatory region of the protein, shares structural homology with a known elastase inhibitor from Anemonia. Therefore, the methods described herein can comprise preventing degradation of Bruch's membrane by administering to a subject a therapeutically effective amount of an elastase inhibitor. For example, and not to be limiting, the elastase inhibitor can be alpha-1 antitrypsin, ZD0892, SPIPm2, ONO-5046, 1,4-bisphenyl-1,4-dihydropyridine, 2-hydroxy and 2-aminodihydropyridines, a dolastatin or dolasatin analog, or a lyngbyastatin or a lyngbyastatin analog.

Also described herein are methods of treating Vascular Associated Maculopathy in a subject, comprising administering an effective amount of an elastase inhibitor to the subject.

In one aspect, the methods of treating Vascular Associated Maculopathy in a subject, can comprise administering an effective amount of alpha-1 antitrypsin, ZD0892, SPIPm2, ONO-5046, 1,4-bisphenyl-1,4-dihydropyridine, 2-hydroxy and 2-aminodihydropyridines, a dolastatin or dolasatin analog, or a lyngbyastatin or a lyngbyastatin analog or DPMFKLboroV to the subject.

Also described herein are methods of treating one or more symptoms associated with Vascular Associated Maculopathy in a subject, comprising administering an effective amount of an elastase inhibitor to the subject.

In one aspect, the methods of treating one or more symptoms associated with Vascular Associated Maculopathy in a subject, can comprise administering an effective amount of alpha-1 antitrypsin, ZD0892, SPIPm2, ONO-5046, 1,4-bisphenyl-1,4-dihydropyridine, 2-hydroxy and 2-aminodihydropyridines, a dolastatin or dolasatin analog, or a lyngbyastatin or a lyngbyastatin analog or DPMFKLboroV to the subject.

Also described herein are methods of treating severe maculopathy or last stage maculopathy in a subject, comprising administering an effective amount of an elastase inhibitor to the subject.

In one aspect, methods of treating severe maculopathy or last stage maculopathy in a subject, can comprise administering an effective amount of alpha-1 antitrypsin, ZD0892, SPIPm2, ONO-5046, 1,4-bisphenyl-1,4-dihydropyridine, 2-hydroxy and 2-aminodihydropyridines, a dolastatin or dolasatin analog, or a lyngbyastatin or a lyngbyastatin analog or DPMFKLboroV to the subject.

Also described herein are methods of resolving aberrant choriocappilaris lobules in a subject, comprising administering an effective amount of an elastase inhibitor to the subject.

In one aspect the methods of resolving aberrant choriocappilaris lobules in a subject, can comprise administering an effective amount of alpha-1 antitrypsin, ZD0892, SPIPm2, ONO-5046, 1,4-bisphenyl-1,4-dihydropyridine, 2-hydroxy and 2-aminodihydropyridines, a dolastatin or dolasatin analog, or a lyngbyastatin or a lyngbyastatin analog or DPMFKLboroV to the subject.

Also described herein are methods of (i) treating Vascular Associated Maculopathy; (ii) treating one or more symptoms associated with Vascular Associated Maculopathy; (iii) treating severe maculopathy or last stage maculopathy or (iv) resolving aberrant choriocappilaris lobules in a subject comprising: (a) selecting a subject with (i) treating Vascular Associated Maculopathy; (ii) treating one or more symptoms associated with Vascular Associated Maculopathy; (iii) treating severe maculopathy or last stage maculopathy or (iv) resolving aberrant choriocappilaris lobules; (b) administering an effective amount of one or more of the disclosed compositions, therapeutic agents, active agents, or functional nucleic acids to the subject, thereby (i) treating Vascular Associated Maculopathy; (ii) treating one or more symptoms associated with Vascular Associated Maculopathy; (iii) treating severe maculopathy or last stage maculopathy or (iv) resolving aberrant choriocappilaris lobules in the subject.

Further described herein are methods of (i) treating Vascular Associated Maculopathy; (ii) treating one or more symptoms associated with Vascular Associated Maculopathy; (iii) treating severe maculopathy or last stage maculopathy or (iv) resolving aberrant choriocappilaris lobules in a subject comprising: (a) selecting a subject with (i) treating Vascular Associated Maculopathy; (ii) treating one or more symptoms associated with Vascular Associated Maculopathy; (iii) treating severe maculopathy or last stage maculopathy or (iv) resolving aberrant choriocappilaris lobules; and (b) administering an effective amount of a pharmaceutical composition comprising one or more of the disclosed compositions, therapeutic agents, active agents, or functional nucleic acids and a pharmaceutically acceptable carrier to the subject, thereby (i) treating Vascular Associated Maculopathy; (ii) treating one or more symptoms associated with Vascular Associated Maculopathy; (iii) treating severe maculopathy or last stage maculopathy or (iv) resolving aberrant choriocappilaris lobules in the subject.

Also described herein are methods of treating or reversing Vascular Associated Maculopathy, or a symptom thereof, in a subject, wherein aberrant choriocapillaris lobules are present in an eye of the subject, comprising administering to a subject a therapeutically effective amount of one or more agents that increase perfusion in choriocapillaris lobules in the eye of the subject. In one aspect, the symptom of Vascular Associated Maculopathy can appear in an eye of the subject. Therefore, also described herein are methods of treating or reversing a symptom of Vascular Associated Maculopathy in an eye of a subject, wherein aberrant choriocapillaris lobules are present in an eye of the subject, comprising administering to a subject a therapeutically effective amount of one or more agents that increase perfusion in choriocapillaris lobules in the eye of the subject.

Additionally, described herein are methods of treating or reversing Vascular Associated Maculopathy, or a symptom thereof, in a subject, wherein aberrant choriocapillaris lobules are present in an eye of the subject, comprising administering to a subject a therapeutically effective amount of one or more agents that inhibit closure of choriocapillaris lobules in the eye of the subject in combination with one or more agents that increase perfusion in choriocapillaris lobules in the eye of the subject. In one aspect, the symptom of Vascular Associated Maculopathy can appear in an eye of the subject. Therefore, also described herein are methods of treating or reversing symptom of Vascular Associated Maculopathyin an eye of a subject, wherein aberrant choriocapillaris lobules are present in an eye of the subject, comprising administering to a subject a therapeutically effective amount of one or more agents that inhibit closure of choriocapillaris lobules in eye of the subject in combination with one or more agents that increase perfusion in choriocapillaris lobules in the eye of the subject.

Furthermore, described herein are methods of treating or reversing Vascular Associated Maculopathy, or a symptom thereof, in a subject, wherein aberrant choriocapillaris lobules are present in an eye of the subject, comprising administering to a subject a therapeutically effective amount of an anti-vascular endothelial growth factor (anti-VEGF) agent in combination with one or more agents that inhibit closure of or decreased perfusion of choriocapillaris lobules in the eye of the subject or one or more agents that increase perfusion in choriocapillaris lobule in the eye of the subject. In one aspect, the symptom of Vascular Associated Maculopathy can appear in an eye of the subject. Therefore, also described herein are methods of treating or reversing a symptom of Vascular Associated Maculopathy in an eye of a subject, wherein aberrant choriocapillaris lobules are present in an eye of the subject, comprising administering to a subject a therapeutically effective amount of an anti-vascular endothelial growth factor (anti-VEGF) agent in combination with one or more agents that inhibit closure of choriocapillaris lobules in the eye of the subject or one or more agents that increase perfusion in choriocapillaris lobules in the eye of the subject. The anti-VEGF agent can be, but is not limited to, bevacizumab, ranibizumab, ramucirumab, aflibercept, sunitinib, sorafenib, vandetanib, vatalanib, tivozanib, axitinib, imatinib or pazopanib.

In one aspect, the methods of treating or reversing Vascular Associated Maculopathy, or a symptom thereof, described herein can further comprise the step of monitoring the aberrant choriocapillaris lobules in the eye of the subject.

Also described herein are methods of decreasing the risk of Vascular Associated Maculopathy, or a symptom thereof, in a subject, wherein aberrant choriocapillaris lobules are present in an eye of the subject, comprising administering to a subject a therapeutically effective amount of one or more agents that inhibit closure of choriocapillaris lobules in the eye of the subject. In one aspect, the symptom of Vascular Associated Maculopathy can appearin an eye of the subject. Therefore, also described herein are methods of decreasing the risk of a symptom of Vascular Associated Maculopathy in an eye of a subject, wherein aberrant choriocapillaris lobules are present in an eye of the subject, comprising administering to a subject a therapeutically effective amount of one or more agents that inhibit closure, either anatomical or functional closure, of choriocapillaris lobules in the eye of the subject.

As used herein, the phrase "decreasing the risk" can mean the same as "prevent" or "preventing" and refers to precluding, averting, obviating, forestalling, stopping, delaying or hindering something from happening, especially by advance action. It is understood that where decrease, inhibit or prevent are used herein, unless specifically indicated otherwise, the use of the other two words is also expressly disclosed.

In one aspect, the one or more agents that inhibit closure of choriocapillaris lobules can be administered directly to the eye. This route of administration, known as ophthalmic administration can be accomplished by a variety of means known to a person of skill in the art. For example, and not to be limiting, the agent or agents can be provided to an eye of a subject in need thereof through the placement of a cream, an ointment, or a liquid drop preparation onto the inner eyelid of the subject, through the use of a mist sprayed onto the eye of the subject, or through intravitreal injection. Ophthalmic administration can further include topical administration, subconjunctival administration, sub-Tenon's administration, epibulbar administration, retrobulbar administration, intra-orbital administration, periocular injection, and intraocular administration, which includes intra-vitreal administration.

In one aspect, ophthalmic administration of an agent or agents can be accomplished through the use of systemic delivery, such intravenous delivery, unidirectional episcleral implant, hollow microneedles, solid coated microneedles, free-floating intravitreal implant, or scleral-fixated intravitreal implant. Ophthalmic administration of an agent or agents can also be accomplished through the use of topical iontophoresis. Iontophoresis is a noninvasive method of delivering compounds into the eye. It can be performed by applying a small electrical current that has the same charge as the compound to create replulsive electromotive forces that enable delivery of the compound to the anterior or posterior segment of the eye. Edelhauser et al., Ophthalmic Drug Delivery Systems for the Treatment of Retinal Diseases: Basic Research to Clinical Applications, IOVS 2010: 51(11) 5403-5419, which is herein incoprated in its entirety by this reference, describes these various methods of ophthalmic administration.

The phrase "subject in need thereof', refers to selection of a subject based upon need for treatment of the disorder. For example, a subject can be identified as having a need for treatment of Vascular Associated Maculopathy, or a symptom thereof, severe maculopathy, late stage maculopathy, or aberrant choriocapillaris lobules, based upon diagnosis by a person of skill and thereafter subjected to treatment for the disorder. It is contemplated that the identification can, in one aspect, be performed by a person different from the person making the diagnosis. It is also contemplated, in a further aspect, that the administration can be performed by one who subsequently performed the administration.

Also described herein are methods of selecting a subject for the disclosed methods of (i) treating Vascular Associated Maculopathy; (ii) treating one or more symptoms associated with Vascular Associated Maculopathy; (iii) treating severe maculopathy or last stage maculopathy or (iv) resolving aberrant choriocappilaris lobules.

Also described herein are methods of diagnosing a subject with (i) Vascular Associated Maculopathy; (ii) one or more symptoms associated with Vascular Associated Maculopathy; (iii) severe maculopathy or last stage maculopathy or (iv) aberrant choriocappilaris lobules.

In one aspect, described herein are methods wherein subject is selected or diagnosed by detecting the presence of aberrant choriocapillaris lobules in the eye of the subject, wherein aberrant choriocapillaris lobules identifies a subject with (i) Vascular Associated Maculopathy; (ii) one or more symptoms associated with Vascular Associated Maculopathy; or (iii) severe maculopathy or last stage maculopathy. The aberrant choriocapillaris lobules in the eye of the subject can be identified using any of the methods disclosed herein, for example, the aberrant choriocapillaris lobules in the eye of the subject can be identified by autofluorescent imaging techniques, infrared imaging techniques, optical coherence tomography (OCT), Stratus optical coherence tomography (Stratus OCT), Fourier-domain optical coherence tomography (Fd-OCT), two-photon-excited fluorescence (TPEF) imaging, adaptive optics scanning laser ophthalmoscopy (AOSLO), scanning laser ophthalmoscopy, near-infrared imaging combined with spectral domain optical coherence tomography (SD-OCT), color fundus photography, fundus autofluorescence imaging, red-free imaging, fluorescein angiography, indocyanin green angiography, multifocal electroretinography (ERG) recording, microperimetry, color Doppler optical coherence tomography (CDOCT), visual field assessment, Heidelberg Spectralis, the Zeiss Cirrus, the Topcon 3D OCT 2000, the Optivue RTVue SD-OCT, the Opko OCT SLO, the NIDEK F-10, or the Optopol SOCT Copernicus HR.

Further described herein are methods wherein subject is selected or diagnosed by determining in the subject the identity of one or more SNPs in the HTRA1, ARMS2, or CFH genes, wherein the one or more SNPs are (i) the rs11200638 in the HTRA1 gene, rs1049331 in the HTRA1 gene, rs2672587 in the HTRA1 gene, rs10490924 in the ARMS2 gene, rs3750848 in the ARMS2 gene, rs1061170 in the CFH gene, or rs800292 in the CFH gene, or (ii) a SNP in linkage disequilibrium with the SNPS of (i), wherein the presence of one or more variants of the SNPs identifies a subject with (a) Vascular Associated Maculopathy; (b) one or more symptoms associated with Vascular Associated Maculopathy; (c) severe maculopathy or last stage maculopathy or (d) aberrant choriocappilaris lobules. For example, disclosed herein are methods wherein subject is selected by determining in the subject the identity of one or more SNPs in the HTRA1, ARMS2, or CFH genes, wherein an A at the rs11200638 SNP, a T at the rs1049331 SNP, a G at the rs2672587 SNP, a T at the rs10490924 SNP, a G at the rs3750848 SNP, a C at the rs1061170 SNP, or a G at the rs800292 SNP identifies a subject with (a) Vascular Associated Maculopathy; (b) one or more symptoms associated with Vascular Associated Maculopathy; (c) severe maculopathy or last stage maculopathy or (d) aberrant choriocappilaris lobules.

Examples of agents that can inhibit closure, either anatomical or functional closure, of the choriocapillaris lobules include, but are not limited to, aspirin, anti-inflammatory medications, cholesterol-lowering medications, anti-hyperglycemic medications, vasodilators, vasopressors, diuretics, anticoagulants, thrombolytic medications, anti-vascular endothelial growth factor medications, anti-post-ischemic injury medications, anti-hypertensive medications, abnormal clotting therapeutics, or other vascular therapeutics.

Examples of vascular therapeutics can include, but are not limited to, Amturnide (aliskiren + amlodipine + hydrochlorothiazide), Pradaxa (dabigatran etexilate mesylate), Tekamlo (aliskiren + amlodipine), Tribenzor (olmesartan medoxomil + amlodipine + hydrochlorothiazide), Cialis (tadalafil), Atryn (antithrombin recombinant lyophilized powder for reconstitution), Efient (prasugrel), Livalo (pitavastatin), Multaq (dronedarone), Tyvaso (treprostinil), Cleviprex (clevidipine), Trilipix (fenofibric acid), Azor (amlodipine besylate; olmesartan medoxomil), Fenofibrate, Letairis (ambrisentan), Soliris (eculizumab), Tekturna (aliskiren), Ranexa (ranolazine), BiDil (isosorbide dinitrate/hydralazine hydrochloride), Caduet (amlodipine/atorvastatin), Crestor (rosuvastatin calcium), Levitra (vardenafil), Altocor (lovastatin), Benicar, Imagent (perflexane lipid microspheres), Inspra (eplerenone tablets), Plavix (clopidogrel bisulfate), Remodulin (treprostinil), Advicor (extended-release niacin/lovastatin), Diovan (valsartan), Natrecor (nesiritide), Teveten (eprosartan mesylate plus hydrochlorothiazide), Tricor (fenofibrate), Angiomax (bivalirudin), Argatroban Injection, Atacand (candesartan cilexetil), Betapace AF Tablet, Diltiazem HCL, Innohep (tinzaparin sodium), Lescol XL (fluvastatin sodium), Micardis HCT (telmisartan and hydrochlorothiazide), Nitrostat (nitroglycerin), Lescol (fluvastatin sodium), Mevacor (lovastatin), Niaspan, Agrylin (anagrelide HCL), Atacand (candesartan cilexetil), Atacand (candesartan cilexetil), CellCept, Diovan HCT (valsartan), Integrilin, Micardis (telmisartan), Rythmol, Tiazac (diltiazem hydrochloride), Tiazac (diltiazem hydrochloride), Tricor (fenofibrate), Viagra, Baycol (cerivastatin sodium), Captopril and hydrochlorotiazide, Cardizem, Corlopam, Diovan (valsartan), DynaCirc CR, EDEX, Lescol (fluvastatin sodium), Lexxel (enalapril maleate-felodipine ER), Microzide (hydrochlorothiazide), Normiflo, Pentoxifylline, Pindolol, Plavix (clopidogrel bisulfate), Posicor, ReoPro, REPRONEX(menotropins for injection, USP), Teveten (eprosartan mesylate), Verapamil, Warfarin Sodium, Zocor, Covera-HS (verapamil), Mavik (trandolapril), Muse, Pravachol (pravastatin sodium), Pravachol (pravastatin sodium), ProAmatine (midodrine), Procanbid (procainamide hydrochloride extended-release tablets), Retavase (reteplase), Teczem (enalapril maleate/diltiazem malate), Tiazac (diltiazem hydrochloride), Visipaque (iodixanol), Androderm (Testosterone Transdermal System), Corvert Injection (ibutilide fumarate injection), Prinivil or Zestril (Lisinopril), or Toprol-XL (metoprolol succinate).

Additional therapeutic agents that can be used in the methods described herein include, but are not limited to, β-adrenergic blockers such as betaxolol, propranolol, nadolol, metoprolol, atenolol, carvedilol, metoprolol, nebivolol, labetalol, sotalol, timolol, esmolol, carteolol, penbutolol, acebutolol, pindolol, and bisoprolol; abnormal clotting drugs such as, heparin, enoxaparin, dalteparin, coumadin, TPA, streptokinase, urokinase, diypyramidole, Ticlopidine (Ticlid), clopidrogel (Plavix), abciximab (Reopro), eptifabitide (Integrilin), and tirofiban (Aggrastat); diuretics, such as acetazolamide, dichlorphenamide, methazolamide, torsemide, furosemide, bumetanide, ethacrynic acid, pamabrom, spironolactone, spironolactone, amiloride, triamterene, indapamide, methyclothiazide, hydrochlorothiazide, chlorothiazide, metolazone, bendroflumethiazide, polythiazide, hydroflumethiazide, and chlorthalidone; Ca²⁺ channel blockers such as diltiazem, nimodipine, verapamil, nifedipine, amlodipine, felodipine, isradipine, clevidipine, bepridil, and nisoldipine; nitrodilators such as nitroglycerin, alprostadil, hydralazine, minoxidil, nesiritide, isosorbide mononitrate , and nitroprusside; α-adrenoceptor antagonists or alpha-blockers such as doxazosin, prazosin, terazosin, alfuzosin, tamsulosin, and silodosin; angiotensin converting enzyme inhibitors such as Trandolapril, fosinopril, enalapril, ramipril, captopril, moexipril, lisinopril, quinapril, benazepril, and perindopril; angiotensin receptor blockers or ARBs such as eprosartan, olmesartan, telmisartan, losartan, valsartan, irbesartan, and candesartan; renin inhibitors such as Aliskiren; peripheral pressors such as cyclandelate, papaverine, and isoxsuprine; β-agonists such as epinephrine, norepinephrine, dopamine, dobutamine, and isoproterenol; cardiac glycosides such as ouabain, digitalis, digoxin, and digitoxin; phosphodiesterase inhibitors such as milrinone, inamrinone, cilostazol, sildenafil, and tadalafil; vassopressin analogs such as arginine vasopressin and terlipressin; anti-coagulants; coumarins and indandiones such as warfarin and anisindione; factor Xa inhibitors such as fondaparinux; heparins such as dalteparin, tinzaparin, enoxaparin, ardeparin, and danaparoid; platelet aggregation inhibitors such as aspirin, prasugrel, cilostazol, clopidogrel, dipyridamole, and ticlopidine; thrombin inhibitors such as argatroban, bivalirudin, desirudin, and lepirudin; anti-inflammatory drugs; steroids such as prednisone, prednisolone, and hydrocortisone; ophthalmic anti-inflammatory agents such as nepafenac, ketorolac, flurbiprofen, suprofen, cyclosporine, triamcinolone, diclofenac, and bromfena; non-steroidal anti-inflammatory drugs (NSAIDS) such as ibuprofen,. ketoprofen, etodolac, fenoprofen, naproxen, sulindac, indomethacin, piroxicam, mefenamic acid, oxaprozin, and tolmetin; cholesterol-lowering medications; statins such as atorvastatin, simvastatin, pravastatin, lovastatin, fluvastatin, cerivastatin, pitavastatin, and rosuvastatin; niacin, aspirin, ezetimibe, and dextrothyroxine; fibric acids such as Gemfibrozil, fenofibrate, and clofibrate; Anti-hyperglycemics; secretagogues such as glipizide, glimepiride, glyburide, chlorpropamide, acetohexamide, tolbutamide, tolazamide, repaglinide, and nateglinide; insulin sensitizers such as rosiglitazone, pioglitazone, troglitazone, metformin, buformin, and phenformin; alpha-glucosidase inhibitors such as miglitol, acarbose; peptide analogs such as exenatide, saxagliptin, sitagliptin, liraglutide, taspoglutide, and pramlinitide; and drugs that inhibit post-ischemic injury such as lycopene, glutamate, Anti-T-Lymphocyte Globulin, EPO, ethyl pyruvate, diannexin, A-002 (Athera Pharmaceuticals, PLA2 inhibition), Pycnogenol®, DP-b99 (D-Pharm, metal chelator), Rivaroxaban alone or in combination with thienopyridine, Viprinex™ (Ancrod), Omecamtiv mecarbil (CK-1827452), Ginsenoside-Rd, Tenecteplase, Rotigaptide, desmoteplase, Otamixaban (XRP0673), eplerenone, SCH 530348, ranibizumab, Alteplase, argatroban, Human Recombinant Fibroblast Growth Factor-1 (FGF 1-141), Eptifibatide, FX06 (fibrin-derived peptide), and apixaban.

It is well known in the art that many of the agents described herein can have more than one effect and function. Specifically, many of the described agents can both prevent or inhibit closure or occlusion of choriocapillaris lobules and increase blood flow in choriocapillaris lobules to increase perfusion to target tissues and organs, for example, the choroid of a human eye, heart, brain, spinal cord, muscle, bone, eye, ear, kidney, uterus, placenta, skin, mucous membranes, stomach, liver, lung, gall bladder, spleen, appendix, small intestine, large intestine, pancreas, prostate, or urinary bladder and the like.

It is also contemplated that agents directed at interrupting various pathways that lead to post-ischemic injury can be used in the disclosed methods. Examples of such pathways include, but are not limited to, those related to NF-KappaB, basic fibroblast growth factor, kinase pathways (PI3, Akt, Ras, MAPK), and other pathways (caspase, SOD, catalase, prostaglandin E1, prostaglandin E2, thrombospondin, and Fas receptor), and the like.

In one aspect, the one or more agents that inhibit closure of the choriocapillaris lobules can be coupled with an agent or agents that hybridizes to HTRA1 or ARMS2 nucleic acids or peptides. The HTRA1 or ARMS2 targeting agent can be, but is not limited to, a nucleic acid, a functional nucleic acid, aptamers, ribozymes, triplex forming molecules, and external guide sequences, a peptide nucleic acid, an antisense molecule, an siRNA, an shRNA, a morpholino, an antibody, a peptide, or a small molecule. Moreover the HTRA1 or ARMS2 targeting agent can be an agent that inhibits the expression or activity of HTRA1 or ARMS2. For example, and not to be limiting, the HTRA1 or ARMS2 expression or activity can be inhibited by about 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%. Thus, HTRA1 or ARMS2 expression or activity can be inhibited by from about 5% to about 100%, including all inhibition percentage values between 5% and 100%.

### 2. FUNCTIONAL NUCLEIC ACIDS

Disclosed are functional nucleic acids that can interact with the disclosed polynucleotides. Functional nucleic acids are nucleic acid molecules that have a specific function, such as binding a target molecule or catalyzing a specific reaction. Functional nucleic acid molecules can be divided into the following categories, which are not meant to be limiting. For example, functional nucleic acids include antisense molecules, aptamers, ribozymes, triplex forming molecules, and external guide sequences. The functional nucleic acid molecules can act as effectors, inhibitors, modulators, and stimulators of a specific activity possessed by a target molecule, or the functional nucleic acid molecules can possess a de novo activity independent of any other molecules.

Functional nucleic acid molecules can interact with any macromolecule, such as DNA, RNA, polypeptides, or carbohydrate chains. Thus, functional nucleic acids can interact with the mRNA of polynucleotide sequences described herein or the genomic DNA of the polynucleotide sequences described herein or they can interact with the polypeptide encoded by the polynucleotide sequences described herein. Often functional nucleic acids are designed to interact with other nucleic acids based on sequence homology between the target molecule and the functional nucleic acid molecule. In other situations, the specific recognition between the functional nucleic acid molecule and the target molecule is not based on sequence homology between the functional nucleic acid molecule and the target molecule, but rather is based on the formation of tertiary structure that allows specific recognition to take place.

### 3. ANTISENSE MOLECULES

Described herein are antisense molecules that interact with the disclosed polynucleotides. Antisense molecules are designed to interact with a target nucleic acid molecule through either canonical or non-canonical base pairing. The interaction of the antisense molecule and the target molecule is designed to promote the destruction of the target molecule through, for example, RNAseH mediated RNA-DNA hybrid degradation. Alternatively the antisense molecule is designed to interrupt a processing function that normally would take place on the target molecule, such as transcription or replication. Antisense molecules can be designed based on the sequence of the target molecule. Numerous methods for optimization of antisense efficiency by finding the most accessible regions of the target molecule exist. Exemplary methods would be in vitro selection experiments and DNA modification studies using DMS and DEPC. It is preferred that antisense molecules bind the target molecule with a dissociation constant (k_{d}) less than or equal to 10⁻⁶, 10⁻⁸, 10⁻¹⁰, or 10⁻¹². A representative sample of methods and techniques which aid in the design and use of antisense molecules can be found in the following non-limiting list of United States patents: 5,135,917, 5,294,533, 5,627,158, 5,641,754, 5,691,317, 5,780,607, 5,786,138, 5,849,903, 5,856,103, 5,919,772, 5,955,590, 5,990,088, 5,994,320, 5,998,602, 6,005,095, 6,007,995, 6,013,522, 6,017,898, 6,018,042, 6,025,198, 6,033,910, 6,040,296, 6,046,004, 6,046,319, and 6,057,437.

Generally, the term "antisense" refers to a nucleic acid molecule capable of hybridizing to a portion of an RNA sequence (such as mRNA) by virtue of some sequence complementarity. The antisense nucleic acids described herein can be oligonucleotides that are double-stranded or single-stranded, RNA or DNA or a modification or derivative thereof, which can be directly administered to a cell (for example by administering the antisense molecule to the subject), or which can be produced intracellularly by transcription of exogenous, introduced sequences (for example by administering to the subject a vector that includes the antisense molecule under control of a promoter).

Antisense nucleic acids are polynucleotides, for example nucleic acid molecules that are at least 6 nucleotides in length, at least 10 nucleotides, at least 15 nucleotides, at least 20 nucleotides, at least 100 nucleotides, at least 200 nucleotides, such as 6 to 100 nucleotides. However, antisense molecules can be much longer. In particular examples, the nucleotide is modified at one or more base moiety, sugar moiety, or phosphate backbone (or combinations thereof), and can include other appending groups such as peptides, or agents facilitating transport across the cell membrane (Letsinger et al., Proc. Natl. Acad. Sci. USA 1989, 86:6553-6; Lemaitre et al., Proc. Natl. Acad. Sci. USA 1987, 84:648-52; WO 88/09810) or blood-brain barrier (WO 89/10134), hybridization triggered cleavage agents (Krol et al., BioTechniques 1988, 6:958-76) or intercalating agents (Zon, Pharm. Res. 5:539-49, 1988). Additional modifications include those set forth in U.S. Patent Nos. 6,608,035, 7,176,296; 7,329,648; 7,262,489, 7,115,579; and 7,105,495.

Examples of modified base moieties include, but are not limited to: 5-fluorouracil, 5-bromouracil, 5-chlorouracil, 5-iodouracil, hypoxanthine, xanthine, acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N∼6-sopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, methoxyarninomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid, pseudouracil, queosine, 2-thiocytosine, 5-methyl-2-thiouracil, 2-thiouracil, 4-thiouracil, 5-methyluracil, uracil-5-oxyacetic acid methylester, uracil-S-oxyacetic acid, 5-methyl-2-thiouracil, 3-(3-amino-3-N-2-carboxypropyl) uracil, and 2,6-diaminopurine.

Examples of modified sugar moieties include, but are not limited to: arabinose, 2-fluoroarabinose, xylose, and hexose, or a modified component of the phosphate backbone, such as phosphorothioate, a phosphorodithioate, a phosphoramidothioate, a phosphoramidate, a phosphordiamidate, a methylphosphonate, an alkyl phosphotriester, or a formacetal or analog thereof.

In a particular example, an antisense molecule is an α-anomeric oligonucleotide. An α-anomeric oligonucleotide forms specific double-stranded hybrids with complementary RNA in which, contrary to the usual β-units, the strands run parallel to each other (Gautier et al., Nucl. Acids Res. 15:6625-41, 1987). The oligonucleotide can be conjugated to another molecule, such as a peptide, hybridization triggered cross-linking agent, transport agent, or hybridization-triggered cleavage agent. Oligonucleotides can include a targeting moiety that enhances uptake of the molecule by host cells. The targeting moiety can be a specific binding molecule, such as an antibody or fragment thereof that recognizes a molecule present on the surface of the host cell.

In a specific example, antisense molecules that recognize a nucleic acid set forth herein, include a catalytic RNA or a ribozyme (for example see WO 90/11364; WO 95/06764; and Sarver et al., Science 247:1222-5, 1990). Conjugates of antisense with a metal complex, such as terpyridylCu (II), capable of mediating mRNA hydrolysis, are described in Bashkin et al. (Appl. Biochem Biotechnol. 54:43-56, 1995). In one example, the antisense nucleotide is a 2'-0-methylribonucleotide (Inoue et al., Nucl. Acids Res. 15:6131-48, 1987), or a chimeric RNA-DNA analogue (Inoue et al., FEBS Lett. 215:327-30, 1987).

Antisense molecules can be generated by utilizing the Antisense Design algorithm of Integrated DNA Technologies, Inc. (1710 Commercial Park, Coralville, IA 52241 USA; (http://www.idtdna.com/Scitools/Applications/AntiSense/Antisense.aspx/).

### 4. SIRNA

Short interfering RNAs (siRNAs), also known as small interfering RNAs, are double-stranded RNAs that can induce sequence-specific post-transcriptional gene silencing, thereby decreasing gene expression (See, for example, U.S. Patent Nos. 6,506,559, 7,056,704, 7,078,196, 6,107,094, 5,898,221, 6,573,099, and European Patent No. 1 144 623). siRNas can be of various lengths as long as they maintain their function. In some examples, siRNA molecules are about 19-23 nucleotides in length, such as at least 21 nucleotides, for example at least 23 nucleotides. In one example, siRNA triggers the specific degradation of homologous RNA molecules, such as mRNAs, within the region of sequence identity between both the siRNA and the target RNA. For example, WO 02/44321 discloses siRNAs capable of sequence-specific degradation of target mRNAs when base-paired with 3' overhanging ends. The direction of dsRNA processing determines whether a sense or an antisense target RNA can be cleaved by the produced siRNA endonuclease complex. Thus, siRNAs can be used to modulate transcription or translation, for example, by decreasing expression of a gene set forth in Table 1. The effects of siRNAs have been demonstrated in cells from a variety of organisms, including *Drosophila* , *C. elegans,* insects, frogs, plants, fungi, mice and humans (for example, WO 02/44321; Gitlin et al., Nature 418:430-4, 2002; Caplen et al., Proc. Natl. Acad. Sei. 98:9742-9747, 2001; and Elbashir et al., Nature 411:494-8, 2001).

Utilizing sequence analysis tools, one of skill in the art can design siRNAs to specifically target any gene set forth in Table 1 for decreased gene expression. siRNAs that inhibit or silence gene expression can be obtained from numerous commercial entities that synthesize siRNAs, for example, Ambion Inc. (2130 Woodward Austin, TX 78744-1832, USA), Qiagen Inc. (27220 Tumberry Lane, Valencia, CA USA) and Dharmacon Inc. (650 Crescent Drive, #100 Lafayette, CO 80026, USA). The siRNAs synthesized by Ambion Inc., Qiagen Inc. or Dharmacon Inc, can be readily obtained from these and other entities by providing a GenBank Accession No. for the mRNA of any gene set forth in Table 1. In addition, siRNAs can be generated by utilizing Invitrogen's BLOCK-IT™ RNAi Designer https://rnaidesigner.invitrogen.com/rnaiexpress.

### 5. SHRNA

shRNA (short hairpin RNA) is a DNA molecule that can be cloned into expression vectors to express siRNA (typically 19-29 nt RNA duplex) for RNAi interference studies. shRNA has the following structural features: a short nucleotide sequence ranging from about 19-29 nucleotides derived from the target gene, followed by a short spacer of about 4-15 nucleotides (*i.e.* loop) and about a 19-29 nucleotide sequence that is the reverse complement of the initial target sequence.

### 6. MORPHOLINOS

Morpholinos are synthetic antisense oligos that can block access of other molecules to small (about 25 base) regions of ribonucleic acid (RNA). Morpholinos are often used to determine gene function using reverse genetics methods by blocking access to mRNA. Morpholinos, usually about 25 bases in length, bind to complementary sequences of RNA by standard nucleic acid base-pairing. Morpholinos do not degrade their target RNA molecules. Instead, Morpholinos act by "steric hindrance", binding to a target sequence within an RNA and simply interfering with molecules which might otherwise interact with the RNA. Morpholinos have been used in mammals, ranging from mice to humans.

Bound to the 5'-untranslated region of messenger RNA (mRNA), Morpholinos can interfere with progression of the ribosomal initiation complex from the 5' cap to the start codon. This prevents translation of the coding region of the targeted transcript (called "knocking down" gene expression). Morpholinos can also interfere with pre-mRNA processing steps, usually by preventing the splice-directing snRNP complexes from binding to their targets at the borders of introns on a strand of pre-RNA. Preventing U1 (at the donor site) or U2/U5 (at the polypyrimidine moiety & acceptor site) from binding can cause modified splicing, commonly leading to exclusions of exons from the mature mRNA. Targeting some splice targets results in intron inclusions, while activation of cryptic splice sites can lead to partial inclusions or exclusions. Targets of U1 1/U12 snRNPs can also be blocked. Splice modification can be conveniently assayed by reverse-transcriptase polymerase chain reaction (RT-PCR) and is seen as a band shift after gel electrophoresis of RT-PCR products. Methods of designing, making and utilizing morpholinos are disclosed in U.S. Patent No. 6,867,349.

### 7. APTAMERS

Also disclosed are aptamers that interact with the disclosed polynucleotides. Aptamers are molecules that interact with a target molecule, preferably in a specific way. Typically aptamers are small nucleic acids ranging from 15-50 bases in length that fold into defined secondary and tertiary structures, such as stem-loops or G-quartets. Aptamers can bind small molecules, such as ATP (United States patent 5,631,146) and theophylline (United States patent 5,580,737), as well as large molecules, such as reverse transcriptase (United States patent 5,786,462) and thrombin (United States patent 5,543,293). Aptamers can bind very tightly with kds from the target molecule of less than 10⁻¹² M. It is preferred that the aptamers bind the target molecule with a kd less than 10⁻⁶, 10⁻⁸, 10⁻¹⁰ or 10⁻¹². Aptamers can bind the target molecule with a very high degree of specificity. For example, aptamers have been isolated that have greater than a 10000 fold difference in binding affinities between the target molecule and another molecule that differ at only a single position on the molecule (United States patent 5,543,293). It is preferred that the aptamer have a kd with the target molecule at least 10, 100, 1000, 10,000, or 100,000 fold lower than the kd with a background binding molecule. It is preferred when doing the comparison for a polypeptide for example, that the background molecule be a different polypeptide. For example, when determining the specificity of aptamers, the background protein could be ef-la. Representative examples of how to make and use aptamers to bind a variety of different target molecules can be found in the following non-limiting list of United States patents: 5,476,766, 5,503,978, 5,631,146, 5,731,424, 5,780,228, 5,792,613, 5,795,721, 5,846,713, 5,858,660, 5,861,254, 5,864,026, 5,869,641, 5,958,691, 6,001,988, 6,011,020, 6,013,443, 6,020,130, 6,028,186, 6,030,776, and 6,051,698.

### 8. RIBOZYMES

Also disclosed are ribozymes that interact with the disclosed polynucleotides. Ribozymes are nucleic acid molecules that are capable of catalyzing a chemical reaction, either intramolecularly or intermolecularly. Ribozymes are thus catalytic nucleic acid. It is preferred that the ribozymes catalyze intermolecular reactions. There are a number of different types of ribozymes that catalyze nuclease or nucleic acid polymerase type reactions which are based on ribozymes found in natural systems, such as hammerhead ribozymes, (for example, but not limited to the following United States patents: 5,334,711, 5,436,330, 5,616,466, 5,633,133, 5,646,020, 5,652,094, 5,712,384, 5,770,715, 5,856,463, 5,861,288, 5,891,683, 5,891,684, 5,985,621, 5,989,908, 5,998,193, 5,998,203, WO 9858058 by Ludwig and Sproat, WO 9858057 by Ludwig and Sproat, and WO 9718312 by Ludwig and Sproat) hairpin ribozymes (for example, but not limited to the following United States patents: 5,631,115, 5,646,031, 5,683,902, 5,712,384, 5,856,188, 5,866,701, 5,869,339, and 6,022,962), and tetrahymena ribozymes (for example, but not limited to the following United States patents: 5,595,873 and 5,652,107). There are also a number of ribozymes that are not found in natural systems, but which have been engineered to catalyze specific reactions de novo (for example, but not limited to the following United States patents: 5,580,967, 5,688,670, 5,807,718, and 5,910,408). Preferred ribozymes cleave RNA or DNA substrates, and more preferably cleave RNA substrates. Ribozymes typically cleave nucleic acid substrates through recognition and binding of the target substrate with subsequent cleavage. This recognition is often based mostly on canonical or non-canonical base pair interactions. This property makes ribozymes particularly good candidates for target specific cleavage of nucleic acids because recognition of the target substrate is based on the target substrate's sequence. Representative examples of how to make and use ribozymes to catalyze a variety of different reactions can be found in the following non-limiting list of United States patents: 5,646,042, 5,693,535, 5,731,295, 5,811,300, 5,837,855, 5,869,253, 5,877,021, 5,877,022, 5,972,699, 5,972,704, 5,989,906, and 6,017,756.

### 9. TRIPLEX FORMING FUNCTIONAL NUCLEIC ACID MOLECULES

Also disclosed are triplex forming functional nucleic acid molecules that interact with the disclosed polynucleotides. Triplex forming functional nucleic acid molecules are molecules that can interact with either double-stranded or single-stranded nucleic acid. When triplex molecules interact with a target region, a structure called a triplex is formed, in which there are three strands of DNA forming a complex dependenton both Watson-Crick and Hoogsteen base-pairing. Triplex molecules are preferred because they can bind target regions with high affinity and specificity. It is preferred that the triplex forming molecules bind the target molecule with a k_{d} less than 10⁻⁶, 10⁻⁸, 10⁻¹⁰, or 10⁻¹². Representative examples of how to make and use triplex forming molecules to bind a variety of different target molecules can be found in the following non-limiting list of United States patents: 5,176,996, 5,645,985, 5,650,316, 5,683,874, 5,693,773, 5,834,185, 5,869,246, 5,874,566, and 5,962,426.

### 10. EXTERNAL GUIDE SEQUENCES

Also disclosed are external guide sequences that form a complex with the disclosed polynucleotides. External guide sequences (EGSs) are molecules that bind a target nucleic acid molecule forming a complex, and this complex is recognized by RNase P, which cleaves the target molecule. EGSs can be designed to specifically target a RNA molecule of choice. RNAse P aids in processing transfer RNA (tRNA) within a cell. Bacterial RNAse P can be recruited to cleave virtually any RNA sequence by using an EGS that causes the target RNA:EGS complex to mimic the natural tRNA substrate. (WO 92/03566 by Yale, and Forster and Altman, Science 238:407-409 (1990)).

Similarly, eukaryotic EGS/RNAse P-directed cleavage of RNA can be utilized to cleave desired targets within eukaryotic cells. (Yuan et al., Proc. Natl. Acad. Sci. USA 89:8006-8010 (1992); WO 93/22434 by Yale; WO 95/24489 by Yale; Yuan and Altman, EMBO J 14:159-168 (1995), and Carrara et al., Proc. Natl. Acad. Sci. (USA) 92:2627-2631 (1995)). Representative examples of how to make and use EGS molecules to facilitate cleavage of a variety of different target molecules can be found in the following non-limiting list of United States patents: 5,168,053, 5,624,824, 5,683,873, 5,728,521, 5,869,248, and 5,877,162.

### 11. PEPTIDE NUCLEIC ACID

Also disclosed are polynucleotides that contain peptide nucleic acids (PNAs) compositions. PNA is a DNA mimic in which the nucleobases are attached to a pseudopeptide backbone (Good and Nielsen, Antisense Nucleic Acid Drug Dev. 1997; 7(4) 431-37). PNA is able to be utilized in a number of methods that traditionally have used RNA or DNA. Often PNA sequences perform better in techniques than the corresponding RNA or DNA sequences and have utilities that are not inherent to RNA or DNA. A review of PNA including methods of making, characteristics of, and methods of using, is provided by Corey (Trends Biotechnol 1997 June; 15(6):224-9). As such, in certain embodiments, one may prepare PNA sequences that are complementary to one or more portions of an mRNA sequence based on the disclosed polynucleotides, and such PNA compositions may be used to regulate, alter, decrease, or reduce the translation of the disclosed polynucleotides transcribed mRNA, and thereby alter the level of the disclosed polynucleotide's activity in a host cell to which such PNA compositions have been administered.

PNAs have 2-aminoethyl-glycine linkages replacing the normal phosphodiester backbone of DNA (Nielsen et al., Science Dec. 6, 1991; 254(5037):1497-500; Hanvey et al., Science. Nov. 27, 1992; 258(5087):1481-5; Hyrup and Nielsen, Bioorg Med Chem. 1996 January; 4(1):5-23). This chemistry has three important consequences: firstly, in contrast to DNA or phosphorothioate oligonucleotides, PNAs are neutral molecules; secondly, PNAs are achiral, which avoids the need to develop a stereoselective synthesis; and thirdly, PNA synthesis uses standard Boc or Fmoc protocols for solid-phase peptide synthesis, although other methods, including a modified Merrifield method, have been used.

PNA monomers or ready-made oligomers are commercially available from PerSeptive Biosystems (Framingham, Mass.). PNA syntheses by either Boc or Fmoc protocols are straightforward using manual or automated protocols (Norton et al., Bioorg Med Chem. 1995 April; 3(4):437-45). The manual protocol lends itself to the production of chemically modified PNAs or the simultaneous synthesis of families of closely related PNAs.

As with peptide synthesis, the success of a particular PNA synthesis will depend on the properties of the chosen sequence. For example, while in theory PNAs can incorporate any combination of nucleotide bases, the presence of adjacent purines can lead to deletions of one or more residues in the product. In expectation of this difficulty, it is suggested that, in producing PNAs with adjacent purines, one should repeat the coupling of residues likely to be added inefficiently. This should be followed by the purification of PNAs by reverse-phase high-pressure liquid chromatography, providing yields and purity of product similar to those observed during the synthesis of peptides.

Modifications of PNAs for a given application may be accomplished by coupling amino acids during solid-phase synthesis or by attaching compounds that contain a carboxylic acid group to the exposed N-terminal amine. Alternatively, PNAs can be modified after synthesis by coupling to an introduced lysine or cysteine. The ease with which PNAs can be modified facilitates optimization for better solubility or for specific functional requirements. Once synthesized, the identity of PNAs and their derivatives can be confirmed by mass spectrometry. Several studies have made and utilized modifications of PNAs (for example, Norton et al., Bioorg Med Chem. 1995 April; 3(4):437-45; Petersen et al., J Pept Sci. 1995 May-June; 1(3):175-83; Orum et al., Biotechniques. 1995 September; 19(3):472-80; Footer et al., Biochemistry. Aug. 20, 1996; 35(33): 10673-9; Griffith et al., Nucleic Acids Res. Aug. 11, 1995; 23(15):3003-8; Pardridge et al., Proc Natl Acad Sci USA. Jun. 6, 1995; 92(12):5592-6; Boffa et al., Proc Natl Acad Sci USA. Mar. 14, 1995; 92(6):1901-5; Gambacorti-Passerini et al., Blood. Aug. 15, 1996; 88(4):1411-7; Armitage et al., Proc Natl Acad Sci USA. Nov. 11, 1997; 94(23):12320-5; Seeger et al., Biotechniques. 1997 September; 23(3):512-7). U.S. Pat. No. 5,700,922 discusses PNA-DNA-PNA chimeric molecules and their uses in diagnostics, modulating protein in organisms, and treatment of conditions susceptible to therapeutics.

Methods of characterizing the antisense binding properties of PNAs are discussed in Rose (Anal Chem. Dec. 15, 1993; 65(24):3545-9) and Jensen et al. (Biochemistry. Apr. 22, 1997; 36(16):5072-7). Rose uses capillary gel electrophoresis to determine binding of PNAs to their complementary oligonucleotide, measuring the relative binding kinetics and stoichiometry. Similar types of measurements were made by Jensen *et al.* using BIAcore™ technology.

Other applications of PNAs that have been described and will be apparent to the skilled artisan include use in DNA strand invasion, antisense inhibition, mutational analysis, enhancers of transcription, nucleic acid purification, isolation of transcriptionally active genes, blocking of transcription factor binding, genome cleavage, biosensors, in situ hybridization, and the like.

### 12. ANTIBODIES

As used herein, the term "antibodies" encompasses chimeric antibodies and hybrid antibodies, with dual or multiple antigen or epitope specificities, and fragments, such as F(ab')2, Fab', Fab and the like, including hybrid fragments. Thus, fragments of the antibodies that retain the ability to bind their specific antigens are provided. Such antibodies and fragments can be made by techniques known in the art and can be screened for specificity and activity according to the methods set forth in the Examples and in general methods for producing antibodies and screening antibodies for specificity and activity (See Harlow and Lane. Antibodies, A Laboratory Manual. Cold Spring Harbor Publications, New York, (1988)).

Also included within the meaning of "antibody" are conjugates of antibody fragments and antigen binding proteins (single chain antibodies) as described, for example, in U.S. Pat. No. 4,704,692.

Optionally, the antibodies are generated in other species and "humanized" for administration in humans. In one aspect, the "humanized" antibody is a human version of the antibody produced by a germ line mutant animal. Humanized forms of non-human (e.g., murine) antibodies are chimeric immunoglobulins, immunoglobulin chains or fragments thereof (such as Fv, Fab, Fab', F(ab')2, or other antigen-binding subsequences of antibodies) which contain minimal sequence derived from non-human immunoglobulin. Humanized antibodies include human immunoglobulins (recipient antibody) in which residues from a CDR of the recipient are replaced by residues from a CDR of a non-human species (donor antibody) such as mouse, rat or rabbit having the desired specificity, affinity and capacity. In one embodiment, described herein are humanized versions of an antibody, comprising at least one, two, three, four, or up to all CDRs of a monoclonal antibody that specifically binds to a protein or fragment thereof encoded by a gene set forth herein. In some instances, Fv framework residues of the human immunoglobulin are replaced by corresponding non-human residues. Humanized antibodies may also comprise residues that are found neither in the recipient antibody nor in the imported CDR or framework sequences. In general, the humanized antibody can comprise substantially all of or at least one, and typically two, variable domains, in which all or substantially all of the CDR regions correspond to those of a non-human immunoglobulin and all or substantially all of the FR regions are those of a human immunoglobulin consensus sequence. The humanized antibody optimally also can comprise at least a portion of an immunoglobulin constant region (Fc), typically that of a human immunoglobulin (Jones et al., Nature, 321:522-525 (1986); Riechmann et al., Nature, 332:323-327 (1988); and Presta, Curr. Op. Struct. Biol., 2:593-596 (1992)).

Methods for humanizing non-human antibodies are well known in the art. Generally, a humanized antibody has one or more amino acid residues introduced into it from a source that is non-human. These non-human amino acid residues are often referred to as "import" residues, which are typically taken from an "import" variable domain. Humanization can be essentially performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. Accordingly, such "humanized" antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies

### 13. PEPTIDES

An HTRA1 or ARMS2 peptide can be a recombinant HTRA1 or ARMS2 peptide, a synthetic HTRA1 or ARMS2 peptide, an HTRA1 or ARMS2 peptide, a purified HTRA1 or ARMS2 peptide, or a commercially available HTRA1 or ARMS2 peptide. An HTRA1 or ARMS2 peptide can have a non-naturally occurring sequence or can have a sequence present in any species (e.g., human, rat, or mouse). In some cases, an HTRA1 or ARMS2 peptide can contain one or more amino acid analogs or other peptidomimetics. As used herein, the term "peptidomimetics" means a molecule that mimics the biological activity of a polypeptide, but that is not peptidic in chemical nature. While, in certain aspects, a peptidomimetic can be a molecule that contains no peptide bonds (that is, amide bonds between amino acids), the term peptidomimetic can include molecules that are not completely peptidic in character, such as pseudo-peptides, semi-peptides and peptoids. Whether completely or partially non-peptide in character, peptidomimetics as described herein can provide a spatial arrangement of reactive chemical moieties that closely resembles the three-dimensional arrangement of active groups in a polypeptide. As a result of this similar active-site geometry, the peptidomimetic can exhibit biological effects that are similar to the biological activity of a polypeptide. The subunits of an HTRA1 or ARMS2 peptide may be linked by peptide bonds or other bonds such as, for example, ester or ether bonds. An HTRA1 or ARMS2 peptide can be a full-length HTRA1 or ARMS2 peptide, a precursor HTRA1 or ARMS2 peptide, or a fragment of an HTRA1 or ARMS2 peptide. In some cases, an HTRA1 or ARMS2 peptide can contain one or more modifications. For example, an HTRA1 or ARMS2 peptide can be modified to be pegylated or to contain additional amino acid sequences such as an albumin sequence (e.g., a human albumin sequence). In some cases, an HTRA1 or ARMS2 peptide can be a fusion polypeptide, such as a fusion polypeptide that contains a fragment of an albumin sequence. In some cases, an HTRA1 or ARMS2 peptide can be covalently attached to oligomers, such as short, amphiphilic oligomers that enable oral administration or improve the pharmacokinetic or pharmacodynamic profile of a conjugated HTRA1 or ARMS2 peptide. The oligomers can comprise water soluble polyethylene glycol (PEG) and lipid soluble alkyls (short chain fatty acid polymers). See, for example, International Patent Application Publication No. WO 2004/047871 which describes variant and modified peptides and peptide analogs that can be used in the treatment of a variety of conditions. In some cases, an HTRA1 or ARMS2 peptide can be fused to the Fc domain of an immunoglobulin molecule (e.g., an IgG1 molecule) such that active transport of the fusion polypeptide occurs across epithelial cell barriers via the Fc receptor.

In one aspect, administering an HTRA1 or AMRS2 peptide to a subject can be designed to produce HTRA1 or ARMS2 antibodies in the subject. For example, an HTRA1 or ARMS2 polypeptide that is foreign to a subject's immune system can be administered to the subject so that the subject produces HTRA1 or ARMS2 antibodies that can inhibit the activity of an HTRA1 or ARMS2 polypeptide in the subject. Polypeptides that can be administered to the subject include, but are not limited to: Ac-EPARSPPQPEHCEG-amide (SEQ ID No. 1), corresponding to the HTRA1 36-49 IGFBP domain; Ac-PASATVRRRAQC-amide (SEQ ID NO. 2), corresponding to the HTRA1 96-106 IGFBP domain; Ac-CGSDANTYANL-amide (SEQ ID NO. 4), corresponding to the HTRA1 119-129 Kazal domain; Ac-SRRSERLHRPPVIC-amide (SEQ ID No. 3), corresponding to the HTRA1 136-148 Kazal domain; Ac-CGQGQEDPNSLRHK-OH (SEQ ID NO. 5), corresponding to the HTRA1 155-168 Linker-protease domain; Ac-SHDRQAKGKAITKC-amide (SEQ ID NO. 6) corresponding to the HTRA1 367-379 Protease-linker domain; Ac-CPDTPAEAGGLKEN-amide (SEQ ID NO. 7), corresponding to the HTRA1 419-431 PDZ domain; or Ac-LDPGVGGEGASDKQRSKC-amide (SEQ ID NO. 8), corresponding to the ARMS2 42-58 domain. In a further aspect, a self HTRA1 or AMRS2 polypeptide can be designed to contain foreign T-cell epitopes so that administration of the polypeptide produces HTRA1 or AMRS2 antibodies that can inhibit the activity of an HTRA1 or ARMS2 polypeptide in the subject. Adjuvants such as alum can be used in combination with HTRA1 or ARMS2 polypeptides. The HTRA1 or ARMS2 peptide activity can be inhibited by any amount. For example, and not to be limiting, the peptide activity can be inhibited by about 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%, and by any percentage between about 5% and 100%.

Also described herein are methods of treating Vascular Associated Maculopathy in a subject comprising administering to the subject decoy-soluble elastin (the elastin trap). Administering the decoy elastin can prevent elastases, for example HTRA1, from binding to their normal substrate, thereby blocking their elastase activity.

Also described herein are methods of treating Vascular Associated Maculopathy, or a symptom thereof, in a subject, wherein aberrant choriocapillaris lobules are present in the eye of the subject, comprising administering to a subject a therapeutically effective amount of one or more polypeptides encoded by an HTRA1, ARMS2, CFH, or C3 protective haplotype. In one aspect, the symptom of Vascular Associaetd Maculopathy can appear in an eye of the subject. Therefore, also described herein are methods of treating a symptom of Vascular Associated Maculopathy in an eye of a subject, wherein aberrant choriocapillaris lobules are present in an eye of the subject, comprising administering to a subject a therapeutically effective amount of one or more polypeptides encoded by an HTRA1, ARMS2, CFH, or C3 protective haplotype.

Additionally, described herein are methods of treating Vascular Associated Maculopathy, or a symptom thereof, in a subject, wherein aberrant choriocapillaris lobules are present in an eye of the subject, comprising administering to a subject a therapeutically effective amount of one or more polypeptides encoded by an HTRA1, ARMS2, CFH, or C3 mutant haplotype. In one aspect, the symptom of Vascular Associaetd Maculopathy can appear in an eye of the subject. Therefore, also described herein are methods of treating a symptom of Vascular Associated Maculopathy in an eye of a subject, wherein aberrant choriocapillaris lobules are present in an eye of the subject, comprising administering to a subject a therapeutically effective amount of one or more polypeptides encoded by an HTRA1, ARMS2, CFH, or C3 mutant haplotype.

A mutant HTRA1, ARMS2, CFH, or C3 haplotype can be encoded by an HTRA1, ARMS2, CFH, or C3 nucleotide sequence with a single nucleotide polymorphism (SNP). More specifically, the HTRA1, ARMS2, CFH, or C3 nucleotide sequence can have an A allele at the rs11200638 SNP in the HTRA1 gene, a T allele at the rs10490924 SNP in the ARMS2 gene, a C allele at the rs1061170 SNP in the CFH gene, or a G allele at the rs2230199 SNP in the C3 gene.

### 14. SMALL MOLECULES

Any small molecule that targets, either directly or indirectly, HTRA1 or ARMS2 nucleic acids or peptides, can be utilized in the methods described herein. These molecules can be identified in the scientific literature, in the StarLite database available from the European Bioinformatics Institute, in DrugBank (Wishart et al. Nucleic Acids Res. 2006 Jan 1;34 (Database issue):D668-72), package inserts, brochures, chemical suppliers (for example, Sigma, Tocris, Aurora Fine Chemicals, to name a few), or by any other means, such that one of skill in the art makes the association between HTRA1 or ARMS2 and inhibition of HTRA1 and ARMS2, either direct or indirect, by a molecule. Preferred small molecules are those small molecules that have IC50 values of less than about ImM, less than about 100 micromolar, less than about 75 micromolar, less than about 50 micromolar, less than about 25 micromolar, less than about 10 micromolar, less than about 5 micromolar or less than about 1 micromolar. The half maximal inhibitory concentration (IC50) is a measure of the effectiveness of a compound in inhibiting biological or biochemical function. This quantitative measure indicates how much of a particular compound or other substance (inhibitor) is needed to inhibit a given biological process (or component of a process, i.e. an enzyme, cell, cell receptor or microorganism) by half. In other words, it is the half maximal (50%) inhibitory concentration (IC) of a substance (50% IC, or IC50).

### • METHODS OF DETERMINING THE EFFICACY OF THERAPEUTICS

Described herein are methods of determining the efficacy of a treatment of Vascular Associated Maculopathy, or a symptom thereof, in a subject diagnosed with Vascular Associated Maculopathy, or a symptom thereof,, comprising: a) determining a number of aberrant choriocapillaris lobules in an eye of the subject before beginning treatment of the disease, or symptom thereof; b) beginning treatment of the disease for an interval of time; c) determining a subsequent number of aberrant choriocapillaris lobules in the eye of step a) after the interval of time; and d) comparing the number of aberrant choriocapillaris lobules in the eye of the subject of step c) to the number of aberrant choriocapillaris lobules in the eye of the subject of step a), wherein detecting no increase or a decrease in the number of aberrant choriocapillaris lobules in the eye of the subject of step c) indicates efficacy of the treatment of Vascular Associated Maculopathy, or a symptom thereof, in the subject.

In one aspect, the methods of determining the efficacy of a treatment of Vascular Associated Maculopathy, or a symptom thereof, in a subject can comprise detecting the regrowth or regeneration of RPE cells overlying and corresponding to the choriocapillaris lobules, following treatment of Vascular Associated Maculopathy, or a symptom thereof,, wherein detecting the regrowth or regeneration of the RPE cells indicates efficacy of the treatment. Various procedures known to persons of ordinary skill in the art can be utilized to detect the regrowth or regeneration of RPE cells, including, but not limited to, autofluorescent imaging techniques, infrared imaging techniques, optical coherence tomography (OCT), Stratus optical coherence tomography (Stratus OCT), Fourier-domain optical coherence tomography (Fd-OCT), two-photon-excited fluorescence (TPEF) imaging, adaptive optics scanning laser ophthalmoscopy (AOSLO), scanning laser ophthalmoscopy, near-infrared imaging combined with spectral domain optical coherence tomography (SD-OCT), color fundus photography, fundus autofluorescence imaging, red-free imaging, fluorescein angiography, indocyanin green angiography, multifocal electroretinography (ERG) recording, microperimetry, color Doppler optical coherence tomography (CDOCT), visual field assessment, the Heidelberg Spectralis, the Zeiss Cirrus, the Topcon 3D OCT 2000, the Optivue RTVue SD-OCT, the Opko OCT SLO, the NIDEK F-10, or the Optopol SOCT Copernicus HR.

In a further aspect, the methods of determining the efficacy of a treatment of Vascular Associated Maculopathy, or a symptom thereof, in a subject can comprise detecting increased perfusion of the choriocapillaris lobules, following treatment of Vascular Associated Maculopathy, or a symptom thereof,, wherein detecting increased perfusion of the choriocapillaris lobules indicates efficacy of the treatment. Various procedures known to persons of ordinary skill in the art can be utilized to detect the increased perfusion of the choriocapillaris lobules, including, but not limited to, autofluorescent imaging techniques, infrared imaging techniques, optical coherence tomography (OCT), Stratus optical coherence tomography (Stratus OCT), Fourier-domain optical coherence tomography (Fd-OCT), two-photon-excited fluorescence (TPEF) imaging, adaptive optics scanning laser ophthalmoscopy (AOSLO), scanning laser ophthalmoscopy, near-infrared imaging combined with spectral domain optical coherence tomography (SD-OCT), color fundus photography, fundus autofluorescence imaging, red-free imaging, fluorescein angiography, indocyanin green angiography, multifocal electroretinography (ERG) recording, microperimetry, color Doppler optical coherence tomography (CDOCT), visual field assessment, the Heidelberg Spectralis, the Zeiss Cirrus, the Topcon 3D OCT 2000, the Optivue RTVue SD-OCT, the Opko OCT SLO, the NIDEK F-10, or the Optopol SOCT Copernicus HR..

In yet a further aspect, the methods of determining the efficacy of a treatment of Vascular Associated Maculopathy, or a symptom thereof,in a subject can comprise detecting a return of complement pathway, HTRA1, or ARMS2 associated activity, such as expression level, biochemical activity (e.g., enzymatic activity of a complement component), or serum auto antibodies against complement pathway, HTRA1, or ARMS2 associated molecules, from abnormal levels to or toward normal levels, following treatment of Vascular Associated Maculopathy, or a symptom thereof, wherein detecting a return of complement pathway, HTRA1, or ARMS2 associated activity to or toward normal levels indicates efficacy of the treatment.

As used herein, the term "diagnosed" means having been subjected to a physical examination, including but not limited to genetic examination, by a person of skill, for example, a physician, and found to have a condition that can be diagnosed or treated by the compounds, compositions, or methods described herein. For example, "diagnosed with Vascular Associated Maculopathy, or a symptom thereof," means having been subjected to a physical examination by a person of skill, for example, a physician utilizing the methods described herein, and found to have a condition that can be diagnosed as Vascular Associated Maculopathy, or a symptom thereof.

As used herein, the term "diagnosed" can also mean having examined a subject's DNA, RNA, or in some cases, protein, to assess the presence or absence of the various SNPs described herein (and, in one aspect, other SNPs and genetic or behavioral characteristics) so as to determine whether the subject has Vascular Associated Maculopathy, or a symptom thereof.

As used herein, "no increase" or "a decrease" means that there is no significant or perceptible increase in the number or size of the aberrant choriocapillaris lobules when an eye is examined by a person of ordinary skill using ophthalmological procedures well known in the art, such as the procedures described herein. Treatment can be in the form of administering one or more therapeutic agents to the subject alone or in combination with other forms of treatments including, but not limited to, exercise, reducing or eliminating smoking, reducing or eliminating alcohol intake, reducing stress, weight loss, controlling blood pressure, or improving diet and nutritional intake.

Thus, a person of skill in the art can determine whether a course of treatment of Vascular Associated Maculopathy, or a symptom thereof,in a subject is effective by following the subject at various time intervals and examining the subject's eyes to compare the number or size aberrant choriocapillaris lobules at each examination to the number or size of aberrant choriocapillaris lobules determined at the initial examination when Vascular Associated Maculopathy, or a symptom thereof, in the subject was first diagnosed. A person of skill in the art can photograph and document the appearance of the subject's macula in one or both eyes using ophthalmological procedures well known in the art, such as the procedures described herein, and then measure the size of the aberrant choriocapillaris lobules or count the number of aberrant choriocapillaris lobules in the macula. When there is no increase in the number or size of the aberrant choriocapillaris lobules, a person of skill in the art can determine that the compositions and methods of treatment are effective.

Also described herein are methods of determining the efficacy of a treatment of a Vascular Associated Maculopathy, or a symptom thereof, in a subject diagnosed with Vascular Associated Maculopathy, comprising: a) determining a number of aberrant choriocapillaris lobules in an eye of the subject before beginning treatment of Vascular Associated Maculopathy, or a symptom thereof,; b) beginning treatment of Vascular Associated Maculopathy, or a symptom thereof, for an interval of time; c) determining a subsequent number of aberrant choriocapillaris lobules in the eye of step a) after the interval of time; and d) comparing the number of aberrant choriocapillaris lobules in the eye of the subject of step c) to the number of aberrant choriocapillaris lobules in the eye of the subject of step a), wherein detecting an increase in the number of aberrant choriocapillaris lobules in the eye of the subject of step c) indicates an agent not effective in treating Vascular Associated Maculopathy, or a symptom thereof, in the subject.

In one aspect, the methods of determining the efficacy of a treatment of Vascular Associated Maculopathy, or a symptom thereof, in a subject can comprise detecting the regrowth or regeneration of RPE cells overlying and corresponding to the choriocapillaris lobule, following treatment of Vascular Associated Maculopathy, or a symptom thereof,, wherein detecting no regrowth or regeneration of the RPE cells indicates an agent not effective in treating Vascular Associated Maculopathy, or a symptom thereof. Various procedures known to persons of ordinary skill in the art can be utilized to detect the regrowth or regeneration of RPE cells, including, but not limited to, autofluorescent imaging techniques, infrared imaging techniques, optical coherence tomography (OCT), Stratus optical coherence tomography (Stratus OCT), Fourier-domain optical coherence tomography (Fd-OCT), two-photon-excited fluorescence (TPEF) imaging, adaptive optics scanning laser ophthalmoscopy (AOSLO), scanning laser ophthalmoscopy, near-infrared imaging combined with spectral domain optical coherence tomography (SD-OCT), color fundus photography, fundus autofluorescence imaging, red-free imaging, fluorescein angiography, indocyanin green angiography, multifocal electroretinography (ERG) recording, microperimetry, color Doppler optical coherence tomography (CDOCT), visual field assessment, the Heidelberg Spectralis, the Zeiss Cirrus, the Topcon 3D OCT 2000, the Optivue RTVue SD-OCT, the Opko OCT SLO, the NIDEK F-10, or the Optopol SOCT Copernicus HR.

In a further aspect, the methods of determining the efficacy of a treatment of Vascular Associated Maculopathy, or a symptom thereof, in a subject can comprise detecting no increase in perfusion of the choriocapillaris lobules, following treatment of Vascular Associated Maculopathy, or a symptom thereof,, wherein detecting no increase in perfusion of the choriocapillaris lobules indicates an agent not effective in treating Vascular Associated Maculopathy, or a symptom thereof. Various procedures known to persons of ordinary skill in the art can be utilized to detect the increased perfusion of the choriocapillaris lobules, including, but not limited to, autofluorescent imaging techniques, infrared imaging techniques, optical coherence tomography (OCT), Stratus optical coherence tomography (Stratus OCT), Fourier-domain optical coherence tomography (Fd-OCT), two-photon-excited fluorescence (TPEF) imaging, adaptive optics scanning laser ophthalmoscopy (AOSLO), scanning laser ophthalmoscopy, near-infrared imaging combined with spectral domain optical coherence tomography (SD-OCT), color fundus photography, fundus autofluorescence imaging, red-free imaging, fluorescein angiography, indocyanin green angiography, multifocal electroretinography (ERG) recording, microperimetry, color Doppler optical coherence tomography (CDOCT), visual field assessment, the Heidelberg Spectralis, the Zeiss Cirrus, the Topcon 3D OCT 2000, the Optivue RTVue SD-OCT, the Opko OCT SLO, the NIDEK F-10, or the Optopol SOCT Copernicus HR.

In yet a further aspect, the methods of determining the efficacy of a treatment of Vascular Associated Maculopathy, or a symptom thereof, in a subject can comprise detecting no return of complement pathway, HTRA1, or ARMS2 associated activity, such as expression level, biochemical activity (e.g., enzymatic activity of a complement component), or serum auto antibodies against complement pathway, HTRA1, or ARMS2 associated molecules, from abnormal levels to or toward normal levels, following treatment of Vascular Associated Maculopathy, or a symptom thereof,, wherein detecting no return of complement pathway, HTRA1, or ARMS2 associated activity to or toward normal levels indicates an agent not effective in treating Vascular Associated Maculopathy, or a symptom thereof.

A person of skill in the art can determine whether a course of treatment of Vascular Associated Maculopathy, or a symptom thereof, in a subject is not effective by following the subject at various time intervals and examining the macula of the subject's eyes to compare the number or size aberrant choriocapillaris lobules at each examination to the number or size of aberrant choriocapillaris lobules determined at the initial examination, when Vascular Associated Maculopathy, or a symptom thereof, was first diagnosed. A person of skill in the art can photograph and document the appearance of the subject's macula in one or both eyes using ophthalmological procedures well known in the art, such as the procedures described herein, and then measure the size of the aberrant choriocapillaris lobules or count the number of aberrant choriocapillaris lobules in the macula. When there an increase in the number or size of the aberrant choriocapillaris lobules, a person of skill in the art can determine that the compositions and methods of treatment are not effective.

Furthermore, described herein are methods of screening for an agent or combination of agents effective in treating Vascular Associated Maculopathy, or a symptom thereof, in a subject diagnosed with Vascular Associated Maculopathy, or a symptom thereof, comprising: a) determining a number or size of aberrant choriocapillaris lobules in an eye of the subject; b) administering an agent or combination of agents to the subject for an interval of time; c) determining a subsequent number or size of aberrant choriocapillaris lobules in the eye of step a) after the interval of time; and d) comparing the number or size of aberrant choriocapillaris lobules in the eye of the subject of step c) to the number or size of aberrant choriocapillaris lobules in the the eye of the subject of step a), wherein detecting no increase in the number or size of aberrant choriocapillaris lobules in the eye of the subject of step c) indicates an agent or combination of agents effective in treating Vascular Associated Maculopathy, or a symptom thereof, in the subject. The methods of screening described herein can also be used to screen for combinations of therapeutic agents in combination with other treatments including, but not limited to, exercise, reducing or eliminating smoking, reducing or eliminating alcohol intake, reducing stress, weight loss, controlling blood pressure, or improving diet and nutritional intake.

Also described herein are methods of screening for an agent or combination of agents effective in (i) treating Vascular Associated Maculopathy; (ii) treating one or more symptoms associated with Vascular Associated Maculopathy; (iii) treating severe maculopathy or last stage maculopathy; or (iv) resolving aberrant choriocappilaris lobules.

Furthermore, described herein are methods of screening for an agent or combination of agents effective in (i) treating Vascular Associated Maculopathy; (ii) treating one or more symptoms associated with Vascular Associated Maculopathy; (iii) treating severe maculopathy or last stage maculopathy; or (iv) resolving aberrant choriocappilaris lobules in a subject diagnosed with (i), (ii), (iii) or (iv) comprising: a) determining a number or size of aberrant choriocapillaris lobules in an eye of the subject; b) administering an agent or combination of agents to the subject for an interval of time; c) determining a subsequent number or size of aberrant choriocapillaris lobules in the eye of step a) after the interval of time; and d) comparing the number or size of aberrant choriocapillaris lobules in the eye of the subject of step c) to the number or size of aberrant choriocapillaris lobules in the eye of the subject of step a), wherein detecting no increase in the number or size of aberrant choriocapillaris lobules in the eye of the subject of step c) indicates an agent or combination of agents effective in (i) treating Vascular Associated Maculopathy; (ii) treating one or more symptoms associated with Vascular Associated Maculopathy; (iii) treating severe maculopathy or last stage maculopathy; or (iv) resolving aberrant choriocappilaris lobules in the subject.

Also described herein are methods of enhancing clinical trials comprising choosing appropriate patient populations for those clinical trials. In one aspect, the methods can be used to ensure patients are identified to participate in clinical trials based upon whether or not they are responsive to the experimental therapeutic agent or agents being studied. The methods can comprise monitoring the condition of subjects receiving treatment for Vascular Associated Maculopathy, or a symptom thereof. A successful treatment outcome can be indicated by return of complement pathway, HTRA1, or ARMS2 associated activity, such as expression level, biochemical activity (e.g., enzymatic activity of a complement component), or serum auto antibodies against complement pathway, HTRA1, or ARMS2 associated molecules, from abnormal levels to or toward normal levels. In one aspect, the methods can comprise measuring an initial value for the level of abnormal activity (e.g., abnormal presence of an autoantibody, or abnormal levels of complement pathway, HTRA1, or ARMS2 molecules) before the subject has received treatment. Repeat measurements can then be made over a period of time. For example, and not to be limiting, that period of time can be about 1 day, 2 days, 5 days, 1 week, 2 weeks, 3 weeks, 1 month, 2 months, 3 months, 4 months, 6 months, 9 months, 1 year, or greater than 1 year. If the initial level is elevated relative to the mean level in a control population, a significant reduction in level in subsequent measurements can indicate a positive treatment outcome. Likewise, if the initial level of a measure marker is reduced relative to the mean in a control population, a significant increase in measured levels relative to the initial level can signal a positive treatment outcome. Subsequently measured levels are considered to have changed significantly relative to initial levels if a subsequent measured level differs by more than one standard deviation from the mean of repeat measurements of the initial level. If monitoring reveals a positive treatment outcome, that indicates a patient that can be chosen to participate in a clinical trial for that particular therapeutic agent or agents. If monitoring reveals a negative treatment outcome, that indicates a patient that should not be chosen to participate in a clinical trial for that particular therapeutic agent or agents.

### D. THERAPEUTIC COMPOSITIONS

Described herein are the components to be used to prepare the compositions described herein as well as the compositions themselves to be used within the methods described herein. These and other materials are described herein, and it is understood that when combinations, subsets, interactions, groups, etc. of these materials are disclosed that while specific reference of each various individual and collective combinations and permutation of these compounds can not be explicitly disclosed, each is specifically contemplated and described herein. For example, if a particular indicator reagent is disclosed and discussed and a modification of that indicator reagent is also discussed, specifically contemplated is each and every combination and permutation of the indicator reagent and the modifications that are possible unless specifically indicated to the contrary. Thus, if a class of indicator reagents A, B, and C are disclosed as well as a class of indicator reagents D, E, and F and an example of a combination indicator reagent, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the compositions described herein. Thus, if there are a variety of additional steps that can be performed, it is understood that each of these additional steps can be performed with any specific embodiment or combination of embodiments of the methods described herein.

The compositions described herein have certain functions, such as treating Vascular Associated Maculopathy or ameliorating a symptom associated with Vascular Associated Maculopathy. Described herein are certain structural requirements for performing the disclosed functions, and it is understood that there are a variety of structures that can perform the same function that are related to the disclosed structures, and that these structures will typically achieve the same result, for example treating Vascular Associated Maculopathy, or a symptom thereof.

In one aspect, the therapeutic agents to treat Vascular Associated Maculopathy, or a symptom thereof, include biological therapies such as gene therapy. For example, DNA containing all or part of the coding sequence for a HTRA1 or ARMS2 polypeptide can be incorporated into a vector for expression of the encoded polypeptide in suitable host cells. A large number of vectors, including bacterial, yeast, and mammalian vectors, are known in the art for replication and/or expression in various host cells or cell-free systems, and may be used for gene therapy. Expression vectors can be any nucleotide construction used to deliver genes into cells (e.g., a plasmid), or as part of a general strategy to deliver genes, e.g., as part of recombinant retrovirus or adenovirus (Ram et al. Cancer Res. 53:83-88, (1993)). For example, disclosed herein are expression vectors comprising an isolated polynucleotide comprising a sequence of one or more of genes described herein, operably linked to a control element.

As used herein, plasmid or viral vectors are agents that transport the disclosed nucleic acids, such as an isolated polynucleotide capable of encoding one or more polypeptides disclosed herein into the cell without degradation and include a promoter yielding expression of the gene in the cells into which it is delivered. In some embodiments the isolated polynucleotides disclosed herein are derived from either a virus or a retrovirus.

### 1. PHARMACEUTICAL CARRIERS/DELIVERY OF PHARMACEUTICAL PRODUCTS

The compositions described herein (alternatively referred to as compositions) can also be administered *in vivo* in a pharmaceutically acceptable carrier. By "pharmaceutically acceptable" is meant a material that is not biologically or otherwise undesirable, i.e. the material may be administered to a subject along with an agent, for example, a peptide, described herein, without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

The compositions can be administered by oral administration, transdermal administration, administration by inhalation, nasal administration, topical administration, intravaginal administration, ophthalmic administration, intraaural administration, intracerebral administration, rectal administration, and parenteral administration, including injectable such as intravenous administration, intra-arterial administration, intramuscular administration, and subcutaneous administration. As used herein, "topical intranasal administration" means delivery of the compositions into the nose and nasal passages through one or both of the areas and can comprise delivery by a spraying mechanism or droplet mechanism, or through aerosolization. Administration of the compositions by inhalant can be through the nose or mouth via delivery by a spraying or droplet mechanism. Delivery can also be directly to any area of the respiratory system (e.g., lungs) via intubation. The compositions can be administered ophthalmicly. Ophthalmic administration can be accomplished by a variety of means known to a person of skill in the art. For example, and not to be limiting, the agent or agents can be provided to an eye of a subject in need thereof through the placement of a cream, an ointment, or a liquid drop preparation onto the inner eyelid of the subject, through the use of a mist sprayed onto the eye of the subject, or through intravitreal injection. Ophthalmic administration can further include, but is not limited to, topical administration, subconjunctival administration, sub-Tenon's administration, epibulbar administration, retrobulbar administration, intra-orbital administration, and intraocular administration, which includes intravitreal administration.

In one aspect, ophthalmic administration of an agent or agents can be accomplished through the use of systemic delivery, such intravenous delivery, unidirectional episcleral implant, hollow microneedles, solid coated microneedles, free-floating intravitreal implant, or scleral-fixated intravitreal implant. Ophthalmic administration of an agent or agents can also be accomplished through the use of topical iontophoresis. Iontophoresis is a noninvasive method of delivering compounds into the eye. It can be performed by applying a small electrical current that has the same charge as the compound to create replulsive electromotive forces that enable delivery of the compound to the anterior or posterior segment of the eye. Edelhauser et al., Ophthalmie Drug Delivery Systems for the Treatment of Retinal Diseases: Basic Research to Clinical Applications, IOVS 2010: 51(11) 5403-5419, which is herein incoprated in its entirety by this reference, describes these various methods of ophthalmic administration.

The exact amount of the compositions required will vary from subject to subject, depending on the species, age, weight and general condition of the subject, the severity of the disease, its mode of administration and the like. Thus, it is not possible to specify an exact amount for every composition. However, an appropriate amount can be determined by one of ordinary skill in the art using only routine experimentation given the teachings herein.

Parenteral administration of the composition, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained. *See, e.g.,* U.S. Patent 3,610,795. In a further aspect, the disclosed compositions are administered by I.V., by injection and/or an I.V. drip.

### a. PHARMACEUTICALLY ACCEPTABLE CARRIERS

The compositions described herein can be used therapeutically in combination with a pharmaceutically acceptable carrier.

Suitable carriers and their formulations are described in Remington: The Science and Practice of Pharmacy (19th ed.) ed. A.R. Gennaro, Mack Publishing Company, Easton, PA 1995. Typically, an appropriate amount of a pharmaceutically-acceptable salt is used in the formulation to render the formulation isotonic. Examples of the pharmaceutically-acceptable carrier include, but are not limited to, saline, Ringer's solution and dextrose solution. The pH of the solution can be from about 5 to about 8, and more preferably from about 7 to about 7.5. Further carriers include sustained release preparations such as semipermeable matrices of solid hydrophobic polymers containing the peptide, which matrices are in the form of shaped articles, e.g., films, liposomes, nanoparticles, or microparticles. It will be apparent to those persons skilled in the art that certain carriers may be more preferable depending upon, for instance, the route of administration and concentration of composition being administered.

Pharmaceutical carriers are known to those skilled in the art. These most typically would be standard carriers for administration of drugs to humans, including solutions such as sterile water, saline, Ringer's solution, dextrose in water, balanced salt solutions, and buffered solutions at physiological pH. The pharmaceutical carrier employed can also be, for example, a solid, liquid, or gas. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen.

Pharmaceutical compositions may include carriers, thickeners, diluents, buffers, preservatives, surface active agents and the like in addition to the one or more molecules of choice. Pharmaceutical compositions may also include one or more active ingredients such as antimicrobial agents, anti-inflammatory agents, anesthetics, and the like.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

Formulations for topical administration may include ointments, lotions, creams, gels, drops, suppositories, sprays, liquids and powders. Conventional pharmaceutical carriers, aqueous, powder or oily bases, thickeners and the like may be necessary or desirable.

In preparing the compositions for oral dosage form, any convenient pharmaceutical media can be employed. For example, water, glycols, oils, alcohols, flavoring agents, preservatives, coloring agents and the like can be used to form oral liquid preparations such as suspensions, elixirs and solutions; while carriers such as starches, sugars, microcrystalline cellulose, diluents, granulating agents, lubricants, binders, disintegrating agents, and the like can be used to form oral solid preparations such as powders, capsules and tablets. Because of their ease of administration, tablets and capsules are the preferred oral dosage units whereby solid pharmaceutical carriers are employed. Optionally, tablets can be coated by standard aqueous or nonaqueous techniques

Some of the compositions may potentially be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono, di-, trialkyl and aryl amines and substituted ethanolamines.

Certain materials, compounds, compositions, and components described herein can be obtained commercially or readily synthesized using techniques generally known to those of skill in the art.

### b. DOSAGES

Effective dosages and schedules for administering the compositions described herein may be determined empirically, and making such determinations is within the skill in the art. The dosage ranges for the administration of the compositions are those large enough to produce the desired effect in which the symptoms of the disorder are affected. The dosage should not be so large as to cause adverse side effects, such as unwanted cross-reactions, anaphylactic reactions, and the like. Generally, the dosage will vary with the age, condition, sex and extent of the Vascular Associated Maculopathy, or a symptom thereof, in the patient, route of administration, or whether other drugs are included in the regimen, and can be determined by one of skill in the art. The dosage can be adjusted by the individual physician in the event of any contraindication. Dosage can vary and can be administered in one or more dose administrations daily for one or several days. Guidance can be found in the literature for appropriate dosages for given classes of pharmaceutical products, particularly peptides. Examples of such guidance can be found throughout the literature.

In one aspect, the dosage level can be about 0.1 to about 250 mg/kg per day; more preferably 0.5 to 100 mg/kg per day. A suitable dosage level can be about 0.01 to 250 mg/kg per day, about 0.05 to 100 mg/kg per day, or about 0.1 to 50 mg/kg per day. Within this range the dosage can be 0.05 to 0.5, 0.5 to 5.0 or 5.0 to 50 mg/kg per day. For oral administration, the compositions are preferably provided in the from of tablets containing 1.0 to 1000 miligrams of the active ingredient, particularly 1.0, 5.0, 10, 15, 20, 25, 50, 75, 100, 150, 200, 250, 300, 400, 500, 600, 750, 800, 900 and 1000 milligrams of the active ingredient for the symptomatic adjustment of the dosage of the patient to be treated. The compound can be administered on a regimen of 1 to 4 times per day, preferably once or twice per day. This dosing regimen can be adjusted to provide the optimal therapeutic response.

It is understood, however, that the specific dose level for any particular patient will depend upon a variety of factors. Such factors include the age, body weight, general health, sex, and diet of the patient. Other factors include the time and route of administration, rate of excretion, drug combination, and the type and severity of the particular disease undergoing therapy.

### • ARRAYS

Also described herein are arrays comprising polynucleotides capable of specifically hybridizing to a HTRAlor ARMS2 or a variant HTRA1 or ARMS2 encoding nucleic acid. For example, described are arrays comprising polynucleotides capable of specifically hybridizing to one or more risk or protective SNPs described in Table 1 and Figures 39-42 herein. Also disclosed are arrays comprising polynucleotides capable of specifically hybridizing to one or more of the one or more risk or protective SNPs described in Table 1 and Figures 39-42 herein.

Also described herein are solid supports comprising one or more polypeptides capable of specifically hybridizing to a HTRA2 or ARMS2 or a variant HTRA1 or ARMS2 peptide.

Solid supports are solid-state substrates or supports with which molecules, such as analytes and analyte binding molecules, can be associated. Analytes, such as calcifying nano-particles and proteins, can be associated with solid supports directly or indirectly. For example, analytes can be directly immobilized on solid supports. Analyte capture agents, such as capture compounds, can also be immobilized on solid supports. For example, described herein are antigen binding agents capable of specifically binding to a a HTRA1 or ARMS2 or a variant HTRAlor ARMS2 peptide.

A preferred form of solid support is an array. Another form of solid support is an array detector. An array detector is a solid support to which multiple different capture compounds or detection compounds have been coupled in an array, grid, or other organized pattern.

Solid-state substrates for use in solid supports can include any solid material to which molecules can be coupled. This includes materials such as acrylamide, agarose, cellulose, nitrocellulose, glass, polystyrene, polyethylene vinyl acetate, polypropylene, polymethacrylate, polyethylene, polyethylene oxide, polysilicates, polyglycolic acid, polylactic acid, polyorthoesters, polypropylfumerate, collagen, glycosaminoglycans, and polyamino acids. Solid-state substrates can have any useful form including thin film, membrane, bottles, dishes, fibers, woven fibers, shaped polymers, particles, beads, nanoparticles, microparticles, or a combination. Solid-state substrates and solid supports can be porous or non-porous. A preferred form for a solid-state substrate is a microtiter dish, such as a standard 96-well type. In preferred embodiments, a multiwell glass slide can be employed that normally contain one array per well. This feature allows for greater control of assay reproducibility, increased throughput and sample handling, and ease of automation.

Different compounds can be used together as a set. The set can be used as a mixture of all or subsets of the compounds used separately in separate reactions, or immobilized in an array. Compounds used separately or as mixtures can be physically separable through, for example, association with or immobilization on a solid support. An array can include a plurality of compounds immobilized at identified or predefined locations on the array. Each predefined location on the array generally can have one type of component (that is, all the components at that location are the same). Each location will have multiple copies of the component. The spatial separation of different components in the array allows separate detection and identification of the polynucleotides or polypeptides described herein.

Although preferred, it is not required that a given array be a single unit or structure. The set of compounds may be distributed over any number of solid supports. For example, at one extreme, each compound may be immobilized in a separate reaction tube or container, or on separate beads or microparticles or nanoparticles. Different modes of the disclosed method can be performed with different components (for example, different compounds specific for different proteins) immobilized on a solid support.

Some solid supports can have capture compounds, such as antibodies, attached to a solid-state substrate. Such capture compounds can be specific for calcifying nano-particles or a protein on calcifying nano-particles. Captured calcifying nano-particles or proteins can then be detected by binding of a second, detection compound, such as an antibody. The detection compound can be specific for the same or a different protein on the calcifying nano-particle.

Methods for immobilizing antibodies (and other proteins) to solid-state substrates are well established. Immobilization can be accomplished by attachment, for example, to aminated surfaces, carboxylated surfaces or hydroxylated surfaces using standard immobilization chemistries. Examples of attachment agents are cyanogen bromide, succinimide, aldehydes, tosyl chloride, avidin-biotin, photocrosslinkable agents, epoxides and maleimides. A preferred attachment agent is the heterobifunctional cross-linker N-[γ-Maleimidobutyryloxy] succinimide ester (GMBS). These and other attachment agents, as well as methods for their use in attachment, are described in Protein immobilization: fundamentals and applications, Richard F. Taylor, ed. (M. Dekker, New York, 1991); Johnstone and Thorpe, Immunochemistry In Practice (Blackwell Scientific Publications, Oxford, England, 1987) pages 209-216 and 241-242, and Immobilized Affinity Ligands; Craig T. Hermanson et al., eds. (Academic Press, New York, 1992). Antibodies can be attached to a substrate by chemically cross-linking a free amino group on the antibody to reactive side groups present within the solid-state substrate. For example, antibodies may be chemically cross-linked to a substrate that contains free amino, carboxyl, or sulfur groups using glutaraldehyde, carbodiimides, or GMBS, respectively, as cross-linker agents. In this method, aqueous solutions containing free antibodies are incubated with the solid-state substrate in the presence of glutaraldehyde or carbodiimide.

A preferred method for attaching antibodies or other proteins to a solid-state substrate is to functionalize the substrate with an amino- or thiol-silane, and then to activate the functionalized substrate with a homobifunctional cross-linker agent such as (Bis-sulfo-succinimidyl suberate (BS³) or a heterobifunctional cross-linker agent such as GMBS. For cross-linking with GMBS, glass substrates are chemically functionalized by immersing in a solution of mercaptopropyltrimethoxysilane (1% vol/vol in 95% ethanol pH 5.5) for 1 hour, rinsing in 95% ethanol and heating at 120 °C for 4 hrs. Thiol-derivatized slides are activated by immersing in a 0.5 mg/ml solution of GMBS in 1% dimethylformamide, 99% ethanol for 1 hour at room temperature. Antibodies or proteins are added directly to the activated substrate, which are then blocked with solutions containing agents such as 2% bovine serum albumin, and air-dried. Other standard immobilization chemistries are known by those of skill in the art.

Each of the components (compounds, for example) immobilized on the solid support preferably is located in a different predefined region of the solid support. Each of the different predefined regions can be physically separated from each of the other different regions. The distance between the different predefined regions of the solid support can be either fixed or variable. For example, in an array, each of the components can be arranged at fixed distances from each other, while components associated with beads will not be in a fixed spatial relationship. In particular, the use of multiple solid support units (for example, multiple beads) will result in variable distances.

Components can be associated or immobilized on a solid support at any density. Components preferably are immobilized to the solid support at a density exceeding 400 different components per cubic centimeter. Arrays of components can have any number of components. For example, an array can have at least 1,000 different components immobilized on the solid support, at least 10,000 different components immobilized on the solid support, at least 100,000 different components immobilized on the solid support, or at least 1,000,000 different components immobilized on the solid support.

Optionally, at least one address on the solid support is the sequences or part of the sequences set forth in any of the nucleic acid sequences described herein. Also disclosed are solid supports where at least one address is the sequences or portion of sequences set forth in any of the peptide sequences described herein. Solid supports can also contain at least one address is a variant of the sequences or part of the sequences set forth in any of the nucleic acid sequences described herein. Solid supports can also contain at least one address as a variant of the sequences or portion of sequences set forth in any of the peptide sequences described herein.

Also disclosed are antigen microarrays for multiplex characterization of antibody responses. For example disclosed are antigen arrays and miniaturized antigen arrays to perform large-scale multiplex characterization of antibody responses directed against the polypeptides, polynucleotides and antibodies described herein, using submicroliter quantities of biological samples as described in Robinson et al., Autoantigen microarrays for multiplex characterization of autoantibody responses, Nat Med., 8(3):295-301 (2002).

Protein variants and derivatives are well understood to those of skill in the art and can involve amino acid sequence modifications. For example, amino acid sequence modifications typically fall into one or more of three classes: substitutional, insertional or deletional variants. Polypeptide variants described herein will typically exhibit at least about 70%, 75%, 80%, 85%, 90%, 91%, 92%, 93%, 94%, 95%, 96%, 97%, 98%, or 99% or more identity (determined as described below), along their length, to the polypeptide sequences set forth herein.

### F. EXAMPLES

The following examples are put forth so as to provide those of ordinary skill in the art with a complete disclosure and description of how the compounds, compositions, articles, devices and/or methods described herein are made and evaluated. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### 1. The Nature of Aberrant Choriocapillaris Lobules

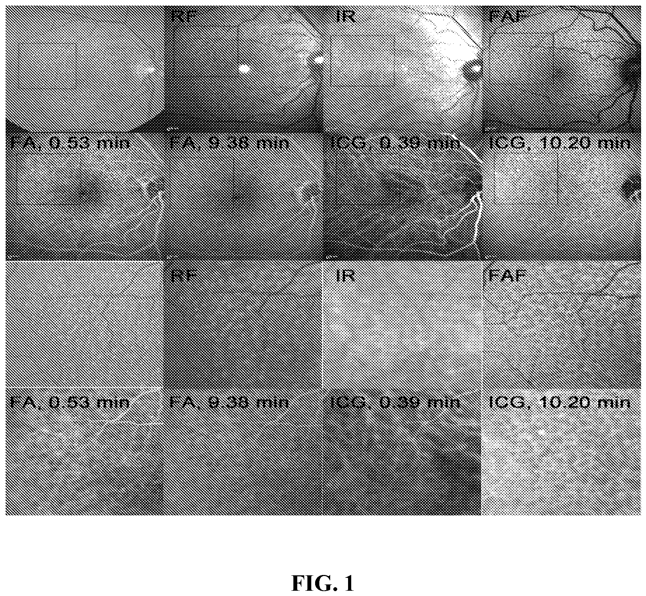

The characteristic presentation of aberrant choriocapillaris lobules on near infrared (IR), fundus autofluorescence (AF), and red-free (RF) images, along with the corresponding fluorescein angiographs (FA) and indocyanin green angiographs (ICG), is depicted in Figure 1. Aberrant choriocapillaris lobules can be observed in color and red-free fundus photographs/images, although visualization by these techniques grossly underestimates their prevalence. Aberrant choriocapillaris lobules are defined in color and red-free fundus photographs/images as regular, white to yellow, interlacing networks of relatively low-contrast.

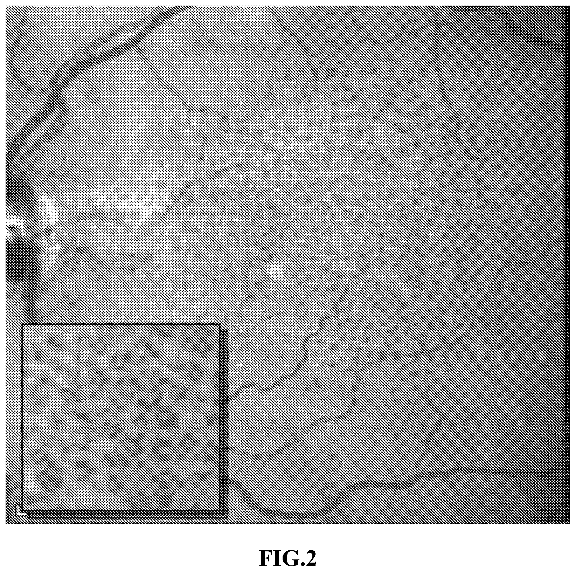

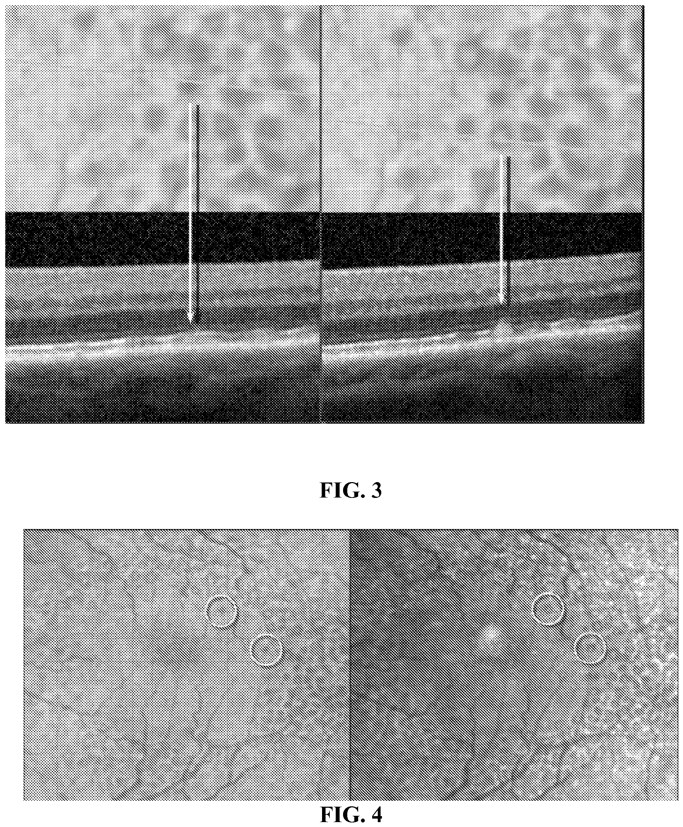

Aberrant choriocapillaris lobules are particularly distinct and most clearly visualized by near infrared imaging, combined with spectral domain optical coherence tomography (SD-OCT) (see Zweifel et al 2010), as compared to other imaging modalities. Infrared-imaged (IR) aberrant choriocapillaris lobules are defined as distinct, 150-400um diameter (or sometimes smaller or larger) units of hypo-reflectance - both with and without a distinct hyper-reflective central 'spot' -- against a background of mild hyper-reflectance (Figure 2). These are now appreciated as distinct stages in the progression of the lesion phenotype over time (Figures 3 and 4).

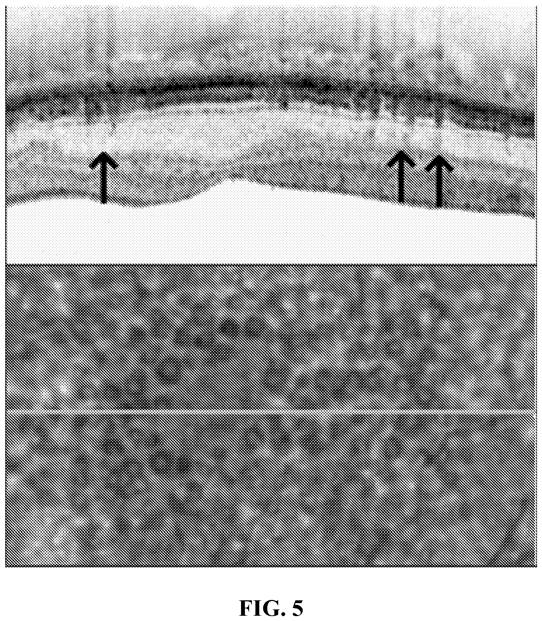

The characteristic "location" of one developmental phenotype of aberrant choriocapillaris lobules - that which appears as a donut-shaped structure by infrared images -- in the subretinal space, as visualized by SD-OCT (Heidelberg Spectralis and other instruments), is depicted (Figure 5). A key discovery here was that the 'subretinal spikes' observed in OCT images correspond to the centers of donut-shaped lobules viewed by near IR (Figure 5). Thus, when projected three dimensionally, one appreciates that the distribution of aberrant choriocapillaris lobules is not random as would be predicted in SD-OCT images alone, but rather exhibit a regular pattern of 150-400 micron diameter (sometimes larger or smaller in diameter) spherical structures that recapitulate the sizes and distribution of the choriocapillaris (Figure 5).

Assessment of aberrant choriocapillaris lobules detected by different imaging modalities was made using image registration. Individual lobules viewed by different imaging techniques had high spatial correspondence (Figure 1). Measurements of aberrant choriocapillaris lobules in near-infrared reflectance images were made using the Heidelberg HRA+OCT/Spectralis instrument.

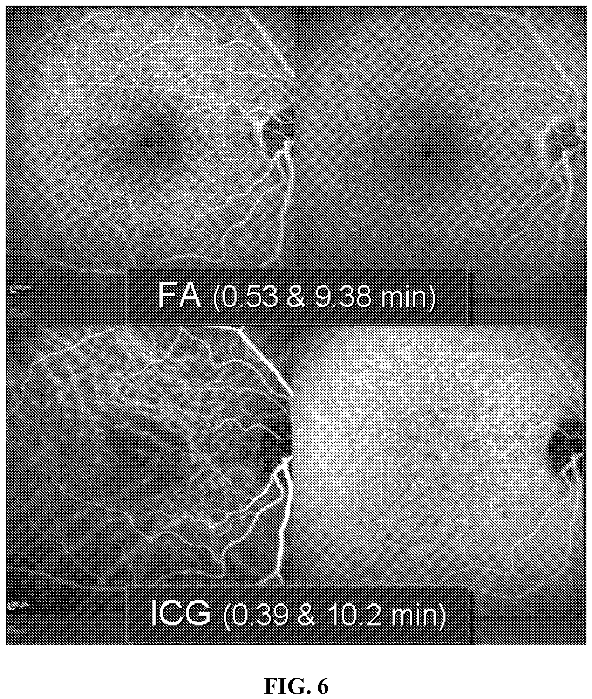

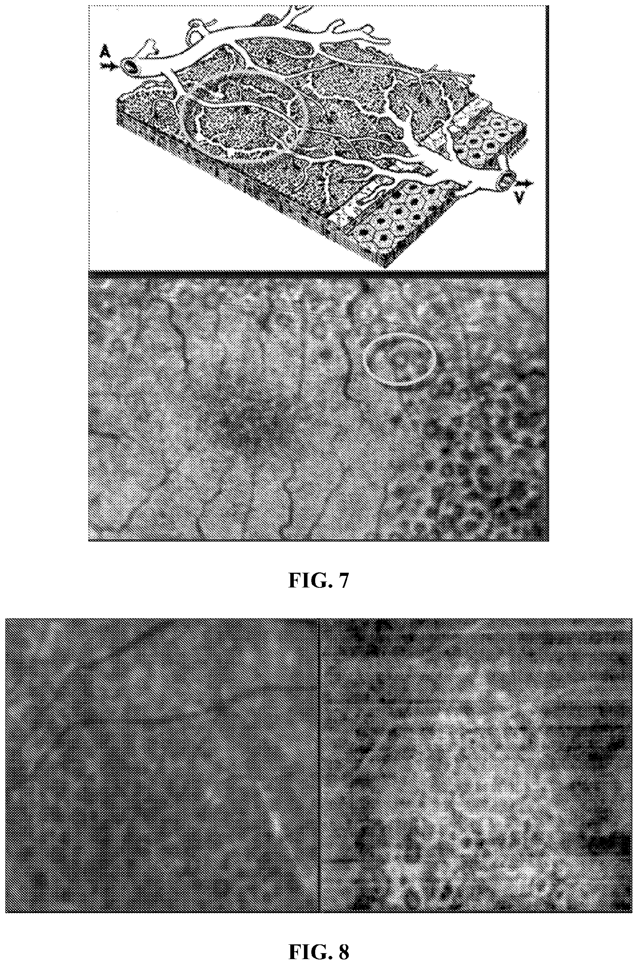

Both early filling (0.39 and 0.53 sec) and late stage (9.38 and 10.20 min) phases of the fluorescein and ICG angiographs show that the aberrant choriocapillaris lobules are characterized by late hypofluorescence, indicating poor perfusion of these choriocapillaris lobules and/or masking of the fluorescent signal (Figures 1 and 6). Importantly, the overall pattern observed in the fundus recapitulates that of the macular choriocapillaris anatomy, which is unique compared to other regions of the choroid. (Figure 7).

Various lines of evidence support the observation that aberrant choriocapillaris lobules represent poorly perfused, non-perfused, dead and/or dysfunctional macular capillary lobules. First, the general spherical morphology and sizes of individual lesions supports this. Second, high resolution OCT 'C' scans taken from patients with macular aberrant choriocapillaris lobules more precisely define the lobular nature and anatomy of aberrant choriocapillaris lobules, again recapitulating macular choriocapillaris morphology (Figure 8).

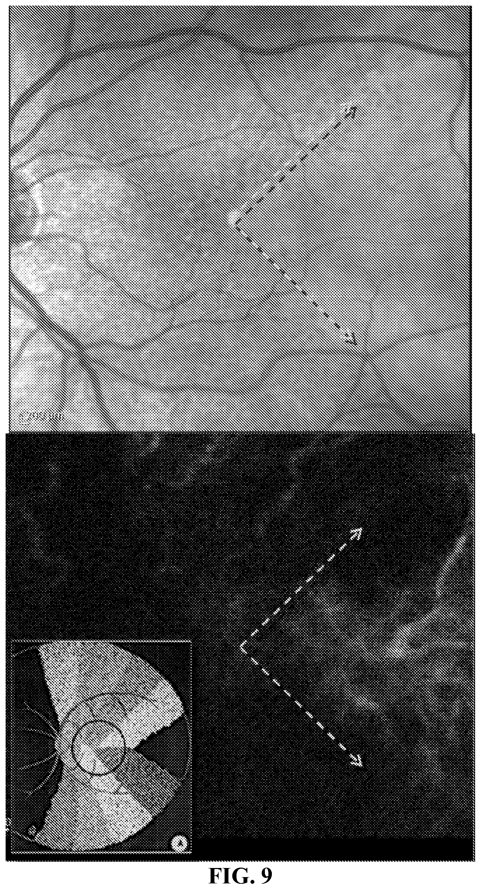

Third, more than 25 study patients have been examined that exhibit a 'segmental' distribution of aberrant choriocapillaris lobules centered on the fovea (Figure 9). These study patients often exhibit a temporal wedge of normal macula, surrounded by macula characterized by the presence of aberrant choriocapillaris lobules. On IGG angiography, filling of the choroid in the region of normal macula is normal, whereas filling of the aberrant choriocapillaris lobules -associated macula is delayed or non-existent (Figure 9; right panel), additional support that it is the choroidal vascular bed that is affected in aberrant choriocapillaris lobule pathology. Moreover, these segmental patterns mimic that of the distribution of temporal/macular choroidal short posterior ciliary arteries and their associated watershed zones as characterized by Hayreh (Brit J Ophthalmol 59:631, 1975) (Figure 9; inset).

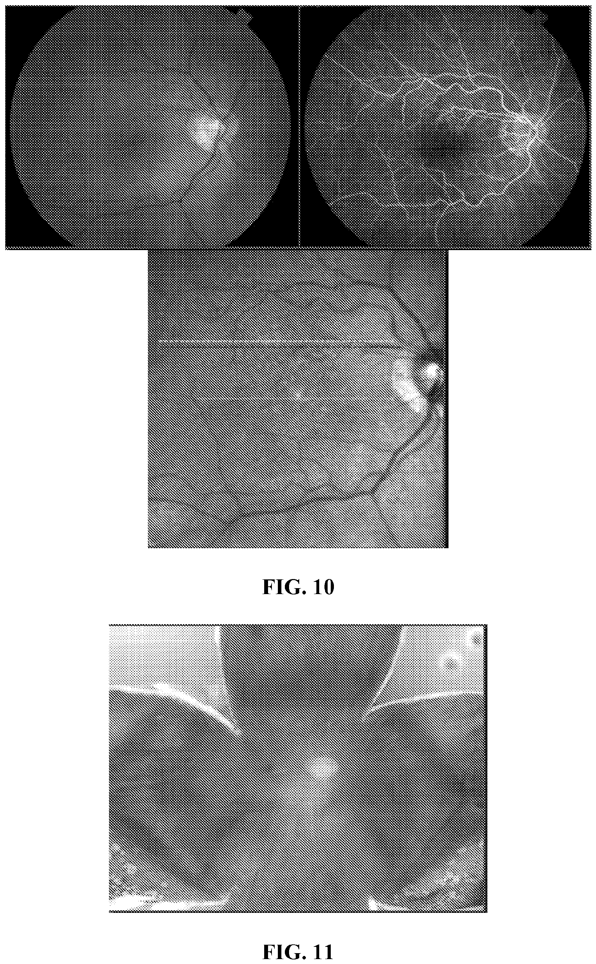

Additional evidence for degeneration of macular choriocapillaris lobules has also been obtained from six Utah study patients who donated their eyes at the time of death. All six patients had been seen at the John A. Moran Eye Center (JMEC) and determined to have aberrant choriocapillaris lobules prior to the time of death based upon color, IR, AF, and red-free images, along with the corresponding FA and/or ICG angiographs imaging. Three of these patients exhibited early stage AMD characterized by aberrant choriocapillaris lobules and three later stages of the disease associated with the presence of aberrant choriocapillaris lobules. An example of one of these eye donors is described herein. This patient was first seen at the JMEC in February 2009, last seen at the JMEC March, 2011 and died from a bowel obstruction in May, 2011. A previous medical history of hypertension and myocardial infarct was noted. Color, FA and IR images taken in March 2011 and depicting the presence of aberrant choriocapillaris lobules are shown in Figure 10 (aberrant choriocapillaris lobules are visible in the panel to the right; a dotted line represents the region from which the histological sections below were made). The patient was diagnosed with soft drusen and RPE changes, in addition to aberrant choriocapillaris lobules.

The eye taken from this donor 3 hours and 45 minutes after the time of death and opened to reveal the inside layers is depicted in Figure 11. The eye is characterized by the presence of cobblestone (pavingstone) degeneration in the far periphery, another feature that characterizes patients with aberrant choriocapillaris lobules. Peripheral cobblestone (pavingstone) also can also derive from abnormal degeneration/function of the choroidal vascular bed in the retinal periphery.

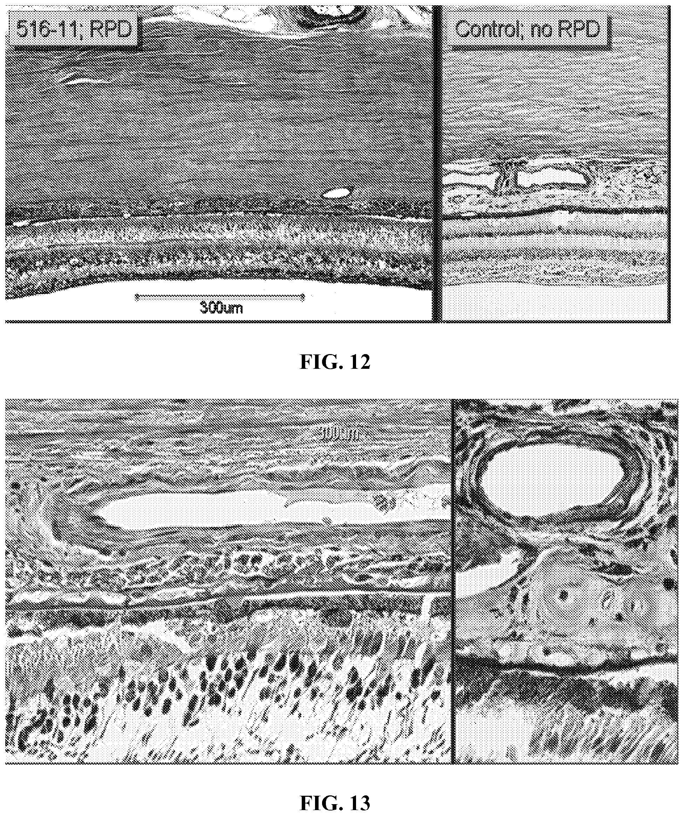

A standard histological section (stained with Mallory trichrome) is taken from the region described above, is depicted in Figure 12, left panel. A section taken from an age-matched donor with no history of aberrant choriocapillaris lobules or AMD is depicted, at the same magnification in Figure 12, right panel. A 300um scale bar, representing the average diameter of a single choriocapillaris lobule is shown. Strikingly, the choroid of the donor with aberrant choriocapillaris lobules is extremely thinned, as compared to the control donor. Thinning of the macular choroid was also assessed in 50 patients with aberrant choriocapillaris lobules (mean thickness 115.3um), as compared to 50 patients without aberrant choriocapillaris lobules (mean thickness 158.2um), providing additional support for the observation of thinned choroid in patients with aberrant choriocapillaris lobules. Moreover, a loss of choroidal vessels, especially the intermediate-sized vessels is obvious in these images, as is the fibrotic nature of the choroidal stroma in the donor with a history of aberrant choriocapillaris lobules (dark pink stroma in the left panel, as compared to lucent stroma in the right panel). In addition, the loss of photoreceptors in the donor with aberrant choriocapillaris lobules is apparent.

A number of distinct morphological features are observed at higher magnification (Figure 13). Importantly, the choriocapillaris in the posterior pole where the aberrant choriocapillaris lobules were present clinically, is severely degenerated (the majority of its capillaries completely so), supporting angiographic findings that macular RPD lesions represent aberrant choriocapillaris lobules (Figure 13, left panel). Moreover, the choroidal stroma is fibrotic, draining venules are dilated and filled with red blood cells, much of the normal choroidal vascular architecture is absent, and the retina exhibits severe photoreceptor degeneration (Figure 13, left panel). No deposits of subretinal debris, or subretinal drusenoid deposits are present (Figuire 13, left panel). The arterioles that occupy the central vascular layer and that feed the choriocapillaris lobules are often hyalinized and exhibit reduced or absent lumena (Figure, 13 right panel). Similar capillary pathology is typical of atherosclerosis in the brain, where thickening and hyalinization of the walls of small arterioles that arise at right angles to parent vessels are often observed. The resulting narrow lumena, which affects constriction, dilation and flow, is often a result of hypertension and/or atherosclerosis.

### 2. Assessment of 'Aberrant Choriocapillaris Lobules' in a Human Donor Eye Repository

A repository comprised of eyes, DNA, blood, medical and ophthalmological records from greater than 4,000 human donors was employed to establish the nature of 'aberrant choriocapillaris lobules' (ACL) and specifically to determine whether ACL was manifest by subretinal debris, also referred to as subretinal drusenoid deposits. Greater than 85% of all eyes in the repository were processed within four hours of death. The gross pathological features, as well as the corresponding fundus photographs and angiograms, when available, of all eyes in this repository were read and classified by retinal specialists. Fundi were classified according to a modified version of the International AMD grading system (Chong et al. 2005). Donors were classified as AMD-unaffected if they were over the age of 65 at the time of death, had no macroscopic or funduscopic signs of macular pathology or any documented ophthalmic history of AMD.

For light microscopic assessment, the entire posterior pole, or a wedge of the posterior pole was fixed in 4% (para)formaldehyde in 100 mmol/L sodium cacodylate, pH 7.4, as described previously (Hageman et al. 1999). After two to four hours in fixative, eyes were transferred to 100 mmol/L sodium cacodylate and embedded in acrylamide and/or paraffin. Tissues spanning between the ora serrata and the macula (in both the superotemporal and inferotemporal quadrants in most cases), were sectioned to a thickness of 6µm to 8µm on a microtome or cryostat.

Oriented, 4mm-diameter, full-thickness punches of sclera-choroid-RPE-retina of all eyes examined were taken in two defined locations in the supero-temporal quadrant -- 1 to 2 mm and 12 to 13 mm from the foveal center using a trephine punch. Punches used for transmission electron microscopic observation were fixed by immersion fixation in one-half strength Karnovsky's fixative for a minimum of 24 hours. Trephine-punched specimens were fixed, transferred to 100 mmol/L sodium cacodylate buffer, pH 7.4, and subsequently dehydrated, embedded in epoxy resin, sectioned and photographed, as described previously (Chong et al. 2005; Hageman et al. 1999). Any type of subretinal debris, regardless of amount, that was identified upon examination of these micrographs was included as 'subretinal drusenoid deposits.'

All eyes in the human donor repository for which there were both light and electron micrographs were reviewed for the presence of subretinal drusenoid deposits. Donors in the repository for which there were both light and electron micrographs available numbered 2,379. Of these donors, 402 (16.9%) had documented clinical histories of AMD and 1,977 (83.1 %) had no clinical histories or histological evidence of AMD. Of the 2,379 donors examined, 22 (0.92%) had evidence of subretinal debris lying within the subretinal space adjacent to the apical aspect of the RPE. Subretinal debris was identified in 14 (3.4%) of the AMD donors and 9 (0.5%) of the 1,977 non-AMD donors. Six of the AMD donors with subretinal debris were advanced cases (3 with geographic atrophy and 3 with CNV) and eight were early stage disease. The data are summarized in Table 2 herein.

When viewed at the substructural level of resolution, the subretinal debris, when present, was thin, of relatively uniform thickness ('flat aggregates,' as per the nomenclature of Zweifel and coworkers), patchy and limited to the apical aspect of the RPE. In no case did the subretinal debris extend to the level of the photoreceptor inner segments or inner limiting membrane, nor were any conical mounds observed by either light or electron microscopic examination in any of the donors. In all cases except one (which was heme), the material was comprised of membranous debris and identifiable portions of photoreceptor outer segments. Importantly, subretinal deposits do exist, but they are not of the appropriate size, number or topographical distribution to recapitulate that of IR-imaged aberrant choriocapillaris lesions.

**Table 2. Distribution of Subretinal Drusenoid Deposit in Donors with Morphologically Detectable Subretinal Debris.**

| ***Condition*** | ***Totals*** | ***ACL*** | |
|---|---|---|---|
| | | ***N*** | ***Percent*** |
| **No AMD** | 1,977 | 9 | 0.46 |
| **All AMD** | 402 | 13 | 3.2 |
| **Early Stage AMD** | 217 | 8 | 3.7 |
| **GA** | 52 | 1 | 1.9 |
| **CNV** | 133 | 3 | 2.3 |

In a second approach, the repository was assessed for donors specifically exhibiting clinical 'reticular pseudodrusen' - a yellowish network of broad, interlacing ribbons -- as defined by Klein and colleagues (Klein et al., 2008). Aberrant choriocapillaris lobules were indentified in color fundus photographs taken prior to death, as well as in postmortem photographs of the posterior fundus in which aberrant choriocapillaris lobules meeting the defined criteria are identified in some cases.

Donors for which gross and/or pre-mortem fundus photographs were available numbered 3,565. Convincing evidence of aberrant choriocapillaris lobules, as defined by Klein and colleagues (2008), was identified in 42 donors, or 1.2% of the total donors examined; of these, 10 were identified from pre-mortem fundus photographs and 32 by gross photographs. 19 of the donors with aberrant choriocapillaris lobules (83.3%) had confirmed clinical histories of AMD, 4 (9.5%) had questionable histories of AMD, and 3 (7.2%) had no evidence or histories of AMD. Both light and electron micrographs were available for 30 of the 42 donors with photographic evidence of aberrant choriocapillaris lobules; these were evaluated for the presence of subretinal debris, or 'subretinal drusenoid deposits.' Only 2 of these 30 aberrant choriocapillaris lobules donors had any evidence of subretinal debris or 'subretinal drusenoid deposits.' Data are summarized in Table 3 herein.

**Table 3. Distribution of Subretinal Drusenoid Deposit in Donors with Photographic Evidence of Aberrant Choriocapillaris Lobules.**

| ***Condition*** | ***Totals*** | ***Percent of Total ACL*** | ***Percent of AMD*** |
|---|---|---|---|
| **All ACL** | 42 | | |
| **No AMD** | 3 | 7.1 | |
| **Unknown** | 4 | 9.5 | |
| **All AMD** | 35 | 83.3 | |
| **Early AMD** | 16 | 38.0 | 45.7 |
| **GA** | 12 | 28.6 | 34.3 |
| **CNV** | 7 | 16.7 | 20.0 |

### 3. Assessment of Aberrant Choriocapillaris Lobules in Iowa and Melbourne Case-Control Cohorts Based Solely on Color Photography

A cohort comprised of over 2,600 AMD cases and AMD-unaffected, age-matched controls was ascertained.

This cohort was assessed to determine the demographics of aberrant choriocapillaris lobules detectable by color fundus photographs only. This same cohort has been used in numerous previous studies to assess genetic associations with AMD (see Hageman et al. 2005). Individuals in this cohort are of European-American descent, over the age of 60, and unrelated. AMD cases and controls are matched for age. Patients were examined and photographed by trained ophthalmologists. Color fundus photographs were graded according to standardized classification systems (Bird et al 1995; Klaver et al 2001) as described previously (Hageman et al 2005); classification of the worst eye was used in this analysis. Fundus photographs from 2,600 subjects were assessed for the presence or absence of aberrant choriocapillaris lobules based on criteria established for color photographs (Klein et al 2008; Smith et al 2009). Aberrant choriocapillaris lobules were identified in 82 of the 2,600 (3.2%) study participants. Of the 82 subjects with defined aberrant choriocapillaris lobules, 68 (83%) were female and 14 (17%) male. 6.1% of all patients with aberrant choriocapillaris lobules did not have a documented diagnosis of AMD and 2.4% were not graded for a variety of reasons. The remaining subjects had clinically documented diagnoses of AMD; 8.5% with AMD (Grade 1a), 22.0% with early stage AMD (Grades 1b, 2a, 2b and 3), 7.3% with geographic atrophy (Grade 4a), 41.5% with CNV (Grade 4b) and 12.2% with both GA and CNV (Grade 4c).

Approximately 60% of patients with aberrant choriocapillaris lobules exhibited a 'tigroid' pattern, while the remaining 40% a 'punctate' pattern on color photography. The lobules occured in the central region of the fundus in approximately 50% of individuals exhibiting the 'punctate' phenotype (the remaining are 40% superior and 10% temporal). They occured in the superior region of the fundus in approximately 70% of the individuals exhibiting the 'tigroid' phenotype (the remaining are approximately 12% temporal, 12% central and the remainder nasal). Greater than 70% of patients with aberrant choriocapillaris lobules detected on color fundus photographs exhibited late stage AMD, including geographic atrophy and/or choroidal neovascularization. Myopia was associated with 28%, of patients with aberrant choriocapillaris lobules; hyperopia and emmetropia were equally distributed. However, we now know based on more advanced imaging modalities (see section on Utah cohort) that detection of aberrant choriocapillaris lobules using color and red-free fundus photographs/images grossly underestimates the prevalence of this phenotype in patient cohorts by 10-20 fold. A similar observation was made using a case-control cohort from University of Melbourne. Only 105 patinets of the greater than 2,000 subject cohort exhibited the aberrant choriocapillaris lobule phenotype on color photographs. A number of patients from this cohort have been reinvited and imaged using SD-OCT. Many additional patients that did not exhibit the aberrant choriocapillaris lobule phenotype by color photography in the initial assessment show the presence of the aberrant choriocapillaris lobule phenotype by SD-OCT. Collectively, these studies provide some information about clinical associations with the aberrant choriocapillaris lobule phenotype, but grossly underestimate the prevalence of this phenotype in the population when using color photography alone.

### 4. Assessment of Aberrant Choriocapillaris Lobules in a Ghanan Cohort

A case/controlcohort comprised of 323 individuals(77 cases, 191 controls, 55 indeterminate) was ascertained in Accra, Ghana under Institutional Review Board-approved protocols. This cohort was assessed to determine the demographics of aberrant choriocapillaris lobules detectable by color fundus photographs only. Individuals in this cohort were of African descent, over the age of 60, and unrelated. AMD cases and controls were matched for age. Patients were examined and photographed by trained ophthalmologists and staff. Color fundus photographs were graded according to standardized classification systems (Bird et al 1995; Klaver et al 2001) as described previously (Hageman et al 2005); classification of the worst eye was used in this analysis. Fundus photographs from 323 subjects (average age = 67) were assessed for the presence or absence of aberrant choriocapillaris lobules based on criteria established for color photographs (Klein et al 2008; Smith et al 2009). Only one putative case of aberrant choriocapillaris lobules was noted.

Genotypes for SNPs listed in Table 1 were assessed for the entire cohort. A portion of the cohort consisting of 42 cases and 107 controls was analyzed for AMD disease association. Single marker associations were assessed using standard chi square, 2x2 table, and double-sided Fisher's exact tests. Haplotypes were constructed using Haploview (http://www.broadinstitute.org/haploview) software.

### 5. Retrospective Assessment of Aberrant Choriocapillaris Lobules Prevalence/Frequency in a Utah Clinical Cohort

A retrospective chart review was conducted of all patients entered into the Willow Database, which includes multimodal images and clinical information on patients that have been seen since April 2007. Only patients in this database that were imaged with color, near-infrared reflectance and SD-OCT between April 2007 and June 2010 were included in the assessment. The database was screened specifically for two groups of subjects: 1) patients diagnosed with any form of AMD; and 2) and age-matched patients diagnosed with macular holes, to serve as a control group.

For inclusion into the analysis, the presence of aberrant choriocapillaris lobules on combined near-infrared reflectance and SD-OCT images had to be either confirmed ('aberrant choriocapillaris lobules') or clearly excluded ('non- aberrant choriocapillaris lobules). A diagnosis of aberrant choriocapillaris lobules was strictly based on 1) the presence of 'subretinal spikes,' as described by Spaide (Ref), in SD-OCT images and 2) clearly visible entities of the appropriate size in IR images. The presence of aberrant choriocapillaris lobules in color, red-free, and AF photographs was recorded, but not used as a criterion for classification. Exclusion criteria included situations where one could not verify or exclude the presence of aberrant choriocapillaris lobules due to poor quality images, signs of diabetic retinopathy, history of retinal vascular occlusions and any signs or history of hereditary retinal dystrophy. The presence of aberrant choriocapillaris lobules and associated features (*e.g.* location, degree of macular involvement, disease association) were assessed and recorded for both groups.

248 subjects diagnosed with AMD and imaged by near-infrared reflectance and SD-OCT were identified. Of the 248 subjects, there was evidence of aberrant choriocapillaris lobules in 204 (82.3%) and no evidence of aberrant choriocapillaris lobules in 44 (17.7%). Data are shown in Table 4 herein. 92.5% of all patients with documented CNV and 97% of those with documented geographic atrophy manifested aberrant choriocapillaris lobules. The vast majority of patients without aberrant choriocapillaris lobules had histories of early stage - not advanced - AMD.

97 subjects diagnosed with macular holes (average age = 72.2) and imaged by near-infrared reflectance and SD-OCT were identified. There was evidence of aberrant choriocapillaris lobules in six patients (6.2%) and no evidence of aberrant choriocapillaris lobules in 91 (93.8%).

Assessment of the Utah cohort revealed the following characteristics of the aberrant choriocapillaris lobules phenotype. The aberrant choriocapillaris lobules phenotype is strongly skewed toward females (approximately 70% female in the Utah cohort and 90% in a cohort ascertained in Melbourne). Additionally, approximately 77% of patients with aberrant choriocapillaris lobules have severe or late stage maculopathy, compared to only 37% of patients that to not have aberrant choriocapillaris lobules.

**Table 4. Distribution of Eye Disease with Aberrant Choriocapillaris Lobules.**

| | ***All Patients*** | | ***With ACL*** | | ***Without ACL*** | |
|---|---|---|---|---|---|---|
| | **Number** | **Percent of Total** | **Number** | **Percent of Total** | **Number** | **Percent of Total** |
| **Total** | 248.0 | | 204.0 | 82.3 | 44.0 | 17.7 |
| **Early AMD** | 77.0 | 31.0 | 47.0 | 61.0 | 30.0 | 39.0 |
| **GA** | 33.0 | 13.3 | 32.0 | 97.0 | 1.0 | 3.0 |
| **CNV** | 134.0 | 54.0 | 124.0 | 92.5 | 10.0 | 7.5 |
| **No Dx** | 1.0 | 0.4 | 1.0 | 100.0 | 0.0 | 0.0 |

### 6. Assessment of SD-OCT-confirmed Aberrant Choriocapillaris Lobules in a Prospective Utah Case-Control Cohort

Patients diagnosed with all stages of AMD, as well age-matched control groups comprised of subjects both with and without macular holes, were recruited from the outpatient clinics. All studies followed the tenets of the Declaration of Helsinki. Informed consent was obtained from each patient after explanation of the nature and possible consequences of the study. Ophthalmological and medical histories were recorded using a standard questionnaire and review of records. Blood samples or saliva were collected for genetic analyses and sera for future biomarker analyses.

Pupils were dilated with 1.0% tropicamide and 2.5% phenylephrine and the study subjects imaged with combined cSLO, near-infrared and SD-OCT. Color fundus photographs, fundus autofluorescence images and red-free images were collected from most patients. Fluorescein angiography, indocyanin green angiography, multifocal ERG recording, microperimetry and visual field assessment were performed in selected cases.

For inclusion in the study data presented, the presence of the reticular pattern on combined near-infrared reflectance and SD-OCT images had to be either confirmed (aberrant choriocapillaris lobules AMD; aberrant choriocapillaris lobules non-AMD) or clearly excluded (non- aberrant choriocapillaris lobules AMD; non-aberrant choriocapillaris lobules control). Exclusion criteria included signs of diabetic retinopathy, history of retinal vascular occlusions, and any signs or history of hereditary retinal dystrophy.

### a. Color Fundus Photography

Color fundus photographs were obtained using a mydriatic (ZeissFF450 50° field or Zeiss FF4 30° field) and/or non-mydriatic (Nidek AFC-210) digital fundus cameras as per standard protocols.

### b. SLO and SD-OCT Imaging

High-resolution imaging was performed using a Heidelberg HRA+OCT/Spectralis scanning laser ophthalmoscope (Heidelberg Engineering, Heidelberg, Germany) that allows for simultaneous recording of cSLO and SD-OCT images (Helb, et al., 2009). For combined imaging, a minimum standardized imaging protocol was performed in all patients, which included acquisition of near-infrared reflectance (830 nm; field of view, 30° x 30°; image resolution, 768 x 768 pixels) and simultaneous SD-OCT scanning using a second, independent pair of scanning mirrors (870 nm; acquisition speed, 40,000 A-scans per seconds; scan depth, 1.8 mm; digital depth resolution, approximately 3.5 µm per pixel). The SD-OCT scans were viewed using the contained Heidelberg software (Spectralis Viewing Module 4.0.0.0; Heidelberg Engineering). Areas of aberrant choriocapillaris lobules pathology in the posterior pole were imaged using sections, each comprising up to 100 averaged scans. Eye movements were registered and corrected automatically; this allowed for pixel-to-pixel correlation of cSLO and OCT images. The point-to point correlation feature of this instrument was used to register corresponding pathology between the near-infrared and SD-OCT images. Depending on different modes (high-resolution and high-speed, respectively), the vertical presentation of the OCT scan was magnified by a factor of up to four. As such, SD-OCT images appear disproportionately high in the vertical dimension, or Z axis.

### c. Autofluorescence Imaging

AF images were captured on the same Heidelberg HRA+OCT/Spectralis instrument using a blue laser light at 488 nm for illumination and a barrier filter at 500 nm. Each image represents an average of 6 to 9 scans composed by the SLO software.

### d. Red-Free Imaging

RF images were captured on the same Heidelberg HRA+OCT/Spectralis instrument using a blue laser light at 488 nm for illumination. RF images were also obtained using the Zeiss digital fundus camera using a standarized protocol.

### e. Fluorescein Angiography

After intravenous injection of 5ml of a 10% sodium fluorescein solution (Akorn Pharmaceuticals; Lake Forest, Illinois), high speed digital images were captured over the first 20-30 second period, followed by still photographs at 1min, 5min, 10min and, at times, 20min on a Zeiss fundus camera using a blue filter or on the Heidelberg HRA+OCT/Spectralis instrument using a blue laser light at 488 nm for illumination and a barrier filter at 530 nm.

### f. Indocyanin Green Angiography

After intravenous injection of 2-4ml solution containing 12.5-25.0mg ICG (Akorn Pharmaceuticals; Lake Forest, Illinois), high speed digital images were captured over the first 20-30 second period, followed by still photographs at 1min, 5min, 10min and, at times, 20min on a Zeiss fundus camera using a blue filter or on the Heidelberg HRA+OCT/Spectralis instrument using a blue laser light at 488 nm for illumination and a barrier filter at 530 nm.

### g. Multifocal Electroretinography

Eyes were analysed for retinal response/sensitivity by multifocal electroretinogrpahy (mERG). mERG responses were obtained with the Veris Version 6.3 Multifocal System (Electro-Diagnostic Imaging Inc; Redwood City, CA) using 103 hexagon fields 40° horizontal and 35° vertical. Some patients were analysed using the RETIscan system (Roland Consult, Elektrophysiologische Diagnostik Systeme, Brandenburg, Germany). Elements were modified independently between two luminance levels following a short corrected binary m-sequence, and local responses of all stimulated retinal areas were extracted from the sum response.

### h. Fundus Controlled Microperimetry

Eyes were analysed for retinal function using fundus-controled microperimetry performed using the MAIA Macuar Integrity Assessment instrument (Ellex; CenterVue SpA; Padova, Italy), a confocal, infrared ophthalmoscope combined with a system for visible light projection to obtain perimetric measurements, using fundus perimetry. A standard 37 stimulus field was employed using the 'Expert Test' protocol as provided by the manufacturer. Some patients were assessed using the Microperimeter 1 (MP1; Nidek Technologies, Padova, Italy), which allows determination of fundus-controlled light increment sensitivity (LIS) of photopic function. The integrated infrared fundus camera allows real-time fundus imaging on a monitor. In addition to predefined testing grids, individual testing points on the fundus image were chosen by the examiner; the fundus images were captured before the perimetric examination. An integrated eye tracking system continuously monitored the patients' eye movements, ensuring exact spatial correlation between the anatomic landmarks and the perimetric sensitivity maps.

### 7. Image Interpretation and Analysis

For the spatial assessment of the reticular pattern, both cSLO and SD-OCT scans were studied simultaneously side by side. The analysis included the topographic distribution and the signal characteristics of the reticular pattern on en face cSLO images over the posterior pole, a distinct pattern visible as an interlacing network of round or oval irregularities with an approximate size between 100 and 400 µm. Corresponding structural changes on SD-OCT scans at the RPE/photoreceptor level and in more inner retinal layers were evaluated. For the former, individual bands below the hyporeflective band of the outer nuclear layer were analyzed according to Pircher et al.(2006): (1) a thin hyperreflective band presumably corresponding to the external limiting membrane, (2) a slightly thicker hyperreflective band presumably corresponding to the interface of the inner and outer segments of the photoreceptor layer (IPRL), (3) a thin -- only occasionally visible -- hyperreflective band presumably corresponding to the outer segment-RPE interdigitation, and (4) a broad hyperreflective band that is thought to correspond to the RPE-Bruch's membrane complex.

The associations of aberrant choriocapillaris lobules (ACL) with the prospective case-control cohort are summarized in Table 10. The association of ACl with the chromosome 10 (rs10490924) risk allele is high (MAF = 0.434). The association with chromosome 1 risk (rs1061170) is less robust. The association of ACL with various homozygote combinations of rs10490924 and rs1061170 is also depicted in Table 10. The most significant association segregates with a homozygous risk genotype (TT; 69.9% to 75.0%). ACL is biased toward females (66.2%) in this cohort, an association we've detected in all cohorts studied to date. Finally, ACL are strongly associated with the late stage macular degeneration phenotypes of geographic atrophy (4A), CNV (4B), or both (4C). For example, 58.2% of all CNV cases are associated with ACL.

**Table 10**

| **rs10490924 genotype** | | | | | | | | |
|---|---|---|---|---|---|---|---|---|
| *ACL* | GG | | *GT* | | *TT* | | *MAF* | |
| No (409) | 204 | 49.9% | 164 | 40.1% | 41 | 10.0% | T=.301 | |
| Yes (377) | 126 | 33.4% | 175 | 46.4% | 76 | 20.2% | T=.434 | |
| MAF - minor allele frequency | | | | | | | | |
| | | | | | | | | |

| **rs1061170 genotype** | | | | | | | | |
|---|---|---|---|---|---|---|---|---|
| *ACL* | *CC* | | *CT* | | *TT* | | *MAF* | |
| No (408) | 111 | 27.2% | 189 | 46.3% | 108 | 26.5% | T=.496 | |
| Yes (379) | 120 | 31.7% | 184 | 48.5% | 75 | 19.8% | T=.441 | |
| MAF - minor allele frequency | | | | | | | | |
| | | | | | | | | |

| **rs1410996 genotype** | | | | | | | | |
|---|---|---|---|---|---|---|---|---|
| *ACL* | *AA* | | *AG* | | *GG* | | *MAF* | |
| No (406) | 34 | 8.4% | 177 | 43.6% | 195 | 48.0% | A=.302 | |
| Yes (382) | 17 | 4.5% | 137 | 35.9% | 228 | 59.7% | A=.224 | |
| MAF - minor allele frequency | | | | | | | | |
| | | | | | | | | |

| **Chr10/Chr1 Combinations** | | | | | | | | |
|---|---|---|---|---|---|---|---|---|
| rs10490924/rs1061170 | | | | | | | | |
| | *No ACL* | | *Yes ACL* | | | | *w*/*o ACL* | *w*/*ACL* |
| GGCC | 53 | 13.1% | 43 | 11.4% | | GGCC | 55.2% | 44.8% |
| GGCT | 89 | 21.9% | 59 | 15.6% | | GGCT | 60.1% | 39.9% |
| GGTT | 60 | 14.8% | 24 | 6.4% | | GGTT | 71.4% | 28.6% |
| GTCC | 46 | 11.3% | 54 | 14.3% | | GTCC | 46.0% | 54.0% |
| GTCT | 78 | 19.2% | 86 | 22.8% | | GTCT | 47.6% | 52.4% |
| GTTT | 39 | 9.6% | 35 | 9.3% | | GTTT | 52.7% | 47.3% |
| TTCC | 11 | 2.7% | 22 | 5.8% | | TTCC | 33.3% | 66.7% |
| TTCT | 21 | 5.2% | 38 | 10.1% | | TTCT | 35.6% | 64.4% |
| TTTT | 9 | 2.2% | 16 | 4.2% | | TTTT | 36.0% | 64.0% |
| Total: | *406* | | *377* | | | | | |
| *chr10: T=risk, chr1: C=risk (e.g. GGCC=chr10 homo non-risk, chr1 homo risk) | | | | | | | | |
| | | | | | | | | |

| rs10490924/rs1410996 Genotype Combinations | | | | | | | | |
|---|---|---|---|---|---|---|---|---|
| | *No ACL* | | *Yes ACL* | | | | *w*/*o ACL* | *w*/*ACL* |
| GGAA | 21 | 3.7% | 6 | 0.8% | | GGAA | 77.8% | 22.2% |
| GGAG | 91 | 16.2% | 38 | 5.3% | | GGAG | 70.5% | 29.5% |
| GGGG | 91 | 16.2% | 84 | 11.8% | | GGGG | 52.0% | 48.0% |
| GTAA | 12 | 2.1% | 8 | 1.1% | | GTAA | 60.0% | 40.0% |
| GTAG | 70 | 12.5% | 64 | 9.0% | | GTAG | 52.2% | 47.8% |
| GTGG | 79 | 14.1% | 103 | 14.5% | | GTGG | 43.4% | 56.6% |
| TTAA | 1 | 0.2% | 3 | 0.4% | | TTAA | 25.0% | 75.0% |
| TTAG | 16 | 2.9% | 35 | 4.9% | | TTAG | 31.4% | 68.6% |
| TTGG | 23 | 4.1% | 39 | 5.5% | | TTGG | 37.1% | 62.9% |
| *chr10: T=risk, chr1: A=protection | | | | | | | | |
| | | | | | | | | |

| **Gender** | | | | | | | | |
|---|---|---|---|---|---|---|---|---|
| | *Female* | | *Male* | | | | | |
| No | 290 | 60.2% | 192 | 39.8% | | | | |
| Yes | 300 | 66.2% | 153 | 33.8% | | | | |
| | 51% have ACL | | 44% have ACL | | | | | |
| | | | | | | | | |

| **Worst Eye** | | | | | | | | |
|---|---|---|---|---|---|---|---|---|
| *No ACL* | *Count* | | *Yes ACL* | *Count* | | | | |
| 0 | 127 | 32.2% | 0 | 2 | 0.5% | | | |
| 1a | 12 | 3.0% | 1a | 1 | 0.3% | | | |
| 1b | 4 | 1.0% | 1b | 0 | 0.0% | | | |
| 2a | 8 | 2.0% | 2a | 8 | 2.1% | | | |
| 2b | 30 | 7.6% | 2b | 19 | 4.9% | | | |
| 3 | 69 | 17.5% | 3 | 57 | 14.8% | | | |
| 4a | 25 | 6.3% | 4a | 56 | 14.5% | | | |
| 4b | 113 | 28.6% | 4b | 224 | 58.2% | | | |
| 4c | 7 | 1.8% | 4c | 18 | 4.7% | | | |

### 8. Functional Consequences of Aberrant Choriocapillaris Lobules: Multifocal Electroretinography

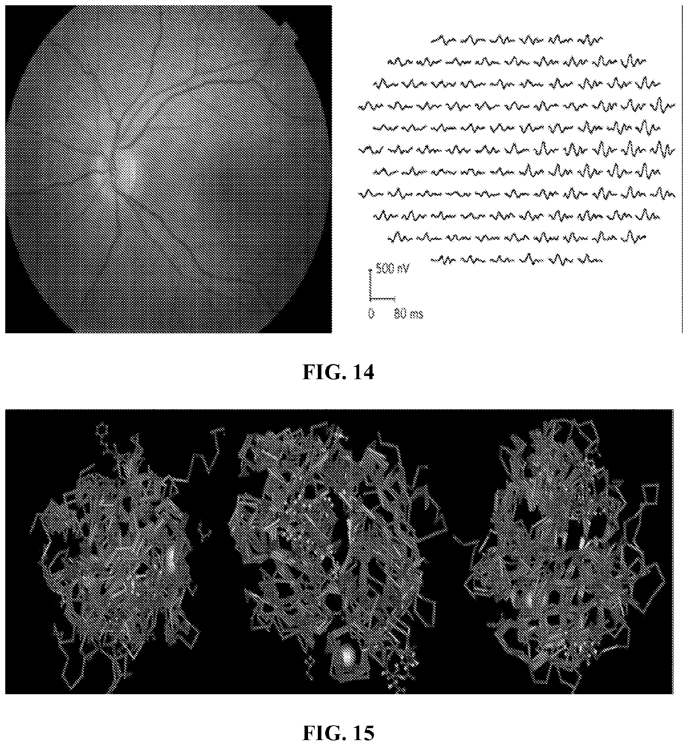

In one study, the paired-samples t-test was conducted to compare multifocal electroretinogram (MERG) responses (N1-P1 amplitudes and PI latencies) from both the superior with the inferior retina in patients with aberrant choriocapillaris lobules manifesting in the superior macula, as compared to control subjects without any aberrant choriocapillaris lobules. Each superior semi-ring was paired with the inferior semi-ring of the same eccentricity for analysis, forming 5 pairs. Variance between superior and inferior groups showed non-significant differences according the Levene's test and there was no violation of normality. There were significant depressions in amplitude (-10.26, -2.35 nV/deg²) in the superior semi-rings 1 and 2 (1-8°) compared with their inferior counterpart in control eyes and solely in superior semi-ring 1 for aberrant choriocapillaris lobules eyes. Though amplitude differences were found between the superior and inferior retina centrally (1-8°) for both control and aberrant choriocapillaris lobules eyes, little to no amplitude differences was found peripherally. Both control and pseudodrusen eyes showed a significant difference in mean latencies between superior and inferior retina at semi-ring 3 (eccentricity of 8-12°). Most interestingly, a significant delay in latency was found in aberrant choriocapillaris lobules eyes for the superior semi-ring 4 (32.3 ±1.9) compared with the inferior semi-ring 4 (31.1 ± 1.9); t(22) = 3.13, p =0.005. A similar significant delay was found between superior semi-ring 5 (31.7 ± 2.0) compared with inferior semi-ring 5 (31.0 ±2.0) in RPD eyes only; t(22) = 2.30, p=0.032. These P1-implicit time delays of the superior peripheral retina compared with the inferior peripheral (12-22°) retina were only found in aberrant choriocapillaris lobules eyes but not control eyes. Subsequent studies have continued to document marked disturbances in retinal function -- as assessed by MERG -- over regions of the macula with aberrant choriocapillaris lobules. An example of reduced macular retinal function (dark area in the right panel in Figure 14) in a patient with extensive aberrant choriocapillaris lobules in the central macula is shown in Figure 14.

### 9. Functional Consequences of Aberrant Choriocapillaris Lobules: Fundus Controlled Microperimetry

Photoreceptor function over areas of aberrant choriocapillaris lobules was compared to regions without aberrant choriocapillaris lobules in 25 patients. Photoreceptor function was consistently compromised in areas of aberrant choriocapillaris lobules as compared to regions without aberrant choriocapillaris lobules.

### 10. Aberrant Choriocapillaris Lobules and RAP

An examination of all patients with a diagnosis of retinal angiomatous proliferation (RAP) in the Willow database of the John Moran Eye Center at the University of Utah was conducted. In all cases observed, RAP was associated with the presence of aberrant choriocapillaris lobules, suggesting that this specific phenotype of neovascularization may derive as a result of aberrant choriocapillaris lobule formation. The typical form of choroidal neovascularization observed in patients with AMD erodes through the retinal pigment epithelium and infiltrates the neurosensory retina. In contrast, RAP has its origin in the deep layers of the retina and the proliferation progresses into the subretinal space before communicating with choroidal vascular networks and forming a retinal-choroidal anastomosis (Yannuzzi L.A. et al., Retinal Angiomatous Proliferation in Age-related Macular Degeneration, Retina 2001, 21(5):416-34).

### 11. The Association of Polymorphisms in AMD-associated Genes with Aberrant Choriocapillaris Lobules in a Small Cohort Detected Solely with Color Photography

The association of various SNPs within the CFH (V62I, rs800292; Y402H, rs1061170), HTRA1 (Promoter, rs11200638), ARMS2 (A69S, rs10490924), C3 (R102G, rs2230199), CFB (L9H, rs4151667; R32Q, rs641153), C2 (IVS10, rs547154; E318D, rs9332739), and APOE genes was determined initially in a small cohort of 49 patients with the aberrant choriocapillaris lobules phenotype based on control cohorts. In this cohort, 10.2% of subjects were male and 89.8% were female, the average age was 74.2 years and 82% had advanced AMD.

Genomic DNA from all subjects was isolated from peripheral blood leukocytes with QIAamp DNA Blood Maxi kits (Qiagen, Valencia, CA). DNA samples were screened for haplotype-tagging SNPs (ht-SNPs) in CFH (V62I, rs800292; Y402H, rs1061170), HTRA1 (Promoter, rs11200638), ARMS2 (A69S, rs10490924), C3 (R102G, rs2230199), CFB (L9H, rs4151667; R32Q, rs641153), C2 (IVS10, rs547154; E318D, rs9332739), and APOE. Genotyping was performed by TaqMan assays (Applied Biosystems, Foster City, California) using 10 ng of template DNA in a 5µL reaction. The thermal cycling conditions in the 384-well thermocycler (PTC-225, MJ Research) consisted of an initial hold at 95°C for 10 minutes, followed by 40 cycles of a 15-second 95°C denaturation step and a 1-minute 60°C annealing and extension step. Plates were read in the 7900HT Fast Real-Time PCR System (Applied Biosystems).

Allele and genotype frequencies of the SNPs were characterized in the aberrant choriocapillaris lobules cohort and compared with data for the same SNPs acquired previously on a cohort of approximately 900 AMD cases and 400 age- and ethnicity-matched controls (Hageman et al 2005). Statistical analyses were performed by standard chi-square, 2x2 table, and double-sided Fisher's exact tests.

The frequencies of the HTRA1 promoter allele (rs11200638) were 52% in the reticular cohort and 40% in the general AMD cohort (p<0.01). The corresponding frequencies of the risk allele for ARMS2 were 54% and 41% (p<0.01). In contrast, the frequencies of the CFH Y402H risk allele were 56% in the aberrant choriocapillaris lobules cohort and 54% in the general AMD cohort and that for the protective I62V allele was 16% in the aberrant choriocapillaris lobules patients and 15% in the general AMD cohort; these data were not significant. Similarly, there were no significant differences in allele frequencies for the C3 and CFB SNPs screened between aberrant choriocapillaris lobules patients and previously published AMD cohorts. It is also noted that the increased female-to-male ratio in this small cohort of aberrant choriocapillaris lobules subjects (10.2% male and 89.8% female) is consistent with that found in previous studies.

These data provided evidence that the chromosome 10 locus, which contains the two genes HTRA1 and ARMS2, is more strongly associated with aberrant choriocapillaris lobules than it is with AMD.

### 12. The Association of Polymorphisms in AMD-associated Genes with Aberrant Choriocapillaris Lobules in a Fully Imaged Prospective Utah Case-Control Cohort

Patients diagnosed with all stages of AMD, as well age-matched control groups comprised of subjects both with and without macular holes, were recruited from an outpatient clinic and imaged as described elsewhere herein The association of various SNPs within the chromosome 1 complement locus (CFH-to-F13B), as well as the HTRA1, ARMS2, C3, CFB, C2 and APOE genes (Figures 39A-39C) was determined in a Utah cohort comprised of 388 patients with the aberrant choriocapillaris lobules phenotype and 416 individuals with no aberrant choriocapillaris lobules as imaged with SD-OCT and IR. In the aberrant lobule cohort, 39.8% of subjects were male and 60.2% were female, the average age was 79.7 years and 77.4% had advanced AMD.

Genomic DNA from all subjects was isolated from peripheral blood leukocytes with QIAamp DNA Blood Maxi kits (Qiagen, Valencia, CA). DNA samples were screened for SNPs in the control of complement region (CFH-to-F13B), as well as the HTRA1, ARMS2, C3, CFB, C2 and APOE genes (Figures 39A-39C). Genotyping was performed by TaqMan assays (Applied Biosystems, Foster City, California) using 10 ng of template DNA in a 5µL reaction. The thermal cycling conditions in the 384-well thermocycler (PTC-225, MJ Research) consisted of an initial hold at 95°C for 10 minutes, followed by 40 cycles of a 15-second 95°C denaturation step and a 1-minute 60°C annealing and extension step. Plates were read in the 7900HT Fast Real-Time PCR System (Applied Biosystems).

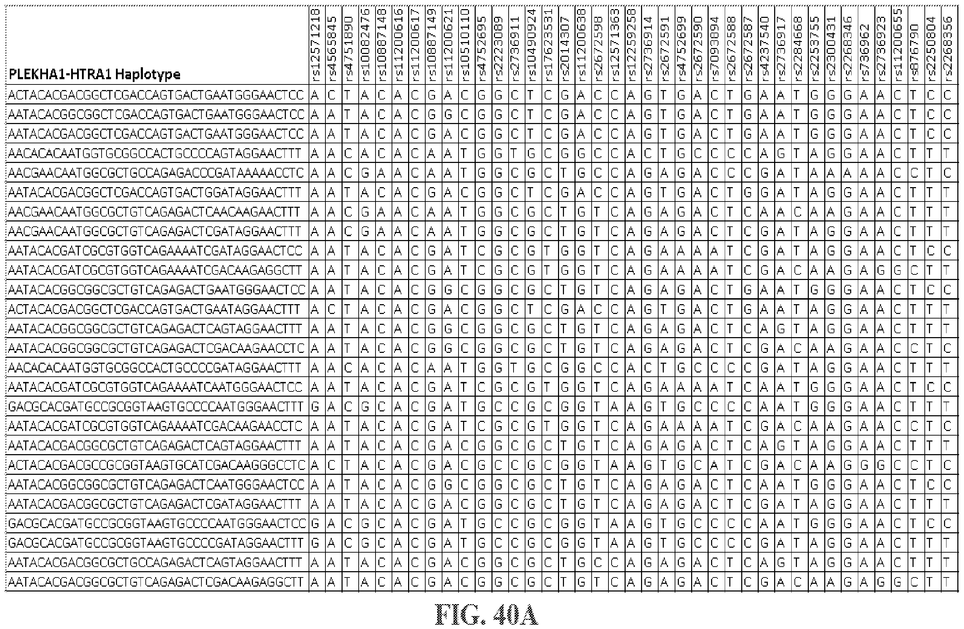

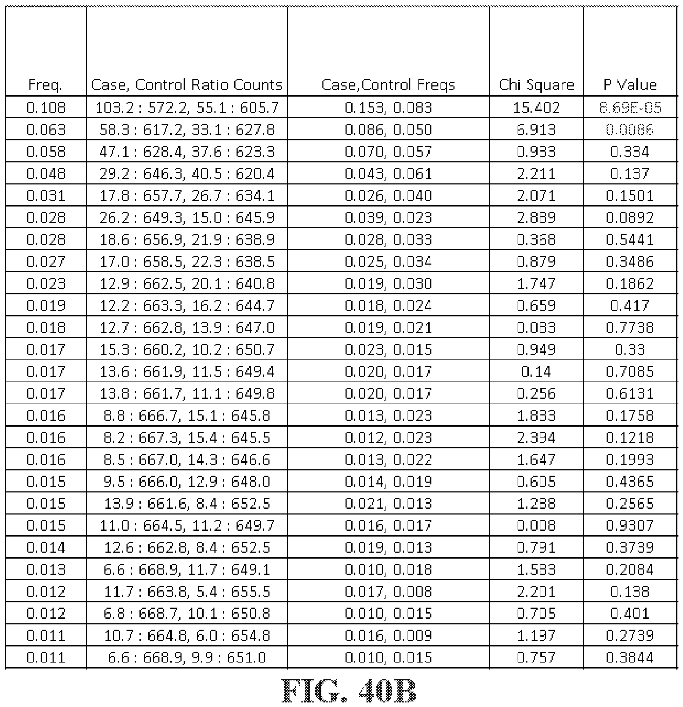

Allele frequencies of the SNPs were characterized in the aberrant choriocapillaris lobules cohort and compared to a cohort of 416 individuals with no aberrant choriocapillaris lobules. Statistical analyses were performed by standard chi-square, 2x2 table, and double-sided Fisher's exact tests (Figures 39A-39C). Where SNPs showed significance, haplotypes were constructed and frequencies calculated. Statistical analyses were performed by standard chi-square, 2x2 table, and double-sided Fisher's exact tests (Figures 40-42).

The frequency of the HTRA1 promoter risk allele (rs11200638) was 44% in the reticular cohort and 32% in the non-reticular cohort (p< 0.000001). The corresponding frequencies of the risk allele for ARMS2 were 44% and 30% (p<0.0000001). In contrast, the frequency of the CFH Y402H risk allele was 56% in the aberrant choriocapillaris lobules cohort and 50% in the non-reticular cohort (p=0.026) and that for the protective CFH I62V allele was 14% in the aberrant choriocapillaris lobules patients and 18% in the cohort with no aberrant lobules (p=0.020). There were no significant differences in allele frequencies for the C2, C3 and CFB SNPs screened between aberrant choriocapillaris lobules patients and the cohort with no aberrant lobules. It is also noted that the skewed female-to-male ratio in this cohort of aberrant choriocapillaris lobules subjects (39.8% male and 60.2% female) is consistent with that we found in other cohorts.

These data provide evidence to suggest that while AMD-associated chromosome 1 SNPs show a significant association with aberrant choriocapillaris lobules, the chromosome 10 locus, especially the two genes HTRA1 and ARMS2, is most strongly associated with aberrant choriocapillaris lobules. These loci, however, can not account for all cases of aberrant choriocapillaris lobules.

### 13. A69S/Y402H Genotypes as Distinguishing Between Aberrent Choriocapillaris Lobules, GA and CNV

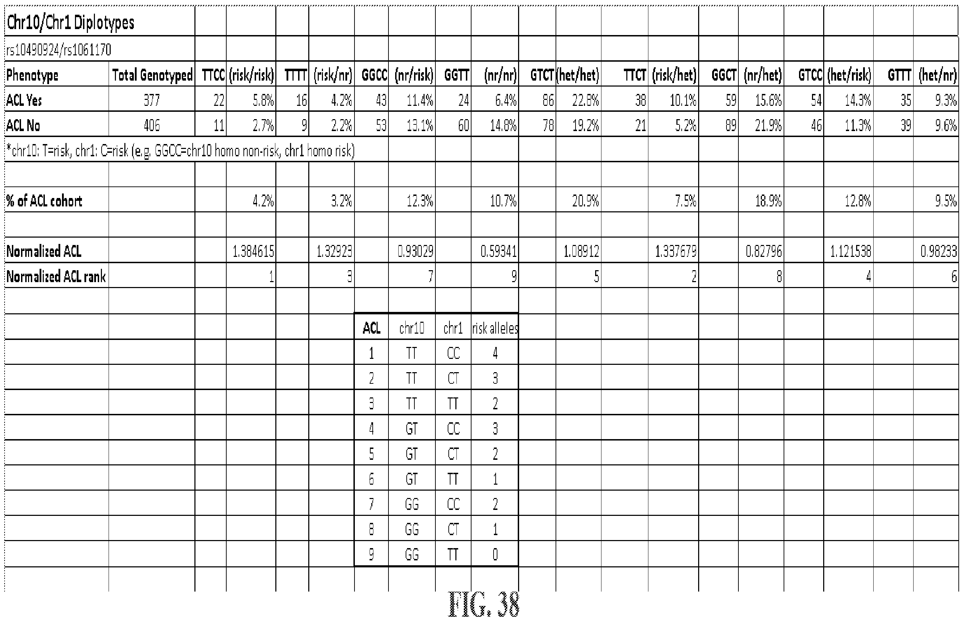

Although chromosomes 1 and 10 harbor the SNPs that are the most strongly and consistently associated with all subtypes of AMD, it is not clear how the combination of alleles at these loci contribute to AMD sub-phenotype risk, such as aberrant choriocapillaris lobules. The data suggests that it is the combination of genotypes or diplotypes at these two major loci that is the distinguishing factor between the AMD sub-phenotypes. Expressly, having specific genotype combinations of A69S and Y402H can show a greater risk for phenotypes like aberrant choriocapillaris lobules. Using normalization and ranking of risk based on the combination of alleles at each of these two loci, it becomes clear that risk alleles for the chromosome 10 A69S variant (rs10490924) are driving ACL risk more than are the risk alleles on chromosome 1 (rs1061170). This is evident by the fact that the largest risk for ACL is any diplotype that is homozygous risk at chromosome 10. See Figure 38.

### 14. HTRA1 Ocular Gene Expression

HTRA1 gene expression was assessed in the RPE-choroid complex of human donor eyes with and without ocular histories of AMD. RPE-choroid and retinal samples were isolated from human eyes obtained from the Lion's Eye Bank at the University of Iowa or the Lion's Oregon Eye Bank. Iowa eyes were classified by retinal specialists using gross pathologic features, and when available, the corresponding ophthalmic histories, including fundus photographs and angiograms. DNA-free RNA was purified using on-column digestion and Qiagen RNeasy preps (Qiagen, Inc., Valencia, CA). Global transcriptome profiling was carried out using Agilent Whole Human Genome 4x44K in situ oligonucleotide arrays (G4112F, Agilent Technologies, Inc., Santa Clara, CA) according to the methods of the manufacturer. Data processing methods and additional technical information are provided in Newman AM et al. (Systems-level analysis of age-related macular degeneration reveals global biomarkers and phenotype-specific functional networks, Genome Med 2012, 24;4(2):16). Sample information, detailed microarray methods, and microarray data are available through the Gene Expression Omnibus (Accession: GSE29801).

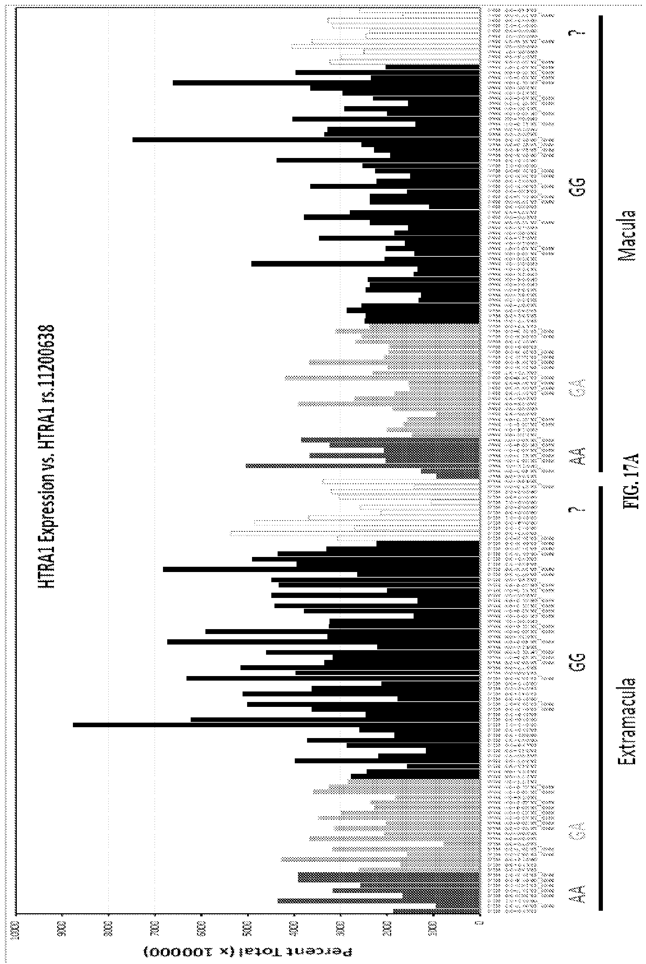

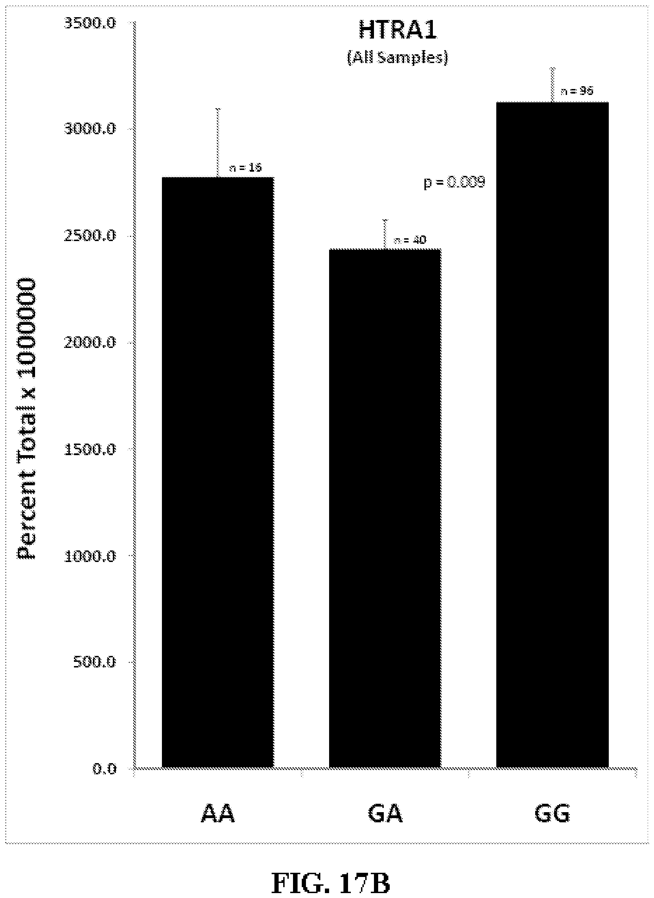

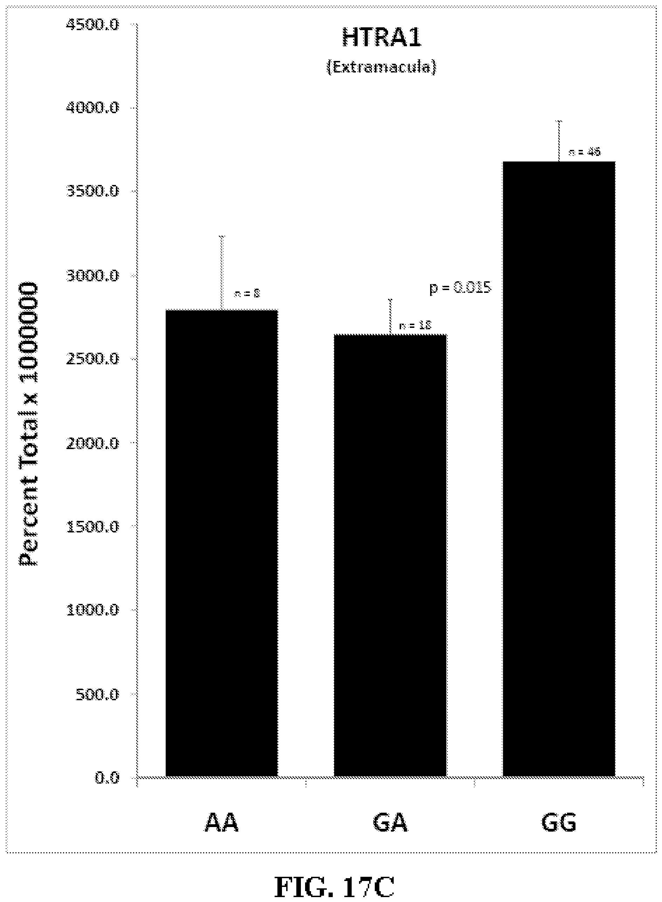

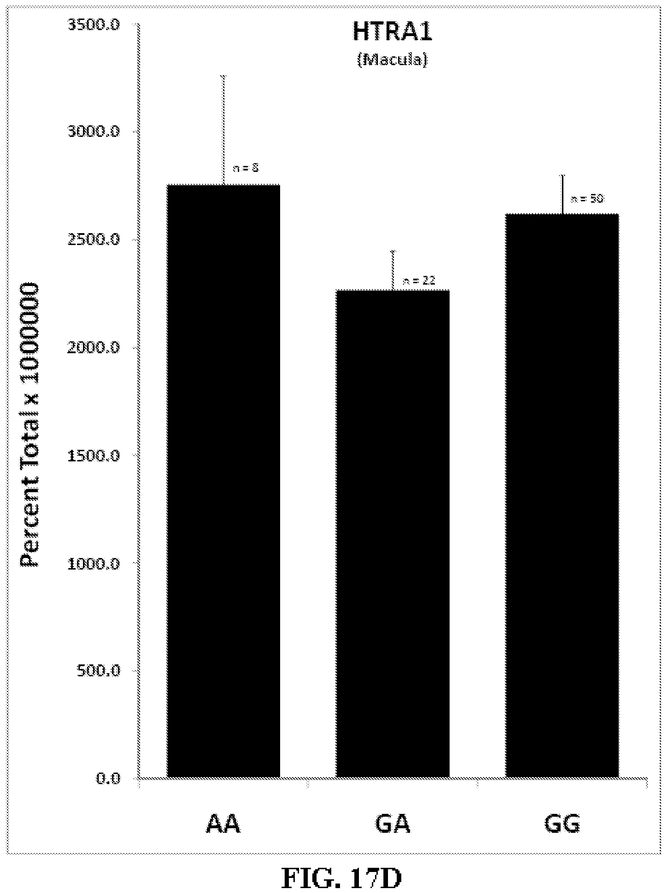

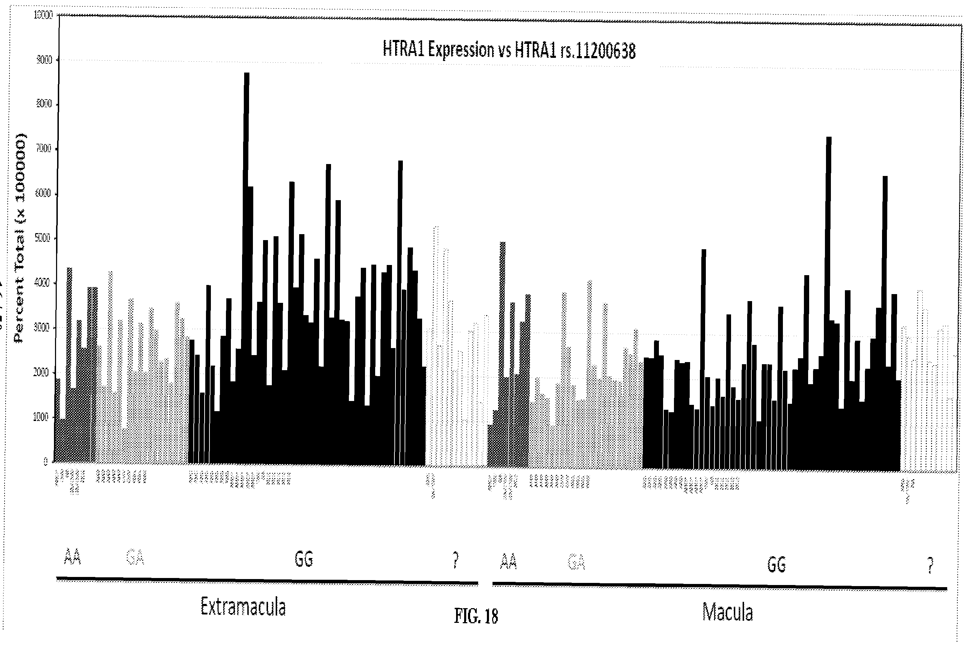

HTRA1 expression data in the extramacular and macular regions is presented in the graphs shown in Figures 17 and 18 Expression data are depicted based on disease status (AMD grade [GA, CNV, MD1MD2)] and controls [not labeled] on the upper chart and AMD and controls on the middle chart) and genotype at the chromosome 10 rs11200638 SNP ('A' is risk allele and 'G' non-risk allele). AMD* indicates the CSMD Oregon eyes for which there is no grade. Association of genotype with expression level is depicted in the lower bar chart. The error bars are standard error of the mean and the p values were determined using student's t-test. There was a slight significant difference (p=0.015) in expression level between donors with the heterozygote 'GA' and the homozygote non-risk 'GG' genotypes in the extramacular, but not the macular region. There does not appear to be any differences in expression levels of donors with CNV or GA, as compared to early stage AMD. See Figures 17 and 18.

### 15. Generation of HTRA1 and ARMS2 Constructs/Plasmids

Constructs designed to express portions of HTRAs 1-4 and ARMS2 were generated. These constructs were codon-optimized to express efficiently in bacteria. See Table 5 herein for a summary of all constructs generated. The amino acid coordinates given in Table 5 for each domain of HTRA1 are in reference to the codon-optimized nucleotide sequence SEQ ID NO. 9. These constructs were tested by western blot analysis for expression of protein. Briefly, cell pellets from uninduced and induced (3 hours with IPTG) were resuspended in 1x sample buffer and boiled for 20 minutes followed by centrifugation. A 5µl sample was loaded per lane for SDS-PAGE. The gels were then transferred to nitrocellulose membranes and blocked using 5% non-fat milk in PBS-Tween 20 (PBST). The membranes were subsequently probed with anti-HisHRP conjugated antibody (1:2000) and proteins were indirectly detected using ECL reagent (SuperSignalWest Dura Chemiluminescent Substrate, Thermo#34076). See Figure 4 herein for a summary of those results. Constructs were expressed in NEB T7 Express lysY cells and induced for 3 hours with ITPG. Expression was detected with an anti-HIS tag antibody (a HIS tag was conjugated to all fusion proteins). Expected bands were identified for all constructs except HTRA3, for which only a small amount of protein was induced, and HTRA2, for which no band was seen. Codon optimized HTRA2 and HTRA3 are being cloned into additional vectors.

**Table 5: HTRA1 and ARMS2 Constructs/Plasmids**

| **Construct** | **Domains** | **Amino acid** | **Plasmid** | **Epitope tag** | **Expression line** |
|---|---|---|---|---|---|
| ARMS2/LOC | full length | full length | pET-21a | 6xHIS | Arctic Express |
| ARMS2/LOC 387715 | full length | full length | pThioHis | TrxA | DE3/BL21AI |
| HTRA1-S328A | full length, no signal peptide, S328A | 23Q-480P | pET-21a | 6x HIS | Arctic Express DE3 |
| HTRA1-S328A | Full length, no signal peptide | 23Q-480P | pThioHis | His-TrxA | BL21/Arcti c Express/ DE3 |
| HTRA2-S306A | full length, S306A | full length | pET-21a | 6x HIS | |
| HTRA3-S305A | full length, S305A | full length | pET-21a | 6x HIS | |
| HTRA4-S326A | full length, S326A | full length | pET-21a | 6x HIS | |
| HTRA1-PDZ | PDZ | 380K-480P | pThioHis | His-TrxA | DE3 |
| HTRA1-ProteaseS328A | Protease | 159Q-372A | pThioHis | His-TrxA | DE3 |
| HTRA1-Protease-156-S328A | Protease-156 | 156G-372A | pThioHis | His-TrxA | DE3 |
| HTRA1-Protease-379-S328A | Protease-379 | 159Q-379K | pThioHis | His-TrxA | DE3 |
| HTRA1-IGFBP | IGFBP | 23Q-111V | pThioHis | His-TrxA | DE3 |
| HTRA1-Kazal | Kazal | 111V-156G | pThioHis | His-TrxA | DE3 |
| HTRA1-Protease-PDZ | Protease-PDZ | 159Q-480P | pThioHis | His-TrxA | |
| HTRA1-IGFBP-Kazal | IGFBP-Kazal | 23Q-156G | pThioHis | His-TrxA | DE3 |
| HTRA1-Kazal-ProteaseS328A-PDZ | Kazal-Protease-PDZ | 111V-480P | pThioHis | His-TrxA | DE3 |

| **Construct** | **Domains** | **Amino acid** | **Plasmid** | **Epitope tag** | |
|---|---|---|---|---|---|
| HTRA1 | Full length, no signal peptide | 23Q-480P | pThioHis | His-TrxA | |
| HTRA1-Protease | Protease | 159Q-372A | pThioHis | His-TrxA | |
| HTRA1-Protease-156 | Protease-156 | 156G-372A | pThioHis | His-TrxA | |
| HTRA1-Protease-379 | Protease-379 | 159Q-379K | pThioHis | His-TrxA | |
| HTRA1-Protease-PDZ | Protease-PDZ | 159Q-480P | pThioHis | His-TrxA | |
| HTRA1-Kazal-Protease-PDZ | Kazal-Protease-PDZ | 111V-480P | pThioHis | His-TrxA | |
| HTRA1-S328A | Full length, no signal peptide | 23Q-480P | pEcoli-Nterm 6xHN | N-terminal 6xHis-Asn | |
| HTRA1-PDZ | PDZ | 380K-480P | pEcoli-Nterm 6xHN | N-terminal 6xHis-Asn | |
| HTRA1-ProteaseS328A | Protease | 159Q-372A | pEcoli-Cterm 6xHN | C-terminal 6xHis-Asn | DE3* |
| HTRA1-Protease-156-S328A | Protease-156 | 156G-372A | pEcoli-Cterm 6xHN | C-terminal 6xHis-Asn | DE3* |
| HTRA1-Protease-379-S328A | Protease-379 | 159Q-379K | pEcoli-Cterm 6xHN | C-terminal 6xHis-Asn | DE3* |
| HTRA1-IGFBP | IGFBP | 23Q-111V | pEcoli-Nterm 6xHN | N-terminal 6xHis-Asn | NEB Shuffle |
| HTRA1-Kazal | Kazal | 111V-156G | pEcoli-Cterm 6xHN | C-terminal 6xHis-Asn | |
| HTRA1-IGFBP-Kazal | IGFBP-Kazal | 23Q-156G | pEcoli-Nterm 6xHN | N-terminal 6xHis-Asn | NEB SHuffle |
| HTRA1-S328A | Full length, no signal peptide | 23Q-480P | pEcoli-Cterm 6xHN | C-terminal 6xHis-Asn | |

### 16. Generation of HTRAl and ARMS2 Adenoviral Constructs

The following adenoviral constructs were designed and constructed with ViraQuest: HTRA1 with and without a Myc-HIS tag, HTRA1-S328A (proteolytically inactive) with and without a Myc-HIS tag, and ARMS2 with and without a Myc-HIS tag. See Table 6 herein for a summary of the adenoviral constructs generated. All constructs were generated with full-length human HTRA1 and ARMS2 sequences (SEQ ID NOs. 10 and 11, respectively) and cloned into the pVQAd CMV vector. Viral particles have been received for all six adenoviral constructs.

**Table 6: HTRA1 and ARMS2 Adenoviral Constructs**

| ***Clone*** | ***Detail*** | ***Vector*** | ***Tag*** |
|---|---|---|---|
| VQAd CMV HTRA 1 | adenovirus, full length HTRA1 (SEQ ID NO. 10) | pVQAd CMV | |
| VQAd CMV HTRA1myc-his | adenovirus, full length HTRA1 (SEQ ID NO. 10) | pVQAd CMV | Myc-HIS |
| VQAd CMV HTRA1-S328A | adenovirus, full length HTRA1, point mutation S328A (SEQ ID NO. 10) | pVQAd CMV | |
| VQAd CMV HTRA1-S328A-myc-his | adenovirus, full length HTRA1, point mutation S328A (SEQ ID NO. 10) | pVQAd CMV | Myc-HIS |
| VQAd CMV ARMS2 | adenovirus, full length ARMS2 (SEQ ID NO. 11) | pVQAd CMV | |
| VQAd CMV ARMS2myc-his | adenovirus, full length ARMS2 (SEQ ID NO. 11) | pVQAd CMV | Myc-HIS |

### 17. The Expression and Purification of HTRA1 using Arctic Express DE3 Cells

Arctic Express DE3 cells were used to express the GeneArt HTRA1 plasmid. Aggregation and co-elution of contaminant proteins have been observed in previous attempts to purify HTRA1 using several different chromatographic strategies. Methods for aggregate prevention/dissolution were explored. An HTRA1 IMAC eluate fraction was concentrated approximately ten fold and run over a SEC column. Several peaks were resolved on the chromatogram. HTRA1 was seen in all the peaks when analyzed with SDS-PAGE and western blots, although the co-eluting proteins were different in the different peaks. HTRA1 did not elute as a single band in any of the peaks. Several agents were evaluated to see if they would disrupt the aggregates: 0.05% Brij-35 (non-ionic detergent), 200mM MgSO4 (Kosmotrope), 100mM CaCl2, and 1M urea (Chaotropes). All were added to IMAC eluate and then passed through a 30,000 mw cut-off filter. In each case all protein was retained, including low mw proteins expected to pass through the membrane.

A benzamidine column had been tried previously for HTRA1 purification. Several contaminants were observed, and the HTRA1 protein precipitated out of solution. Precipitation could have been caused by the pH changes to elute the protein (pH 3) and then neutralize the acidic pH (pH 9 tris). The theoretical pI for HTRA1 is 8.09. The benzamidine column experiment was repeated with a lower pH 8.2 tris buffer in the fraction tubes. The volume added to give a final pH of 7.4 and avoid passing through the pI of HTRA1. No precipitate was seen in this run.

Arctic Express cells were grown and induced following the protocol supplied by Strategene. Cell pellets were stored at -80°C. Thawed pellets were resuspended in protein sample buffer containing Benzonase nuclease and lysed using an Avestin homogenizer. CHAPS (10mM final) was added prior to centrifugation and cleared lysate was run over an immobilized metal ion affinity column (IMAC) followed by protein elution using imidazole. Eluate fractions were analyzed by SDS-PAGE using Syprostain (BioRad)orimmunoblotting with HTRA1 antibody #1693 (1:1000 dilution and 3 min exposure). IMAC eluate was incubated with Benzamidinesepharose for 1 hour at 4°C. The slurry was placed in a BioRad column and washed with binding buffer. Protein was eluted with 20mM para-amino benzamidine in binding buffer. See Figure 5 herein for the HTRA1 expression and purification results.

HTRA1 expression constructs designed to express individual domains and particular combinations of domains were generated. These were used to test the specificity of antibodies and to generate protein for crystal structure determination. See Table 7 herein for a summary of these HTRA1 expression constructs. The amino acid coordinates given in Table 7 for each domain of HTRA1 are in reference to the codon-optimized nucleotide sequence SEQ ID NO.9.

**Table 7: HTRA1 Expression**

| ***HTRA1 Expression*** | | |
|---|---|---|
| **Construct** | **Domains** | **Amino Acids** |
| 1 | Full length, no signal peptide | 23Q-480P |
| 2 | PDZ | 380K-480P |
| 3 | Protease | 159Q-372A |
| 4 | Protease-156 | 156G-372A |
| 5 | Protease-379 | 159Q-379K |
| 6 | IGFBP | 23Q-111V |
| 7 | Kazal | 111V-156G |
| 8 | Protease-PDZ | 159Q-480P |
| 9 | IGFBP-Kazal | 23Q-156G |
| 10 | Kazal-Protease-PDZ | 111V-480P |

### 18. The Expression and Purification of ARMS2

Western blot analysis using the ARMS2-directed 42-58 antibody showed no expression of the ARMS2 expression construct in Invitrogen DE3 cells. BL21 AI cells showed that most of the ARMS2 is in the insoluble portion of the cell lysate with only a small amount seen in the soluble fraction. Soluble ARMS2 was extracted with NiNTA magnetic beads and eluted with 150mM imidazole. Due to its insolubility, ARMS2 was cloned into a vector with a Thioredoxin tag and enterokinase cleavage site (Invitrogen). Cultures were grown in DE3 cells, induced with 1mM IPTG, and harvested 4 hours post induction. Whole cell lysates showed a strong band at the expected molecular weight of 23KDa, while no band was seen in the pre-induced controls on coomassie stained SDS-PAGE gels. Western blots also gave a strong 23KDa signal using the anti-ARMS2 antibody. See Figure 6 herein for a summary of the ARMS2 expression and purification results.

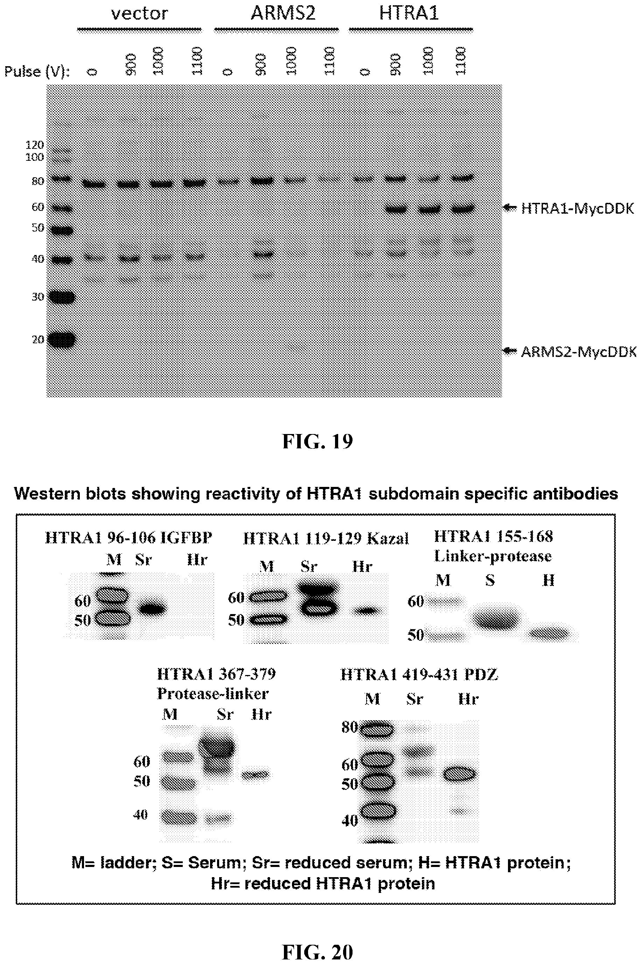

Additional HTRA1 and ARMS2 expression experiments were conducted. HEK293 cells were cultured in Advanced MEM with 5% fetal bovine serum and 1 mMGlutamax. On the day of transfection, cells were washed in PBS, treated with 0.25% trypsin and resuspended in electroporation buffer 'R' at 4 x 10⁷ cells/ml. 10 µg of plasmid DNA encoding pCMV6-Entry-MycDDK, pCMV6-HTRA1-MycDDK (Origene catalog # RC222362) or pCMV6-ARMS2-MycDDK (Origene catalog # RC223747) in a volume of 10 µl was added to 100 µl of cells. Cells were electroporated using a Neon (Invitrogen) with two pulses at 900 or 1000 volts, 20 ms. Alternatively, cells were pulsed with a single pulse at 1100 volts, 20 ms. As a negative control, cells were also left unpulsed. Cells were resuspended in 10 ml of culture media and incubated at 37°C. At 24 hours (1000 and 1100 v samples) or 48 hours (900 v samples), cells were washed in PBS and lysed in 0.5 ml M-PER lysis buffer (Pierce PI-78501) containing protease inhibitor cocktail. Following centrifugation, the protein yield in the supernatants was quantified by Bradford assay. A total of 10 µg of protein for each sample was electrophoresed on a 4-12% NuPage gel (Invitrogen), transferred by Western blot to nitrocellulose membrane and incubated for 1 hour in Starting Block (Pierce #PI-37539). The membrane was probed for 1 hour with anti-DDK antibody (cat. no. TA50011) diluted 1:1000 in PBS+0.1% Tween-20. Following washes in PBS + 0.1% Tween-20, the membrane was then probed with HRP-conjugated goat-anti-mouse antibody diluted 1:10,000 in PBS + 0.1% Tween-20. Again, the membrane was washed and then treated with Super Signal West Dura (Thermo #34076). The membrane was exposed for 5 minutes and imaged on an LAS4000. See Figure 19.

### 19. Generation of HTRA1 and ARMS2 Antibodies

Polyclonal antibodies were generated to distinguish the various HTRA1 and ARMS2 proteins. Epitopes for HTRA1 and ARMS2 were designed based on structure. Six epitopes in the HTRA1 and ARMS2 proteins were employed for the generation of antisera in rabbits. Rabbits were immunized with three injections over a 28-day period. Production bleeds were made on days 35 and 40 and ELISA employed to determine titers. Animals were immunized a fourth time on day 68, exsanguinated on day 78, blood collected and antibodies prepared by affinity purification using the columns made from the same peptides that were used as immunogens. See Table 8 herein for a summary of these antibody generation data.

**Table 8: HTRA1 & ARMS2 Antibody Generation**

| ***Protein Domains & Epitope Sequences*** | | | | | ***Affinity Purified Antibodies*** | |
|---|---|---|---|---|---|---|
| **Number** | **Target Protein** | **Protein Domain** | **Amino Acids** | **Protein Sequence** | ***Serum Titer*** | **mg of antibody** |
| #2414 | HTRA1 | IGFBP | 36-49 | Ac-EPARSPPQPEHCEG-amide (SEQ ID NO.1) | | 5.26 |
| | | | | | | |
| #1684 | HTRA1 | IGFBP | 96-106 | Ac-PASATVRRRAQC-amide (SEQ ID NO. 2) | 3,665,500 | 19.55 |
| | | | | | | |
| #1688 | HTRA1 | Kazal | 119-129 | Ac-CGSDANTYANL-amide (SEQ ID NO. 4) | 109,100 | 1.28 |
| | | | | | | |
| #2413 | HTRA1 | Kazal | 136-148 | Ac-SRRSERLHRPPVIC-amide(SEQ ID NO. 3) | | 2.58 |
| | | | | | | |
| #1693 | HTRA1 | Linker-Protease | 155-168 | Ac-CGQGQEDPNSLRHK-OH (SEQ ID NO. 5) | 118,200 | 11.13 |
| | | | | | | |
| #1695 | HTRA1 | Protease-Linker | 367-379 | Ac-SHDRQAKGKAITKC-amide (SEQ ID NO. 6) | 1,474,100 | 20.28 |
| | | | | | | |
| #1694 | HTRA1 | PDZ | 419-431 | Ac-CPDTPAEAGGLKEN-amide (SEQ ID NO. 7) | 408,300 | 10.30 |
| | | | | | | |
| SC- | HTRA1 | C- | Not | C-terminus | | |
| 15465 | | Terminus | Known | | | |
| | | | | | | |
| SC-50335 | HTRA1 | C-Terminus | 386-480 | C-terminus | | |
| | | | | | | |
| #1685 | ARMS2 | No Domains | 42-58 | Ac-LDPGVGGEGASDKQRSKC-amide (SEQ ID NO. 8) | 128,200 | 6.21 |

Of the five HTRA1-directed antibodies designed to recognize specific domains, all five detected HTRA1 in serum. The ARMS2 antibody detected bacteria-expressed ARMS2 protein. Serum samples were removed from storage at -80°C and thawed on ice. Each sample was diluted 1/40 with PBS and an equal volume of 2x sample buffer containing 50 mMDTT (reduced conditions) or sample buffer lacking DTT (non-reduced conditions). The samples were denatured for 7 minutes at 95°C followed by 5 minutes at 4°C. A sample of recombinant HtrA1 protein was prepared as a positive control. Samples was loaded on 4-12% Bis-TrisNuPage gels (Invitrogen) along with MagicMarkmolecular weight markers (Invitrogen). Gels were run in MES running buffer with antioxidant for 35 minutes at 200 V. Proteins were transferred to nitrocellulose membranes for 1 hour at 30V by Western blot in transfer buffer containing 15% methanol. The membranes were blocked for 1 hour in Starting Block (Pierce #PI-37539). The membrane was probed for 1 hour at room temperature with primary antibody diluted 1:1000 in PBS+0.1% Tween-20. Following washes in PBS + 0.1% Tween-20, the membrane was then probed with HRP-conjugated secondary antibody diluted 1:10,000 in PBS + 0.1% Tween-20. Lastly, the membrane was washed and treated with Super Signal West Dura (Thermo#34076). The membrane was exposed for various amounts of time and imaged on a FujiFilmLAS-4000 analyzer. See Table 9 and Figure 20 herein for a summary of the HTRA1 and ARMS2 detection data.

**Table 9: Detection of HTRA1 and ARMS2 Proteins by Directed Antibodies**

| **Antibody Number** | **Target Protein** | **Protein Domain** | **Amino Acids** | **Recombinant Protein** | **Human Serum** |
|---|---|---|---|---|---|
| #2414 | HTRA1 | IGFBP | 36-49 | Yes | Weak |
| #1684 | HTRA1 | IGFBP | 96-106 | Yes | |
| | | | | | |
| #1688 | HTRA1 | Kazal | 119-129 | Yes | Weak |
| | | | | | |
| #2413 | HTRA1 | Kazal | 136-148 | Yes | |
| | | | | | |
| #1693 | HTRA1 | Linker-Protease | 155-168 | Yes | Weak |
| | | | | | |
| #1695 | HTRA1 | Protease-Linker | 367-379 | Yes | Yes |
| | | | | | |
| #1694 | HTRA1 | PDZ | 419-431 | Yes | Yes |
| | | | | | |
| SC-15465 | HTRA1 | C-Terminus | | Yes | Yes |
| | | | | | |
| SC-50335 | HTRA1 | C-Terminus | 389-480 | Yes | Yes |
| | | | | | |
| #1685 | ARMS2 | NA | 42-58 | Yes | No |

### 20. Serum Levels of HTRA1 and AMD

The distribution of HTRA1 and any potential HTRA1 peptides in serum and plasma samples derived from subjects with and without AMD was examined. Samples from 10 AMD subjects from each genotype group and 10 control subjects were evaluated using six commercial, domain-specific HTRA1-directed antibodies.

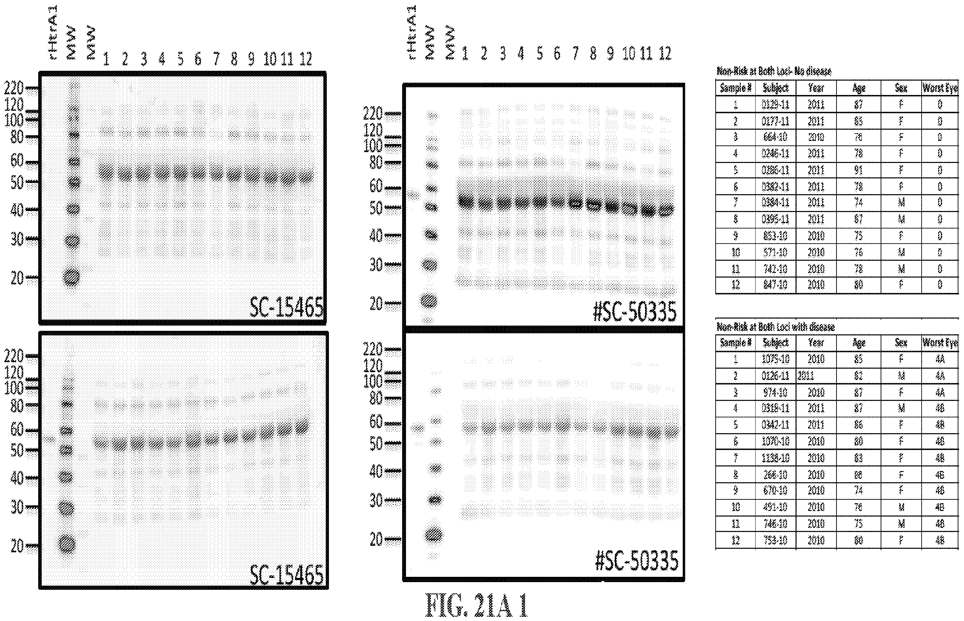

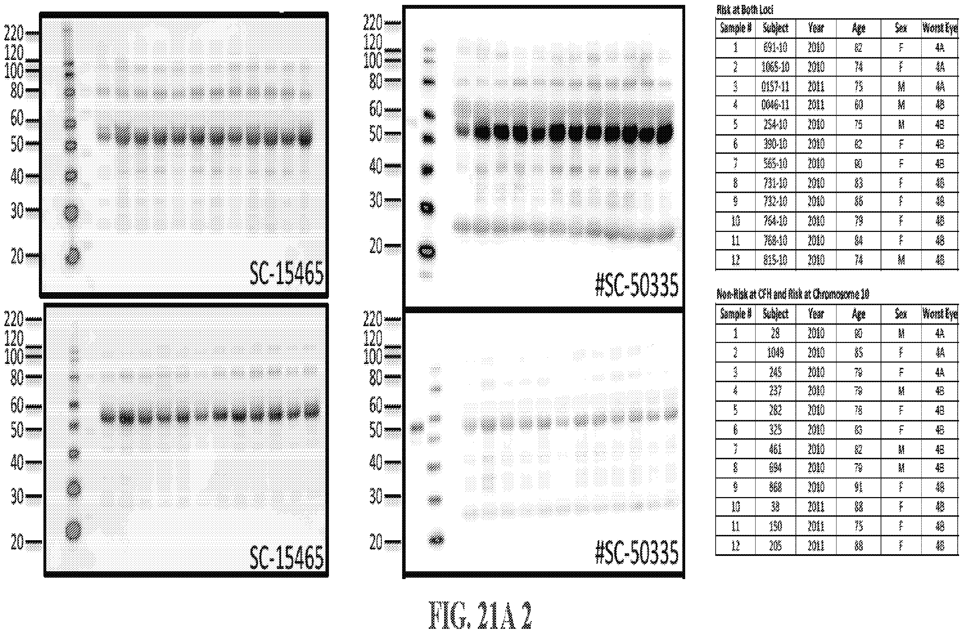

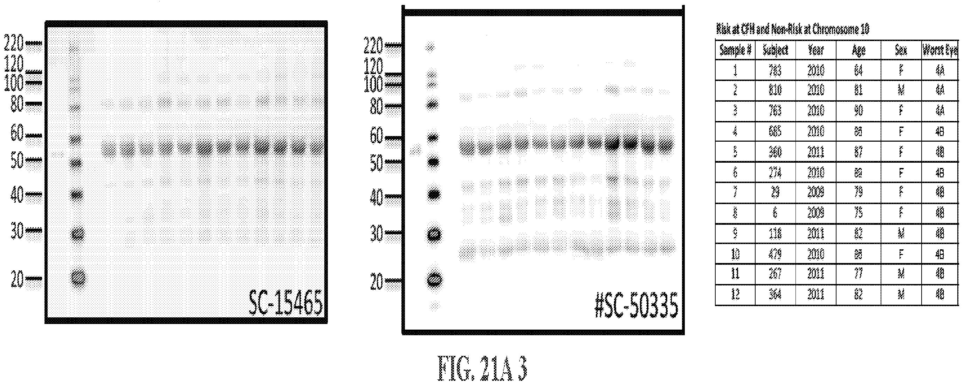

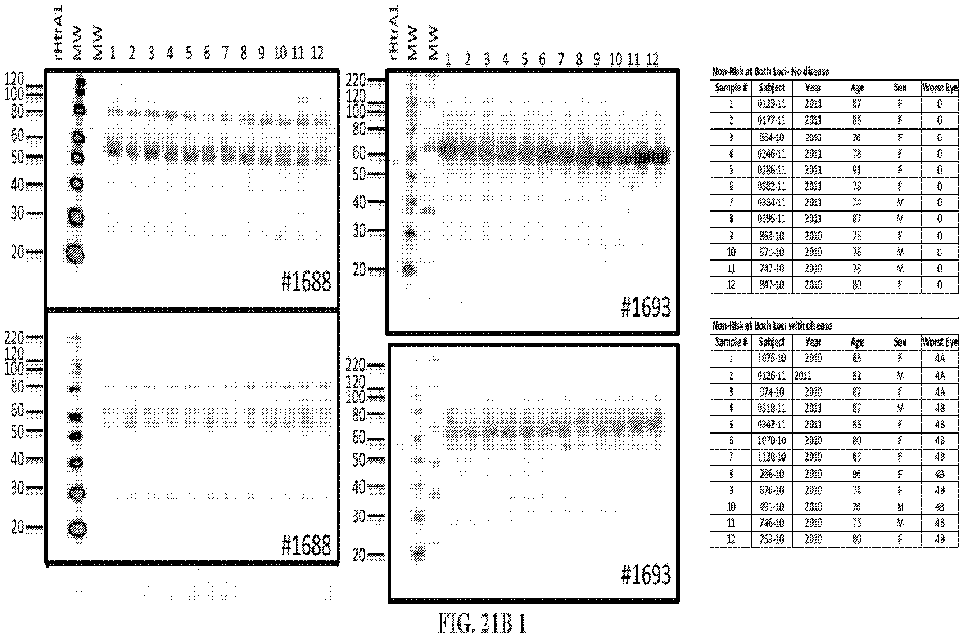

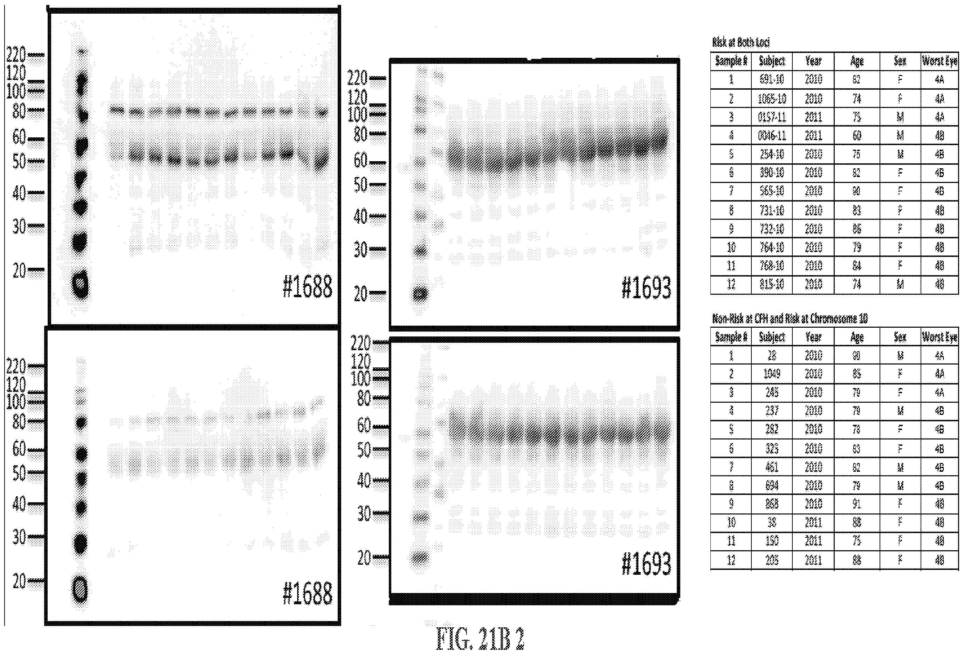

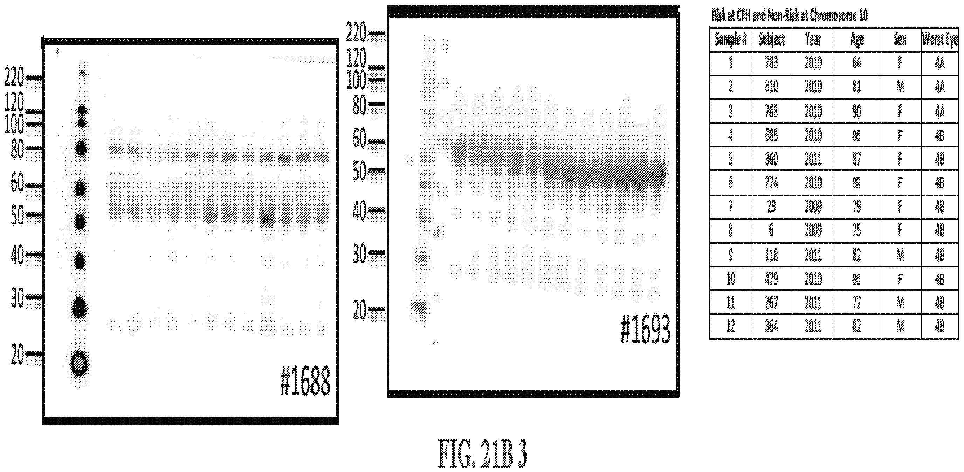

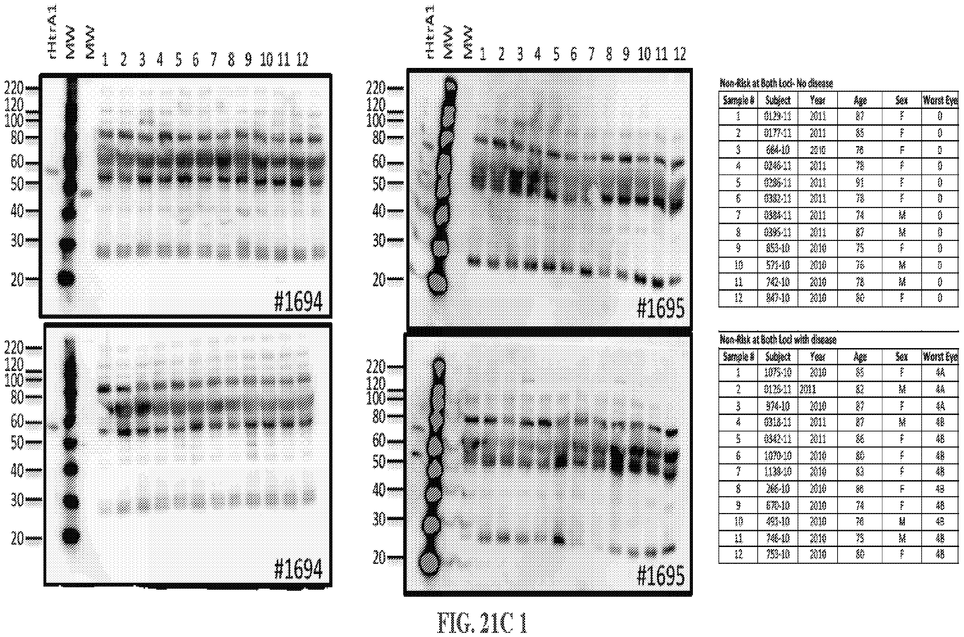

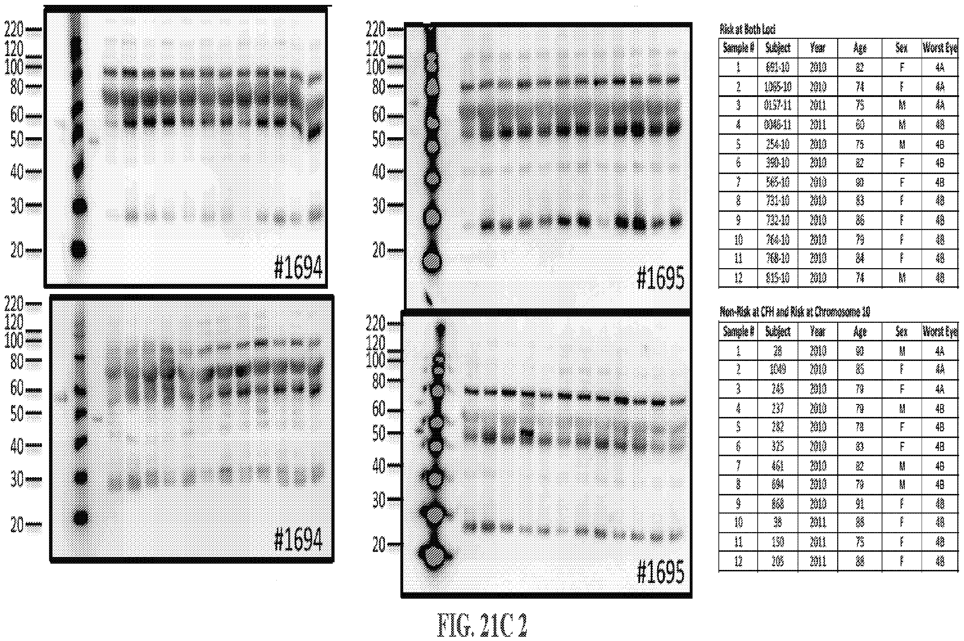

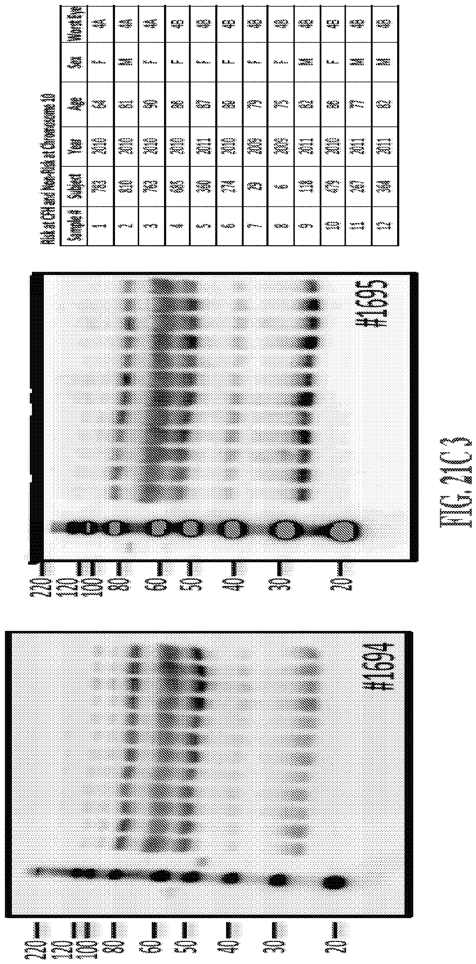

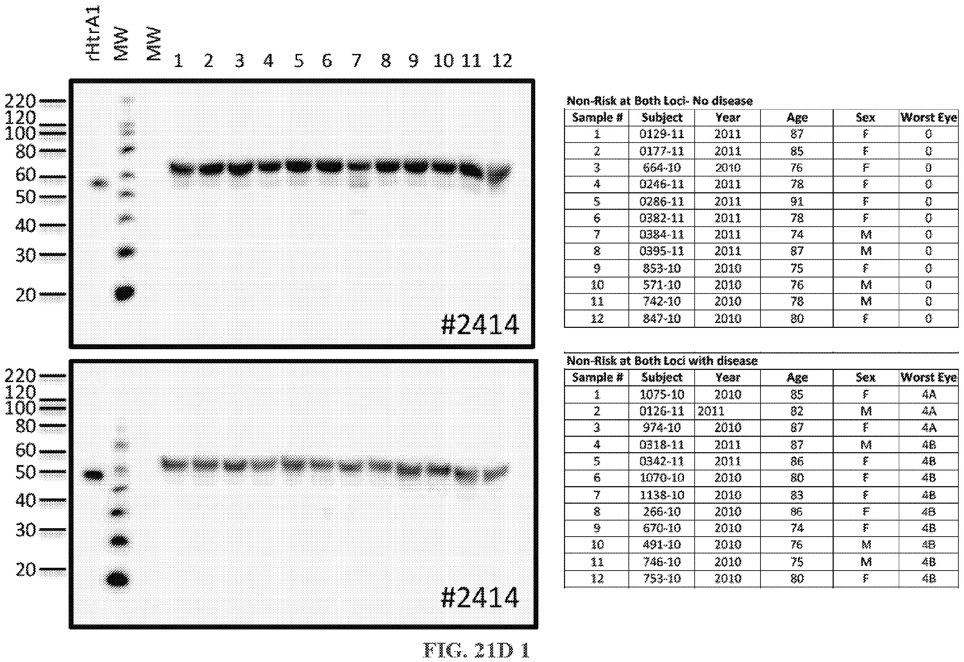

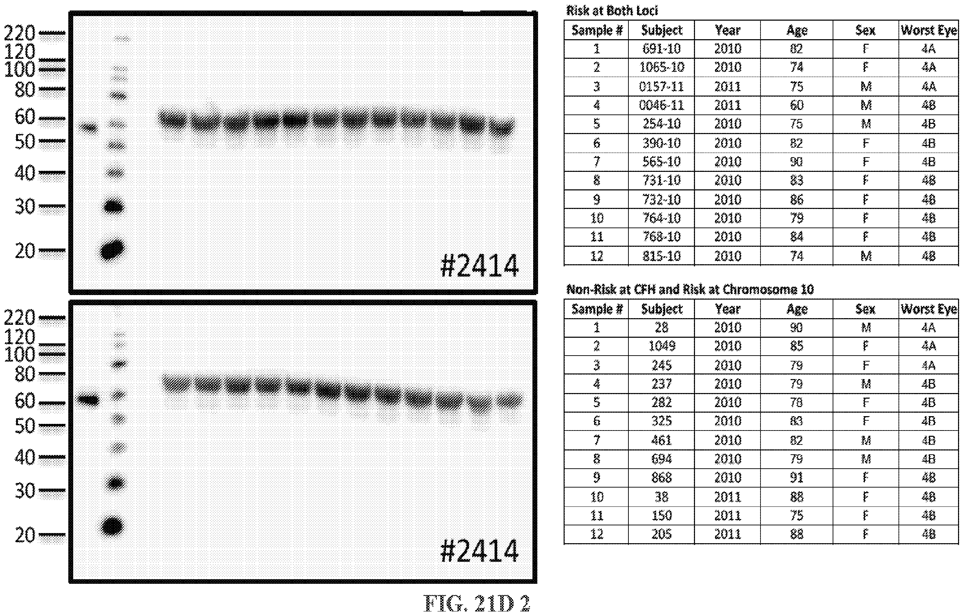

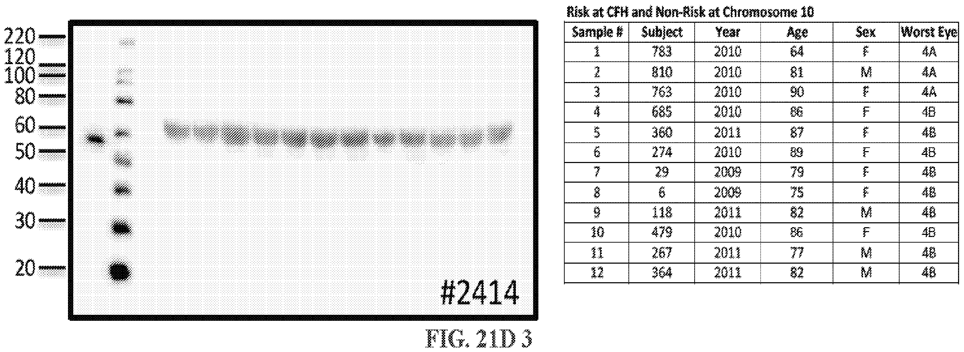

Serum samples were removed from storage at -80°C and thawed on ice. 2µL of each sample was diluted 100 fold in PBS for protein concentration determination with the microplate Coomassie Plus Protein Assay (Pierce). Each sample was then diluted with PBS to give a final protein concentration of 2µg/µL in 150µL. An equal volume of 2x sample buffer containing DTT (50mM final), and loading control protein, FUS1 (1ng/lane final) was added to each sample. The samples were divided into 2x150µL aliquots in PCR plates and loaded in a MJ Research PCR instrument and denatured for 7 minutes at 95°C followed by 5 minutes at 4°C. Each sample was then divided in 12.5µl aliquots stored at -80°C. A sample of HtrA1 protein was prepared for a positive control by harvesting cell culture supernatants from HEK293 cells expressing human recombinant HtrA1 protein. A total of 208 µl of the 48 hour supernatant was mixed with 25 µl 500 mMdithiothreitol and 75 ml 4X sample buffer. The mixture was denatured at 95°C for 7minutes before were denatured at 95°C for 7 minutes. A total of 10 µl of the serum samples was loaded onto 4-12% Bis-TrisNuPage gels (Invitrogen) along with 10 µl of the HtrA1 positive control and molecular weight markers. Gels were run in MES running buffer with antioxidant for 35 minutes at 200 V. Proteins were transferred to nitrocellulose membranes for 1 hour at 30V by Western blot in transfer buffer containing 15% methanol. The membranes were blocked for 1 hour in Starting Block (Pierce #PI-37539). The membrane was probed for 1 hour at room temperature with primary antibody diluted 1:1000 in PBS+0.1% Tween-20. Following washes in PBS + 0.1% Tween-20, the membrane was then probed with HRP-conjugated secondary antibody diuted 1:10,000 in PBS + 0.1% Tween-20. Again, the membrane was washed and then treated with Super Signal West Dura (Thermo #34076). The membrane was exposed for various amounts of time and imaged on an LAS4000. See Figure 21. Serum samples from patients with different genotypes were analyzed by Western blot with antibodies: A- SC-15465 (left), SC-50335 (right), B- NEP-1688 (left), NEP-1693 (right), C- NEP-1694 (left), NEP-1695 (right), D- NEP-2414.

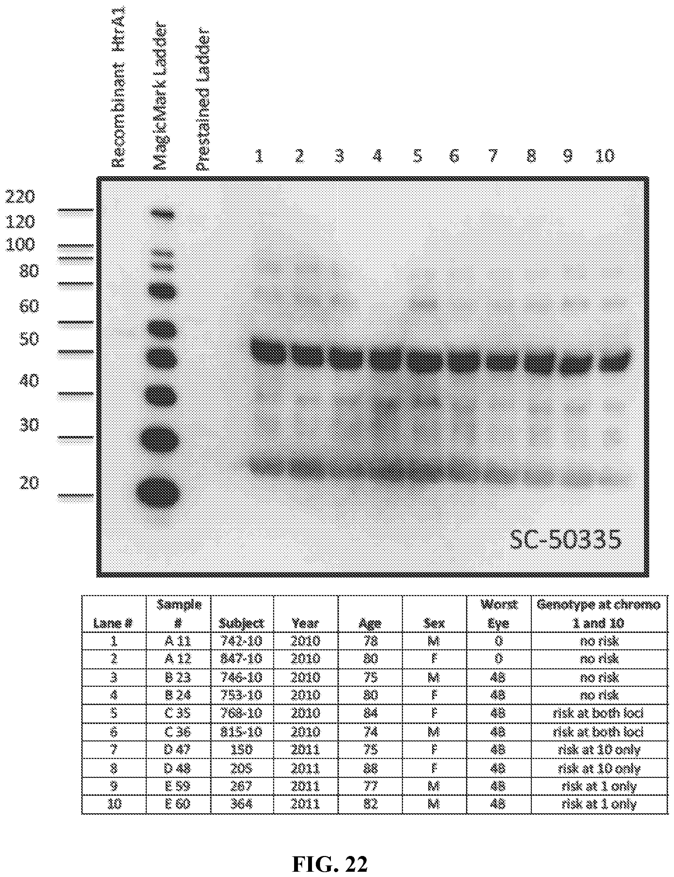

In Figure 21, the intensities of the HtrA1 band varied on the different blots. To determine whether this was due to differences in the levels of HtrA1 with the different genotypes or if it was due to differences in the efficiencies of the transfer of proteins to nitrocellulose, 2 samples from each genotype group were selected and run on the same Western blot. Probing with anti-HtrAl antibody failed to demonstrate differences in the levels of HtrA1 between the different genotype groups. Thus, the differences shown in Figure 21 were likely due to transfer efficiency. See Figure 22.

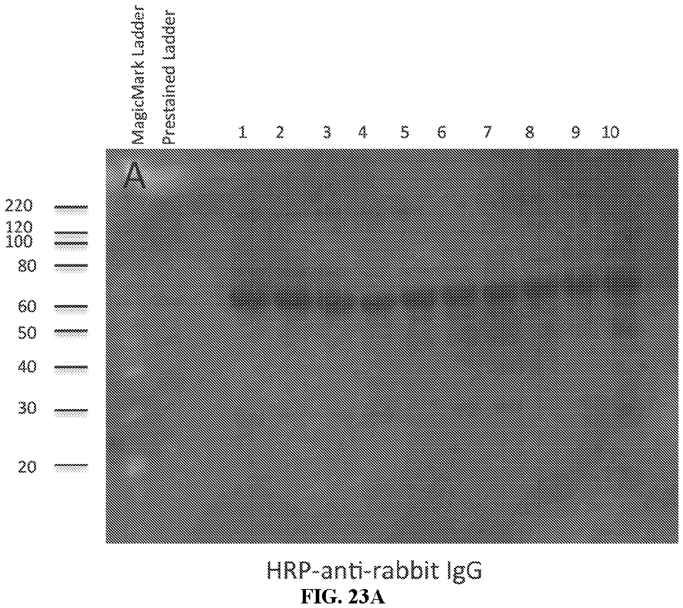

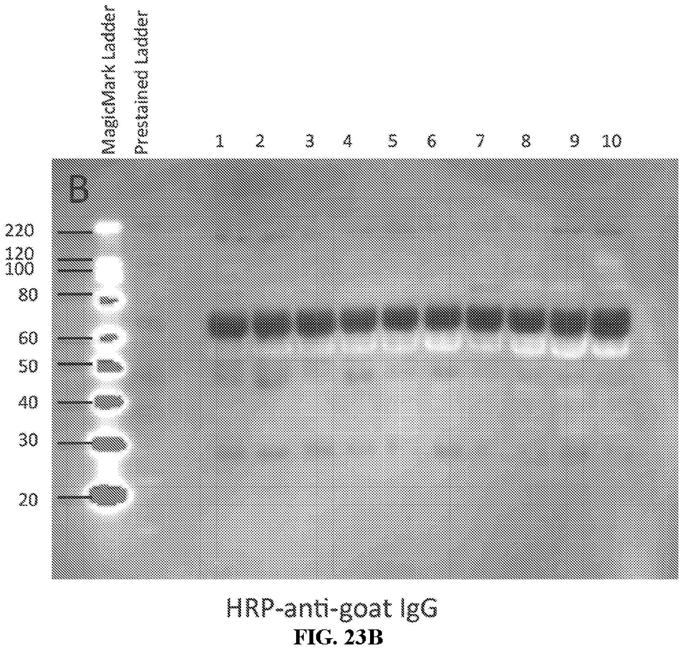

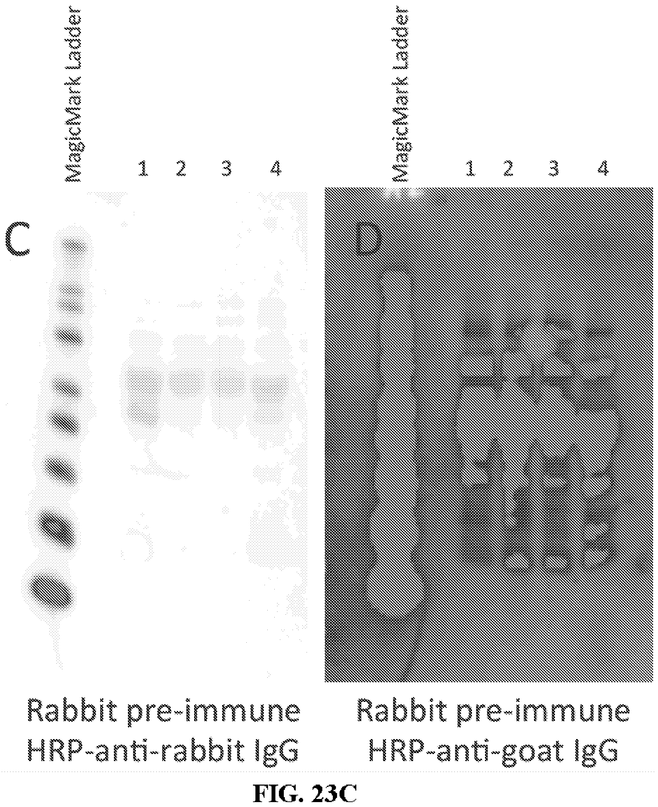

Additionally, in Figure 21, there were a significant number of bands that are not of the correct size. To determine whether this was due to non-specific reactivity of the primary or secondary antibodies, samples were run on a gel, transferred to nitrocellulose membranes, the membranes were blocked with Starting Block and probed with secondary antibody alone (diluted 1:10,000 in PBS + 0.1% tween-20). Alternatively, the blocked membranes were first incubated over night with rabbit pre-immune serum and then incubated with secondary antibody diluted 1:10,000 in PBS + 0.1% tween-20. The results showed that much of the background bands seen in Figure 21 were due to non-specific reactivity of the secondary antibodies. See Figure 23.

### 21. Detection of HtrA1 and ARMS2 Proteins in Retina/RPE Protein Extracion and Westen Blots

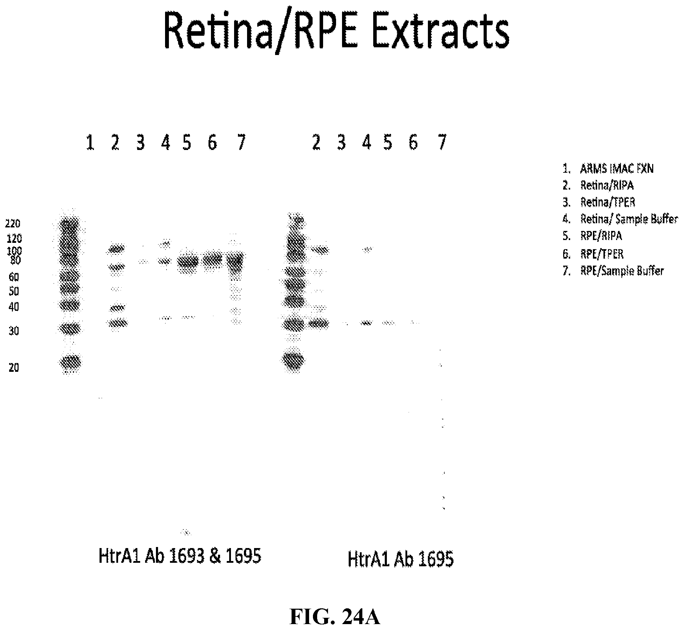

Retina and RPE (punch 9) were collected 4 hours post mortem and frozen in liquid nitrogen, and stored at -80C prior to protein extraction. The tissues were thawed on ice. Retina was washed with PBS to remove excess vitreous. Three extraction buffers were compared: RIPA, TPer, and SDS-PAGE sample buffer. Ice cold protein extraction buffer, with Complete protease inhibitor, was added to the samples (1ml/40mg tissue). The samples were ground with polypropylene pestles in microfuge tubes and centrifuged. The supernatant was loaded on gels for Western blots. Western blots were probed with anti-HTRA1 antibodies #1693 and #1695 combined or with #1695 alone, all diluted 1:1000. See Figure 24A.

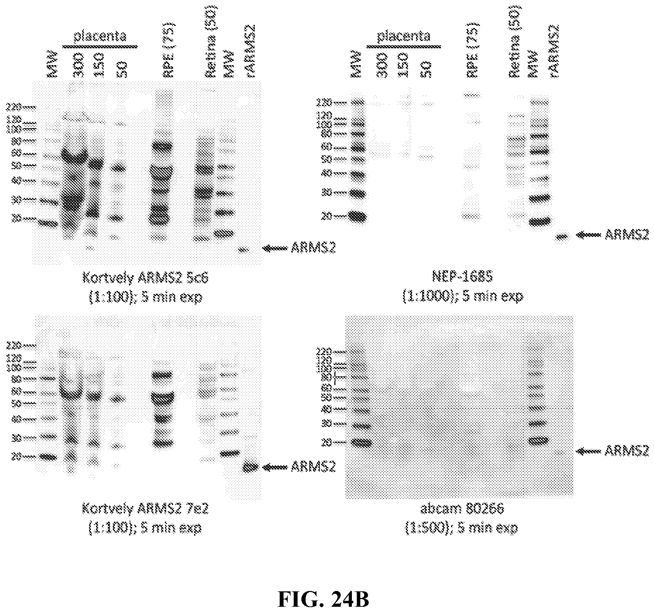

Extracts of placenta tissue, RPE, and retina were prepared with RIPA buffer as described above and quantitated with a BCA assay kit. A total of 50 to 300 µg of protein was run on a 4-12% Bis-Tris SDS gel, transferred by Western blot to a nitrocellulose membrane, and immunoblotted with various anti-ARMS2 antibodies at the indicated dilutions. As a positive control, recombinant ARMS2 protein was also run on the gel. Recombinant ARMS2 protein was detected with all antibodies tested. The ARMS2 antibodies provided by E. Kortvely et al. (Investigative Ophthalmology & Visual Science 51: 79-88, 2010) detected recombinant ARMS2 and possibly a similar sized band in RPE and placenta, though it was very faint. See Figure 24B. Various amounts of extracts (indicated in parenthesis as total µg of protein) from placenta, RPE, and retina tissue were analyzed by immunoblotting with anti-ARMS2 antibodies. A recombinant ARMS2 protein (rARMS2) was included as a positive control. The NEP-1685 antibody was made as described in Table 8. The Abcam #80266 antibody was purchased from Abeam.

### 22. Detecting HtrA1 and ARMS2 Protein Expression in Adult and Fetal Human Tissues

The natureof HtrA1 and ARMS2 proteins in adult and fetal tissues was examined to provide a basic understanding of expression patterns and levels for chromosome 10 high priority candidate targets. Identification of alternative sizes of splice variants between ARMS2 and HtrA1 provided a causal link between increased risk of ACL and chromosome 10 haplotypes. This hypothesis was tested by identification of novel protein/mRNAs of HtrA1 and/or ARMS2.

The antibodies used in these studies are listed in Table 9 herein. Generation of polyclonal antibodies targeting HtrA1 and ARMS2 were described in previous reports. Briefly, peptides corresponding to antigenic regions of HtrA1 and ARMS2 were synthesized and used to immunize rabbits. Polyclonal antibodies were enriched from rabbit serum via affinity column purification by using the original peptide antigen. The tissue panel blots were purchased from BioChainInc. (Cat. # W1234404 and W1244425). Membrane blocking was carried out using 2% nonfat milk (NFM) in PBS-T. Blots were probed with respective antibodies (1:100 or 1:1000) for 1 hr at room temperature (RT) or overnight at 4°C. The blots were subsequently washed 3 x 5mins in PBS-T and secondary antibody conjugated to HRP (Santa Cruz goat anti-rabbit; 1:10,000 for adult tissues and 1:5000 for fetal tissues) was added for 1 hr at RT or overnight at 4°C. The blots were then washed 3 x 5 mins in PBS-T and developed up to 10 mins using SuperSignalWest Dura ECL reagent from Pierce (Cat. #34075). Blots were exposed from 10 mins to 2 hrs, depending on signal intensity,using a FujiFilm LAS4000 digital imager. Blots were stripped for 15 mins using Restore Plus Western Blot Stripping Buffer (Pierce Cat. #46430) and reprobed with additional antibodies up to 4 times with no obvious loss of signal. Some of the longer exposure experiments did show residual signal from incomplete stripping of primary antibody and bands are depicted with an asterisk. To keep track of any "leak-through" from a previous experiment, we labeled blots as A, B, or C and numbered them sequentially 1, 2, 3, or 4 in the order blots were probed. GAPDH was used as the final antibody tested forblots to confirm equal loading of tissue lysates and that proteins were not inadvertently removed during the stripping procedure.

Of the seven HtrA1 antibodies tested, there were four major bands migrating at ∼25, ∼30, ∼52, and ∼70 kDa. The expected size for full-length HtrA1 was ∼52 kDa. The other immunoreactive bands may represent different forms of HtrA1 generated by post-translational medications or alternative splicing of HtrA1 mRNA, mRNA hybrids with ARMS2 or any combination of the above. Alternatively, these antibodies may recognize different sized bands because of cross-reacting epitopes and extremely long exposure times due to poor antibody titers.

Starting at the N-terminus of HtrA1, antibody #2414 targeting the IGFBP domain detected one major band at ∼52kDa and a second less robust band ∼70 kDa in all fetal human tissues. The ∼52kDa was predicted to be full-length HtrA1 protein. The adult tissue blot only showed bands at ∼25-30 kDa, which have been consistently seen in other tissues.

Antibody #1688, which recognizes the Kazal domain, detected numerous bands in fetal tissue with a major antigen at ∼25 kDa and some tissues detecting bands from ∼40-50 kDa. In the adult tissue blot only skeletal muscle showed a positive signal at ∼52kDa, all other tissues were negative for HtrA1 expression. This antibody has been successfully used to detect a ∼52 kDa recombinant HtrA1 protein (1:100 dilution) and an appropriately sized band in reduced serum samples from patient samples.

The newly generated Kazal domain specific antibody #2413 reacted with a major band at ∼30kDa and smaller bands in several different fetal tissues. The faint bands detected at ∼25, ∼52 and ∼70 kDa may represent leftover signal from the previous blot. The adult tissue blot did not show any positive bands.

The #1693 antibody that targets the linker-protease region detected a major band ∼70kDa in all fetal tissues tested. A robust band at ∼70 kDa was abundantly expressed in all adult tissues, except brain. Multiple smaller, less abundant bands were also detected in several of the adult tissues. The ∼70 kDa band was not consistent with HtrA1, but previous western blots using RPE and retina protein lysates did detect a ∼70 kDa band with this antibody.

Antibody #1695 detected several bands in fetal tissues, but no consistent banding pattern was recognized. Conversely, the adult tissue showed a very clean banding pattern, with a single band ∼30 kDa in most tissues and a few tissues showing extra bands between ∼15 and 35 kDa. The ∼30 kDa band was also detected in previous studies using protein lysates from RPE, retina, brain, and serum sources. Under optimal conditions an appropriately sized band was detected in reduced serum sample conditions.

The #1694 antibody generated by NEP recognized the PDZ domain (Figure 6). The antibody showed very weak bands at ∼70 and ∼52kDa in some fetal tissues. The adult tissues expressed a dominant ∼70 kDa band in all tissues, with a less intense band at ∼52kDa in most tissues types. Several additional bands were detected with this antibody. A faint band corresponding to ∼52 kDa was detected in patient serum samples under reducing conditions using starting block as the blocking agent.

As a comparison for the NEP developedpolyclonal antibodies, a commercially available HtrA1 polyclonal antibody from Santa Cruz (SC-50335) was also tested. Unlike the peptide generated NEP antibodies, this antibody was developed using the entire PDZ domain as antigen. The antibody detected two major bands at ∼70 and ∼52 kDa in adult tissues. Most of the tissues expressed both of these bands, but some did not, including brain. The liver predominantly expresseed the larger ∼70 kDa band, whereas placenta, reported to express high levels of HtrA1, predominantly expressed a ∼52kDa protein band.

Endogenous ARMS2 protein was detected by western blotting. Western blots with antibody #1685 using the human fetal tissue panel showed several bands between ∼10 and 25 kDa in liver tissue. None of the other fetal tissues in the panel showed a positive signal. The adult tissue panel did show a positive signal ∼17 kDa in liver tissue. A ∼28 kDaband was also detected in skeletal muscle. All other tissues were negative for ARMS2 expression, including placenta that had shown ARMS2 mRNA.

In this preliminary assessment, several HtrA1-directed antibodies did not recognize a full-length HtrA1 protein (e.g. #1688, #2413, and #1695) while others detected full-length protein (#2414, #1693, #1694 and SC-50335). The ARMS2-directed antibody did detect several bands slightly larger than expected in liver.

### 23. Elastase Activity of the HTRA1 Protein

The HTRA1 protein, which is comprised of four different modules: IGFBP, poorly defined) possesses several unique features that distinguish it from other serine proteases, making it a realistic therapeutic 'target'. These features include the following: 1) it has the smallest protease domain of the class, containing approximately 200 residues; 2) it does not possess disulfide-mediated stabilization of the protease domain or of the recognition pocket, unlike all other serine proteases; 3) it is not synthesized as a pro-enzyme; 4) zymogen activation occurs as a result of conformational changes; 5) activation is reversible; 6) it contains PDZ domains that may be involved in the activation process as well as in substrate recognition; and 7) the protease domain exhibits high sequence homology only to the corresponding domain of the other HtrAs, and not to any other serine protease.

HTRA1 expression data suggest that the risk haplotype results in increased levels of mRNA and protein in ocular tissues derived from donors with a documented clinical history of AMD and a risk HTRA1 haplotype. Various lines of evidence have suggested that Bruch's membrane, an extracellular layer comprised of the structural proteins elastin and collagen, functions as a physical barrier to the egress of cells and vessels from the choroid into the sub-RPE and subretinal spaces. Disruption of, or damage to, this barrier is associated with loss of vision in AMD, often resulting from the growth of new blood vessels from the choroid into the sub-RPE and/or subretinal spaces (a process referred to as choroidal neovascularization, or CNV). Moreover, choroidal neovascular membrane (CNVM) formation can be induced experimentally by thermal damage to Bruch's membrane in normal monkey eyes following laser photocoagulation or following laser treatment of human CNVMs. Extramacular laser treatment is relatively ineffective at inducing CNV, suggesting that the macular Bruch's membrane is uniquely susceptible to damage. Recent studies have provided a hypothesis for this susceptibility. Morphometric assessment of the macular and extramacular regions of human donor eyes, with and without AMD, revealed a statistically significant difference in both the integrity and thickness of the elastin-containing layer (EL) of Bruch's membrane between the macular and extramacular regions. The EL was 3-6 times thinner and 2-5 times less abundant in the macula than in the periphery in individuals of all ages. Importantly, the integrity and thickness of the macular EL is significantly lower yet in donors with early stage AMD, as compared to age-matched controls (Chong et al., 2005). These structural properties of the macular EL (thin and porous) correspond spatially to the distribution of macular lesions associated with AMD and provide strong evidence that degradation of elastin may 1) play a key role in the pathobiology of AMD and 2) be an important 'inducer' of CNVM genesis and growth. Additional studies showing the presence of higher levels of elastin degradation peptides, or EDPs, in the plasma and urine of AMD patients - especially patients with advanced stages of the disease - provide additional support for this concept. In addition, the protease recognition pocket of HTRA1, which plays a critical role in substrate recognition, resembles that of elastase. It has also been shown that the Kazal domain, an integral part of the regulatory region of the protein, shares structural homology with a known elastase inhibitor from anemonia.

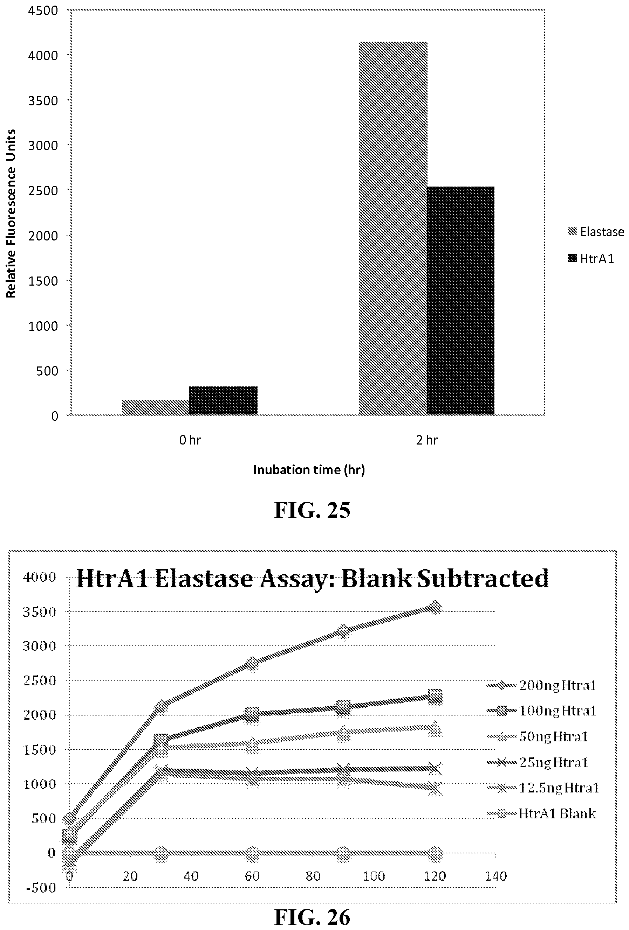

Therefore, studies were performed to determine whether HTRA1 degrades elastin, a major component of Bruch's membrane. Recombinant HTRA1 was incubated with dye-labeled elastin at 37°C for two hrs and the fluorescence of the released elastin peptides was monitored at 513 nm. It was found that HTRA1 degraded dye-labeled elastin. In an additional positive control assay, HTRA1 elastase activity was measured by fluorescence using various concentrations of elastase enzyme starting with 0.04ng/well of porcine pancreatic elastase and 100ng/well of ProteaImmun HtrA1. DQ Elastin substrate was 25ug/ml. See Figure 25 for the results.

Recombinant HtrA1 protein expressed and purified from Sf9 cells was purchased from ProteaImmun (Cat. # 30600103). The HtrA1 elastase activity was measured using a fluorescent EnzChekElastase Assay Kit from Invitrogen (Cat. #E12056) using the protocol provided, except substrate concentration was 12.5µg/mL. HtrA1 concentration was titrated from 200-12.5 ng/well in two-fold dilutions. Activity was measured in 30-minute increments for a total of 2 hours. See Figure 26 for the results.

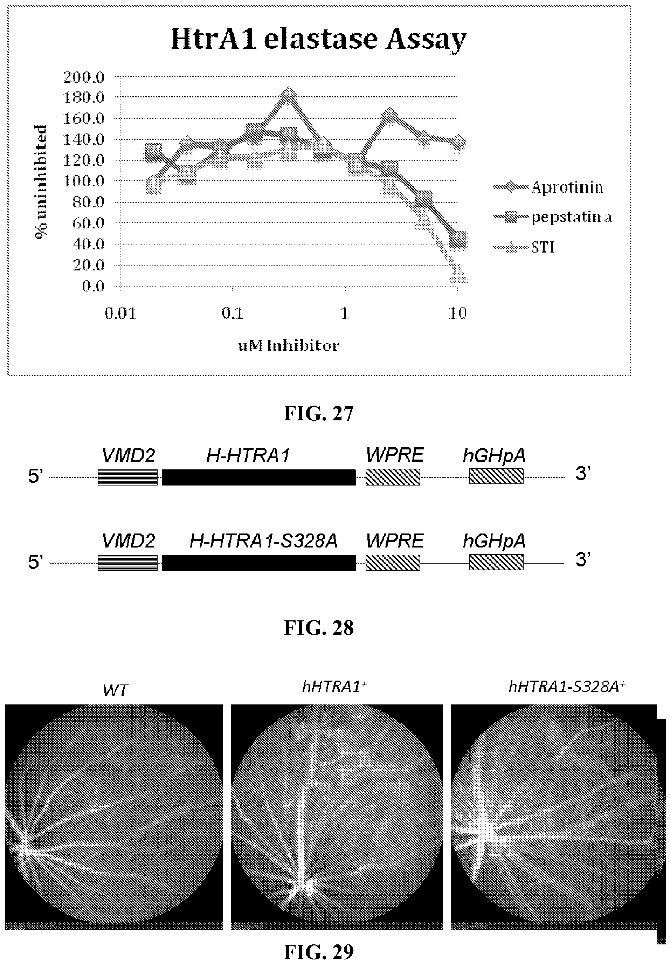

To test inhibition of HTRA1 elastase activity, HtrA1 was also run against several protease inhibitors. Recombinant HtrA1 protein expressed and purified from Sf9 cells was purchased from ProteaImmun (Cat. # 30600103). The HtrA1 elastase activity was measured using a fluorescent EnzChekElastase Assay Kit from Invitrogen (Cat. #E12056) using the protocol provided, except substrate concentration was 12.5 µg/mL. For protease inhibitor studies, recombinant HtrA1 at 100 ng/well was used. The following inhibitors were testedfor HtrA1 inhibition; Aprotinin, Pepstatin A, and Soy Trypsin Inhibitor (STI) all purchased from Sigma. Inhibitor concentration was titrated from 10-0.01 µM in two-fold dilutions and percent inhibition was measured after 2 hours and normalized to untreated samples. See Figure 27 for the results.

### 24. HTRA1 Modeling

HtrA1 Protease domain (Protein Databank ID 3NZI) modeled in complex with HtrA1 Kazal domain (homology model based on Anemonia elastase inhibitor, Proteion Databank ID 1Y1C.) The large ribbon (middle) represents the DPMFKLboroV peptide, which attaches to the active site serine.

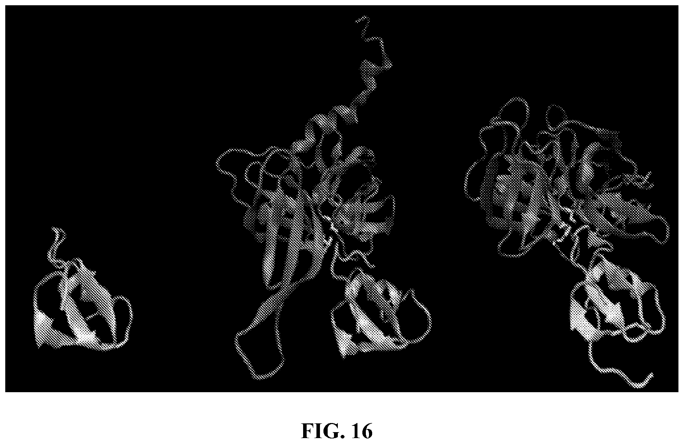

HtrA1 Protease domain (light blue, Protein Databank ID 3NZI) modeled in complex with HtrA1 Kazal domain (dark blue, homology model based on Anemonia elastase inhibitor, Proteion Databank ID 1Y1C.) The red ribbon represents the DPMFKLboroV peptide (SEQ ID NO. 13), which attaches to the active site serine. Specifically, HtrA1 protease domain was aligned with the co-structure *of Bos taurus* trypsinogen and *Sus scrofa* pancreatic secretory trypsin inhibitor using 3NZI Chain B residues 326-340 and 1TGS Chain Z residues 193-210. The HtrA1 Kazal homology model was then overlaid onto 1TGS Chain I. Structural alignments were conducted usingDeepView 4.0. Homology modeling was conducted using Swiss-Model (2-4). Sequence-based homology between trypsinogen and HtrA1 protease domain was identified using the Smith-Waterman algorithm. See Figures 15 and 16.

### a. Structural Comparison of the HtrA1 Protease Domain with Elastase Homologs

The NCBI structure comparison tool VAST (Vector Alignment Search Tool) was queried using structure 7EST (Interaction Of The Peptide Cf3-Leu-Ala-Nh-C6h4-Cf3(Tfla) With Porcine Pancreatic Elastase). Seven structures from the medium redundancy dataset were selected and aligned with HtrA1 protease domain structure 3NZI, as well as 1TGS, which is the co-structure of trypsinogen in complex with Pancreatic Secretory Inhibitor (Kazal type) used to model the complex between HtrA1 protease and Kazal domains. Three views of the structural alignment are shown in Figures 15 and 16; structures included 7EST, 1ELT, 1GI3, 1AZZ, 3GOV, 2BZ6, 1TGS, 3NZI, and 2HAL.

The NCBI structure comparison tool VAST (Vector Alignment Search Tool) was queried using structure 7EST (Interaction Of The Peptide Cf3-Leu-Ala-Nh-C6h4-Cf3(Tfla) With Porcine Pancreatic Elastase). Seven structures from the medium redundancy dataset were selected and aligned with HtrA1 protease domain structure 3NZI, as well as 1TGS, which is the co-structure of trypsinogen in complex with Pancreatic Secretory Inhibitor (Kazal type) used to model the complex between HtrA1 protease and Kazal domains. Three views of the structural alignment are shown in Figure 16; structures included 7EST, 1ELT, 1GI3, 1AZZ, 3GOV, 2BZ6, 1TGS, 3NZI, and 2HAL.

### b. HtrA1 Kazal Domain- Structural Comparison with Elastase Inhibitor Homologs and Redefinition of Boundaries

Templates for structural modeling of the HtrA1 Kazal domain were identified through a protein BLAST against Protein DataBank-deposited structures using HtrA1 residues 114-155 as the query sequence, which is the region currently identified through NCBI as the HtrA1 Kazal domain. Of all deposited structures, the sequences of Anemonia elastase inhibitor structures 1Y1B and 1Y1C best match the query sequence. The amino acid sequences of 1Y1B and 1Y1C are identical through the aligned region, which spans HtrA1 residues 116 - 155. Alignment statistics were: Identities = 18/40 (45%); Positives = 24/40 (60%); Gaps = 4/40 (10%). These analyses suggested that the HtrA1 Kazal domain functions as an elastase inhibitor, with the capability of inhibiting the 'protease' domain that functions as an elastase.

The 1Y1C structure was selected to generate a sequence-based structural alignment using the program Swiss-Model (2-4). Structural modeling indicated the presence of a disulfide bond in 1Y1C that could not be formed using HtrA1 residues 114-155. Extending the Kazal sequence to residues 111 - 155, including a proximal cysteine residue, allowed the missing disulfide to be modeled. Thus, residues 111 - 155 may more accurately reflect the boundaries of the HtrA1 Kazal domain.

### 25. HTRA1 Transgenic Mouse

A transgenic mice (*hHTRA1*⁺) overexpressing human HTRA1 (*hHTRA1*) in mouse RPE was generated. The transgene was driven by the RPE-specific human vitelliform macular dystrophy 2 (VMD2) promoter. To generate the HTRA1 transgene, a 1.5-kb human *HTRA1* cDNA (TAG stop codon was removed) (SEQ ID NO. 12) was amplified using primers with BamHI and XhoI overhangs from pcDNA3.1-CMV-VMD-hHtra1-*myc*-*His₆*, which was described in Jones, A. et al., Proc Natl Acad Sci U S A 108, 14578-14583 (2011). A myc-his₆ fragment was amplified from the same template using primers with XhoI and EcoRI overhangs. The destination plasmid, pAAV-CAG-Shuttle-WPRE was digested with BamHI and EcoRI and ligated with the amplified human *HTRA1* cDNA and the myc-his fragment in a three-piece ligation reaction. Subsequently, the human VMD2 promoter (-585 to +38 bp) was inserted as a KpnI-BamHI fragment before the HTRA1 cDNA. The transgene regions containing the VMD2 promoter, *hHTPAl-myc-His₆,* woodchuck post-transcriptional regulatory element (WPRE), and human growth hormone poly(A) were sequence-verified. See Figure 28. The transgene construct was excised by KpnI -RsrII digestion, gel-purified, and injected into C57BL/6 × CBA embryos at the University of Utah Transgenic/Gene Targeting Core Facility.

The phenotypes of the new transgenic *hHTRA1*⁺ mice were similar to those described in Jones, A. et al. Increased expression of multifunctional serine protease, HTRA1, in retinal pigment epithelium induces polypoidal choroidal vasculopathy in mice. Proc Natl Acad Sci U S A 108, 14578-14583 (2011), which is herein incoprated in its entirety by this reference.

### a. PCV Phenotype

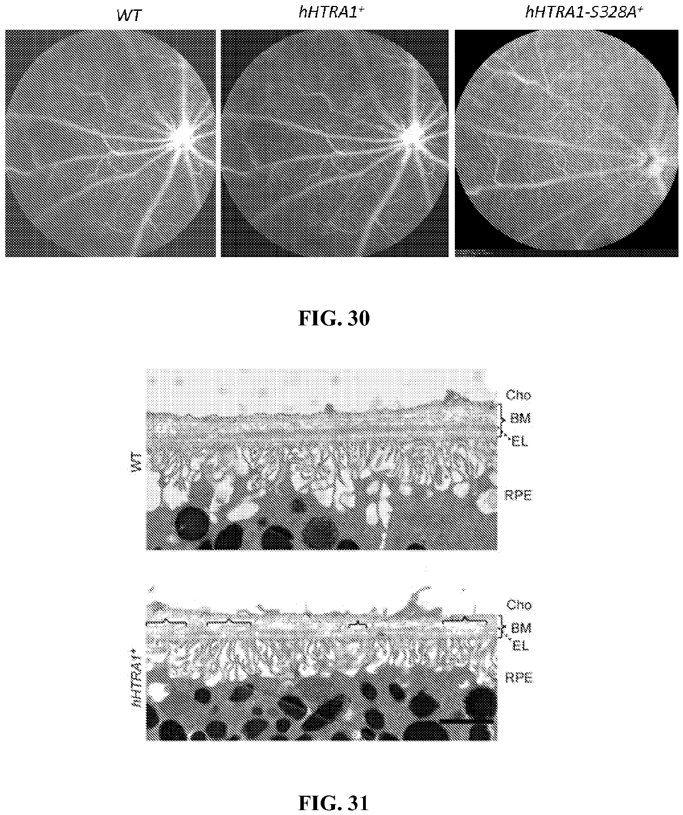

On indocyanine green angiography (ICGA), *hHTRA1*⁺ mice (1-4 month old) exhibited cardinal features of PCV bilaterally: 1) Numerous small hyperfluorescent dots (diameter less than 0.25 mm) consistent with microaneurysmal dilations ; 2) Large polypoidal lesions (diameter more than 0.45 mm) resembling grape clusters. None of the above features were observed in wild-type (WT) littermates. See Figure 29. Fluorescein angiography (FA) was normal, suggesting the retinal vasculature was not affected in those mice. See Figure 30.

### b. Degradation of Elastic Lamina of Bruch's Membrane

On ultrastructural analysis, the elastic lamina (EL) of the *hHTRA1*⁺ mice was fragmented, and interrupted by gaps of varying sizes. The degradation of EL shares close similarity to macular EL disruption associated with AMD lesions (see Chong et al. 2005). See Figure 31.

### c. Degradation of the Elastic Lamina of the Choroidal Vessels in hHTRA1⁺ Mice

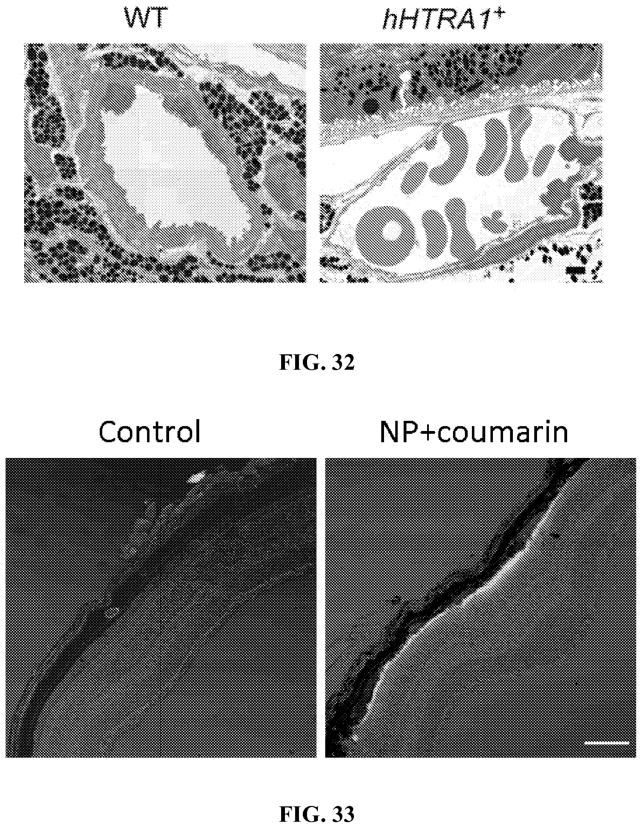

The PCV lesions in *hHTRA1*⁺ mice resulted from the exudates of compromised choroidal vessels. Ultrastructural analysis of *hHTRA1*⁺ mice showed marked attenuation of the choroidal vessels. The choroidal arteries in atrophic regions had reduced elastin content in both the elastic interna and elastic externa. The tunica media was severely degenerated or missing. See Figure 32.

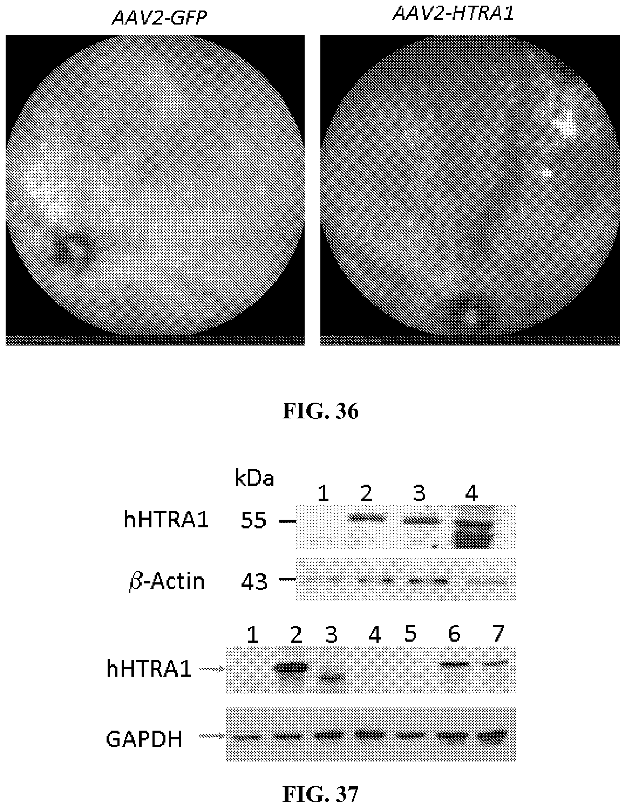

The explanation for the elastin degradation in both the Bruch's membrane and the choroid vessels in the HTRA1 mice is that the protease activity of overexpressed HTRA1, which is secreted from RPE, caused the degeneration. Since HTRA1 was not known to have elastase activity, an *in vitro* elastin degradation assay was performed using DQ elastin, a soluble elastin labeled with quenched BODIPY FL dye, as a substrate. Purified recombinant human HTRA1 degraded elastin with a specific activity of 4.4 ± 0.8 u/mg (n=3). This activity was ∼30 times less than that of porcine pancreas elastase (135 ± 5.5 u/mg, n=4), suggesting that the basal elastase activity of HTRA1 was low. To eliminate the possibility that the depletion of the elastic layers of the choroidal vessels and Bruch's membrane was the result of downregulation of elastin expression, we analyzed the protein level of soluble elastin in the RPE/choroid of and WT mice by western blot. The level of tropoelastin, the soluble precursor of elastin, was similar in both the *hHTRA1*⁺ mice and WT (Fig. 37), suggesting that the biosynthesis of elastin was not altered by HTRA1 overexpression. However, there was an increase of degraded elastin products (Fig. 31, bracket) in *hHTRA1*⁺*-PCV*⁺ mice in comparison with the WT and PCV-mice, suggesting that elastin degradation rather than elastin downregulation was likely the cause for the observed lesions in the Bruch's membrane and the choroid vessels in PCV+ mice. This is similar to what has been observed in human donors with AMD and with aberrant choriocapillaris lobules.

### d. HTRA1 Expression in the Mouse RPE

The expression levels of human HTRA1 in mouse RPE were estimated to be 50%- 80% of the HTRA1 expression in the published mouse line (Jones, A. et al.), which was 5.3 times that of HTRA1 protein in human RPE. In other words, the expression levels of human HTRA1 in mouse RPE of the transgenic lines were 2.7 - 4.2 times of the HTRA1 protein in human RPE.

### e. Negative Control hHTRA1-S328A⁺ Mouse

To test the hypothesis that the protease activity of HTRA1 was responsible for its role in PCV, we generated a negative control mice, h*HTRA1-S328A*⁺*.* The method in generating the mutant h*HTRA1-S328A*⁺ mice was the same as that used in generating the *hHTRA1*⁺ mice except that the S328 residue in the transgene was mutated to alanine by a PCR-based mutagenesis method.

There was no evidence of PCV in the h*HTRA1-S328A*⁺ mice by ICGA. FA was also normal. Additionally, the expression level of HTRA1-S328A (line 26) in mouse RPE was approximately 3 times that of HTRA1 in the new transgenic *hHTRA1*⁺ mice.

### f. Treating the hHTRA1⁺ mice with HTRA1 inhibitors reverses PCV phenotype

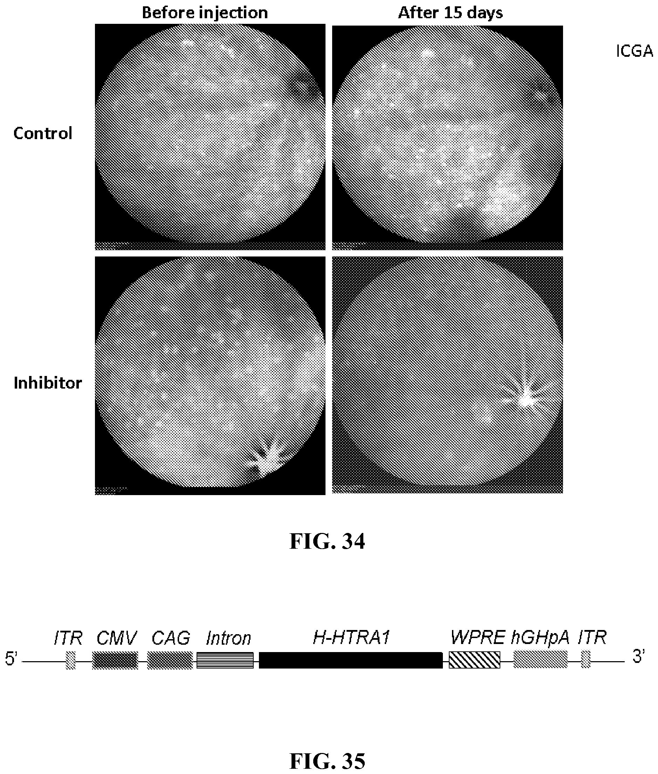

Truebestein et al (Nat Struct Mol Biol 18, 386-388, 2011) identified a peptide, DPMFKLboroV (SEQ ID NO. 13), that binds covalently to the active-site serine of HTRA1 via its C-terminal boronic acid group (IC50 = 2.6 µM). Since PCV occurs in the choroid, biodegradable polymer-based nanoparticles (NPs) were used to deliver DPMFKLboroV to the posterior segment of the eye of hHTRA1+ mice to achieve long term sustained release. Previous work demonstrated that intravitreally administered NPs transiently moved across the retina and preferentially localized to the RPE. NPs remained in the RPE for weeks to months after a single injection. Two types of NPs were prepared using polylactic acid/polylactic acid-polyethylene oxide (PLA/ PLA-PEO) following a published procedure: 1) NPs encapsulating a lipophilic fluorescent marker, 6- coumarin; 2) NPs encapsulating coumarin and HTRA1 inhibitor. The NPs were ∼ 100 nm in size based on electron microscopy. See Figure 34.

Adult WT mice were injected intravitreally with 1.5 µL NPs containing coumarin. See Figure 33. Four days after injection, NPs were concentrated in the RPE and outer segment. NPs were mainly located in the RPE afterward and were detectable at least 36 days after injection. Consistent with previous work, NP-injected eyes had normal retinal architecture with no signs of toxicity compared to the control eye. hHTRA1+ mice (2-month old) were injected with NPs containing inhibitor and coumarin or NPs containing only coumarin. Fifteen days after inhibitor injection, the lesion numbers were significantly reduced. In control NP-injected eyes, PCV lesions remained This observation supports supported the hypothesis that the elastase/protease activity of HTRA1 is responsible for the development of PCV.

### g. AAV2-HTRA1 infected mice

The human HTRA1 cDNA containing a full-length coding region and the C-terminal myc-His₆ tag² was subcloned into an AAV shuttle vector pAAV-CAG-Shuttle-WPRE under the CAG promoter. See Figure 35. AAV2-HTRA1 virus was produced commercially. The viral titer was 7.6 × 10¹² GC/mL. CD1 mice were injected with 2 µL AAV2-HTRA1 or control AAV2-GFP by subretinal injection at the age of ∼ 2 months as previously described. One month after injection, mice were imaged by ICGA. PCV lesions were observed similar to those in the mice. See Figure 36.

### 26. Genotype & Response to Anti-VEGF Therapy in Patients with Exudative AMD

Three hundred and thirty-nine study participants were recruited from consecutive patients presenting for initial or maintenance anti-VEGF therapy (Pegaptanib, ranibizumab, and/or Bevacizumab) for exudative AMD. Subjects were treated by one of four study-associated vitreoretinal surgeons according to current practice patterns for exudative AMD between 2005 and 2008. The treatment strategy employed by the participating physicians most closely resembled an 'as needed' treatment strategy as was employed in the PRONTO study (Fung et al., 2007). For subjects enrolled at the time of initial anti-VEGF therapy in one eye, all data was collected prospectively. For subjects enrolled during maintenance therapy, data were collected both retrospectively to the time of evaluation for the initial anti-VEGF treatment and prospectively from the time of enrollment. Eyes for the analyses presented herein were excluded for one or more of the following exclusion criteria: prior photodynamic therapy with Visudyne, prior therapy with intravitreal triamcinolone, prior periocular injection with anecortave acetate, advanced disciform scarring at the initiation of intravitreal anti-VEGF therapy, or insufficient follow-up to meet one of the study endpoints. Data collection included visual acuity, assessment of CNV leakage (either subretinal fluid on OCT or determined by fluorescein angiography), retinal pigment epithelial detachment (RPED) status (persistent or resolved), color photographs, and treatment regimen analysis during the treatment for one year or more after the initial treatment using anti-VEGF therapeutics. Visual acuity was measured on Snellen or ETDRS charts and values converted to LogMar values. A 0.2 LogMar change in vision (corresponding to a 2 line change) was considered to be a significant change in vision. CNV leakage was assessed primarily by Optical Coherence Tomography (OCT) and, when available, by fluorescein angiography. OCT was performed for nearly all eyes using a Stratus OCT 3 instrument. CNV leakage was assessed utilizing one or more of the following variables for each scan: central macular thickness, subretinal fluid, intraretinal fluid, and RPED status. For some eyes with large amounts of subretinal fluid, large RPED, or poor fixation, central macular thickness measurements were either unavailable (off the scale) or unreliable due to poor fixation. As such, it was necessary to make an assessment based on qualitative features (i.e., diminution or resolution of fluid or cysts) rather than an absolute central macular thickness measurement.

Subjects were categorized according to one of the following four anti-VEGF response patterns, which are based on rigid criteria developed by the PI and clinical collaborators: 1) *Early Responder without Maintenance:* Visual Acuity (VA) improved ≥ 0.2 LogMar after ≤3 injections regardless of retinal/subretinal/RPED fluid status or complete resolution of intraretinal/subretinal/RPED fluid and VA within 0.2 LogMar of pre-treatment VA. Additionally, these subjects never developed recurrent subretinal fluid after the 3rd injection so were designated as not requiring maintenance (even if some investigators continued injections for other reasons such as patient request or monocular status); 2) *Early Responder with Maintenance:* Visual acuity (VA) improved ≥ 0.2 LogMar after ≤3 injections regardless of retinal/subretinal/RPED fluid status or complete resolution of intraretinal/subretinal/RPED fluid and VA within 0.2 LogMar of pre-treatment VA. Additionally, these subjects developed recurrent subretinal fluid each time attempts were made to stop or extend treatment so were designated as requiring maintenance therapy beyond the third injection; 3) *Delayed Responder:* Visual acuity ≥ 0.2 LogMar with four or more treatments over a one year period or VA within 0.2 LogMar (no change in vision) with resolution of intraretinal/subretinal fluid (RPED may persist but not increase); or 4) *Nonresponder:* Visual acuity ≥ 2 LogMar worse after three or more injections within a one year period or VA within 0.2 LogMar (no change in vision) with no change or worsening in subretinal fluid or RPED after three or more injections within a one year period.

SNPs in the CFH (rs800292, rs12029785, rs551397 & rs106117), ARMS2 (rs10490923, rs2736911, and rs10490924), C3 (rs2230199), CFB (rs415667) and APOE genes, were genotyped by a combination of Applied Biosystems (Foster City, CA) Taqman® 5' nucleotidase assay, SSCP, direct sequencing, and Molecular Inversion Probe Genotyping (ParAllele Biosciences/Affymetrix). Genotypes and anti-VEGF response categories were tabulated in Microsoft Excel and imported into SAS 9.1.3. Analyses were run using each qualifying eye as a unit of observation. The SAS procedure FREQ was used to cross-tabulate genotype frequencies. The Pearson chi-square test was used for comparisons of each of the categorical variables and genotype frequencies for each of the SNPs assessed. Allele frequencies were tested using both the Pearson chi-square and Fisher's Exact tests. All P values were calculated with two-sided tests, and no correction was made for multiple testing. The three response categories ('early responder without maintenance', 'early responder with maintenance' and 'delayed responder') were compared individually and in combination with the 'non-responders'. Logistic-regression analysis was used to estimate odds ratios and 95% confidence intervals on the odds of significant genotypes. Odds ratios for categorical variables were estimated in relation to a reference category. Data were analyzed with the use of the SAS statistical software package, version 9.1.3.

A total of 242 eyes undergoing anti-VEGF therapy for exudative AMD (University of Iowa cohort), and that had no exclusion criteria, were included in this analysis. Strikingly, the analyses revealed a strongly significant (P= 0.0034) association between C3 genotype and the response of pre-existing exudative AMD to treatment with intravitreal anti-VEGF therapeutic agents. Specifically, the common functional polymorphism rs2230199 (Arg80Gly), which encodes for the C3S (slow) and C3F (fast) protein isoforms respectively, was associated with differential response to treatment when all three categories of 'responders' were compared to 'non-responders'. Individuals are more likely to respond to anti-VEGF therapy if they are GC [P=0.0016; odds ratio (OR) = 5.48, 95% confidence interval (1.90-15.75)] or CC [P=0.01; OR = 3.65, 95% CI (1.35-9.86)] for the rs2230199 C3 variant. Put another way, patients are more likely not to respond to therapy with anti-VGF agents if they carry a GG genotype. When distinguishing between the sub-categories of responders, the C3 association was most significant in the 'early responders with maintenance' group (P=0.0004).

No associations in response to treatment between the responder and nonresponder groups were observed for any of the CFH, CFB, ARMS2 or APOE genotypes assessed. There was a trend toward significance (P = 0.035) between responders and non-responders, however, for the ARMS2 rs10490923 variant.

### 27. The ratio of the Diameters of Retinal Arteries or Veins Further Indicates the Diagnosis of the Aberrent Choriocapillaris Lobule Disease

The diameters of retinal arteries and veins, and the ratio of these parameters (A:V), were measured in fundus photographs of 19 patients with aberrant choriocapillaris lobules, using IVAN (Vessel Measurement System, Department of Ophthalmology, University of Wisconsin, Madison, WI), a semi-automated system used to measure retinal vessel widths from a digital (or digitized) retinal images (Knudtson, MD et al; Variation associated with measurement of retinal vessel diameters at different points in the pulse cycle 2004; Br J Ophthalmol 88:57). This studv was conducted to determine whether an objective assessment of retinal vessel calibers from photographs would provide cardiovascular risk information that is unique to a specific phenotype. Computer assisted measurements of example, have shown that retinal arteriolar narrowing was associated with incident coronary heart disease in women (Wong TY, et al. Retinal arteriolar narrowing and incident coronary heart disease in men and women: The Atherosclerosis Risk in Communities Study, JAMA 2002; 287:1153-59) and incident stroke and diabetes in men and women (Wong TY, et al. Retinal microvascular abnormalities and incident stroke: The Atherosclerosis Risk in the Communities Study, Lancet 2001; 358:1134-40; Wong TY, et al. Retinal arteriolar narrowing and risk of diabetes mellitus in middle-aged persons, JAMA 2002; 287:2528-33; De Silva DA, et al., Neurology 2011 Aug 30; 77:896-903).

The automated components included placement of the overlaying grid centered on the optic disc, vessel type identification, and width measurements for vessels. The color blue was used to denote veins/venules (V) and the color red was used to denote arteries/arterioles (A) in the screen displays. The data table on the control window displayed the mean width and standard deviation for each measured vessel and located each vessel by its clockwise angle in degrees. The vessels were automatically typed as A or V; in the case of uncertainty, the vessel was typed as an A.

Manual components included the option to override any of the initial automated decisions or measurements. This included adjusting the placement of the grid, changing the vessel type, deleting vessels, re-measuring vessels, and adding significant vessels missed in the initial automation. The Modified ARIC grid was composed of three concentric circles, which demarcated an average optic disc, Zone A defined as the region from the disc margin to 1h diameter from the disc, and Zone B defined as the region from 1h disc diameter from the disc to 1 disc diameter from the disc. All retinal vessels were measured in Zone B. IVAN was coded to represent a grid with a center circle (disc diameter) of 1800 microns, the size of which was set by the scale factor. A scale factor was used to adjust for the magnification differences introduced by camera optics, scanner resolution, or patient position. A configuration procedure was used to set parameters for vessel tracing for each dataset (i.e. images from the same study obtained using the same photographic method). Only the width data from the six largest V and six largest A are required to calculate the A:V ratio using the Knudtson formula. No branch (daughter) width data is required.

Patients with aberrant choriocapillaris lobules exhibited a mean average A:V ratio of 0.88 (0.60-1.48) (Scott & Jason - see file 'RPD_ArteryVeinRatios_2.22.10.xlsx'). This ratio was significantly different than that previously published for patients without AMD (mean A:V=0.64), with early stage AMD (mean A:V=0.63), and with late stage AMD (mean A:V=0.64) (Jeganthan V.S.E. et al., Retinal Vascular Caliber and Age-related Macular Degeneration: The Singapore Malay Eye Study, Am J Ophthalmol 2008, 146:954-959).

A preliminary assessment of 20 patients with aberrant choriocapillaris lobules detected on color photographs (visible only in the superior macula), 20 patients with AMD but no aberrant choriocapillaris lobules, and 20 patients without AMD or aberrant choriocapillaris lobules, was conducted. Measurements of an average of six vessels coming out of the disc were made. The superior artery (but not the superior vein) was significantly dilated compared to that of the inferior artery only in the patients with superior aberrant choriocapillaris lobules. These data support a concept that the retina/choroid is ischemic, causing arterial dilation, when aberrant choriocapillaris lobules occur only in the superior macula.

Other aspects of the invention will be apparent to those skilled in the art from consideration of the specification and practice of the invention described herein.

### G. REFERENCES

1. Arnold JJ, Sarks SH, Killingsworth MC, Sarks JP. Reticular pseudodrusen: a risk factor in age-related maculopathy. Retina 1995;15(3):183-91.
2. Bird AC, Bressler NM, Bressler SB, Chisholm IH, Coscas G, Davis MD, et al. An international classification and grading system for age-related maculopathy and age-related macular degeneration. The International ARM Epidemiological Study Group. Surv Ophthalmol. 1995 Mar-Apr;39(5):367-74.
3. Cameron DJ, Yang Z, Gibbs D, Chen H, Kaminoh Y, Jorgensen A, Zeng J, Luo L, Brinton E, Brinton G, Brand JM, Bernstein PS, Zabriskie NA, Tang S, Constantine R, Tong Z, Zhang K. HTRA1 variant confers similar risks to geographic atrophy and neovascular age-related macular degeneration. Cell Cycle. 2007;6(9):1122-5.
4. Chan CC, Shen D, Zhou M, Ross RJ, Ding X, Zhang K, Green WR, Tuo J. Human HtrA1 in the archived eyes with age-related macular degeneration. Trans Am Ophthalmol Soc. 2007;105:92-7; discussion 97-8.
5. Chong NH, Keonin J, Luthert PJ, Frennesson CI, Weingeist DM, Wolf RL, Mullins RF, Hageman GS. Decreased thickness and integrity of the macular elastic layer of Bruch's membrane correspond to the distribution of lesions associated with age-related macular degeneration. Am J Pathol. 2005 Jan; 166(1):241-51.
6. Cohen SY, Dubois L, Tadayoni R, et al. Prevalence of reticular pseudodrusen in age-related macular degeneration with newly diagnosed choroidal neovascularization. Br J Ophthalmol 2007;91(3):354-9.
7. Fung AE, Lalwani GA, Rosenfeld PJ, Dubovy SR, Michels S, Feuer WJ, Puliafito CA, Davis JL, Flynn HW Jr, Esquiabro M. An optical coherence tomography-guided, variable dosing regimen with intravitreal ranibizumab (Lucentis) for neovascular age-related macular degeneration. Am J Ophthalmol. 2007;143(4):566-83.
8. Hageman GS, Anderson DH, Johnson LV, et al. A common haplotype in the complement regulatory gene factor H (HF1/CFH) predisposes individuals to age-related macular degeneration. Proc Natl Acad Sci U S A 2005;102(20):7227-32.
9. Hageman G, Mullins R. Molecular composition of drusen as related to substructural phenotype. Mol Vis. 1999;5:28.
10. Hageman G, Mullins R, Russell S, Johnson L, Anderson D. Vitronectin is a constituent of ocular drusen and the vitronectin gene is expressed in human retinal pigmented epithelial cells. FASEB J. 1999;13:477-484.
11. Helb, H. M., P. C. Issa, M. Fleckenstein, S. Schmitz-Valckenberg, H. P. Scholl, C. H. Meyer, N. Eter, and F. G. Holz. 2009. Clinical evaluation of simultaneous confocal scanning laser ophthalmoscopy imaging combined with high-resolution, spectral-domain optical coherence tomography. Acta Ophthalmol.
12. Klaver CC, Assink JJ, van Leeuwen R, Wolfs RC, Vingerling JR, Stijnen T, et al. Incidence and progression rates of age-related maculopathy: the Rotterdam Study. Invest Ophthalmol Vis Sci. 2001 Sep;42(10):2237-41.
13. Klein R, Meuer SM, Knudtson MD, et al. The epidemiology of retinal reticular drusen. Am J Ophthalmol 2008;145(2):317-26.
14. Leveziel N, Puche N, Richard F, et al. Genotypic influences on severity of exudative age-related macular degeneration. Invest Ophthalmol Vis Sci Epub 2009 Dec 30:09-4423.
15. Mimoun G, Soubrane G, Coscas G. Macular drusen [in French]. J Fr Ophtalmol 1990;13:511-30.
16. Pircher, M., E. Gotzinger, O. Findl, S. Michels, W. Geitzenauer, C. Leydolt, U. Schmidt-Erfurth, and C. K. Hitzenberger. 2006. Human macula investigated in vivo with polarization-sensitive optical coherence tomography. Invest Ophthalmol Vis Sci 47 (12):5487-5494.
17. Prenner JL, Rosenblatt BJ, Tolentino MJ, Ying GS, Javornik NB, Maguire MG, Ho AC; CNVPT Research Group. Risk factors for choroidal neovascularization and vision loss in the fellow eye study of CNVPT. Retina. 2003 Jun;23(3):307-14.
18. Rivera A, Fisher SA, Fritsche LG, et al. Hypothetical LOC387715 is a second major susceptibility gene for age-related macular degeneration, contributing independently of complement factor H to disease risk. Hum Mol Genet 2005;14(21):3227-36.
19. Rudolf M, Malek G, Messinger JD, et al. Sub-retinal drusenoid deposits in human retina: organization and composition. Exp Eye Res 2008;87:402- 8.
20. Russell S, Mullins R, Schneider B, Hageman G. Basal laminar drusen are indistinguishable in location, substructure, and composition from drusen associated with aging and age-related macular degeneration. Am J Ophthalmol. 2002;129:205-214.
21. Smith RT, Sohrab M, Busuioc M, Barile G. Reticular macular disease. Am J Ophthalmol 2009;148(5):733-43.
22. Smith RT, Chan JK, Busuoic M, et al. Autofluorescence characteristics of early, atrophic, and high-risk fellow eyes in age-related macular degeneration. Invest Ophthalmol Vis Sci 2006;47(12):5495-504.
23. Tuo J, Ross RJ, Reed GF, Yan Q, Wang JJ, Bojanowski CM, Chew EY, Feng X, Olsen TW, Ferris FL 3rd, Mitchell P, Chan CC. The HtrA1 promoter polymorphism, smoking, and age-related macular degeneration in multiple case-control samples. Ophthalmology. 2008;115(11):1891-8.
24. Wang JJ, Rochtchina E, Lee AJ, et al. Ten-year incidence and progression of age-related maculopathy: the Blue Mountains Eye Study. Ophthalmology 2007;114:92- 816.
25. Yang Z, Camp NJ, Sun H, Tong Z, Gibbs D, Cameron DJ, Chen H, Zhao Y, Pearson E, Li X, Chien J, Dewan A, Harmon J, Bernstein PS, Shridhar V, Zabriskie NA, Hoh J, Howes K, Zhang K. A variant of the HTRA1 gene increases susceptibility to age-related macular degeneration. Science 2006;314(5801):992-3.
26. Zweifel SA, Spaide RF, Curcio CA, et al. Reticular pseudodrusen are subretinal drusenoid deposits. Ophthalmology 2010a;117:303-12.
27. Zweifel SA, Imamura Y, Spaide TC, Fujiwara T, Spaide RF. Prevalence and Significance of Subretinal Drusenoid Deposits (Reticular Pseudodrusen) in Age-Related Macular Degeneration. Ophthalmology. 2010b May 14. [Epub ahead of print]

## Claims

1. An elastase inhibitor for use in the treatment of a disease **characterized by** the presence of aberrant choriocapillaris lobules in the macula of an eye of a subject, wherein said aberrant choriocapillaris lobules are **characterized by** decreased or absent perfusion associated with partial or complete closure or occlusion of the choriocapillaris capillaries, or choroidal arterioles,
wherein the elastase inhibitor inhibits the elastase activity of High Temperature Requirement Serine Peptidase 1 (HTRA1).

2. The elastase inhibitor for use of claim 1, wherein the elastase inhibitor is alpha-1 antitrypsin, ZD0892, SPIPm2, ONO-5046, 1,4-bisphenyl-1,4-dihydropyridine, 2-hydroxy and 2-aminodihydropyridines, a dolastatin or dolasatin analog, or a lyngbyastatin or a lyngbyastatin analog.

3. The elastase inhibitor for use of claim 1, wherein the elastase inhibitor is DPMFKLboroV.

4. The elastase inhibitor for use of claim 1, wherein the elastase inhibitor hybridizes or binds to the protease recognition pocket of HTRA1.

5. The elastase inhibitor for use of any of the preceding claims, wherein the aberrant choriocapillaris lobules present in the eye of the subject are identified by auto fluorescent imaging
techniques, infrared imaging techniques, optical coherence tomography (OCT), Stratus optical coherence tomography (Stratus OCT), Fourier-domain optical coherence tomography (Fd-OCT), two-photon-excited fluorescence (TPEF) imaging, adaptive optics scanning laser ophthalmoscopy (AOSLO), scanning laser ophthalmoscopy, near-infrared imaging combined with spectral domain optical coherence tomography (SD-OCT), color fundus photography, fundus auto fluorescence imaging, red-free imaging, fluorescein angiography, indocyanin green angiography, multifocal electroretinography (ERG) recording, microperimetry, color Doppler optical coherence tomography (CDOCT), visual field assessment, Heidelberg Spectralis, the Zeiss Cirrus, the Topcon 3D OCT 2000, the Optivue RTVue SD-OCT, the Opko OCT SLO, the NIDEK F-10, or the Optopol SOCT Copernicus HR.

6. The elastase inhibitor for use of any of the preceding claims, wherein the partial closure or occlusion of the choroidal arterioles is represented by a diameter ratio of arteries/ arterioles to veins/venules of 0.6 to 1.48.

## Patentansprüche

1. Elastase-Inhibitor zur Anwendung bei der Behandlung einer Krankheit, die durch das Vorhandensein von anormalen Choriocapillaris-Lobuli in der Makula eines Auges eines Subjekts gekennzeichnet ist, wobei die besagten anormalen Choriocapillaris-Lobuli durch verminderte oder fehlende Perfusion gekennzeichnet sind, die mit teilweisem oder vollständigen Verschluss oder Okklusion der Choriocapillaris-Kapillaren oder choroidalen Arteriolen verbunden ist,
wobei der Elastase-Inhibitor die Elastaseaktivität der Hochtemperatur-abhängigen-Peptidase 1 (HTRA1) hemmt.

2. Elastaseinhibitor zur Anwendung nach Anspruch 1, wobei der Elastaseinhibitor Alpha-1-Antitrypsin, ZD0892, SPIPm2, ONO-5046, 1,4-Bisphenyl-1,4-dihydropyridin, 2-Hydroxy- und 2-Aminodihydropyridine, ein Dolastatin oder ein Dolasatin-Analogon oder ein Lyngbyastatin oder ein Lyngbyastatin-Analogon ist.

3. Elastase-Inhibitor zur Anwendung nach Anspruch 1, wobei der Elastase-Inhibitor DPMFKLboroV ist.

4. Elastase-Inhibitor zur Anwendung nach Anspruch 1, wobei der Elastase-Inhibitor an die Proteas-Erkennungstasche von HTRA1 hybridisiert oder daran bindet.

5. Elastase-Inhibitor zur Anwendung nach einem der vorangehenden Ansprüche, wobei die anormalen Choriocapillaris-Lobuli, die im Auge des Subjekts vorhanden sind, durch automatische Fluoreszenzbildgebung, Infrarot-Bildgebungstechniken, optische Kohärenztomographie (OCT), Stratus optische Kohärenztomographie (Stratus OCT), Fourier-Domänen optische Kohärenztomographie (Fd-OCT), Zweiphotonen-angeregte Fluoreszenztomographie (TPEF), Scan-Laser-Ophthalmoskopie mit adaptiver Optik (AOSLO), Scan-Laser-Ophthalmoskopie, Nahinfrarot-Bildgebung in Kombination mit optischer Kohärenztomographie (SD-OCT), Farb-Fundusfotografie, Fundus-Autofluoreszenz-Bildgebung, rotfreie Bildgebung, Fluorescein-Angiographie, Indocyaningrün-Angiographie, Aufzeichnung mit Multifokaler Elektroretinographie (ERG), Mikroperimetrie, Farb-Doppler-Optische Kohärenztomographie (CDOCT), Gesichtsfelduntersuchung, Heidelberg Spectralis, Zeiss Cirrus, das Topcon 3D OCT 2000, Optivue RTVue SD-OCT, Opko OCT SLO, das NIDEK F-10 oder das Optopol SOCT Copernicus HR identifiziert werden.

6. Elastase-Inhibitor zur Anwendung nach einem der vorangehenden Ansprüche, wobei der teilweise Verschluss oder die Okklusion der choroidalen Arteriolen durch ein Durchmesserverhältnis von Arterien/Arteriolen zu Venen/Venolen von 0,6 bis 1,48 repräsentiert wird.

## Revendications

1. Inhibiteur d'élastase à utiliser dans le traitement d'une maladie **caractérisée par** la présence de lobules de choriocapillaire anormaux dans la macula d'un oeil d'un sujet, dans lequel lesdits lobules de choriocapillaire anormaux sont **caractérisés par** une perfusion diminuée ou absente associée à une fermeture ou une occlusion partielle ou complète des capillaires de choriocapillaire, ou des artérioles choroïdiennes,
dans lequel l'inhibiteur d'élastase inhibe l'activité d'élastase de la Sérine Peptidase à Exigence de Température Élevée 1 (HTRA1).

2. Inhibiteur d'élastase à utiliser selon la revendication 1, dans lequel l'inhibiteur d'élastase est l'alpha-1 antitrypsine, ZD0892, SPIPm2, ONO-5046, 1,4-bisphényl-1,4-dihydropyridine, 2-hydroxy et 2-aminodihydropyridines, une dolastatine ou un analogue de dolastatine, ou une lyngbyastatine ou un analogue de lyngbyastatine.

3. Inhibiteur d'élastase à utiliser selon la revendication 1, dans lequel l'inhibiteur d'élastase est DPMFKLboroV.

4. Inhibiteur d'élastase à utiliser selon la revendication 1, dans lequel l'inhibiteur d'élastase s'hybride ou se lie à la poche de reconnaissance de protéase de HTRA1.

5. Inhibiteur d'élastase à utiliser selon l'une quelconque des revendications précédentes, dans lequel les lobules de choriocapillaire anormaux présents dans l'oeil du sujet sont identifiés par des techniques d'imagerie par autofluorescence, des techniques d'imagerie par infrarouge, une tomographie par cohérence optique (OCT), une tomographie par cohérence optique Stratus (Stratus OCT), une tomographie par cohérence optique dans le Domaine de Fourier (Fd-OCT), une imagerie par fluorescence à excitation biphotonique (TPEF), une ophtalmoscopie laser à balayage optique adaptatif (AOSLO), une ophtalmoscopie laser à balayage, une imagerie par infrarouge proche combinée à une tomographie par cohérence optique dans le domaine spectral (SD-OCT), une photographie en couleurs du fond d'oeil, une imagerie par autofluorescence du fond d'oeil, une imagerie en lumière anérythre, une angiographie à la fluorescéine, une angiographie au vert d'indocyanine, un enregistrement d'électrorétinographie multifocale (ERG), une micropérimétrie, une tomographie en couleurs par cohérence optique à effet Doppler (CDOCT), une évaluation de champ visuel, le Spectralis d'Heidelberg, le Cirrus de Zeiss, le Topcon 3D OCT 2000, l'Optivue RTVue SD-OCT, l'Opko OCT SLO, le NIDEK F-10, ou l'Optopol SOCT Copernicus HR.

6. Inhibiteur d'élastase à utiliser selon l'une quelconque des revendications précédentes, dans lequel la fermeture ou l'occlusion partielle des artérioles choroïdiennes est représentée par un rapport de diamètre des artères/artérioles sur les veines/veinules de 0,6 à 1,48.


### SEQUENCE LISTING

<110> University of Utah Research Foundation
   Hagemen, Gregory
   Fu, Yingbin
<120> METHODS OF DIAGNOSING AND TREATING VASCULAR ASSOCIATED MACULOPATHY AND SYMPTOMS THEREOF
<130> 21101.0227P1
<150> 61/452,944
   <151> 2011-03-15
<150> 61/452,964
   <151> 2011-03-15
<150> 61/452,950
   <151> 2011-03-15
<160> 13
<170> PatentIn version 3.5
<210> 1
   <211> 14
   <212> PRT
   <213> Artificial Sequence
<220>
   <223> Description of Artificial Sequence: note = Synthetic Construct
<400> 1
<210> 2
   <211> 12
   <212> PRT
   <213> Artificial Sequence
<220>
   <223> Description of Artificial Sequence: note = Synthetic Construct
<400> 2
   Pro Ala Ser Ala Thr Val Arg Arg Arg Ala Gln Cys
   1               5                   10
<210> 3
   <211> 14
   <212> PRT
   <213> Artificial Sequence
<220>
   <223> Description of Artificial Sequence: note = Synthetic Construct
<400> 3
   Ser Arg Arg Ser Glu Arg Leu His Arg Pro Pro Val Ile Cys
           1               5                   10
<210> 4
   <211> 11
   <212> PRT
   <213> Artificial Sequence
<220>
   <223> Description of Artificial Sequence: note = Synthetic Construct
<400> 4
   Cys Gly Ser Asp Ala Asn Thr Tyr Ala Asn Leu
   1               5                   10
<210> 5
   <211> 14
   <212> PRT
   <213> Artificial Sequence
<220>
   <223> Description of Artificial Sequence: note = Synthetic Construct
<400> 5
   Cys Gly Gln Gly Gln Glu Asp Pro Asn Ser Leu Arg His Lys
   1               5                   10
<210> 6
   <211> 14
   <212> PRT
   <213> Artificial Sequence
<220>
   <223> Description of Artificial Sequence: note = Synthetic Construct
<400> 6
   Ser His Asp Arg Gln Ala Lys Gly Lys Ala Ile Thr Lys Cys
   1               5                   10
<210> 7
   <211> 14
   <212> PRT
   <213> Artificial Sequence
<220>
   <223> Description of Artificial Sequence: note = Synthetic Construct
<400> 7
   Cys Pro Asp Thr Pro Ala Glu Ala Gly Gly Leu Lys Glu Asn
   1               5                   10
<210> 8
   <211> 18
   <212> PRT
   <213> Artificial Sequence
<220>
   <223> Description of Artificial Sequence: note = Synthetic Construct
<400> 8
   Leu Asp Pro Gly Val Gly Gly Glu Gly Ala Ser Asp Lys Gln Arg Ser
   1               5                   10                  15
   Lys Cys
<210> 9
   <211> 1450
   <212> DNA
   <213> Artificial Sequence
<220>
   <223> Description of Artificial Sequence: note = Synthetic Construct
<400> 9
   atggtaccat atgcagattc cgcgtgcagc actgctgcca ctgttactgc tgctgctggc      60
   cgcaccggcg agcgcgcaac tgtctcgtgc gggtcgtagc gcaccgctgg cagcgggttg     120
   tccggatcgt tgcgaaccgg cgcgttgtcc gccgcagccg gaacattgcg aaggcggtcg     180
   tgcgcgtgat gcgtgcggct gctgcgaagt ttgcggtgcg ccggaaggtg cggcgtgtgg     240
   cctgcaagaa ggcccgtgcg gtgaaggtct gcaatgcgtg gtgccgtttg gtgttccggc     300
   gtctgccacc gttcgtcgtc gtgcgcaggc gggtctgtgt gtgtgcgcga gcagcgaacc     360
   ggtgtgcggc agcgatgcga acacctatgc gaacctgtgc caactgcgtg ctgcgagccg     420
   tcgtagcgaa cgtctgcatc gtccgccggt gattgtgctg caacgtggcg cgtgtggtca     480
   gggccaggaa gatccgaaca gcctgcgtca taaatataac tttatcgcgg atgtggtgga     540
   aaaaattgca ccggcggtgg tgcatattga actgtttcgc aaactgccgt ttagcaaacg     600
   tgaagtgccg gtggcgagcg gcagcggctt tattgtgagc gaagatggcc tgattgtgac     660
   caacgcgcat gtggtgacca acaaacatcg tgtgaaagtg gaactgaaaa acggcgcgac     720
   ctatgaagcg aaaatcaaag atgtggatga aaaagcggat attgcgctga tcaaaattga     780
   tcatcagggc aaactgccgg ttctgttact gggccgttct agcgaactgc gtccgggcga     840
   atttgtggtg gcgattggca gcccgtttag cctgcaaaac accgtgacca ccggcattgt     900
   gagcaccacc cagcgtggcg gcaaagaact gggcctgcgt aacagcgata tggattacat     960
   tcagaccgat gcgattatta actatggcaa cgcgggcggt ccgctggtga acctggatgg    1020
   cgaagtgatt ggcattaaca ccctgaaagt gaccgcgggc attagcttcg cgattccgag    1080
   cgataaaatc aaaaaattcc tgaccgaaag ccatgatcgt caggcgaaag gcaaagcgat    1140
   caccaaaaaa aaatacatcg gcatccgtat gatgagcctg accagcagca aagcgaaaga    1200
   actgaaagat cgtcatcgtg attttccgga tgtgattagc ggcgcgtata ttattgaagt 1260
   gattccggat actccggcgg aagcgggcgg tctgaaagaa aacgatgtga tcattagcat 1320
   taacggccag agcgtggtga gcgcgaacga tgtgagcgat gtgattaaac gtgaaagcac 1380
   cctgaacatg gtggtgcgtc gtggcaacga agatattatg attaccgtga tcccggaaga 1440
   aattgatccg 1450
<210> 10
   <211> 7083
   <212> DNA
   <213> Artificial Sequence
<220>
   <223> Description of Artificial Sequence: note = Synthetic Construct
<400> 10
   tacgtagagc agaaactcat ctctgaagag gatctgcatc accatcacca tcactagtga 60
   tccgttcaac tagcagacca ttatcaacaa aatactccaa ttggcgatgg ccctgtcctt 120
   ttaccagaca accattacct gtcgacacaa tctgcccttt ccaaagatcc caacgaaaag 180
   cgtgaccaca tggtccttct tgagtttgta actgctgctg ggattacaca tggcatggat 240
   gagctctaca aataatgaat taaacccgct gatcagcctc gactgtgcct tctagttgcc 300
   agccatctgt tgtttgcccc tcccccgtgc cttccttgac cctggaaggt gccactccca 360
   ctgtcctttc ctaataaaat gaggaaattg catcgcattg tctgagtagg tgtcattcta 420
   ttctgggggg tggggtgggg caggacagca agggggagga ttgggaagac aatagcaggc 480
   atgctgggga tgcggtgggc tctatggctt ctgaggcgga aagaaccagc tggggctcta 540
   gggggtatcc ccacgcgccc tgtagcggcg cattaagcgc ggcgggtgtg gtggttacgc 600
   gcagcgtgac cgctacactt gccagcgccc tagcgcccgc tcctttcgct ttcttccctt 660
   cctttctcgc cacgttcgcc ggctttcccc gtcaagctct aaatcggggg ctccctttag 720
   ggttccgatt tagtgcttta cggcacctcg accccaaaaa acttgattag ggtgatggtt 780
   cacgtagtgg gccatcgccc tgatagacgg tttttcgccc tttgacgttg gagtccacgt 840
   tctttaatag tggactcttg ttccaaactg gaacaacact caaccctatc tcggtctatt 900
   cttttgattt ataagggatt ttgccgattt cggcctattg gttaaaaaat gagctgattt 960
   aacaaaaatt taacgcgaat taattctgtg gaatgtgtgt cagttagggt gtggaaagtc 1020
   cccaggctcc ccagcaggca gaagtatgca aagcatgcat ctcaattagt cagcaaccag 1080
   gtgtggaaag tccccaggct ccccagcagg cagaagtatg caaagcatgc atctcaatta 1140
   gtcagcaacc atagtcccgc ccctaactcc gcccatcccg cccctaactc cgcccagttc 1200
   cgcccattct ccgccccatg gctgactaat tttttttatt tatgcagagg ccgaggccgc 1260
   ctctgcctct gagctattcc agaagtagtg aggaggcttt tttggaggcc taggcttttg 1320
   caaaaagctc ccgggagctt gtatatccat tttcggatct gatcaagaga caggatgagg 1380
   atcgtttcgc atgattgaac aagatggatt gcacgcaggt tctccggccg cttgggtgga 1440
   gaggctattc ggctatgact gggcacaaca gacaatcggc tgctctgatg ccgccgtgtt 1500
   ccggctgtca gcgcaggggc gcccggttct ttttgtcaag accgacctgt ccggtgccct 1560
   gaatgaactg caggacgagg cagcgcggct atcgtggctg gccacgacgg gcgttccttg 1620
   cgcagctgtg ctcgacgttg tcactgaagc gggaagggac tggctgctat tgggcgaagt 1680
   gccggggcag gatctcctgt catctcacct tgctcctgcc gagaaagtat ccatcatggc 1740
   tgatgcaatg cggcggctgc atacgcttga tccggctacc tgcccattcg accaccaagc 1800
   gaaacatcgc atcgagcgag cacgtactcg gatggaagcc ggtcttgtcg atcaggatga 1860
   tctggacgaa gagcatcagg ggctcgcgcc agccgaactg ttcgccaggc tcaaggcgcg 1920
   catgcccgac ggcgaggatc tcgtcgtgac ccatggcgat gcctgcttgc cgaatatcat 1980
   ggtggaaaat ggccgctttt ctggattcat cgactgtggc cggctgggtg tggcggaccg 2040
   ctatcaggac atagcgttgg ctacccgtga tattgctgaa gagcttggcg gcgaatgggc 2100
   tgaccgcttc ctcgtgcttt acggtatcgc cgctcccgat tcgcagcgca tcgccttcta 2160
   tcgccttctt gacgagttct tctgagcggg actctggggt tcgcgaaatg accgaccaag 2220
   cgacgcccaa cctgccatca cgagatttcg attccaccgc cgccttctat gaaaggttgg 2280
   gcttcggaat cgttttccgg gacgccggct ggatgatcct ccagcgcggg gatctcatgc 2340
   tggagttctt cgcccacccc aacttgttta ttgcagctta taatggttac aaataaagca 2400
   atagcatcac aaatttcaca aataaagcat ttttttcact gcattctagt tgtggtttgt 2460
   ccaaactcat caatgtatct tatcatgtct gtataccgac gacctctagc tagagcttgg 2520
   cgtaatcatg gtcatagctg tttcctgtgt gaaattgtta tccgctcaca attccacaca 2580
   acatacgagc cggaagcata aagtgtaaag cctggggtgc ctaatgagtg agctaactca 2640
   cattaattgc gttgcgctca ctgcccgctt tccagtcggg aaacctgtcg tgccagctgc 2700
   attaatgaat cggccaacgc gcggggagag gcggtttgcg tattgggcgc tcttccgctt 2760
   cctcgctcac tgactcgctg cgctcggtcg ttcggctgcg gcgagcggta tcagctcact 2820
   caaaggcggt aatacggtta tccacagaat caggggataa cgcaggaaag aacatgtgag 2880
   caaaaggcca gcaaaaggcc aggaaccgta aaaaggccgc gttgctggcg tttttccata 2940
   ggctccgccc ccctgacgag catcacaaaa atcgacgctc aagtcagagg tggcgaaacc 3000
   cgacaggact ataaagatac caggcgtttc cccctggaag ctccctcgtg cgctctcctg 3060
   ttccgaccct gccgcttacc ggatacctgt ccgcctttct cccttcggga agcgtggcgc 3120
   tttctcatag ctcacgctgt aggtatctca gttcggtgta ggtcgttcgc tccaagctgg 3180
   gctgtgtgca cgaacccccc gttcagcccg accgctgcgc cttatccggt aactatcgtc 3240
   ttgagtccaa cccggtaaga cacgacttat cgccactggc agcagccact ggtaacagga 3300
   ttagcagagc gaggtatgta ggcggtgcta cagagttctt gaagtggtgg cctaactacg 3360
   gctacactag aagaacagta tttggtatct gcgctctgct gaagccagtt accttcggaa 3420
   aaagagttgg tagctcttga tccggcaaac aaaccaccgc tggtagcggt ggtttttttg 3480
   tttgcaagca gcagattacg cgcagaaaaa aaggatctca agaagatcct ttgatctttt 3540
   ctacggggtc tgacgctcag tggaacgaaa actcacgtta agggattttg gtcatgagat 3600
   tatcaaaaag gatcttcacc tagatccttt taaattaaaa atgaagtttt aaatcaatct 3660
   aaagtatata tgagtaaact tggtctgaca gttaccaatg cttaatcagt gaggcaccta 3720
   tctcagcgat ctgtctattt cgttcatcca tagttgcctg actccccgtc gtgtagataa 3780
   ctacgatacg ggagggctta ccatctggcc ccagtgctgc aatgataccg cgagacccac 3840
   gctcaccggc tccagattta tcagcaataa accagccagc cggaagggcc gagcgcagaa 3900
   gtggtcctgc aactttatcc gcctccatcc agtctattaa ttgttgccgg gaagctagag 3960
   taagtagttc gccagttaat agtttgcgca acgttgttgc cattgctaca ggcatcgtgg 4020
   tgtcacgctc gtcgtttggt atggcttcat tcagctccgg ttcccaacga tcaaggcgag 4080
   ttacatgatc ccccatgttg tgcaaaaaag cggttagctc cttcggtcct ccgatcgttg 4140
   tcagaagtaa gttggccgca gtgttatcac tcatggttat ggcagcactg cataattctc 4200
   ttactgtcat gccatccgta agatgctttt ctgtgactgg tgagtactca accaagtcat 4260
   tctgagaata gtgtatgcgg cgaccgagtt gctcttgccc ggcgtcaata cgggataata 4320
   ccgcgccaca tagcagaact ttaaaagtgc tcatcattgg aaaacgttct tcggggcgaa 4380
   aactctcaag gatcttaccg ctgttgagat ccagttcgat gtaacccact cgtgcaccca 4440
   actgatcttc agcatctttt actttcacca gcgtttctgg gtgagcaaaa acaggaaggc 4500
   aaaatgccgc aaaaaaggga ataagggcga cacggaaatg ttgaatactc atactcttcc 4560
   tttttcaata ttattgaagc atttatcagg gttattgtct catgagcgga tacatatttg 4620
   aatgtattta gaaaaataaa caaatagggg ttccgcgcac atttccccga aaagtgccac 4680
   ctgacgacga cggatcggga gatctcccga tcccctatgg tgcactctca gtacaatctg 4740
   ctctgatgcc gcatagttaa gccagtatct gctccctgct tgtgtgttgg aggtcgctga 4800
   gtagtgcgcg agcaaaattt aagctacaac aaggcaaggc ttgaccgaca attgcatgaa 4860
   gaatctgctt agggttaggc gttttgcgct gcttcgcgat gtacgggcca gatatacgcg 4920
   ttgacattga ttattgacta gttattaata gtaatcaatt acggggtcat tagttcatag 4980
   cccatatatg gagttccgcg ttacataact tacggtaaat ggcccgcctg gctgaccgcc 5040
   caacgacccc cgcccattga cgtcaataat gacgtatgtt cccatagtaa cgccaatagg 5100
   gactttccat tgacgtcaat gggtggagta tttacggtaa actgcccact tggcagtaca 5160
   tcaagtgtat catatgccaa gtacgccccc tattgacgtc aatgacggta aatggcccgc 5220
   ctggcattat gcccagtaca tgaccttatg ggactttcct acttggcagt acatctacgt 5280
   attagtcatc gctattacca tggtgatgcg gttttggcag tacatcaatg ggcgtggata 5340
   gcggtttgac tcacggggat ttccaagtct ccaccccatt gacgtcaatg ggagtttgtt 5400
   ttggcaccaa aatcaacggg actttccaaa atgtcgtaac aactccgccc cattgacgca 5460
   aatgggcggt aggcgtgtac ggtgggaggt ctatataagc agagctctct ggctaactag 5520
   agaacccact gcttactggc ttatcgaaat taatacgact cactataggg agacccaagc 5580
   tggctagtta agcttgatca aacaagtttg tacaaaaaag caggcttgga aggagttcga 5640
   accatgcaga tcccgcgcgc cgctcttctc ccgctgctgc tgctgctgct ggcggcgccc 5700
   gcctcggcgc agctgtcccg ggccggccgc tcggcgcctt tggccgccgg gtgcccagac 5760
   cgctgcgagc cggcgcgctg cccgccgcag ccggagcact gcgagggcgg ccgggcccgg 5820
   gacgcgtgcg gctgctgcga ggtgtgcggc gcgcccgagg gcgccgcgtg cggcctgcag 5880
   gagggcccgt gcggcgaggg gctgcagtgc gtggtgccct tcggggtgcc agcctcggcc 5940
   acggtgcggc ggcgcgcgca ggccggcctc tgtgtgtgcg ccagcagcga gccggtgtgc 6000
   ggcagcgacg ccaacaccta cgccaacctg tgccagctgc gcgccgccag ccgccgctcc 6060
   gagaggctgc accggccgcc ggtcatcgtc ctgcagcgcg gagcctgcgg ccaagggcag 6120
   gaagatccca acagtttgcg ccataaatat aactttatcg cggacgtggt ggagaagatc 6180
   gcccctgccg tggttcatat cgaattgttt cgcaagcttc cgttttctaa acgagaggtg 6240
   ccggtggcta gtgggtctgg gtttattgtg tcggaagatg gactgatcgt gacaaatgcc 6300
   cacgtggtga ccaacaagca ccgggtcaaa gttgagctga agaacggtgc cacttacgaa 6360
   gccaaaatca aggatgtgga tgagaaagca gacatcgcac tcatcaaaat tgaccaccag 6420
   ggcaagctgc ctgtcctgct gcttggccgc tcctcagagc tgcggccggg agagttcgtg 6480
   gtcgccatcg gaagcccgtt ttcccttcaa aacacagtca ccaccgggat cgtgagcacc 6540
   acccagcgag gcggcaaaga gctggggctc cgcaactcag acatggacta catccagacc 6600
   gacgccatca tcaactatgg aaactcggga ggcccgttag taaacctgga cggtgaagtg 6660
   attggaatta acactttgaa agtgacagct ggaatctcct ttgcaatccc atctgataag 6720
   attaaaaagt tcctcacgga gtcccatgac cgacaggcca aaggaaaagc catcaccaag 6780
   aagaagtata ttggtatccg aatgatgtca ctcacgtcca gcaaagccaa agagctgaag 6840
   gaccggcacc gggacttccc agacgtgatc tcaggagcgt atataattga agtaattcct 6900
   gataccccag cagaagctgg tggtctcaag gaaaacgacg tcataatcag catcaatgga 6960
   cagtccgtgg tctccgccaa tgatgtcagc gacgtcatta aaagggaaag caccctgaac 7020
   atggtggtcc gcaggggtaa tgaagatatc atgatcacag tgattcccga agaaattgac 7080
   cca 7083
<210> 11
   <211> 1195
   <212> **DNA**
   <213> Artificial Sequence
<220>
   <223> Description of Artificial Sequence: note = Synthetic Construct
<400> 11
   ctsaatkgag caatgggcgg taggcgtgta cggtgggagg tctatataag cagagctcgt 60
   ttagtgaacc gtcagaattt tgtaatacga ctcactatag ggcggccgcg aattcgtccc 120
   tgtaccctac atgctgcgcc tatacccagg accgatggta actgaggcgg aggggaaagg 180
   agggcctgag atggcaagtc tgtcctcctc ggtggttcct gtgtccttca tttccactct 240
   gcgagagtct gtgctggacc ctggagttgg tggagaagga gccagtgaca agcagaggag 300
   caaactgtct ttatcacact ccatgatccc agctgctaaa atccacactg agctctgctt 360
   accagccttc ttctctcctg ctggaaccca gaggaggttc cagcagcctc agcaccacct 420
   gacactgtct atcatccaca ctgcagcaag gtgactcgac tctagattgc ggccgcggtc 480
   atagctgttt cctgaacaga tcccgggtgg catccctgtg acccctcccc agtgcctctc 540
   ctggccctgg aagttgccac tccagtgccc accagccttg tcctaataaa attaagttgc 600
   atcattttgt ctgactaggt gtccttctat aatattatgg ggtggagggg ggtggtatgg 660
   agcaaggggc aagttgggaa gacaacctgt agggcctgcg gggtctattg ggaaccaagc 720
   tggagtgcag tggcacaatc ttggctcact gcaatctccg cctcctgggt tcaagcgatt 780
   ctcctgcctc agcctcccga gttgttggga ttccaggcat gcatgaccag gctcagctaa 840
   tttttgtttt tttggtagag acggggtttc accatattgg ccaggctggt ctccaactcc 900
   taatctcagt gatctaccca ccttggcctc caaattgctg ggattacagg cgtgaaccac 960
   tgctccttcc tgtccttctg atttaaaata actatacagc agagacgtca gacaagcata 1020
   gctactgtca tgcccacggt gggamattgg tgctgctgca ctgtcctctc atgcgttggg 1080
   tccactcata atgctgtgga tgggtaccgg cagcttggct gtgcatgkgt cagtagggtg 1140
   gtagtccaag tcgacagcag atgcagcatg gmtttcatta gtcagcaaca gttga 1195
<210> 12
   <211> 1524
   <212> DNA
   <213> Artificial Sequence
<220>
   <223> Description of Artificial Sequence: note = Synthetic Construct
<400> 12
   atgcagatcc cgcgcgccgc tcttctcccg ctgctgctgc tgctgctggc ggcgcccgcc      60
   tcggcgcagc tgtcccgggc cggccgctcg gcgcctttgg ccgccgggtg cccagaccgc     120
   tgcgagccgg cgcgctgccc gccgcagccg gagcactgcg agggcggccg ggcccgggac     180
   gcgtgcggct gctgcgaggt gtgcggcgcg cccgagggcg ccgcgtgcgg cctgcaggag     240
   ggcccgtgcg gcgaggggct gcagtgcgtg gtgcccttcg ggtgccagcc ctcggccacg      300
   gtgcggcggc gcgcgcaggc cggcctctgt gtgtgcgcca gcagcgagcc ggtgtgcggc     360
   agcgacgcca acacctacgc caacctgtgc cagctgcgcg ccgccagccg ccgctccgag     420
   aggctgcacc ggccgccggt catcgtcctg cagcgcggag cctgcggcca agggcaggaa     480
   gatcccaaca gtttgcgcca taaatataac tttatcgcgg acgtggtgga gaagatcgcc     540
   cctgccgtgg ttcatatcga attgtttcgc aagcttccgt tttctaaacg agaggtgccg     600
   gtggctagtg ggtctgggtt tattgtgtcg gaagatggac tgatcgtgac aaatgcccac     660
   gtggtgacca acaagcaccg ggtcaaagtt gagctgaaga acggtgccac ttacgaagcc     720
   aaaatcaagg atgtggatga gaaagcagac atcgcactca tcaaaattga ccaccagggc     780
   aagctgcctg tcctgctgct tggccgctcc tcagagctgc ggccgggaga gttcgtggtc     840
   gccatcggaa gcccgttttc ccttcaaaac acagtcacca ccgggatcgt gagcaccacc     900
   cagcgaggcg gcaaagagct ggggctccgc aactcagaca tggactacat ccagaccgac     960
   gccatcatca actatggaaa ctcgggaggc ccgttagtaa acctggacgg tgaagtgatt    1020
   ggaattaaca ctttgaaagt gacagctgga atctcctttg caatcccatc tgataagatt    1080
   aaaaagttcc tcacggagtc ccatgaccga caggccaaag gaaaagccat caccaagaag    1140
   aagtatattg gtatccgaat gatgtcactc acgtccagca aagccaaaga gctgaaggac    1200
   cggcaccggg acttcccaga cgtgatctca ggagcgtata taattgaagt aattcctgat    1260
   accccagcag aagctggtgg tctcaaggaa aacgacgtca taatcagcat caatggacag    1320
   tccgtggtct ccgccaatga tgtcagcgac gtcattaaaa gggaaagcac cctgaacatg    1380
   gtggtccgca ggggtaatga agatatcatg atcacagtga ttcccgaaga aattgaccca    1440
   gctcgaggtc acccattcga acaaaaactc atctcagaag aggatctgaa tatgcatacc    1500
   ggtcatcatc accatcacca ttga                                           1524
<210> 13
   <211> 6
   <212> PRT
   <213> Artificial Sequence
<220>
   <223> Description of Artificial Sequence: note = Synthetic Construct
<400> 13
   Asp Pro Met Phe Lys Leu
   1               5